# EUROPEAN PATENT SPECIFICATION

(11) **EP 2 477 478 B1**
(45) Date of publication and mention of the grant of the patent: **30.11.2016**
(21) Application number: 10817726.2
(22) Date of filing: 14.09.2010
(51) Int. Cl.: C12N 15/82, C12N 9/30

(54) **A SYSTEM FOR TRANSFORMATION OF THE CHLOROPLAST GENOME OF SCENEDESMUS SP. AND DUNALIELLA SP.**
SYSTEM ZUR TRANSFORMATION DES CHLOROPLASTENGENOMS VON SCENEDESMUS SP. UND DUNALIELLA SP.
SYSTÈME DE TRANSFORMATION DU GÉNOME CHLOROPLASTIQUE DES ESPÈCES SCENEDESMUS ET DUNALIELLA

(30) Priority: 15.09.2009 US 242735 P
(43) Date of publication of application: 25.07.2012
(73) Proprietor: Sapphire Energy, Inc., San Diego, CA 92121 (US)
(72) Inventor: BOTSCH, Kyle, San Diego, California 92121 (US); SZYJKA, Shawn, San Diego, California 92121 (US); LEVINE, Wendy, San Diego, California 92121 (US); CURRAN, Amy, San Diego, California 92121 (US); O'NEILL, Bryan, San Diego, California 92121 (US); MENDEZ, Michael, San Diego, California 92121 (US)
(74) Representative: Chapman, Paul William
(86) International application number: PCT/US2010/048828
(87) International publication number: WO 2011/034863

(56) References cited:
- WO-A1-2008/124526
- WO-A1-2010/051489
- WO-A1-2010/104763
- WO-A1-2010/104763
- WO-A1-2010/111707
- WO-A2-2007/133558
- WO-A2-2009/036067
- WO-A2-2009/036385
- WO-A2-2009/045550
- TAN CONGPING ET AL: "Establishment of a micro-particle bombardment transformation system for Dunaliella saliva", JOURNAL OF MICROBIOLOGY, vol. 43, no. 4, August 2005 (2005-08), pages 361-365, XP002689502, ISSN: 1225-8873
- LAPIDOT MIRI ET AL: "Stable chloroplast transformation of the unicellular red alga Porphyridium species", PLANT PHYSIOLOGY, AMERICAN SOCIETY OF PLANT PHYSIOLOGISTS, ROCKVILLE, MD, US, vol. 129, no. 1, 1 May 2002 (2002-05-01), pages 7-12, XP009093306, ISSN: 0032-0889, DOI: 10.1104/PP.011023
- TARA L WALKER ET AL: "Isolation and characterisation of components of the Dunaliella tertiolecta chloroplast genome", JOURNAL OF APPLIED PHYCOLOGY, KLUWER ACADEMIC PUBLISHERS, DO, vol. 17, no. 6, 1 December 2005 (2005-12-01), pages 495-508, XP019247831, ISSN: 1573-5176
- COLL J M: "Methodologies for transferring DNA into eukaryotic microalgae", SPANISH JOURNAL OF AGRICULTURAL RESEARCH, MINISTERIO DE EDUCACION Y CIENCIA, INSTITUTO NACIONAL DE INVESTIGACION Y TECNOLOGIA AGRARIA Y ALIMENTARIA (I N I A), ES, vol. 4, no. 4, 1 December 2006 (2006-12-01), pages 316-330, XP002485485, ISSN: 1695-971X

### SEQUENCE LISTING

<110> Sapphire Energy, Inc. Botsch, Kyle Szyjka, Shawn LeVine, Wendy Curran, Amy O'Neill, Bryan Mendez, Michael
<120> A SYSTEM FOR TRANSFORMATION OF THE CHLOROPLAST GENOME OF SCENEDESMUS SP. AND DUNALIELLA SP.
<130> 0802WO1
<150> 61/242735
   <151> 2009-09-15
<160> 170
<170> PatentIn version 3.5
<210> 1
   <211> 21
   <212> DNA
   <213> Artificial Sequence
<220>
   <223> PCR primer
<400> 1
   agaaggagct tctacagatg c 21
<210> 2
   <211> 26
   <212> DNA
   <213> Artificial Sequence
<220>
   <223> PCR primer
<400> 2
   tcattagtta cttcatcttt aatccg 26
<210> 3
   <211> 21
   <212> DNA
   <213> Artificial Sequence
<220>
   <223> PCR primer
<400> 3
   gaactacaac taattatttt c 21
<210> 4
   <211> 24
   <212> DNA
   <213> Artificial Sequence
<220>
   <223> PCr primer
<400> 4
   tgaaaccagt ctttgtaaag ctca 24
<210> 5
   <211> 22
   <212> DNA
   <213> Artificial Sequence
<220>
   <223> PCR primer
<400> 5
   ctaaattcca gcaaccagca tt 22
<210> 6
   <211> 22
   <212> DNA
   <213> Artificial Sequence
<220>
   <223> PCR primer
<400> 6
   cgttcttctg agaaatggct ta 22
<210> 7
   <211> 27
   <212> DNA
   <213> Artificial Sequence
<220>
   <223> PCR primer
<400> 7
   tgtaaattta aggctgcctg tgatgtg 27
<210> 8
   <211> 28
   <212> DNA
   <213> Artificial Sequence
<220>
   <223> PCR primer
<400> 8
   gaggttcgaa gaatgggtca aagataag 28
<210> 9
   <211> 24
   <212> DNA
   <213> Artificial Sequence
<220>
   <223> PCR primer
<400> 9
   caactaccac tggagataaa tttc 24
<210> 10
   <211> 25
   <212> DNA
   <213> Artificial Sequence
<220>
   <223> PCR primer
<400> 10
   aatcactcta ccaactgagt tatgg 25
<210> 11
   <211> 23
   <212> DNA
   <213> Artificial Sequence
<220>
   <223> PCR primer
<400> 11
   gcactacctg atgaaaaata acc 23
<210> 12
   <211> 33
   <212> DNA
   <213> Artificial Sequence
<220>
   <223> PCR primer
<400> 12
   ttggtttcta gattacgccc cgccctgcca ctc 33
<210> 13
   <211> 20
   <212> DNA
   <213> Artificial Sequence
<220>
   <223> PCR primer
<400> 13
   gtgaatcaac aactgattgg 20
<210> 14
   <211> 24
   <212> DNA
   <213> Artificial Sequence
<220>
   <223> PCR primer
<400> 14
   tgaattgcat aaatttacac atac 24
<210> 15
   <211> 24
   <212> DNA
   <213> Artificial Sequence
<220>
   <223> PCR primer
<400> 15
   ccacctcgta tggtaaaata attg 24
<210> 16
   <211> 24
   <212> DNA
   <213> Artificial Sequence
<220>
   <223> PCR primer
<400> 16
   gaaagaatta tggacagtcc tgct 24
<210> 17
   <211> 42
   <212> DNA
   <213> Artificial Sequence
<220>
   <223> PCR primer
<400> 17
   ttgttgcggc cgcttttgaa gccgaaatac tttattttta tg 42
<210> 18
   <211> 35
   <212> DNA
   <213> Artificial Sequence
<220>
   <223> PCR primer
<400> 18
   catcattaat ggagaaaaaa atcactggat atacc 35
<210> 19
   <211> 35
   <212> DNA
   <213> Artificial Sequence
<220>
   <223> PCR primer
<400> 19
   aattcctagg ttacgccccg ccctgccact catcg 35
<210> 20
   <211> 87
   <212> DNA
   <213> Artificial Sequence
<220>
   <223> FLAG epitope tag linked to a MAT epitope tag by a TEV protease site
<400> 20
   ggtaccggtg attacaaaga tgatgacgat aaaagtggtg aaaaccttta ttttcaaggc        60
   cataatcacc gtcacaaaca caccggt                                              87
<210> 21
   <211> 762
   <212> DNA
   <213> Artificial Sequence
<220>
   <223> codon optimized endoxylanase from T. reesei
<220>
   <221> misc_feature
   <222> (673)..(759)
   <223> tag
<400> 21
   atggtaccag tatctttcac aagtctttta gcagcatctc caccttcacg tgcaagttgc        60
   cgtccagctg ctgaagtgga atcagttgca gtagaaaaac gtcaaacaat tcaaccaggt       120
   acaggttaca ataacggtta cttttattct tactggaatg atggacacgg tggtgttaca       180
   tatactaatg gacctggtgg tcaatttagt gtaaattgga gtaactcagg caattttgtt       240
   ggaggaaaag gttggcaacc tggtacaaag aataaggtaa tcaatttctc tggtagttac       300
   aaccctaatg gtaattctta tttaagtgta tacggttgga gccgtaaccc attaattgaa       360
   tattatattg tagagaactt tggtacatac aacccttcaa caggtgctac taaattaggt       420
   gaagttactt cagatggatc agtttatgat atttatcgta ctcaacgcgt aaatcaacca       480
   tctataattg gaactgccac tttctaccaa tactggagtg taagacgtaa tcatcgttca       540
   agtggtagtg ttaatacagc aaaccacttt aatgcatggg ctcaacaagg tttaacatta       600
   ggtacaatgg actatcaaat tgtagctgtt gaaggttatt tttcatcagg tagtgcttct       660
   atcactgtta gcggtaccgg tgattacaaa gatgatgacg ataaaagtgg tgaaaacctt       720
   tattttcaag gccataatca ccgtcacaaa cacaccggtt aa                            762
<210> 22
   <211> 81
   <212> DNA
   <213> Artificial Sequence
<220>
   <223> TEV protease site linked to a FLAG epitope tag
<400> 22
   ggtaccggtg aaaacttata ctttcaaggc tcaggtggcg gtggaagtga ttacaaagat 60
   gatgatgata aaggaaccgg t 81
<210> 23
   <211> 1191
   <212> DNA
   <213> Artificial Sequence
<220>
   <223> codon optimized FPP synthase from G. gallus
<220>
   <221> misc_feature
   <222> (1108)..(1188)
   <223> tag
<400> 23
   atggtaccac acaagttcac aggtgttaac gctaaattcc agcaaccagc attaagaaat      60
   ttatctccag tggtagttga gcgcgaacgt gaggaatttg taggattctt tccacaaatt     120
   gttcgtgact taactgaaga tggtattggt catccagaag taggtgacgc tgtagctcgt     180
   cttaaagaag tattacaata caacgcacct ggtggtaaat gcaatagagg tttaacagtt     240
   gttgcagctt accgtgaact ttctggacca ggtcaaaaag acgctgaaag tcttcgttgt     300
   gctttagcag taggatggtg tattgaatta ttccaagcct ttttcttagt tgctgacgat     360
   ataatggacc agtcattaac tagacgtggt caattatgtt ggtacaagaa agaaggtgtt     420
   ggtttagatg caataaatga ttcttttctt ttagaaagct ctgtgtatcg cgttcttaaa     480
   aagtattgcc gtcaacgtcc atattatgta catttattag agctttttct tcaaacagct     540
   taccaaacag aattaggaca aatgttagat ttaatcactg ctcctgtatc taaggtagat     600
   ttaagccatt tctcagaaga acgttacaaa gctattgtta agtataaaac tgctttctat     660
   tcattctatt taccagttgc agcagctatg tatatggttg gtatagattc taaagaagaa     720
   catgaaaacg caaaagctat tttacttgag atgggtgaat acttccaaat tcaagatgat     780
   tatttagatt gttttggcga tcctgcttta acaggtaaag taggtactga tattcaagat     840
   aacaaatgtt catggttagt tgtgcaatgc ttacaaagag taacaccaga acaacgtcaa     900
   cttttagaag ataattacgg tcgtaaagaa ccagaaaaag ttgctaaagt taaagaatta     960
   tatgaggctg taggtatgag agccgccttt caacaatacg aagaaagtag ttaccgtcgt    1020
   cttcaagagt taattgagaa acattctaat cgtttaccaa aagaaatttt cttaggttta    1080
   gctcagaaaa tatacaaacg tcaaaaaggt accggtgaaa acttatactt tcaaggctca    1140
   ggtggcggtg gaagtgatta caaagatgat gatgataaag gaaccggtta a             1191
<210> 24
   <211> 36
   <212> DNA
   <213> Artificial Sequence
<220>
   <223> streptavidin epitope tag
<400> 24
   accggtagtg cttggtcaca ccctcaattt gagaaa 36
<210> 25
   <211> 2196
   <212> DNA
   <213> Artificial Sequence
<220>
   <223> codon optimized fusicoccadiene synthase from P. amygdali
<220>
   <221> misc_feature
   <222> (2158)..(2193)
   <223> tag
<400> 25
   atggaattta aatattcaga agttgttgaa ccatcaacat attatacaga aggtttatgt      60
   gaaggtattg atgttcgtaa atcaaaattt acaacattag aagatcgtgg tgctattcgt     120
   gctcatgaag attggaataa acatattggt ccatgtggtg aatatcgtgg tacattaggt     180
   ccacgttttt catttatttc agttgctgtt ccagaatgta ttccagaacg tttagaagtt     240
   atttcatacg ctaatgaatt tgctttttta catgatgatg ttacagatca tgttggtcat     300
   gatacaggtg aagttgaaaa tgatgaaatg atgacagttt ttttagaagc tgctcataca     360
   ggtgctattg atacatcaaa taaagttgat attcgtcgtg ctggtaaaaa acgtattcaa     420
   tcacaattat ttttagaaat gttagctatt gatccagaat gtgctaaaac aacaatgaaa     480
   tcatgggctc gttttgttga agttggttca tcacgtcaac atgaaacacg ttttgttgaa     540
   ttagctaaat atattccata tcgtattatg gatgttggtg aaatgttttg gtttggttta     600
   gttacatttg gtttaggttt acatattcca gatcatgaat tagaattatg tcgtgaactt     660
   atggctaatg cttggattgc tgttggttta caaaatgata tttggtcatg gccaaaagaa     720
   cgtgatgctg ctacattaca tggtaaagat catgttgtta atgctatttg ggttttaatg     780
   caagaacatc aaacagatgt tgatggtgct atgcaaattt gtcgtaaact tattgttgaa     840
   tatgttgcta aatatttaga agttattgaa gctacaaaaa atgatgaatc aatttcatta     900
   gatttacgta aatatttaga tgctatgtta tattcaattt caggtaatgt tgtttggtca     960
   ttagaatgtc cacgttataa tccagatgtt tcatttaata aaacacaatt agaatggatg    1020
   cgtcaaggtt taccatcatt agaatcatgt ccagttttag ctcgttcacc agaaattgat    1080
   tcagatgaat cagcagtttc accaactgct gatgaatcag attcaacaga agattcatta    1140
   ggttcaggtt cacgtcaaga ttcatcatta tcaacaggtt tatcattatc accagttcat    1200
   tcaaatgaag gtaaagattt acaacgtgtt gatacagatc atattttttt tgaaaaagct    1260
   gttttagaag ctccatacga ttatattgct tcaatgccat caaaaggtgt tcgtgaccaa    1320
   tttattgatg ctttaaatga ttggttacgt gttccagatg ttaaagttgg taaaattaaa    1380
   gatgctgttc gtgttttaca taattcatca ttattattag atgattttca agataattca    1440
   ccattacgtc gtggtaaacc atcaacacat aatatttttg gttcagctca aacagttaat    1500
   acagctacat attcaattat taaagctatt ggtcaaatta tggaattttc tgctggtgag    1560
   tcagttcaag aagttatgaa ctcaattatg attttatttc aaggtcaagc tatggattta      1620
   ttttggacat ataatggtca tgttccatca gaagaagaat attatcgtat gattgaccaa      1680
   aaaacaggtc aattattttc aattgctaca tcattattat taaatgctgc tgataatgaa      1740
   attccacgta caaaaattca atcatgttta catcgtttaa cacgtttatt aggtcgttgt      1800
   tttcaaattc gtgatgatta tcaaaattta gtttctgctg attacactaa acaaaaagga      1860
   ttctgtgaag atttagatga aggtaaatgg tcattagctt taattcacat gattcataaa      1920
   caacgttcac acatggcttt attaaatgtt ttatcaacag gtcgtaaaca tggtggtatg      1980
   acattagaac aaaaacaatt tgttttagat attattgaag aagaaaaatc attagattat      2040
   acacgttcag ttatgatgga tcttcatgtt caattacgtg ctgaaattgg tcgtattgaa      2100
   attttattag attcaccaaa tccagctatg cgtttattat tagaattatt acgtgttacc      2160
   ggtagtgctt ggtcacaccc tcaatttgag aaataa                                2196
<210> 26
   <211> 1704
   <212> DNA
   <213> Artificial Sequence
<220>
   <223> codon optimized phytase from E. coli
<220>
   <221> misc_feature
   <222> (1615)..(1701)
   <223> tag
<400> 26
   atggtaccaa tgcgtatctc tcttaaaaaa tcaggaatgt taaaacttgg tttatctctt      60
   gtagctatga cagtagctgc ttctgtacaa gctaaaacat tagtatattg ttcagaaggc     120
   tctccagaag gttttaatcc tcaacttttt acttcaggta caacttatga cgcttcttca     180
   gtacctttat acaaccgttt agtagagttc aaaatcggta caacagaagt tattccaggt     240
   ttagctgaaa aatgggaagt atcagaagac ggtaaaactt acacattcca tttaagaaaa     300
   ggtgttaaat ggcacgataa taaagagttt aaaccaacaa gagaattaaa tgctgatgat     360
   gtagttttct catttgaccg tcagaaaaat gctcaaaatc catatcataa agtttcagga     420
   ggatcttacg aatatttcga aggcatggga ttaccagaac ttatttctga agttaaaaaa     480
   gttgatgata acacagttca atttgtttta actagaccag aagctccatt tttagctgat     540
   ttagctatgg atttcgctag tattttatca aaagaatatg cagatgctat gatgaaagct     600
   ggtacacctg aaaaacttga tcttaatcca attggtactg gtccattcca attacaacaa     660
   tatcagaaag attcacgtat tcgttacaaa gcatttgacg gctattgggg tacaaaacct     720
   caaattgata ctttagtatt ttcaattaca ccagacgcat cagttcgtta cgcaaaatta     780
   cagaaaaatg aatgccaagt aatgccatat ccaaatccag ctgatattgc acgtatgaaa     840
   caagataaat ctatcaattt aatggaaatg cctggtttaa atgttggtta tttatcatat     900
   aacgttcaaa aaaaaccatt agatgatgta aaagttcgtc aagcattaac ttatgcagtt     960
   aataaagacg caatcattaa agcagtatat caaggtgctg gagttagtgc taaaaatctt    1020
   attccaccaa caatgtgggg ttacaacgat gacgttcaag attatactta cgaccctgag    1080
   aaagctaaag cattacttaa agaagcaggt ttagaaaaag gtttctcaat tgatttatgg    1140
   gcaatgccag tacaacgtcc ttacaatcca aatgctagac gtatggcaga aatgattcaa    1200
   gcagactggg ctaaagtagg tgttcaagca aaaattgtta catacgaatg gggtgaatac    1260
   ttaaaacgtg ctaaagatgg tgaacaccaa actgttatga tgggttggac aggtgataat    1320
   ggtgatcctg acaatttctt tgcaacatta ttttcatgtg ctgcttcaga acaaggttca    1380
   aattattcaa aatggtgtta taaacctttt gaagacttaa ttcaacctgc tcgtgctaca    1440
   gacgatcaca ataaacgtgt tgaattatac aaacaagcac aagttgtaat gcacgaccaa    1500
   gctccagctc ttattattgc tcattcaaca gtattcgaac cagttagaaa agaagttaaa    1560
   ggttatgtag tagatccatt aggtaaacat cactttgaaa acgtatcaat tgaaggtacc    1620
   ggtgactata aagatgatga tgacaaaagt ggagagaact tatactttca aggtcataat    1680
   caccgtcaca aacacaccgg ttaa                                           1704
<210> 27
   <211> 87
   <212> DNA
   <213> Artificial Sequence
<220>
   <223> FLAG epitope tag linked to a MAT epitope tag by a TEV protease site
<400> 27
   ggtaccggtg actataaaga tgatgatgac aaaagtggag agaacttata ctttcaaggt 60
   cataatcacc gtcacaaaca caccggt 87
<210> 28
   <211> 660
   <212> DNA
   <213> Artificial Sequence
<220>
   <223> modified chloramphenicol acetyl transferase gene from E. coli
<400> 28
   atggagaaaa aaatcactgg atataccacc gttgatatat cccaatggca tcgtaaagaa 60
   cattttgagg catttcagtc agttgctcaa tgtacctata accagaccgt tcagctggat 120
   attacggcct ttttaaagac cgtaaagaaa aataagcaca agttttatcc ggcctttatt 180
   cacattcttg cccgcctgat gaatgctcat ccggagttcc gtatggcaat gaaagacggt 240
   gagctggtga tatgggatag tgttcaccct tgttacaccg ttttccatga gcaaactgaa 300
   acgttttcat cgctctggag tgaataccac gacgatttcc ggcagtttct acacatatat 360
   tcgcaagatg tggcgtgtta cggtgaaaac ctggcctatt tccctaaagg gtttattgag 420
   aatatgtttt tcgtcagcgc caatccctgg gtgagtttca ccagttttga tttaaacgtg 480
   gccaatatgg acaacttctt cgcccccgtt ttcactatgg gcaaatatta tacgcaaggc 540
   gacaaggtgc tgatgccgct ggcgattcag gttcatcatg ccgtttgtga tggcttccat 600
   gtcggcagaa tgcttaatga attacaacag tactgcgatg agtggcaggg cggggcgtaa 660
<210> 29
   <211> 1260
   <212> DNA
   <213> Artificial Sequence
<220>
   <223> modified erythromycin esterase gene from E. coli
<400> 29
   atgaggttcg aagaatgggt caaagataag catattcctt tcaaactgaa tcaccctgat 60
   gataattacg atgattttaa gccattaaga aaaataattg gagatacccg agttgtagca 120
   ttaggtgaaa attctcattt cataaaagaa ttttttttgt tacgacatac gcttttgcgt 180
   ttttttatcg aagacctcgg ttttactacg tttgcttttg aatttggttt tgctgagggt 240
   caaatcatca ataactggat acatggacaa ggaactgacg atgaaatagg cagattctta 300
   aaacacttct attatccaga agagctcaaa accacatttc tatggctaag ggagtacaat 360
   aaagcagcaa aagaaaaaat cacatttctt ggcattgata tacccagaaa tggaggttca 420
   tacttaccaa atatggagat agtgcatgac ttttttagaa cagcggataa agaagcacta 480
   cacattatcg atgatgcatt taatattgca aaaaagattg attacttctc cacatcacag 540
   gcagccttaa atttacatga gctaacagat tctgagaaat gccgtttaac tagccaatta 600
   gcacgagtaa aagttcgcct tgaagctatg gctccaattc acattgaaaa atatgggatt 660
   gataaatatg agacaattct gcattatgcc aacggtatga tatacttgga ctataacatt 720
   caagctatgt cgggctttat ttcaggaggc ggaatgcagg gcgatatggg tgcaaaagac 780
   aaatacatgg cagattctgt gctgtggcat ttaaaaaacc cacaaagtga gcagaaagtg 840
   atagtagtag cacataatgc acatattcaa aaaacaccca ttctgtatga tggatttcta 900
   agttgcctac caatgggcca aagacttaaa aatgccattg gtgatgatta tatgtcttta 960
   ggtattactt cttatagtgg gcatactgca gccctctatc cggaagttga tacaaaatat 1020
   ggttttcgag ttgataactt ccaactgcag gaaccaaatg aaggttctgt cgagaaagct 1080
   atttctggtt gtggagttac taattctttt gtctttttta gaaatattcc tgaagattta 1140
   caatccatcc cgaacatgat tcgatttgat tctatttaca tgaaagcaga acttgagaaa 1200
   gcattcgatg gaatatttca aattgaaaag tcatctgtat ctgaggtcgt ttatgaataa 1260
<210> 30
   <211> 2844
   <212> DNA
   <213> Artificial Sequence
<220>
   <223> modified genomic DNA from S. dimorphus
<400> 30
   gcctttaggt atttcaggaa ctttcaactt catgaaaata aacgtgacaa ataaaaacct 60
   tttttatttg aaaattgggg aaattgatga aaaattgaaa aaaataaata aaatataaaa 120
   tactttatga caaaaaaatg aacaaaattt gagatagaac aaattgcaat tctttttttc 180
   aaaaattgtt ttttcaataa tcatcaaccg agcttatttt tgtaagcagg ttcagagact 240
   aaaaaaatag agaaattttt tttgttccca aaaacagaaa aagaaacaaa agatttcttc 300
   acctgtttgt gagatagtcc gatctttttt gaaaaaaaaa gagaaactta agctttcata 360
   acaaaatgga tcgttttcca agctgaacac aacattttaa tgcacccttt ccacatgtta 420
   ggtgtagctg gtgtatttgg tggttcatta ttctctgcaa tgcataggta ctgagaaact 480
   gtgccctttg ttttttttgt gttttttctc ttcttttttt ctttgtgcct gctctctttt 540
   tttctacaaa caagtaaaaa aaaagcaaaa acgcataaaa atgcacgcaa aaacgtatcg 600
   ttgtgcatct aaaaagtata aaaaagaaac aaaaacgctt tgcttcgcta ctttttttct 660
   tcttcatttt ttgcaaagca aaaaatgata aaaagtagcg aagcgtagca aaaaaagctt 720
   ctaaaaacaa ttgaaaatcg agtgaattgc ataaatttac acatacaaaa aaataaaaaa 780
   caagaataaa aaatatgaaa acaacaaaag cacttgacga ttttaacaaa aacgcgtcaa 840
   acaagtattt agattttatt gctctctgca aacaaaaagc agaagagtat tgaccagaac 900
   aacgcggaac ctgtacgtat caccatattg tgcctcgcca tcactacaag gcaaatgagc 960
   tctcttggga aaactttgag agtccagaaa atttagtttt ggtgaccttt gaagaccaca 1020
   ttaaagcaca cgaacttcgt tttgaagttt atggtgaaaa cggggacaat attgctgctc 1080
   ttcgaatggc tggtcacaag gaagagagca tgcgagctat gcaacaagca ggtggtcaag 1140
   cagtaaacaa aattttcaaa gaaaaaaaac aactaatgca taatcaagaa tttcaaaaag 1200
   aaatggctcg ccgctcaatg gcccgtccag atgctcgtca aattcgcagc gaaggaggaa 1260
   agaagggtgc gcatgttcgt caccaaaacc gaactgttcg agcaacagat cgatttcttt 1320
   ggcgttttaa aagagaagac tttttatgta cctttggttt tgacaatgct ggtgatcttt 1380
   gttgagaatt gaatttggca aagccagaaa tttttgttgg tcgtttgagc ggttttctta 1440
   ctggaaaacg aaagacgaac aggggttggt cgtgtgaaaa aattgaagaa tcttccgaaa 1500
   acgaagaaca aacatgaccc aatccacact aaatttttac aaaaaagtgt acgctctttc 1560
   ctttttgttt ttgtgcaaag ttctttgttt ttattttttt ttgtgaaaaa tatgcaacga 1620
   attccagttg taaaagactg gactcgttca gagactagtt ttatttttat gaaacaaagt      1680
   actcgagaga aaaaataagc tttatttttt cttatgatat agtcctcaag aaactttttt      1740
   cttttaaaaa aaaagaaagt tcttgaaggg ttcattagtt acttcatctt taatccgtga      1800
   aacaactgaa aacgaatctg ctaacgaagg atacaaattc ggtcaagaag aagaaactta      1860
   caacatcgta gctgcacacg gttactttgg tcgtttaatc ttccaatacg ctgctttcaa      1920
   caactctaga tcattacact tcttcttagc tgcttggcca gttgtaggta tttggttcac      1980
   tgctttaggt atttcaacta tggctttcaa ccttaacggt ttcaacttca accaatcagt      2040
   tgttgattca caaggtcgtg ttttaaacac atgggctgac atcatcaacc gtgctaactt      2100
   aggtatggaa gttatgcacg aacgtaacgc tcacaacttc ccattagact tagcatctgt      2160
   agaagctcct tctgtaaacg cttagtttta ttttttatga aaaactcagg cttaatttag      2220
   gcttgagttt ttcattcttt ttgaagctct gaaattttaa aatttctagt cttctttaat      2280
   gtttttaaat tttaaaaaat aaatttcttc tctgctgtgt ttttcttttt ttttgaaaaa      2340
   acaaagaaaa aaaatttttt tgttttcttc tttgtttttt tatttctttt tgttttgttt      2400
   attttttagt ttcagaatct ttgattcaaa aaaaaattta gtccgattac tccataggag      2460
   caagcagtaa aaaataaaaa ctgtaataaa aaataaaaca aaaattttat ttctttttgt      2520
   tttgcttgaa cttttcaaaa aaaaattgaa aaattcaagc aaaacaaaaa gaaacaaata      2580
   aaaaatttat gaattttcta ctttttcagg agttgaaatt tctcctttac ttaaaacata      2640
   ttttgctaaa aaaagcgctt gtgttgcttt ttttgctact ttttgtttcc aagcattttt      2700
   tcgaatattt ttttttgatt ttgatgtgcg tttttgttaa cctaaaatct tgaaaagatt      2760
   tactcttttc aaatttttat gtttttattt tttttattca taaaaaaaaa caatacataa      2820
   aaataaagta tttcggcttc aaaa                                             2844
<210> 31
   <211> 1284
   <212> DNA
   <213> Artificial Sequence
<220>
   <223> codon optimized cytosine deaminase from E. coli
<400> 31
   atgtctaaca acgctttaca aactattatc aacgcaagat taccaggtaa agaaggttta        60
   tggcaaatcc accttcaaga tggtaaaatt tctgctattg acgcacaatc aggtgtaatg       120
   ccaataacag aaaattcatt agatgctgaa caaggcttag ttattcctcc tttcgttgag       180
   ccacatatac accttgacac aactcaaaca gctggtcaac caaattggaa tcaatcaggc       240
   acactttttg aaggtattga acgttgggct gagcgtaaag cattattaac acatgacgat       300
   gttaaacaac gtgcttggca aacattaaaa tggcaaattg caaatggtat acaacatgtt       360
   cgtactcatg ttgacgtttc agacgcaact ttaacagcat taaaagctat gttagaagtt       420
   aaacaagaag tagctccatg gatagactta caaattgtag cttttccaca agagggtatt       480
   ttatcatatc caaatggcga agcattatta gaagaggctt tacgtcttgg tgctgacgta       540
   gtaggtgcta ttcctcactt cgaatttact cgtgaatatg gtgtagaaag tttacataaa       600
   actttcgcac ttgctcaaaa atatgatcgt cttattgatg ttcactgtga tgaaattgat       660
   gacgaacaat cacgtttcgt tgaaacagtt gctgcattag ctcaccgtga aggtatgggt       720
   gctcgtgtta cagctagtca tactacagct atgcattctt ataatggagc atacacttct       780
   cgtcttttta gattacttaa aatgtcaggt attaacttcg ttgcaaatcc tttagtaaac       840
   attcacttac aaggtagatt tgatacatat cctaaacgta gaggtattac acgtgttaaa       900
   gaaatgttag aatctggtat caatgtatgt tttggtcacg atgatgtatt cgatccatgg       960
   tatccattag gcacagctaa tatgttacaa gttttacaca tgggtttaca tgtttgtcaa      1020
   cttatgggtt acggtcaaat caacgatggt cttaacttaa ttacacatca ttctgcacgt      1080
   actttaaatc ttcaagatta tggtattgca gctggtaatt cagctaacct tattattctt      1140
   ccagcagaaa atggttttga tgctcttcgt cgtcaagtac ctgttcgtta ttctgttcgt      1200
   ggcggcaaag ttattgctag tacacaacct gcacaaacaa ctgtatattt agaacagcca      1260
   gaagctattg attacaaacg ttaa                                             1284
<210> 32
   <211> 1653
   <212> DNA
   <213> Artificial Sequence
<220>
   <223> codon optimized betaine aldehyde dehydrogenase from S. oleracea
<220>
   <221> misc_feature
   <222> (1510)..(1650)
   <223> tag
<400> 32
   atgatggctt tcccaattcc agctcgtcaa ttattcattg acggtgaatg gcgtgaacca        60
   ttattaaaaa accgtattcc aattattaac ccatcaacag aagaaattat tggtgacatt       120
   ccagctgcta cagctgaaga cgtagaagta gctgtagtag ctgctcgtaa agctttcaaa       180
   cgtaacaaag gtcgtgactg ggctgcttta tggtcacacc gtgctaaata cttacgtgct       240
   attgctgcta aaattacaga aaaaaaagac cacttcgtaa aattagaaac attagactca       300
   ggtaaaccac gtgacgaagc tgtattagac attgacgacg tagctacatg tttcgaatac       360
   ttcgaatact tcgctggtca agctgaagct ttagacgcta aacaaaaagc tccagtaaca       420
   ttaccaatgg aacgtttcaa atcacacgta ttacgtcaac caattggtgt agtaggttta       480
   atttcaccat ggaactaccc attattaatg gacacatgga aaattgctcc agctttagct       540
   gctggttgta caacagtatt aaaaccatca gaattagctt cagtaacatg tttagaattc       600
   ggtgaagtat gtaacgaagt aggtttacca ccaggtgtat taaacatttt aacaggttta       660
   ggtccagacg ctggtgctcc aattgtatca cacccagaca ttgacaaagt agctttcaca       720
   ggttcatcag ctacaggttc aaaaattatg gcttcagctg ctcaattagt aaaaccagta       780
   acattagaat taggtggtaa atcaccagta attatgttcg aagacattga cattgaaaca       840
   gctgtagaat ggacattatt cggtgtattc tggacaaacg gtcaaatttg ttcagctaca       900
   tcacgtttat tagtacacga atcaattgct gctgaattcg tagaccgtat ggtaaaatgg       960
   acaaaaaaca ttaaaatttc agacccattc gaagaaggtt gtcgtttagg tccagtaatt      1020
   tcaaaaggtc aatacgacaa aattatgaaa ttcatttcaa cagctaaatc agaaggtgct      1080
   acaattttat gtggtggttc acgtccagaa cacttaaaaa aaggttacta cattgaacca      1140
   acaattatta cagacattac aacatcaatg caaatttgga aagaagaagt attcggtcca      1200
   gtaatttgtg taaaaacatt caaaacagaa gacgaagcta ttgaattagc taacgacaca      1260
   gaatacggtt tagctggtgc tgtattctca aaagacttag aacgttgtga acgtgtaaca      1320
   aaagctttag aagtaggtgc tgtatgggta aactgttcac aaccatgttt cgtacacgct      1380
   ccatggggtg gtgtaaaacg ttcaggtttc ggtcgtgaat taggtgaatg gggtattgaa      1440
   aactacttaa acattaaaca agtaacatca gacatttcag acgaaccatg gggttggtac       1500
   aaatcaccaa ccggttaccc atacgacgta cctgactatg cttaccctta cgacgtacca      1560
   gactatgctt atccatacga cgtaccagac tacgctgaaa acttatactt ccaaggtcac      1620
   caccaccacc accatcacca cccaccaggt taa                                   1653
<210> 33
   <211> 141
   <212> DNA
   <213> Artificial Sequence
<220>
   <223> 3xHA tag linked to a 6xHIS tag by a TEV protease site
<400> 33
   accggttacc catacgacgt acctgactat gcttaccctt acgacgtacc agactatgct 60
   tatccatacg acgtaccaga ctacgctgaa aacttatact tccaaggtca ccaccaccac 120
   caccatcacc acccaccagg t 141
<210> 34
   <211> 1647
   <212> DNA
   <213> Artificial Sequence
<220>
   <223> codon optimized betaine aldehyde dehydrogenase from B. vulgaris
<220>
   <221> misc_feature
   <222> (1504)..(1644)
   <223> tag
<400> 34
   atgatgtcaa tgccaattcc atcacgtcaa ttattcattg acggtgaatg gcgtgaacca 60
   attaaaaaaa accgtattcc aattattaac ccatcaaacg aagaaattat tggtgacatt 120
   ccagctggtt catcagaaga cattgaagta gctgtagctg ctgctcgtcg tgctttaaaa 180
   cgtaacaaag gtcgtgaatg ggctgctaca tcaggtgctc accgtgctcg ttacttacgt 240
   gctattgctg ctaaagtaac agaacgtaaa gaccacttcg taaaattaga aacaattgac 300
   tcaggtaaac cattcgacga agctgtatta gacattgacg acgtagctac atgtttcgaa 360
   tacttcgctg gtcaagctga agctatggac gctaaacaaa aagctccagt aacattacca 420
   atggaacgtt tcaaatcaca cgtattacgt caaccaattg gtgtagtagg tttaattaca 480
   ccatggaact acccattatt aatggctaca tggaaaattg ctccagcttt agctgctggt 540
   tgtacagctg tattaaaacc atcagaatta gcttcaatta catgtttaga attcggtgaa 600
   gtatgtaacg aagtaggttt accaccaggt gtattaaaca ttgtaacagg tttaggtcca 660
   gacgctggtg ctccattagc tgctcaccca gacgtagaca aagtagcttt cacaggttca 720
   tcagctacag gttcaaaagt aatggcttca gctgctcaat tagtaaaacc agtaacatta 780
   gaattaggtg gtaaatcacc aattattgta ttcgaagacg tagacattga ccaagtagta 840
   gaatggacaa tgttcggttg tttctggaca aacggtcaaa tttgttcagc tacatcacgt 900
   ttattagtac acgaatcaat tgctgctgaa ttcattgacc gtttagtaaa atggacaaaa 960
   aacattaaaa tttcagaccc attcgaagaa ggttgtcgtt taggtccagt aatttcaaaa 1020
   ggtcaatacg acaaaattat gaaattcatt tcaacagcta aatcagaagg tgctacaatt 1080
   ttatgtggtg gttcacgtcc agaacactta aaaaaaggtt acttcattga accaacaatt 1140
   atttcagaca tttcaacatc aatgcaaatt tggcgtgaag aagtattcgg tccagtatta 1200
   tgtgtaaaaa cattctcatc agaagacgaa gctttagact tagctaacga cacagaatac 1260
   ggtttagctt cagctgtatt ctcaaaagac ttagaacgtt gtgaacgtgt atcaaaatta 1320
   ttagaatcag gtgctgtatg ggtaaactgt tcacaaccat gtttcgtaca cgctccatgg 1380
   ggtggtatta aacgttcagg tttcggtcgt gaattaggtg aatggggtat tgaaaactac 1440
   ttaaacatta aacaagtaac atcagacatt tcaaacgaac catggggttg gtacaaatca 1500
   ccaaccggtt acccatacga cgtacctgac tatgcttacc cttacgacgt accagactat 1560
   gcttatccat acgacgtacc agactacgct gaaaacttat acttccaagg tcaccaccac 1620
   caccaccatc accacccacc aggttaa 1647
<210> 35
   <211> 2541
   <212> DNA
   <213> Artificial Sequence
<220>
   <223> codon optimized E-alpha-biabolene synthase from A. grandis
<220>
   <221> misc_feature
   <222> (2458)..(2538)
   <223> tag
<400> 35
   atggtaccag caggtgtatc agctgtgtca aaagtttctt cattagtatg tgacttaagt 60
   agtactagcg gcttaattcg tagaactgca aatcctcacc ctaatgtatg gggttatgac 120
   ttagttcatt ctttaaaatc tccatatatt gatagtagct atcgtgaacg tgctgaagtg 180
   cttgtaagtg aaataaaagc tatgttaaat ccagcaatta ctggagatgg tgaatcaatg 240
   attacacctt cagcttatga cactgcttgg gttgcacgtg taccagcaat tgatggtagc 300
   gcacgtccac aatttccaca aacagtagat tggattttaa agaatcaatt aaaagatggt 360
   tcttggggta ttcaatcaca ctttttactt tcagaccgtt tattagctac tcttagctgt 420
   gttttagttt tacttaaatg gaatgttggt gatttacagg ttgagcaagg tattgagttt 480
   attaagtcaa accttgaatt agtaaaagat gaaactgatc aagattcttt agtgactgat 540
   tttgagatta ttttccctag cttacttcgt gaggcccaaa gtttacgttt aggtcttcca 600
   tacgatttac cttacatcca cttattacaa acaaaacgtc aggaacgttt agcaaaatta 660
   agccgtgaag aaatatatgc agttccaagt ccacttttat attctttaga gggtattcaa 720
   gatattgttg agtgggaacg tattatggaa gtacaatctc aggatggatc atttttaagt 780
   tctccagcat caaccgcatg tgtttttatg catacaggtg acgctaagtg tttagaattt 840
   cttaacagtg taatgattaa gtttggtaat tttgtaccat gcctttatcc tgtagattta 900
   ttagaacgtt tacttatagt agataatata gttcgtcttg gtatttaccg tcacttcgaa 960
   aaagaaatta aagaagcatt agattatgta tatcgccatt ggaatgaacg tggtattggt 1020
   tggggtcgtt taaatccaat tgctgactta gaaacaactg ctttaggttt tcgtttatta 1080
   cgtttacacc gttataatgt atctccagca atctttgata atttcaaaga tgccaatggc 1140
   aaattcattt gtagcactgg tcagtttaat aaggatgtgg cttcaatgtt aaacttatac 1200
   cgtgcatcac aattagcatt cccaggcgaa aacattttag atgaagctaa atcttttgcc 1260
   accaaatact tacgtgaagc ccttgaaaaa tctgaaactt catcagcttg gaacaataaa 1320
   cagaatttaa gtcaagaaat caagtatgca ttaaaaactt catggcacgc ttctgtacca 1380
   cgtgttgaag caaaacgtta ttgtcaagtt tatcgtcctg attacgctcg tattgctaag 1440
   tgtgtataca aattaccata cgttaacaac gaaaaattct tagaattagg taaattagat 1500
   tttaacatca ttcaatcaat tcatcaagaa gaaatgaaaa atgtgacaag ttggtttcgt 1560
   gattctggct taccattatt tactttcgct cgcgaacgtc ctttagaatt ttacttctta 1620
   gttgctgctg gtacttatga acctcaatat gctaaatgtc gtttcttatt cacaaaagta     1680
   gcttgtcttc aaacagtatt agacgatatg tacgatactt acggtacttt agacgaatta     1740
   aaacttttta ccgaggctgt gcgtcgttgg gatttatctt ttacagaaaa tttacctgac     1800
   tatatgaaat tatgttatca aatctattat gacatcgttc atgaagtggc ttgggaagct     1860
   gaaaaagaac aaggtagaga attagtgtca ttcttccgta aaggctggga agactactta     1920
   ttaggttact atgaagaagc agaatggtta gcagcagaat acgttccaac attagatgaa     1980
   tacattaaaa acggtattac atcaatcggc caacgtatct tattactttc aggtgtgtta     2040
   attatggatg gccaactttt atcacaagaa gcattagaaa aagttgatta ccctggtcgt     2100
   cgtgttttaa ctgagttaaa ctcacttatt agccgtttag ctgacgacac taaaacttat     2160
   aaagcagaaa aagctcgtgg agaattagcc tcatcaattg aatgctacat gaaagatcat     2220
   cctgaatgta cagaagaaga agccttagac cacatttatt ctattcttga accagccgta     2280
   aaagaattaa ctcgtgaatt tcttaaacca gacgacgttc catttgcttg taaaaagatg     2340
   ttattcgaag aaactcgtgt tacaatggtg atctttaaag atggtgatgg ttttggtgta     2400
   tctaagttag aagttaaaga tcacatcaaa gaatgcttaa ttgaaccatt accattaggt     2460
   accggtgaaa acttatactt tcaaggctca ggtggcggtg gaagtgatta caaagatgat     2520
   gatgataaag gaaccggtta a                                               2541
<210> 36
   <211> 542
   <212> DNA
   <213> Artificial Sequence
<220>
   <223> modified endogenous promoter from the psbA gene of S. dimorphus
<400> 36
   tcgttgagta gtttttcaga ttaattgcta tgcaacccat gtgaattaaa aatataacat        60
   aaatttcaaa aatgtcaatt tttagtctaa aaaatattat tcgatgtttt tttatgacaa       120
   ttttttttaa atttttcaat aaaaacaaaa atatattatt aaagaataca taaaaaaatc       180
   aaaaattcat aaataaatca ccaaaaaatt tattttttaa tatttgattt caatattttt       240
   atttgaatta aaaatttaat tatttaaaat ttttatttat atatttgaat ttatacttca       300
   gtttttatta aacttaagtt ttcaaatcat aaatttaata gttaatattt ttttaaactc       360
   taaattatta atctttaaaa tttcaaatct ttaacacttg aaattataaa cttcattgtt       420
   tttgtttgaa ttttttttta agtttgaaaa ctttataaaa ttaaataaac taaatagaat       480
   tttgaattgt ataaaaatta aaatgaaaag tttgtgtttt tcagattaaa tgtagcaacc       540
   aa                                                                      542
<210> 37
   <211> 559
   <212> DNA
   <213> Scenedesmus dimorphus
<400> 37
   atttaatctg aaaaacacaa acttttcatt ttaattttta tacaattcaa aattctattt        60
   agtttattta attttataaa gttttcaaac ttaaaaaaaa attcaaacaa aaacaatgaa       120
   gtttataatt tcaagtgtta aagatttgaa attttaaaga ttaataattt agagtttaaa       180
   aaaatattaa ctattaaatt tatgatttga aaacttaagt ttaataaaaa ctgaagtata       240
   aattcaaata tataaataaa aattttaaat aattaaattt ttaattcaaa taaaaatatt       300
   gaaatcaaat attaaaaaat aaattttttg gtgatttatt tatgaatttt tgattttttt       360
   atgtattctt taataatata tttttgtttt tattgaaaaa tttaaaaaaa attgtcataa       420
   aaaaacatcg aataatattt tttagactaa aaattgacat ttttgaaatt tatgttatat       480
   ttttaattaa caagagttca attgaattca tgaaaaatct tttaaagatt cggagaattt       540
   taaattttta tttttttat                                                     559
<210> 38
   <211> 716
   <212> DNA
   <213> Artificial Sequence
<220>
   <223> modified endogenous promoter for the psbB gene of S. dimorphus
<400> 38
   ttaaattact ttatttatgg aaaaatgtat tttttcgatt tctagagttt ttttacactt 60
   tttgaattgt gtgttttttt cttttctaaa agaaaaaaga caattgaaag tcttgtttct 120
   tcatttttat tattttatca taaatctaga atttttaaaa tattttttat tttttaccgc 180
   ggagcggact aaattttttt taaacaatat tttttaattg aaaacatttt ttttcttcaa 240
   aatataataa taaaatttga aaaaaaagaa aaacaaaaaa taaaagcttt cctctgctta 300
   agttgtattt ttttatgttt ttattttttt tgaaagtttt gaaaaaagta caaaaaagtt 360
   taaatttttt ttcatttttt tcacattttc tttatcatat tatcgtctta agtttttttc 420
   ttttttttca gtttttttta agaaactgaa aaaaaagaga aaaacttaag acaataaaaa 480
   agattttttt aacagaaaaa agtttctttt ttaaataaaa tgaaaacaaa agttgtaggc 540
   aaccaaacat ttatttaatt cataaagaga gtacaactta agcagaggaa agcttttatt 600
   ttttgttttt cttttttttc aatttttatt aatatatttt gaagaaaaaa aatgtttttc 660
   aattaaaaaa tattgtttaa aaaaaaattt aatcccgctc cgcggtaaac agtaaa 716
<210> 39
   <211> 550
   <212> DNA
   <213> Scenedesmus dimorphus
<400> 39
   aacttttgtt ttcattttat ttaaaaaaga aacttttttc tgttaaaaaa atctttttta 60
   ttgtcttaag tttttctctt ttttttcagt ttcttaaaaa aaactgaaaa aaaagaaaaa 120
   aacttaagac gataatatga taaagaaaat gtgaaaaaaa tgaaaaaaaa tttaaacttt 180
   tttgtacttt tttcaaaact ttcaaaaaaa ataaaaacat aaaaaaatac aacttaagca 240
   gaggaaagct tttatttttt gtttttcttt tttttcaaat tttattatta tattttgaag 300
   aaaaaaaatg ttttcaatta aaaaatattg tttaaaaaaa atttagaaaa aaataaaaaa 360
   tattttaaaa attctagatt tatgataaaa taataaaaat gaagaaacaa gactttcaat 420
   tgtctttttt cttttagaaa agaaaaaaac acacaattca aaaagtgtaa aaaaactcta 480
   gaaatcgaaa aaatacattt ttccataaat aaagtaatta agtaaacaaa aattcttttt 540
   tcattaattt 550
<210> 40
   <211> 597
   <212> DNA
   <213> Scenedesmus dimorphus
<400> 40
   tttttgaatt agataaatga gtgttctcaa tttttttttc tttgcatttt ttgtttgtgt 60
   tgatttacaa aaacaataga aaaaagaaaa caatattttc tttctaaaaa aaaacaaaat 120
   tgatgaaaaa tagacatgaa caaaaaattt tgaaagttga ctttttaaaa aaatttttgg 180
   tataatacaa aaaaagaatt tttggaaagg tggcagagtg gttgaatgct ctggttttga 240
   aaaccagcgt ggctttacgg tcaccggggg ttcgaatccc tccctttccg ataatatata 300
   caaaaatttt taaagttttt tgtttatttt gtatagataa aaaatctgca ataaaaattt 360
   cgttttttat ttattcaaaa attctgtttt tttgaaaaga aaataaaaaa aatgccaaaa 420
   gtgagttttt tattcaaata ttagaaaaag tttttgaaaa atttaaaaaa atagaaaaaa 480
   tttttttatt tttttcataa tttaaaaaat tatgttataa tttaaattac aaataggttt 540
   tattaaaaaa tttttacgta cagatgaatt ctataaaatt attttggaga tcaccat 597
<210> 41
   <211> 537
   <212> DNA
   <213> Artificial Sequence
<220>
   <223> modified endogenous promoter for the tufA gene of S. dimorphus
<400> 41
   ttaaaccaat tttccaagta actttacttt atcaaaaatt aaaaaattaa aaaactttta 60
   ttgaacttaa aataaaattt ttaacaaaat ttattttaaa aaaaagaaaa aattttttta 120
   ttttggtttt atttatttct ttttttttac aaacaaaaat ttttttaaac agaataataa 180
   aaaaaatttt atttaaagaa tggtttttta atattttgct catgacaaat gattttttac 240
   tacttttatg ctttttttca aaaaaagcag caaagcaaaa aagttataaa aagtgtatgg 300
   agcaaacagt taaattgaca ctttttaaaa gtatttatag gcccaaccgg acttgaaccg 360
   atgacctatt gcttgtaagg caatcactct accaactgag ttatgggcct aaaaaatatt 420
   atttatattt tataatagaa tataaaatct aacaacttct ttatcctcct cttttctcac 480
   ctatcttttt tggttggggg taagtgaaaa aggaaatggt tgctcgcaac gaaaccc 537
<210> 42
   <211> 492
   <212> DNA
   <213> Scenedesmus dimorphus
<400> 42
   taaagaagtt gttagatttt atattctatt ataaaatata aataatattt tttaggccca 60
   taactcagtt ggtagagtga ttgccttaca agcaataggt catcggttca agtccggttg 120
   ggcctataaa tacttttaaa aagtgtcaat ttaaccgctt gctccataca ctttttataa 180
   cttttttgct ttgctgcttt ttttaaaaaa aagcataaaa gtagtaaaaa atcatttgtc 240
   atgagcaaaa tattaaaaaa ccattcttta aataaaattt tttttattat tctgtttaaa 300
   aaaatttttg tttgtaaaaa aaaagaaata aataaaacca aaataaaaaa attttttctt 360
   ttttttaaaa taaattttgt taaaaatttt attttaagtt caataaaagt tttttaattt 420
   tttaattttt gataaagtaa agttacttgg aaaattggtt tatacagaaa aaattataaa 480
   ttatttattc at 492
<210> 43
   <211> 557
   <212> DNA
   <213> Artificial Sequence
<220>
   <223> modified endogenous promoter of the rpoA of S. dimorphus
<400> 43
   cttgtttttt tttctaaatt tacctttaac ttgaaagatc taaaatgaca aaaatttttt 60
   ttagagagca agttttactt ttgttgtcta aaaaactcaa attacacttt gtcttaagaa 120
   ccttttttta ttaaaaaaca aaattttttt attttgtttc tttttattca tttgaaaaaa 180
   aagttttcta aacttaactg ataattcaag aaaaaaaata tgcctgctac tagattcgaa 240
   ctagtgactt tccgcgtatg aaacggatgc tctggccaac tgagctaagc aggcttaata 300
   tcatatatta tacaaaattt ttttcaacaa aaacaagttt ttttttcgaa ttttagaatt 360
   ttttgattat ttttgtttgt ttgaatattc caaacaaaaa taaagttttt ctcttttttt 420
   ttactttttt cctgcttcca aatactaaaa aagtttaata gaaaaactct ttttttgaaa 480
   gttgtcaaat ttttcctttc aaaaaattat ttttttgttg ggctattttt gaaaataaat 540
   gtgagctcgg aaaaaaa 557
<210> 44
   <211> 532
   <212> DNA
   <213> Scenedesmus dimorphus
<400> 44
   cgagctcaca tttattttca aaaatagccc aacaaaaaat aattttttga aaggaaaaat 60
   ttgacaactt tcaaaaaaag agtttttcta ttaaactttt ttagtatttg gaagcaggaa 120
   aaaagtaaaa aaaaagagaa aaactttatt tttgtttgga atattcaaac aaacaaaaat 180
   aatcaaaaaa ttctaaaatt cgaaaaaaaa acttgttttt gttgaaaaaa attttgtata 240
   atatatgata ttaagcctgc ttagctcagt tggccagagc atccgtttca tacgcggaaa 300
   gtcactagtt cgaatctagt agcaggcata tttttttctt gaattatcag ttaagtttag 360
   aacaaatgaa taaaaagaaa caaaataaaa aaattttgtt ttttaataaa aaaaggttct 420
   taagacaaag tgtaatttga gttttttaga caacaaaagt aaaacttgtt ctctaaaaaa 480
   aatttttgtc attttagatc tttcaagtta aaggtaaatt tagaaaaaaa aa 532
<210> 45
   <211> 483
   <212> DNA
   <213> Scenedesmus dimorphus
<400> 45
   aaaagaaatt tttttttatt cctttatttt tttgtattcc aaaaaatatt ttgtttttct 60
   tcaatacagt ttttttgcta ttgctttttc tcactttttt gctttgctgc tttttttttt 120
   aagcgtaaaa gtgaaaaaaa aagtttgtaa aacaaaaaaa ttgaaaaaaa aagattgatt 180
   ttttatacag taaaaatttt ttgtttttta aatatttatt taaattcaac tcaatttcaa 240
   aaaaaaatgt ctttgttgaa tgcaaatttt tttgaacaaa caacaattca aaataaaaca 300
   atttttggtt taaattcaaa atttttaaat tcatctacag aaaattcttc catgagtatt 360
   tttaaaactt taaagggagt ttgttctgga aaaagaaata tactttgcaa aagtacacct 420
   cgtcctatga attctgattt tttaaaaaat aatagttcta ttccttctca aaaacaaaaa 480
   gga 483
<210> 46
   <211> 669
   <212> DNA
   <213> Artificial Sequence
<220>
   <223> modified endogenous promoter of the ftsH gene in S. dimorphus
<400> 46
   agtagaaaaa atgatgtact tgtataaata aaaaataatt atttatacaa aaaatttttt 60
   tttattttgt ttttactttt tttcattttt ttgctttgct gttttttatg aattttttac 120
   tttactgctt tttttcttct ttacttttac gctttttatc acttttttga tttgctgctt      180
   tttttcttct tcattttttg ctttgctgct ttttttcttc ttcatttttt gctttgcaaa      240
   aaatgataaa aagcgtaaaa tgataaaaag cgtaaaatga taaaaagcgt aaaatgataa      300
   aaagcgtaaa agtaaaaaag aaaaaaagca gcaaagtaaa aaagtgataa aaagtttaaa      360
   agtgctaaaa agcgtaagta aacaaaaaaa gcaaaagaca aaaaaaagtt ttcaaaaaaa      420
   aatttttatt tttgtttttt tataaaaatt ttaacaagtt ttattttcta aataaaacaa      480
   aaaattgtga gttttaaaaa aacgaaaaag ttcaaaaatt tttttttgat ggacaaaaag      540
   aaagaaattt taaatttatt aatttttcaa ataactaata aattttgttc aacaggagag      600
   aactattgaa aaaaagccta ctatcggagt tgaaccgata accttcgatt tagctagcaa      660
   ccaacaaac                                                               669
<210> 47
   <211> 557
   <212> DNA
   <213> Scenedesmus dimorphus
<400> 47
   ctgtttcaca acgtttgcat ccaacgcaat cttcagtacg tggtgctgaa gccatttgat       60
   ttgctttaca accattccat ggaaccattt ctaaaacatc taatggacaa gcacgtacac      120
   attgtgtaca accaatacaa gtatcataaa tttttacaat atgtgacatt ttttattttt      180
   aatagaacta ttcaaaagaa gattgagaat ttcaaaaaag aattttttta catgaaaaac      240
   gattttttag aaattttact ataaaaaagt aaaaaaaaat attaaatttt gtagtttttt      300
   gcaggagcag gatttgaacc tgcgacattg ggattatgag ccccacgagc taccagactg      360
   ctctatcctg cgttttttgt tttaaaaagt tttattcgta aaaaaaattt cttttatata      420
   tattacttta ttgattttta tttgtcaaat ttttttttgt ttgaattttt atttcaaatt      480
   ttttaaatat agaaaaaatt tttttttatg ataatataaa actacagttt gaattataaa      540
   taaaaaaaaa aaatgaa                                                    557
<210> 48
   <211> 580
   <212> DNA
   <213> Artificial Sequence
<220>
   <223> modified endogenous promoter of the rbcL gene in S. dimorphus
<400> 48
   tttttttggg gttggttacg gtttttttta cttttgcttt ttttgctttt gttcaaagaa      60
   aaaaaaatac aaaataaaaa aaactaaaat gaaaaaacaa agaattctaa aattcataaa     120
   aaaaattaaa acccaatttt ttttttggaa acttttccaa ataataaaaa aatcaaaaaa     180
   aaatttttct agtatttttt tcatattttg aaactttttt tgagtttata aaaaaataga     240
   aaaaacaaat agatgaaaat ttagaaaaat tataaaccaa taaaaatgaa gttttgcgta     300
   gaaaaaaaat ttagtttact tgttccccaa gagcaagtgg taactttgaa aaaaatattt     360
   aaacttaaaa atttgctaaa gttttgaatt tatgttaaaa ttttaaaaaa ataaaaattt     420
   ttaaactatt tttttatgtt aaaaaaatag tttttattat tttctataat atagtttagt     480
   tttttatttt tttcaatttc tttttttttt ttcaaagaaa aaagttttcc acggatagat     540
   ttttatagga tcgacaaaat gttctatgaa ctttaaaaaa                           580
<210> 49
   <211> 555
   <212> DNA
   <213> Scenedesmus dimorphus
<400> 49
   ggtttttttt acttttgctt tttttgcttt tgttcaaaga aaaaaaaata caaaataaaa      60
   aaaactaaaa tgaaaaaaca aagaattcta aaattcataa aaaaaattaa aacccaattt     120
   tttttttgga aacttttcca aataataaaa aaatcaaaaa aaaatttttc tagtattttt     180
   ttcatatttt gaaacttttt ttgagtttat aaaaaaatag aaaaaacaaa tagatgaaaa     240
   tttagaaaaa ttataaacca ataaaaatga agttttgcgt agaaaaaaaa tttagtttac     300
   ttgttcccca agagcaagtg gtaactttga aaaaaatatt taaacttaaa aatttgctaa     360
   agttttgaat ttatgttaaa atttaaaaaa aataaaaatt tttaaactat ttttttatgt     420
   taaaaaaata gtttttatta ttttctataa tatagtttag ttttttattt ttttcaattt    480
   cttttttttt ttcaaagaaa aaagttttcc acggatagat ttttatagga tcgacaaaat     540
   gttctatgaa ctttt                                                      555
<210> 50
   <211> 532
   <212> DNA
   <213> Artificial Sequence
<220>
   <223> modified endogenous promoter for the chlB gene from S. dimorphus
<400> 50
   cccagtcttc agtacgtggt gctgaagcca tttgatttgc tttacaacca ttccatggaa 60
   ccatttctaa aacatctaat ggacaagcac gtacacattg tgtacaacca atacaagtat 120
   cataaatttt tacaatatgt gacatttttt atttttaata gaactattca aaagaagatt 180
   gagaatttca aaaaagaatt tttttttaca tgaaaaacga ttttttagaa attttactat 240
   aaaaaagtaa aaaaaaatat taaattttgt agttttttgc aggagcagga tttgaacctg 300
   cgacattggg attatgagcc ccacgagcta ccagactgct ctatcctgcg ttttttgttt 360
   taaaaagttt tattcgtaaa aaaaatttct tttatatata ttactttatt gatttttatt 420
   tgtcaaattt ttttttgttt gaatttttat ttcaaatttt ttaaatatag agaaaatttt 480
   tttttatgat aatataaaac tacagtttga attataaata aaaaaaaaaa at 532
<210> 51
   <211> 615
   <212> DNA
   <213> Scenedesmus dimorphus
<400> 51
   taaaccgaag gttatcgggt caactccgat agtaggcttt ttttcaatag ttctctcctg 60
   tgaacaaaat ttattagtta tttgacaaaa ttaataaatt taaaattctt tccttttgtc 120
   catcaaaaaa aaaatttttg aactttttcg tttttttaaa actcacaatt ttttgtttta 180
   ttagaaaata aaacttgtta aaatttttat aaaaaaacaa aaataaaaat ttttttttga 240
   aaactttttt ttgtcttttg ctttttttgt ttacttacgc tttttagcac ttttaaattt 300
   tttatcactt ttttactttg ctgctttttt tcttctttac ttttacgctt tttatcattt 360
   tacgcttttt atcattttac gctttgcaaa aaatgaagaa gaaaaaaagc agcaaagcaa 420
   aaaatgaaga agaaaaaaag cagcaaatca aaaaagtgat aaaaagcgta aaagtaaaga 480
   agaaaaaaag cagtaaagta aaaaattcat aaaaaacagc aaagcaaaaa aatgaaaaaa 540
   agtaaaaaca aaataaaaaa aaattttttg tataaataat tattttttat ttatacaagt 600
   acatcatttt ttcta 615
<210> 52
   <211> 533
   <212> DNA
   <213> Artificial Sequence
<220>
   <223> modified endogenous promoter of the petA gene in S. dimorphus
<400> 52
   tgcacaaaag aaatcaaatg ttttcaaatc gtgcaaacat caaattgcac aaaataattt      60
   ttaaaattca ttttatgaaa gttcgttctt cagttaaaaa aatttgtact aaatgtcgtt     120
   taattcgtcg aaaaggtaca gtaatggtta tttgtacaaa tcctaaacat aaacaacgtc     180
   aaggataatt ttttttaatg gaagaaatgt tttttacttc ttccattaaa aaagaaaaaa     240
   gaaaagtgca gaactttttt tttgatcaaa aaaaagatac aaaaaatttt tttgttttaa     300
   ttttatgata taattctatt tcagagaaga aaaaaaaatt taaacaaaac aaaaaaatac     360
   agagaagttt aaatttagac ctaaaggtag atttccataa attcattttt ctcatttgtt     420
   tttttctttt ttttgcattg cgaaaaaaaa aagaaaaaag caaaaagtca ttttaaagag     480
   araaatggag aaagataaaa gtttttaact ttttttgaac aattccatgg ggg            533
<210> 53
   <211> 521
   <212> DNA
   <213> Scenedesmus dimorphus
<400> 53
   acaaaagaaa tcaaatgttt tcaaatcgtg caaacatcaa attgcacaaa ataattttta      60
   aaattcattt tatgaaagtt cgttcttcag ttaaaaaaat ttgtactaaa tgtcgtttaa     120
   ttcgtcgaaa aggtacagta atggttattt gtacaaatcc taaacataaa caacgtcaag     180
   gataattttt tttaatggaa gaaatgtttt ttacttcttc cattaaaaaa gaaaaaagaa     240
   aagtgcagaa cttttttttt gatcaaaaaa aagatacaaa aaattttttt gttttaattt     300
   tatgatagaa ttctatttca gagaagaaaa aaaaatttaa acaaaacaaa aaaatacaga     360
   gaagtttaaa tttagaccta aaggtagatt tccataaatt catttttctc atttgttttt     420
   ttcttttttt tgcattgcaa aaaaaaaaga aaaaagcaaa aagtcatttt aaagagaaaa     480
   atggagaaag ataaaagttt ttaacttttt ttgaacaatt c                         521
<210> 54
   <211> 503
   <212> DNA
   <213> Artificial Sequence
<220>
   <223> modified endogenous promoter for the petB gene from S. dimorphus
<400> 54
   aagaaagttt aaaaaaattt acataagaag aagatacaaa aacaaattat tttcaatttt      60
   tgttttgtaa aaaaaaaatt tcgtttttta gaatttctat gttttttttt attttttgtt     120
   ttctgaaaaa aaaagttttt tcagaaaaca agaagaaaat taaaggtcta caaaataaaa     180
   attattttgt tacaaacgac caatgtttat tattttttgt ttttttcatt ttctatttct     240
   cacttatctt tttaaagtca aaagaaaatt tagaatgaaa gtaaaaaaaa gaaaaaacaa     300
   taaaaaacaa aaaagcaaaa aaaattcttt gcaatttttg tttaaaaatt tttggtttca     360
   attttaaaat ttatgaaaaa tgtacaccat aagtctaaaa tttacaaaaa ttatctaaaa     420
   aaacttcttt cttttgtaac aaaaggcaat aagcccataa ttcgagcact tttaattgtt     480
   tttgtaaaca aaccaaaaaa aaa                                             503
<210> 55
   <211> 513
   <212> DNA
   <213> Scenedesmus dimorphus
<400> 55
   aaaaacaatt aaaagtgctc gaattatggg cttattgcct tttgttacaa aagaaagaag      60
   tttttttaga taatttttgt aaattttaga cttatggtgt acatttttca taaattttaa   120
   aattgaaacc aaaaattttt aaacaaaaat tgcaaagaat tttttttgct tttttgtttt   180
   tttattgttt tttctttttt ttactttcat tctaaatttt cttttgactt taaaaagata     240
   agtgagaaat agaaaatgaa aaaaacaaaa aataataaac attggtcgtt tgtaacaaaa     300
   taatttttat tttgtagacc tttaattttc ttcttgtttt ctgaaaaaac tttttttttc      360
   agaaaacaaa aaataaaaaa aaacatagaa attctaaaaa acaaattttt tttttacaaa     420
   acaaaattga aaaaatttgt ttttgaatct tattcctatg aattttttta actttttcgt     480
   ttaatggtat tataaaaaat tttctttttc tat                                  513
<210> 56
   <211> 537
   <212> DNA
   <213> Artificial Sequence
<220>
   <223> modified endogenous terminator region for the rbcL gene from S. dimorphus
<400> 56
   tttttaaaat acttcctctt taaaggggaa gtattttttt cttgatttta gaactctaaa 60
   aacacaaatt gtttattttt gtttttcatt ttcatttatt tattgataaa aacaaaagaa 120
   gcagcaaagc aaaaaaacaa aaaaaactaa aaaaatttgt tttgttcatt tcaatagaat 180
   aaaaaaaaca aaatttttca aaaaaattta taaaattatt gtaagttttc aatacaaaaa 240
   attttttaaa aatatttttt aatatgtttt ttttttaaat tgaaaaaaaa aattcaaaac 300
   aaagaatttt aaaaaaatag aagagttttt aattaaaaaa aattgaaatt tcaaaaattt 360
   tttgttaata taatctttag tataattcat gcaaaataaa tgacaaatga gtcaaaaaaa 420
   agcatgggcc gatgcccgag tggttaatgg gggcggattg taaatccgct ggttacgcct 480
   acgttggttc gaatccgact cggcccaaaa atacaaaata cattgtttga aagacta 537
<210> 57
   <211> 538
   <212> DNA
   <213> Scenedesmus dimorphus
<400> 57
   ttttttttta aaatacttcc tctttaaagg ggaagtattt ttttcttgat tttagaactc 60
   taaaaacaca aattgtttat ttttgttttt cattttcatt tatttattga taaaaacaaa 120
   agaagcagca aagcaaaaaa acaaaaaaaa ctaaaaaaat ttgttttgtt catttcaata 180
   gaataaaaaa aacaaaattt ttcaaaaaaa tttataaaat tattgtaagt tttcaataca 240
   aaaaattttt taaaaatatt ttttaatatg tttttttttt aaattgaaaa aaaaaattca 300
   aaacaaagaa ttttaaaaaa atagaagagt ttttaattaa aaaaaattga aatttcaaaa 360
   attttttgtt aatataatct ttagtataat tcatgcaaaa taaatgatca aatgagtcaa 420
   aaaaaagcat gggccgatgc ccgagtggtt aatgggggcg gattgtaaat ccgctggtta 480
   cgcctacgtt ggttcgaatc cgactcggcc caaaaataca aaatacattg tttgaaag 538
<210> 58
   <211> 669
   <212> DNA
   <213> Scenedesmus dimorphus
<400> 58
   ctagatttta ttttttatga aaaactcagg cttaatttag gcttgagttt ttcattcttt 60
   ttgaagctct gaaattttaa aatttctagt cttctttaat gtttttaaat tttaaaaaat 120
   aaatttcttc tctgctgtgt ttttcttttt ttttgaaaaa acaaagaaaa aaaatttttt 180
   tgttttcttc tttgtttttt tatttctttt tgttttgttt attttttagt ttcagaatct 240
   ttgattcaaa aaaaaattta gtccgattac tccataggag caagcagtaa aaaataaaaa 300
   ctgtaataaa aaataaaaca aaaattttat ttctttttgt tttgcttgaa cttttcaaaa 360
   aaaaattgaa aaattcaagc aaaacaaaaa gaaacaaata aaaaatttat gaattttcta 420
   ctttttcagg agttgaaatt tctcctttac ttaaaacata ttttgctaaa aaaagcgctt 480
   gtgttgcttt ttttgctact ttttgtttcc aagcattttt tcgaatattt ttttttgatt 540
   ttgatgtgcg tttttgttaa cctaaaatct tgaaaagatt tactcttttc aaatttttat 600
   gtttttattt tttttattca taaaaaaaaa caatacataa aaataaagta tttcggcttc 660
   aaaaactag 669
<210> 59
   <211> 597
   <212> DNA
   <213> Scenedesmus dimorphus
<400> 59
   aatttccatt tttttcattt ttttttagaa aagttgtatt tttcttgatg aaatgaaaat 60
   ttcaaaaaag aaaaatacaa tttttctcta cttttttttg attctttact tttttttgaa 120
   ttttttttgt gcttcgtttt tgaaaaaaac tttttatttg aaaaattttg ttgaattaaa 180
   aaaaatcaac tcaatacatt ttttttgaac ttctttttac ttttttgctt tgttgctttt 240
   cttttcattt ttatcatttt ttgcttcgct gctttttatc attttttgct ttgcaaaaaa 300
   tgaagaagaa aaaaagcgta aaatgaaaaa gaaaaaaagt ttcaaagaaa aaaaagcgaa 360
   aaagcaagag aagataaaaa acaagaacaa aaaaattgtt caaaaacaca atagaaaatt 420
   ctttaaaaat tttttgattt tttatagaat ttgtgagaaa tagtaaaaaa agtcaaaaaa 480
   tcacaacatt ataaatagaa aaaatcttgt tttgtaaaaa tttataattt tttatattat 540
   ttcaatttta aataagatct tttggagctg aaaaaatatg agaaatagtt tggacaa 597
<210> 60
   <211> 12
   <212> DNA
   <213> Artificial Sequence
<220>
   <223> nucleic acid linker
<400> 60
   gaggcagatc aa 12
<210> 61
   <211> 12
   <212> DNA
   <213> Artificial Sequence
<220>
   <223> nucleic acid linker
<400> 61
   gaggtaatac tt 12
<210> 62
   <211> 514
   <212> DNA
   <213> Dunaliella tertiolecta
<400> 62
   gtatacgaaa cctttaaggt taaagagata tatgttaaat taaacataaa cgaaaagact      60
   ttaaattttt caaataaaaa aaaagataca gagggtacta atatttaata ttatgacctt     120
   ctgtatccta tacttaataa gtataaatta taatatagat taataaatct attcaagtta     180
   ataaactgtg tttttatttt atttaatgat tttctctact aaatattaaa tatgttatta     240
   tttatacata gtgttttttc tttttttttt ttaagcctgt ttaactcaat cggtagagta     300
   ttggttttgt aaaccaaagg ttgcgggttc gattcctgta gcaggctact aattttttaa     360
   gatattttat attttaaaaa tatcttttta aaataaaaaa aaaatttttt aaatcgattt     420
   taaaaataaa aaaagctata cttataaatg caataaaggt taaaaaaaaa attaaacgat     480
   atgatgaatt ataaaaatta ttatggagat gcac                                 514
<210> 63
   <211> 491
   <212> DNA
   <213> Dunaliella tertiolecta
<400> 63
   ctctagctag gacatctccc tttcacggag gaaacaggga ttcgaattcc cttgggggta      60
   aaaaaaaaat agtatatata taggtattta tacatttttc aataaatatt tgattgagag     120
   ttactaaata atctaatttg aattaaaata aattgaatgt ataattctat gtttcgctct     180
   cccacaggtt tataataact attattttat ttgaattatt tttttgaagt atatttcctt     240
   atttaaagtg cttagaaata attaattatt aataacatct gttttttttt tacttatttt     300
   tgaaagttca gtttcaatta taaaaatatt atttatataa tattcagaat aaaaattatg     360
   agtctaatta tgaattaata aaaaaaaaaa aaataaattc atcaaaagta tattaaaaaa     420
   taaaaaaggc taaaattaaa tatactcgaa aaattggctt atatataaaa atatattaat     480
   aatgataaac a                                                          491
<210> 64
   <211> 495
   <212> DNA
   <213> Dunaliella tertiolecta
<400> 64
   tttttctttt taggcgggtc cgaagtcctt aggcttattc gaaggaaaaa cgagaaaaat      60
   ttacgtagta aattttcttt gctggccctg ccaaaaacaa caccattaac ctataagtag     120
   taataattct ttagtattac ttttaggtta tttataaatt tgagaagtat agaagaatct     180
   atagattttg cttatgtgtt tatctataga ttcttctata cttctcattt ttaacaaatt     240
   tttattaaga tttttttaaa caaaaaaaaa gttttcaact tatataatta aacctaaaca     300
   acgttgtata ttttttattt taagttttgg taaagtatgt ataccagtaa acctttagta     360
   aattttttta ccgcttaggc taggacctat aaaatttagc gcggcgccct aagagctagg    420
   ataaagatcc gttgtctgtc cgctgcgcta aattttcttt agacgaagcg aagcgtcgag     480
   tctcacgcac ccacc                                                      495
<210> 65
   <211> 311
   <212> DNA
   <213> Dunaliella
<400> 65
   ttctaatctc ttaacgagat tagattaatc ccttaatgtc taagggatta atacttatcc        60
   catcctgtta aacccaggat gggataaaaa aatttacaag acaaggtatt aaaaatgaat       120
   aaaaatagat tatatgtatt taaacgtata taataaaatt atgagtaaaa taacagagta       180
   aaccaaaaca ataagatttt tttttttttt tttcaattct aaatatcaat atagaaggtt       240
   aggaaatttc cccggaaaaa atcctttgga ttttttccta agggcgaaaa aatattctct       300
   gaatattttt t                                                            311
<210> 66
   <211> 40
   <212> DNA
   <213> Artificial Sequence
<220>
   <223> PCR primer
<400> 66
   ttggttgggc ggccgccgtt taggtgtaac acaatcttgg 40
<210> 67
   <211> 40
   <212> DNA
   <213> Artificial Sequence
<220>
   <223> PCR primer
<400> 67
   ttggttgggc ggccgctagc cgtggtattt acttcactca 40
<210> 68
   <211> 35
   <212> DNA
   <213> Artificial Sequence
<220>
   <223> PCR primer
<400> 68
   ttggttggct cgaggccctt tcgtcttcaa gaaat 35
<210> 69
   <211> 34
   <212> DNA
   <213> Artificial Sequence
<220>
   <223> PCR primer
<400> 69
   ttggttggct cgagaagtct tgcgccttaa acca 34
<210> 70
   <211> 77
   <212> DNA
   <213> Artificial Sequence
<220>
   <223> PCR primer
<400> 70
   ttggttggct gcagttaatt aaggatccac tagtatttaa attcctgatg cggtattttc        60
   tccttacgca tctgtgc                                                       77
<210> 71
   <211> 116
   <212> DNA
   <213> Artificial Sequence
<220>
   <223> PCR primer
<400> 71
   ttggttgggc ggccgctcac agatgagaag atatttgctc gataatcaat actctaggca        60
   tctaactttt cccattgtct taaaccgact taccttagga atcatagttt catgat          116
<210> 72
   <211> 116
   <212> DNA
   <213> Artificial Sequence
<220>
   <223> PCR primer
<400> 72
   ttggttgggc ggccgctgct ggttgtcatt gcctctggat aatttttctc gaactatgcc        60
   tgcgcgttga taccaatcca atggatctac aggcagaacg gcctctagcg gttttt          116
<210> 73
   <211> 62
   <212> DNA
   <213> Artificial Sequence
<220>
   <223> PCR primer
<400> 73
   ttggttggat ttaaatacta gtggatcctt aattaactgc agggccggcc tctagtttta 60
   tt 62
<210> 74
   <211> 100
   <212> DNA
   <213> Artificial Sequence
<220>
   <223> PCR primer
<400> 74
   ttctgcctgt agatccattg gattggtatc aacgcgcagg catagttcga gaaaaattat 60
   ccagaggcaa tgacaaccag cacgggctcg agactagtgg 100
<210> 75
   <211> 100
   <212> DNA
   <213> Artificial Sequence
<220>
   <223> PCR primer
<400> 75
   gaatttttca aaaaattcat actttgtttt tttatttttt ctgagttttt aatcaaaaaa 60
   ctttttgtat aaaattcgta agatctccta ggaaaatgaa 100
<210> 76
   <211> 100
   <212> DNA
   <213> Artificial Sequence
<220>
   <223> PCR primer
<400> 76
   tttttttgaa aatagttttt ctcaattttt tatttttttt gttttttctc taaaaatcaa 60
   aaattcaatt ttgagaaaag ggctcgagac tagtttgtcc 100
<210> 77
   <211> 100
   <212> DNA
   <213> Artificial Sequence
<220>
   <223> PCR primer
<400> 77
   ggtaagtcgg tttaagacaa tgggaaaagt tagatgccta gagtattgat tatcgagcaa 60
   atatcttctc atctgtgact agtgctagct aaagaagttg 100
<210> 78
   <211> 80
   <212> DNA
   <213> Artificial Sequence
<220>
   <223> PCR primer
<400> 78
   gattggtatc aacgcgcagg catagttcga gaaaaattat ccagaggcaa tgacaaccag 60
   cacgggctcg agactagtgg 80
<210> 79
   <211> 83
   <212> DNA
   <213> Artificial Sequence
<220>
   <223> PCR primer
<400> 79
   cattaggaat tatctttttc gcaattttct ttagagaacc acctcgtatg gtaaaataac 60
   gtaagatctc ctaggaaaat gaa 83
<210> 80
   <211> 80
   <212> DNA
   <213> Artificial Sequence
<220>
   <223> PCR primer
<400> 80
   aaaagttaga tgcctagagt attgattatc gagcaaatat cttctcatct gtgatctcct 60
   agtgctagct aaagaagttg 80
<210> 81
   <211> 80
   <212> DNA
   <213> Artificial Sequence
<220>
   <223> PCR primer
<400> 81
   tcaacgccta tggcaactct gtagaatatt catcagcgta acgccttaga atatcatacg 60
   ggctcgagac tagtttgtcc 80
<210> 82
   <211> 80
   <212> DNA
   <213> Artificial Sequence
<220>
   <223> PCR primer
<400> 82
   acgctgatga atattctaca gagttgccat aggcgttgaa cgctacacgg acgatacgaa 60
   tttttgaatt agataaatga 80
<210> 83
   <211> 80
   <212> DNA
   <213> Artificial Sequence
<220>
   <223> PCR primer
<400> 83
   tcatactttg tttttttatt ttttctgagt ttttaatcaa aaaacttttt gtataaaatt 60
   ttttgaagcc gaaatacttt 80
<210> 84
   <211> 80
   <212> DNA
   <213> Artificial Sequence
<220>
   <223> PCR primer
<400> 84
   cattaggaat tatctttttc gcaattttct ttagagaacc acctcgtatg gtaaaataag 60
   ggctcgagac tagtttgtcc 80
<210> 85
   <211> 80
   <212> DNA
   <213> Artificial Sequence
<220>
   <223> PCR primer
<400> 85
   cattaccaga gtgctccgca gacgagtatg gcacatggct ccgcgaggct attttctcct 60
   agtgctagct aaagaagttg 80
<210> 86
   <211> 80
   <212> DNA
   <213> Artificial Sequence
<220>
   <223> PCR primer
<400> 86
   actctggtaa tgcatatggt ccacaggaca ttcgtcgctt ccgggtatgc gctctatgaa 60
   ttcccgtttt aagagcttgg 80
<210> 87
   <211> 80
   <212> DNA
   <213> Artificial Sequence
<220>
   <223> PCR primer
<400> 87
   actgattgac acggtttagc agaacgtttg aggactaggt caaattgagt ggtagttcaa 60
   gagaaaaaaa aagaaaaagc 80
<210> 88
   <211> 98
   <212> DNA
   <213> Artificial Sequence
<220>
   <223> PCR primer
<400> 88
   accactcaat ttgacctagt cctcaaacgt tctgctaaac cgtgtcaatc agtgtctgct 60
   tcctgagtga aacccgtaag atctcctagg aaaatgaa 98
<210> 89
   <211> 80
   <212> DNA
   <213> Artificial Sequence
<220>
   <223> PCR primer
<400> 89
   acgctgatga atattctaca gagttgccat aggcgttgaa cgctacacgg acgatacgaa 60
   agcagttgct ttctcctatg 80
<210> 90
   <211> 80
   <212> DNA
   <213> Artificial Sequence
<220>
   <223> PCR primer
<400> 90
   tctttggccg ttgtgccggg agcgctcatg tcaatacttt tctcctctag aagttgaaag 60
   tacaataaac caagatgaag 80
<210> 91
   <211> 80
   <212> DNA
   <213> Artificial Sequence
<220>
   <223> PCR primer
<400> 91
   aaagtattga catgagcgct cccggcacaa cggccaaaga agtctccaat ttcttatttc 60
   tttttgaatt agataaatga 80
<210> 92
   <211> 102
   <212> DNA
   <213> Artificial Sequence
<220>
   <223> PCR primer
<400> 92
   ccattttttt gaaaatagtt tttctcaatt ttttattttt tttgtttttt ctctaaaaat 60
   caaaaattca attttgagaa aagggtaata actgatataa tt 102
<210> 93
   <211> 102
   <212> DNA
   <213> Artificial Sequence
<220>
   <223> PCR primer
<400> 93
   aggtatgaat ttttcaaaaa attcatactt tgttttttta ttttttctga gtttttaatc 60
   aaaaaacttt ttgtataaaa ttgatcctcg agagatctta tg 102
<210> 94
   <211> 80
   <212> DNA
   <213> Artificial Sequence
<220>
   <223> PCR primer
<400> 94
   acgctgatga atattctaca gagttgccat aggcgttgaa cgctacacgg acgatacgaa 60
   tttttgaatt agataaatga 80
<210> 95
   <211> 34
   <212> DNA
   <213> Artificial Sequence
<220>
   <223> PCR primer
<400> 95
   ttggttggac tagtgggtaa taactgatat aatt 34
<210> 96
   <211> 34
   <212> DNA
   <213> Artificial Sequence
<220>
   <223> PCR primer
<400> 96
   ttggttggac tagtgatcct cgagagatct tatg 34
<210> 97
   <211> 83
   <212> DNA
   <213> Artificial Sequence
<220>
   <223> PCR primer
<400> 97
   ttgtattttt aaatttttag tttgaactac aactaattat tttctacgta agatctacta 60
   gtgggctcga gactagtttg tcc 83
<210> 98
   <211> 83
   <212> DNA
   <213> Artificial Sequence
<220>
   <223> PCR primer
<400> 98
   ttgtattttt aaatttttag tttgaactac aactaattat tttctacgta agatctacta 60
   gtgggctcga gactagtttg tcc 83
<210> 99
   <211> 43
   <212> DNA
   <213> Artificial Sequence
<220>
   <223> PCR primer
<400> 99
   ggttggttgc ggccgccggt ccggtagcag ttaataatgt agg 43
<210> 100
   <211> 59
   <212> DNA
   <213> Artificial Sequence
<220>
   <223> PCR primer
<400> 100
   ccggcctcta gaggccggcc cctaggagat cttacgtaga aaataattag ttgtagttc 59
<210> 101
   <211> 57
   <212> DNA
   <213> Artificial Sequence
<220>
   <223> PCR primer
<400> 101
   ccggcctcta gaggccggcc actagtctcg agcccgggtt gaactatcaa gtttagg 57
<210> 102
   <211> 44
   <212> DNA
   <213> Artificial Sequence
<220>
   <223> PCR primer
<400> 102
   ccaaccaagc ggccgcggcg cgcctttatt atgggcgaac gacg 44
<210> 103
   <211> 1291
   <212> DNA
   <213> Artificial Sequence
<220>
   <223> codon optimized sequence comprising URA3
<400> 103
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accataccac cttttcaatt catcattttt tttttattct tttttttgat ttcggtttcc      240
   ttgaaatttt tttgattcgg taatctccga acagaaggaa gaacgaagga aggagcacag     300
   acttagattg gtatatatac gcatatgtag tgttgaagaa acatgaaatt gcccagtatt     360
   cttaacccaa ctgcacagaa caaaaacctg caggaaacga agataaatca tgtcgaaagc     420
   tacatataag gaacgtgctg ctactcatcc tagtcctgtt gctgccaagc tatttaatat     480
   catgcacgaa aagcaaacaa acttgtgtgc ttcattggat gttcgtacca ccaaggaatt     540
   actggagtta gttgaagcat taggtcccaa aatttgttta ctaaaaacac atgtggatat     600
   cttgactgat ttttccatgg agggcacagt taagccgcta aaggcattat ccgccaagta     660
   caatttttta ctcttcgaag acagaaaatt tgctgacatt ggtaatacag tcaaattgca     720
   gtactctgcg ggtgtataca gaatagcaga atgggcagac attacgaatg cacacggtgt     780
   ggtgggccca ggtattgtta gcggtttgaa gcaggcggca gaagaagtaa caaaggaacc     840
   tagaggcctt ttgatgttag cagaattgtc atgcaagggc tccctatcta ctggagaata     900
   tactaagggt actgttgaca ttgcgaagag cgacaaagat tttgttatcg gctttattgc     960
   tcaaagagac atgggtggaa gagatgaagg ttacgattgg ttgattatga cacccggtgt    1020
   gggtttagat gacaagggag acgcattggg tcaacagtat agaaccgtgg atgatgtggt    1080
   ctctacagga tctgacatta ttattgttgg aagaggacta tttgcaaagg gaagggatgc 1140
   taaggtagag ggtgaacgtt acagaaaagc aggctgggaa gcatatttga gaagatgcgg 1200
   ccagcaaaac taaaaaactg tattataagt aaatgcatgt atactaaact cacaaattag 1260
   agcttcaatt taattatatc agttattacc c 1291
<210> 104
   <211> 2309
   <212> DNA
   <213> Artificial Sequence
<220>
   <223> codon optimized sequence comprising ADE2
<400> 104
   aagcttgcat gcctgcaggt cgatcgactc tagaaatcga tagatctgaa ttaattcttg 60
   aataatacat aacttttctt aaaagaatca aagacagata aaatttaaga gatattaaat 120
   attagtgaga agccgagaat tttgtaacac caacataaca ctgacatctt taacaacttt 180
   taattatgat acatttctta cgtcatgatt gattattaca gctatgctga caaatgactc 240
   ttgttgcatg gctacgaacc gggtaatact aagtgattga ctcttgctga ccttttatta 300
   agaactaaat ggacaatatt atggagcatt tcatgtataa attggtgcgt aaaatcgttg 360
   gatctctctt ctaagtacat cctactataa caatcaagaa aaacaagaaa atcggacaaa 420
   acaatcaagt atggattcta gaacagttgg tatattagga gggggacaat tgggacgtat 480
   gattgttgag gcagcaaaca ggctcaacat taagacggta atactagatg ctgaaaattc 540
   tcctgccaaa caaataagca actccaatga ccacgttaat ggctcctttt ccaatcctct 600
   tgatatcgaa aaactagctg aaaaatgtga tgtgctaacg attgagattg agcatgttga 660
   tgttcctaca ctaaagaatc ttcaagtaaa acatcccaaa ttaaaaattt acccttctcc 720
   agaaacaatc agattgatac aagacaaata tattcaaaaa gagcatttaa tcaaaaatgg 780
   tatagcagtt acccaaagtg ttcctgtgga acaagccagt gagacgtccc tattgaatgt 840
   tggaagagat ttgggttttc cattcgtctt gaagtcgagg actttggcat acgatggaag 900
   aggtaacttc gttgtaaaga ataaggaaat gattccggaa gctttggaag tactgaagga 960
   tcgtcctttg tacgccgaaa aatgggcacc atttactaaa gaattagcag tcatgattgt 1020
   gaggtctgtt aacggtttag tgttttctta cccaattgta gagactatcc acaaggacaa 1080
   tatttgtgac ttatgttatg cgcctgctag agttccggac tccgttcaac ttaaggcgaa 1140
   gttgttggca gaaaatgcaa tcaaatcttt tcccggttgt ggtatatttg gtgtggaaat 1200
   gttctattta gaaacagggg aattgcttat taacgaaatt gccccaaggc ctcacaactc 1260
   tggacattat accattgatg cttgcgtcac ttctcaattt gaagctcatt tgagatcaat 1320
   attggatttg ccaatgccaa agaatttcac atctttctcc accattacaa cgaacgccat 1380
   tatgctaaat gttcttggag acaaacatac aaaagataaa gagctagaaa cttgcgaaag    1440
   agcattggcg actccaggtt cctcagtgta cttatatgga aaagagtcta gacctaacag    1500
   aaaagtaggt cacataaata ttattgcctc cagtatggcg gaatgtgaac aaaggctgaa    1560
   ctacattaca ggtagaactg atattccaat caaaatctct gtcgctcaaa agttggactt    1620
   ggaagcaatg gtcaaaccat tggttggaat catcatggga tcagactctg acttgccggt    1680
   aatgtctgcc gcatgtgcgg ttttaaaaga ttttggcgtt ccatttgaag tgacaatagt    1740
   ctctgctcat agaactccac ataggatgtc agcatatgct atttccgcaa gcaagcgtgg    1800
   aattaaaaca attatcgctg gagctggtgg ggctgctcac ttgccaggta tggtggctgc    1860
   aatgacacca cttcctgtca tcggtgtgcc cgtaaaaggt tcttgtctag atggagtaga    1920
   ttctttacat tcaattgtgc aaatgcctag aggtgttcca gtagctaccg tcgctattaa    1980
   taatagtacg aacgctgcgc tgttggctgt cagactgctt ggcgcttatg attcaagtta    2040
   tacaacgaaa atggaacagt ttttattaaa gcaagaagaa gaagttcttg tcaaagcaca    2100
   aaagttagaa actgtcggtt acgaagctta tctagaaaac aagtaatata taagtttatt    2160
   gatatacttg tacagcaaat aattataaaa tgatatacct attttttagg ctttgttatg    2220
   attacatcaa atgtggactt catacataga aatcaacgct tacaggtgtc cttttttaag    2280
   aatttcatac ataagatctc tcgaggatc                                      2309
<210> 105
   <211> 3688
   <212> DNA
   <213> Artificial Sequence
<220>
   <223> codon optimized sequence comprising URA3-ADE2
<220>
   <221> misc_feature
   <222> (1292)..(1379)
   <223> nucleic acid linker
<400> 105
   gggtaataac tgatataatt aaattgaagc tctaatttgt gagtttagta tacatgcatt      60
   tacttataat acagtttttt agttttgctg gccgcatctt ctcaaatatg cttcccagcc     120
   tgcttttctg taacgttcac cctctacctt agcatccctt ccctttgcaa atagtcctct     180
   tccaacaata ataatgtcag atcctgtaga gaccacatca tccacggttc tatactgttg     240
   acccaatgcg tctcccttgt catctaaacc cacaccgggt gtcataatca accaatcgta     300
   accttcatct cttccaccca tgtctctttg agcaataaag ccgataacaa aatctttgtc     360
   gctcttcgca atgtcaacag tacccttagt atattctcca gtagataggg agcccttgca     420
   tgacaattct gctaacatca aaaggcctct aggttccttt gttacttctt ctgccgcctg     480
   cttcaaaccg ctaacaatac ctgggcccac cacaccgtgt gcattcgtaa tgtctgccca     540
   ttctgctatt ctgtatacac ccgcagagta ctgcaatttg actgtattac caatgtcagc 600
   aaattttctg tcttcgaaga gtaaaaaatt gtacttggcg gataatgcct ttagcggctt 660
   aactgtgccc tccatggaaa aatcagtcaa gatatccaca tgtgttttta gtaaacaaat 720
   tttgggacct aatgcttcaa ctaactccag taattccttg gtggtacgaa catccaatga 780
   agcacacaag tttgtttgct tttcgtgcat gatattaaat agcttggcag caacaggact 840
   aggatgagta gcagcacgtt ccttatatgt agctttcgac atgatttatc ttcgtttcct 900
   gcaggttttt gttctgtgca gttgggttaa gaatactggg caatttcatg tttcttcaac 960
   actacatatg cgtatatata ccaatctaag tctgtgctcc ttccttcgtt cttccttctg 1020
   ttcggagatt accgaatcaa aaaaatttca aggaaaccga aatcaaaaaa aagaataaaa 1080
   aaaaaatgat gaattgaaaa ggtggtatgg tgcactctca gtacaatctg ctctgatgcc 1140
   gcatagttaa gccagccccg acacccgcca acacccgctg acgcgccctg acgggcttgt 1200
   ctgctcccgg catccgctta cagacaagct gtgaccgtct ccgggagctg catgtgtcag 1260
   aggttttcac cgtcatcacc gaaacgcgcg acgtaactat aacggtccta aggtagcgaa 1320
   ggccggcctt aattaaattt aaatcgccga gttacgctag ggataacagg gtaatataga 1380
   agcttgcatg cctgcaggtc gatcgactct agaaatcgat agatctgaat taattcttga 1440
   ataatacata acttttctta aaagaatcaa agacagataa aatttaagag atattaaata 1500
   ttagtgagaa gccgagaatt ttgtaacacc aacataacac tgacatcttt aacaactttt 1560
   aattatgata catttcttac gtcatgattg attattacag ctatgctgac aaatgactct 1620
   tgttgcatgg ctacgaaccg ggtaatacta agtgattgac tcttgctgac cttttattaa 1680
   gaactaaatg gacaatatta tggagcattt catgtataaa ttggtgcgta aaatcgttgg 1740
   atctctcttc taagtacatc ctactataac aatcaagaaa aacaagaaaa tcggacaaaa 1800
   caatcaagta tggattctag aacagttggt atattaggag ggggacaatt gggacgtatg 1860
   attgttgagg cagcaaacag gctcaacatt aagacggtaa tactagatgc tgaaaattct 1920
   cctgccaaac aaataagcaa ctccaatgac cacgttaatg gctccttttc caatcctctt 1980
   gatatcgaaa aactagctga aaaatgtgat gtgctaacga ttgagattga gcatgttgat 2040
   gttcctacac taaagaatct tcaagtaaaa catcccaaat taaaaattta cccttctcca 2100
   gaaacaatca gattgataca agacaaatat attcaaaaag agcatttaat caaaaatggt 2160
   atagcagtta cccaaagtgt tcctgtggaa caagccagtg agacgtccct attgaatgtt 2220
   ggaagagatt tgggttttcc attcgtcttg aagtcgagga ctttggcata cgatggaaga 2280
   ggtaacttcg ttgtaaagaa taaggaaatg attccggaag ctttggaagt actgaaggat 2340
   cgtcctttgt acgccgaaaa atgggcacca tttactaaag aattagcagt catgattgtg 2400
   aggtctgtta acggtttagt gttttcttac ccaattgtag agactatcca caaggacaat 2460
   atttgtgact tatgttatgc gcctgctaga gttccggact ccgttcaact taaggcgaag 2520
   ttgttggcag aaaatgcaat caaatctttt cccggttgtg gtatatttgg tgtggaaatg 2580
   ttctatttag aaacagggga attgcttatt aacgaaattg ccccaaggcc tcacaactct 2640
   ggacattata ccattgatgc ttgcgtcact tctcaatttg aagctcattt gagatcaata 2700
   ttggatttgc caatgccaaa gaatttcaca tctttctcca ccattacaac gaacgccatt 2760
   atgctaaatg ttcttggaga caaacataca aaagataaag agctagaaac ttgcgaaaga 2820
   gcattggcga ctccaggttc ctcagtgtac ttatatggaa aagagtctag acctaacaga 2880
   aaagtaggtc acataaatat tattgcctcc agtatggcgg aatgtgaaca aaggctgaac 2940
   tacattacag gtagaactga tattccaatc aaaatctctg tcgctcaaaa gttggacttg 3000
   gaagcaatgg tcaaaccatt ggttggaatc atcatgggat cagactctga cttgccggta 3060
   atgtctgccg catgtgcggt tttaaaagat tttggcgttc catttgaagt gacaatagtc 3120
   tctgctcata gaactccaca taggatgtca gcatatgcta tttccgcaag caagcgtgga 3180
   attaaaacaa ttatcgctgg agctggtggg gctgctcact tgccaggtat ggtggctgca 3240
   atgacaccac ttcctgtcat cggtgtgccc gtaaaaggtt cttgtctaga tggagtagat 3300
   tctttacatt caattgtgca aatgcctaga ggtgttccag tagctaccgt cgctattaat 3360
   aatagtacga acgctgcgct gttggctgtc agactgcttg gcgcttatga ttcaagttat 3420
   acaacgaaaa tggaacagtt tttattaaag caagaagaag aagttcttgt caaagcacaa 3480
   aagttagaaa ctgtcggtta cgaagcttat ctagaaaaca agtaatatat aagtttattg 3540
   atatacttgt acagcaaata attataaaat gatataccta ttttttaggc tttgttatga 3600
   ttacatcaaa tgtggacttc atacatagaa atcaacgctt acaggtgtcc ttttttaaga 3660
   atttcataca taagatctct cgaggatc 3688
<210> 106
   <211> 88
   <212> DNA
   <213> Artificial Sequence
<220>
   <223> nucleic acid linker
<400> 106
   cgtaactata acggtcctaa ggtagcgaag gccggcctta attaaattta aatcgccgag 60
   ttacgctagg gataacaggg taatatag 88
<210> 107
   <211> 3011
   <212> DNA
   <213> Artificial Sequence
<220>
   <223> codon optimized comprising TRP1-ARS1-CEN4
<210> 108
   <211> 2103
   <212> DNA
   <213> Artificial Sequence
<220>
   <223> codon optimized sequence comprising LEU2
<400> 108
   cctgatgcgg tattttctcc ttacgcatct gtgcggtatt tcacaccgca tatcgaccgg      60
   tcgaggagaa cttctagtat atctacatac ctaatattat tgccttatta aaaatggaat     120
   cccaacaatt acatcaaaat ccacattctc ttcaaaatca attgtcctgt acttccttgt     180
   tcatgtgtgt tcaaaaacgt tatatttata ggataattat actctatttc tcaacaagta     240
   attggttgtt tggccgagcg gtctaaggcg cctgattcaa gaaatatctt gaccgcagtt     300
   aactgtggga atactcaggt atcgtaagat gcaagagttc gaatctctta gcaaccatta     360
   tttttttcct caacataacg agaacacaca ggggcgctat cgcacagaat caaattcgat     420
   gactggaaat tttttgttaa tttcagaggt cgcctgacgc atataccttt ttcaactgaa     480
   aaattgggag aaaaaggaaa ggtgagagcg ccggaaccgg cttttcatat agaatagaga     540
   agcgttcatg actaaatgct tgcatcacaa tacttgaagt tgacaatatt atttaaggac 600
   ctattgtttt ttccaatagg tggttagcaa tcgtcttact ttctaacttt tcttaccttt 660
   tacatttcag caatatatat atatatattt caaggatata ccattctaat gtctgcccct 720
   aagaagatcg tcgttttgcc aggtgaccac gttggtcaag aaatcacagc cgaagccatt 780
   aaggttctta aagctatttc tgatgttcgt tccaatgtca agttcgattt cgaaaatcat 840
   ttaattggtg gtgctgctat cgatgctaca ggtgttccac ttccagatga ggcgctggaa 900
   gcctccaaga aggctgatgc cgttttgtta ggtgctgtgg gtggtcctaa atggggtacc 960
   ggtagtgtta gacctgaaca aggtttacta aaaatccgta aagaacttca attgtacgcc 1020
   aacttaagac catgtaactt tgcatccgac tctcttttag acttatctcc aatcaagcca 1080
   caatttgcta aaggtactga cttcgttgtt gtcagagaat tagtgggagg tatttacttt 1140
   ggtaagagaa aggaagacga tggtgatggt gtcgcttggg atagtgaaca atacaccgtt 1200
   ccagaagtgc aaagaatcac aagaatggcc gctttcatgg ccctacaaca tgagccacca 1260
   ttgcctattt ggtccttgga taaagctaat gttttggcct cttcaagatt atggagaaaa 1320
   actgtggagg aaaccatcaa gaacgaattc cctacattga aggttcaaca tcaattgatt 1380
   gattctgccg ccatgatcct agttaagaac ccaacccacc taaatggtat tataatcacc 1440
   agcaacatgt ttggtgatat catctccgat gaagcctccg ttatcccagg ttccttgggt 1500
   ttgttgccat ctgcgtcctt ggcctctttg ccagacaaga acaccgcatt tggtttgtac 1560
   gaaccatgcc acggttctgc tccagatttg ccaaagaata aggtcaaccc tatcgccact 1620
   atcttgtctg ctgcaatgat gttgaaattg tcattgaact tgcctgaaga aggtaaggcc 1680
   attgaagatg cagttaaaaa ggttttggat gcaggtatca gaactggtga tttaggtggt 1740
   tccaacagta ccaccgaagt cggtgatgct gtcgccgaag aagttaagaa aatccttgct 1800
   taaaaagatt ctcttttttt atgatatttg tacataaact ttataaatga aattcataat 1860
   agaaacgaca cgaaattaca aaatggaata tgttcatagg gtagacgaaa ctatatacgc 1920
   aatctacata catttatcaa gaaggagaaa aaggaggatg taaaggaata caggtaagca 1980
   aattgatact aatggctcaa cgtgataagg aaaaagaatt gcactttaac attaatattg 2040
   acaaggagga gggcaccaca caaaaagtta ggtgtaacag aaaatcatga aactatgatt 2100
   cct 2103
<210> 109
   <211> 1833
   <212> DNA
   <213> Artificial Sequence
<220>
   <223> codon optimized sequence comprising CC-93
<400> 109
   atggcaaaaa tgcgtgctgt tgatgcagca atgtatgtat tagagaaaga aggtattact      60
   acagctttcg gtgtaccagg tgcagcaatc aatccatttt attctgctat gcgtaaacat     120
   ggtggcattc gtcacatttt agctcgtcat gtagaaggtg ctagtcacat ggcagaaggt     180
   tatacacgtg ctacagcagg taatattggt gtttgtttag gtacatctgg cccagctggt     240
   acagatatga ttactgcatt atatagtgct tcagctgata gtattcctat cttatgtatt     300
   actggtcaag cacctcgtgc tcgtcttcat aaagaagatt ttcaagcagt tgatattgaa     360
   gctattgcta aacctgtaag taaaatggct gtaactgttc gtgaggctgc tttagtacca     420
   cgtgtattac aacaggcttt tcacttaatg cgttcaggtc gtcctggtcc tgtattagtt     480
   gatttacctt tcgatgttca agtagctgaa attgaattcg atccagacat gtatgaacct     540
   ttacctgtat ataaacctgc tgcttctcgt atgcaaattg aaaaagcagt agaaatgtta     600
   attcaagctg aacgtcctgt aatcgttgct ggtggtggtg ttattaatgc tgatgcagct     660
   gctcttttac agcaatttgc agaattaact tcagtacctg taattcctac tttaatgggc     720
   tggggttgta ttccagatga tcacgaatta atggcaggca tggtaggttt acaaacagct     780
   caccgttacg gtaatgctac attattagca tctgatatgg tattcggtat tggaaatcgt     840
   ttcgctaatc gtcatactgg ttcagtagag aaatatactg aaggacgtaa aattgttcac     900
   attgacatcg aacctacaca aattggtcgt gtattatgcc ctgatttagg cattgtaagt     960
   gatgcaaaag ctgctttaac tttacttgtt gaggttgctc aagaaatgca aaaagcaggt    1020
   cgtttacctt gtagaaaaga atgggtagct gactgccaac aacgtaaacg tactttatta    1080
   agaaaaactc attttgataa tgttcctgtt aaacctcaac gtgtatacga agaaatgaat    1140
   aaagcatttg gtcgtgatgt ttgctatgtt actacaattg gtttaagtca aatagctgct    1200
   gcacaaatgc ttcatgtatt taaagaccgt cattggatta actgtggcca agctggccct    1260
   ttaggttgga caattcctgc agctttaggt gtatgtgcag ctgatccaaa acgtaatgta    1320
   gttgctattt caggtgattt tgactttcaa tttcttattg aggaattagc tgttggtgca    1380
   caatttaaca ttccatatat ccatgtttta gtaaataacg cttatttagg tttaattcgt    1440
   caatcacaac gtgcttttga tatggattac tgtgttcaat tagcatttga aaatataaac    1500
   agttcagaag ttaacggtta tggtgtagat cacgtaaaag ttgctgaagg cttaggatgt    1560
   aaagctattc gtgtttttaa accagaagat atagctccag cttttgaaca ggctaaagca    1620
   ttaatggctc aatatcgtgt tcctgtagta gttgaagtta ttttagaaag agttacaaac    1680
   atttcaatgg gttcagaatt agataatgtt atggagtttg aagatattgc agataatgct    1740
   gctgacgcac ctactgaaac ttgctttatg cattatgaag attataaaga tgatgatgat    1800
   aaaggacaca accaccgtca caaacattaa tct                                 1833
<210> 110
   <211> 934
   <212> DNA
   <213> Artificial Sequence
<220>
   <223> codon optimized sequence comprising CC-94
<400> 110
   atgaaagtag gatttatagg attaggtatt atgggcaaac caatgtctaa aaacttactt      60
   aaagctggtt actcattagt tgttgctgat cgtaacccag aagcaatcgc tgacgtaatt     120
   gcagctggag ctgaaacagc ttcaactgct aaagctattg cagaacaatg tgatgttatt     180
   attactatgt tacctaattc acctcacgta aaagaagtag ctttaggcga aaatggaatt     240
   attgaaggtg ctaaaccagg cacagtatta atagatatgt catcaattgc tccacttgca     300
   tcacgtgaaa tttctgaagc attaaaagca aaaggtattg atatgttaga tgctccagtt     360
   agtggtggtg aaccaaaagc tatcgatggt acacttagtg ttatggtagg cggtgataaa     420
   gcaatttttg acaaatacta cgatttaatg aaagctatgg ctggttctgt agtacacact     480
   ggtgaaatcg gtgcaggtaa cgtaactaaa ttagctaacc aggttattgt tgcattaaat     540
   atagctgcaa tgtcagaagc tcttacttta gctacaaaag caggtgtaaa tcctgattta     600
   gtatatcagg caattcgtgg cggtttagca ggcagtactg tattagacgc aaaagctcca     660
   atggttatgg atcgtaattt caaaccaggt tttagaattg atttacatat taaagacctt     720
   gctaatgctt tagatacatc acacggtgta ggagctcaat taccattaac tgcagctgtt     780
   atggaaatga tgcaagcatt acgtgctgat ggtttaggta cagcagatca ctcagcttta     840
   gcttgttatt atgagaaatt agcaaaagtt gaagttacac gtgattataa agatgatgat     900
   gataaaggac acaaccaccg tcacaaacat taat                                 934
<210> 111
   <211> 8765
   <212> DNA
   <213> Artificial Sequence
<220>
   <223> codon optimized sequence comprising CC93-CC94
<400> 111
<210> 112
   <211> 8759
   <212> DNA
   <213> Artificial Sequence
<220>
   <223> codon optimized sequence comprising CC93-CC97
<400> 112
   tccaaactat ttctcatatt ttttcagctc caaaagatct tatttaaaat tgaaataata 60
   taaaaaatta taaattttta caaaacaaga ttttttctat ttataatgtt gtgatttttt 120
   gacttttttt actatttctc acaaattcta taaaaaatca aaaaattttt aaagaatttt 180
   ctattgtgtt tttgaacaat ttttttgttc ttgtttttta tcttctcttg ctttttcgct 240
   tttttttctt tgaacttttt tttctttttc attttacgct ttttttcttc ttcatttttt 300
   gcaaagcaaa aaatgataaa aagcagcgaa gcaaaaaatg ataaaaatga aaagaaaagc 360
   aacaaagcaa aaaagtaaaa agaagttcaa aaaaaatgta ttgagttgat tttttttaat 420
   tcaacaaaat ttttcaaata aaaagttttt ttcaaaaacg aagcacaaaa aaaattcaaa 480
   aaaaagtaaa gaatcaaaaa aaagtagaga aaaattgtat ttttcttttt tgaaattttc 540
   atttcatcaa gaaaaataca acttttctaa aaaaaaatga aaaaaatgga aatttctaga 600
   ttaatgtttg tgacggtggt tgtgtccttt atcatcatca tctttataat cttcataatg 660
   cataaagcaa gtttcagtag gtgcgtcagc agcattatct gcaatatctt caaactccat 720
   aacattatct aattctgaac ccattgaaat gtttgtaact ctttctaaaa taacttcaac 780
   tactacagga acacgatatt gagccattaa tgctttagcc tgttcaaaag ctggagctat 840
   atcttctggt ttaaaaacac gaatagcttt acatcctaag ccttcagcaa cttttacgtg 900
   atctacacca taaccgttaa cttctgaact gtttatattt tcaaatgcta attgaacaca 960
   gtaatccata tcaaaagcac gttgtgattg acgaattaaa cctaaataag cgttatttac 1020
   taaaacatgg atatatggaa tgttaaattg tgcaccaaca gctaattcct caataagaaa 1080
   ttgaaagtca aaatcacctg aaatagcaac tacattacgt tttggatcag ctgcacatac 1140
   acctaaagct gcaggaattg tccaacctaa agggccagct tggccacagt taatccaatg 1200
   acggtcttta aatacatgaa gcatttgtgc agcagctatt tgacttaaac caattgtagt 1260
   aacatagcaa acatcacgac caaatgcttt attcatttct tcgtatacac gttgaggttt 1320
   aacaggaaca ttatcaaaat gagtttttct taataaagta cgtttacgtt gttggcagtc 1380
   agctacccat tcttttctac aaggtaaacg acctgctttt tgcatttctt gagcaacctc 1440
   aacaagtaaa gttaaagcag cttttgcatc acttacaatg cctaaatcag ggcataatac 1500
   acgaccaatt tgtgtaggtt cgatgtcaat gtgaacaatt ttacgtcctt cagtatattt 1560
   ctctactgaa ccagtatgac gattagcgaa acgatttcca ataccgaata ccatatcaga 1620
   tgctaataat gtagcattac cgtaacggtg agctgtttgt aaacctacca tgcctgccat 1680
   taattcgtga tcatctggaa tacaacccca gcccattaaa gtaggaatta caggtactga 1740
   agttaattct gcaaattgct gtaaaagagc agctgcatca gcattaataa caccaccacc 1800
   agcaacgatt acaggacgtt cagcttgaat taacatttct actgcttttt caatttgcat 1860
   acgagaagca gcaggtttat atacaggtaa aggttcatac atgtctggat cgaattcaat 1920
   ttcagctact tgaacatcga aaggtaaatc aactaataca ggaccaggac gacctgaacg 1980
   cattaagtga aaagcctgtt gtaatacacg tggtactaaa gcagcctcac gaacagttac 2040
   agccatttta cttacaggtt tagcaatagc ttcaatatca actgcttgaa aatcttcttt 2100
   atgaagacga gcacgaggtg cttgaccagt aatacataag ataggaatac tatcagctga 2160
   agcactatat aatgcagtaa tcatatctgt accagctggg ccagatgtac ctaaacaaac 2220
   accaatatta cctgctgtag cacgtgtata accttctgcc atgtgactag caccttctac 2280
   atgacgagct aaaatgtgac gaatgccacc atgtttacgc atagcagaat aaaatggatt 2340
   gattgctgca cctggtacac cgaaagctgt agtaatacct tctttctcta atacatacat 2400
   tgctgcatca acagcacgca tttttgccat atgaataaat aatttataat tttttctgta 2460
   taaaccaatt ttccaagtaa ctttacttta tcaaaaatta aaaaattaaa aaacttttat 2520
   tgaacttaaa ataaaatttt taacaaaatt tattttaaaa aaaagaaaaa atttttttat 2580
   tttggtttta tttatttctt tttttttaca aacaaaaatt tttttaaaca gaataataaa 2640
   aaaaatttta tttaaagaat ggttttttaa tattttgctc atgacaaatg attttttact 2700
   acttttatgc ttttttttaa aaaaagcagc aaagcaaaaa agttataaaa agtgtatgga 2760
   gcaagcggtt aaattgacac tttttaaaag tatttatagg cccaaccgga cttgaaccga 2820
   tgacctattg cttgtaaggc aatcactcta ccaactgagt tatgggccta aaaaatatta 2880
   tttatatttt ataatagaat ataaaatcta acaacttctt tagctagcac taggagatca 2940
   cagatgagaa gatatttgct cgataatcaa tactctaggc atctaacttt tcccattgtc 3000
   ttaaaccgac ttaccttagg aatcatagtt tcatgatttt ctgttacacc taactttttg 3060
   tgtggtgccc tcctccttgt caatattaat gttaaagtgc aattcttttt ccttatcacg 3120
   ttgagccatt agtatcaatt tgcttacctg tattccttta catcctcctt tttctccttc 3180
   ttgataaatg tatgtagatt gcgtatatag tttcgtctac cctatgaaca tattccattt 3240
   tgtaatttcg tgtcgtttct attatgaatt tcatttataa agtttatgta caaatatcat 3300
   aaaaaaagag aatcttttta agcaaggatt ttcttaactt cttcggcgac agcatcaccg 3360
   acttcggtgg tactgttgga accacctaaa tcaccagttc tgatacctgc atccaaaacc 3420
   tttttaactg catcttcaat ggccttacct tcttcaggca agttcaatga caatttcaac 3480
   atcattgcag cagacaagat agtggcgata gggttgacct tattctttgg caaatctgga 3540
   gcagaaccgt ggcatggttc gtacaaacca aatgcggtgt tcttgtctgg caaagaggcc 3600
   aaggacgcag atggcaacaa acccaaggaa cctgggataa cggaggcttc atcggagatg 3660
   atatcaccaa acatgttgct ggtgattata ataccattta ggtgggttgg gttcttaact 3720
   aggatcatgg cggcagaatc aatcaattga tgttgaacct tcaatgtagg gaattcgttc 3780
   ttgatggttt cctccacagt ttttctccat aatcttgaag aggccaaaac attagcttta 3840
   tccaaggacc aaataggcaa tggtggctca tgttgtaggg ccatgaaagc ggccattctt 3900
   gtgattcttt gcacttctgg aacggtgtat tgttcactat cccaagcgac accatcacca 3960
   tcgtcttcct ttctcttacc aaagtaaata cctcccacta attctctgac aacaacgaag 4020
   tcagtacctt tagcaaattg tggcttgatt ggagataagt ctaaaagaga gtcggatgca 4080
   aagttacatg gtcttaagtt ggcgtacaat tgaagttctt tacggatttt tagtaaacct 4140
   tgttcaggtc taacactacc ggtaccccat ttaggaccac ccacagcacc taacaaaacg 4200
   gcatcagcct tcttggaggc ttccagcgcc tcatctggaa gtggaacacc tgtagcatcg 4260
   atagcagcac caccaattaa atgattttcg aaatcgaact tgacattgga acgaacatca 4320
   gaaatagctt taagaacctt aatggcttcg gctgtgattt cttgaccaac gtggtcacct 4380
   ggcaaaacga cgatcttctt aggggcagac attagaatgg tatatccttg aaatatatat 4440
   atatatattg ctgaaatgta aaaggtaaga aaagttagaa agtaagacga ttgctaacca 4500
   cctattggaa aaaacaatag gtccttaaat aatattgtca acttcaagta ttgtgatgca 4560
   agcatttagt catgaacgct tctctattct atatgaaaag ccggttccgg cgctctcacc 4620
   tttccttttt ctcccaattt ttcagttgaa aaaggtatat gcgtcaggcg acctctgaaa 4680
   ttaacaaaaa atttccagtc atcgaatttg attctgtgcg atagcgcccc tgtgtgttct 4740
   cgttatgttg aggaaaaaaa taatggttgc taagagattc gaactcttgc atcttacgat 4800
   acctgagtat tcccacagtt aactgcggtc aagatatttc ttgaatcagg cgccttagac 4860
   cgctcggcca aacaaccaat tacttgttga gaaatagagt ataattatcc tataaatata 4920
   acgtttttga acacacatga acaaggaagt acaggacaat tgattttgaa gagaatgtgg 4980
   attttgatgt aattgttggg attccatttt taataaggca ataatattag gtatgtagat 5040
   atactagaag ttctcctcga ccggtcgata tgcggtgtga aataccgcac agatgcgtaa 5100
   ggagaaaata ccgcatcagg aatttaaata ctagtggatc cttaattaac tgcagggccg 5160
   gcctctagtt ttatttgtaa accaaaaaaa atgaaaagcc aaaaatttaa gaaataaaaa 5220
   gtcaaagtta tttaacaaaa atgaatttcc aaaacttgca cgagataaaa aagataactc 5280
   tttaaatgaa aaaagaatgc ttttttcaaa aaaagtttta aacaatacgt aaaaacttta 5340
   ttttttttaa actttttttt gaaaaaaggc attctttttt ttttaagaaa ttttaagtaa 5400
   tactttcata tttttttagt atttttttat tgaataaaaa aaaactttaa agtaaaaaat 5460
   tggtcacttt gaaagtccca gctttttttt aattcacttt tttctttatt tattttcctg 5520
   tttaaaagaa aataaaattt ttaaaaattt taaaaaataa aagaacaaac tttcttgaga 5580
   taacactaaa tcatctcaag aaagtttaat atttttgaaa agagttcgtt caaaaatttt 5640
   tcagttttaa atacaaattc tcaaatatct aggttacgcc ccgccctgcc actcatcgca 5700
   gtactgttgt aattcattaa gcattctgcc gacatggaag ccatcacaaa cggcatgatg 5760
   aacctgaatc gccagcggca tcagcacctt gtcgccttgc gtataatatt tgcccatagt 5820
   gaaaacgggg gcgaagaagt tgtccatatt ggccacgttt aaatcaaaac tggtgaaact 5880
   cacccaggga ttggcgctga cgaaaaacat attctcaata aaccctttag ggaaataggc 5940
   caggttttca ccgtaacacg ccacatcttg cgaatatatg tgtagaaact gccggaatcg 6000
   tcgtggtatt cactccagag cgatgaaaac gtttcagttt gctcatggaa aacggtgtaa 6060
   caagggtgaa cactatccca tatcaccagc tcaccgtctt tcattgccat acggaactcc 6120
   ggatgagcat tcatcaggcg ggcaagaatg tgaataaagg ccggataaaa cttgtgctta 6180
   tttttcttta cggtctttaa aaaggccgta atatccagct gaacggtctg gttataggta 6240
   cattgagcaa ctgactgaaa tgcctcaaaa tgttctttac gatgccattg ggatatatca 6300
   acggtggtat atccagtgat ttttttctcc attatgaaaa gttcatagaa cattttgtcg 6360
   atcctataaa aatctatccg tggaaaactt ttttctttga aaaaaaaaaa gaaattgaaa 6420
   aaaataaaaa actaaactat attatagaaa ataataaaaa ctattttttt aacataaaaa 6480
   aatagtttaa aaatttttat tttttttaaa ttttaacata aattcaaaac tttagcaaat 6540
   ttttaagttt aaatattttt ttcaaagtta ccacttgctc ttggggaaca agtaaactaa 6600
   attttttttc tacgcaaaac ttcattttta ttggtttata atttttctaa attttcatct 6660
   atttgttttt tctatttttt tataaactca aaaaaagttt caaaatatga aaaaaatact 6720
   agaaaaattt ttttttgatt tttttattat ttggaaaagt ttccaaaaaa aaaattgggt 6780
   tttaattttt tttatgaatt ttagaattct ttgttttttc attttagttt tttttatttt 6840
   gtattttttt ttctttgaac aaaagcaaaa aaagcaaaag taaaaaaact agaggccgtt 6900
   ctgcctgtag atccattgga ttggtatcaa cgcgcaggca tagttcgaga aaaattatcc 6960
   agaggcaatg acaaccagca cgggctcgag actagtggcc ggcctctagt gctagcacaa 7020
   aagaaatcaa atgttttcaa atcgtgcaaa catcaaattg cacaaaataa tttttaaaat 7080
   tcattttatg aaagttcgtt cttcagttaa aaaaatttgt actaaatgtc gtttaattcg 7140
   tcgaaaaggt acagtaatgg ttatttgtac aaatcctaaa cataaacaac gtcaaggata 7200
   atttttttta atggaagaaa tgttttttac ttcttccatt aaaaaagaaa aaagaaaagt 7260
   gcagagcttt ttttttgatc aaaaaaaaga tacaaaaaat ttttttgttt taattttatg 7320
   atataattct atttcagaga agaaaaaaaa atttaaacaa aacaaaaaaa tatagagaag 7380
   tttaaattta gacctaaagg tagatttcca taaattcatt tttctcattt gtttttttct 7440
   tttttttgca ttgcaaaaaa aaaagaaaaa agcaaaaagt cattttaaag agaaaaatgg 7500
   agaaagataa aagtttttaa ctttttttga acaattccat atgaaattag gtttcatcgg 7560
   tttaggtatt atgggtactc caatggcaat taatcttgct agagctggtc atcaattaca 7620
   tgttactact attggtcctg ttgcagacga acttttatct ttaggtgctg tatcagttga 7680
   aacagcacgt caagttacag aagcatcaga cataatattt attatggttc cagatactcc 7740
   acaagtagaa gaagttttat ttggtgaaaa tggttgcact aaagcatctt taaaaggtaa 7800
   aactattgtt gatatgtctt ctatctcacc tattgaaact aaacgttttg caagacaagt 7860
   aaatgaatta ggtggtgact atcttgacgc tccagtttca ggtggtgaaa ttggtgctcg 7920
   tgaaggtaca ttatctatca tggtaggtgg agatgaagct gttttcgaac gtgtaaaacc 7980
   tttatttgaa ttacttggca aaaatatcac attagttgga ggtaatggtg atggacaaac 8040
   atgtaaagtt gcaaatcaaa ttattgtagc acttaatatt gaagcagttt ctgaagctct 8100
   tttatttgct tcaaaagctg gtgcagatcc tgttcgtgtt cgtcaagctc ttatgggcgg 8160
   ttttgcttca tcaagaattt tagaagtaca tggtgagcgt atgattaaac gtacattcaa 8220
   tcctggtttc aaaattgctt tacatcaaaa agatttaaac ttagctttac agtcagctaa 8280
   agcattagct ttaaacttac ctaatactgc tacttgccaa gaacttttca atacatgcgc 8340
   tgctaatggt ggcagtcagt tagatcattc agctttagtt caagcattag aacttatggc 8400
   taaccataaa ttagcagatt ataaagatga cgacgataaa ggtcacaatc accgtcataa 8460
   acactaatct agaaaaaaaa gcatctttca aaattaaact ttaagttttt ttcttttttt 8520
   tattttttcc ttttcttttt attttattta acaaaaagaa aaggaaaaaa taaaaaaaaa 8580
   ttttaagcga cttatgtttt taagtttcat tttttttatt tttatttatt ttttatttct 8640
   ttttttacaa aacttaaaaa aagtttaaaa ataaaaaatt tttgacaaaa gaaatcaaat 8700
   gttttcaaat cgtgcaaaca tcaaattgca caaaataatt tttaaaattc attttccta 8759
<210> 113
   <211> 927
   <212> DNA
   <213> Artificial Sequence
<220>
   <223> codon optimized sequence comprising CC-97
<400> 113
   atgaaattag gtttcatcgg tttaggtatt atgggtactc caatggcaat taatcttgct      60
   agagctggtc atcaattaca tgttactact attggtcctg ttgcagacga acttttatct     120
   ttaggtgctg tatcagttga aacagcacgt caagttacag aagcatcaga cataatattt     180
   attatggttc cagatactcc acaagtagaa gaagttttat ttggtgaaaa tggttgcact     240
   aaagcatctt taaaaggtaa aactattgtt gatatgtctt ctatctcacc tattgaaact     300
   aaacgttttg caagacaagt aaatgaatta ggtggtgact atcttgacgc tccagtttca     360
   ggtggtgaaa ttggtgctcg tgaaggtaca ttatctatca tggtaggtgg agatgaagct     420
   gttttcgaac gtgtaaaacc tttatttgaa ttacttggca aaaatatcac attagttgga     480
   ggtaatggtg atggacaaac atgtaaagtt gcaaatcaaa ttattgtagc acttaatatt     540
   gaagcagttt ctgaagctct tttatttgct tcaaaagctg gtgcagatcc tgttcgtgtt     600
   cgtcaagctc ttatgggcgg ttttgcttca tcaagaattt tagaagtaca tggtgagcgt     660
   atgattaaac gtacattcaa tcctggtttc aaaattgctt tacatcaaaa agatttaaac     720
   ttagctttac agtcagctaa agcattagct ttaaacttac ctaatactgc tacttgccaa     780
   gaacttttca atacatgcgc tgctaatggt ggcagtcagt tagatcattc agctttagtt     840
   caagcattag aacttatggc taaccataaa ttagcagatt ataaagatga cgacgataaa     900
   ggtcacaatc accgtcataa acactaa                                         927
<210> 114
   <211> 16417
   <212> DNA
   <213> Artificial Sequence
<220>
   <223> codon optimized sequence comprising CC90-CC91-CC92
<400> 114
   cgtttaggtg taacacaatc ttggggtgga tggacaatta gcggtgaaac agcaacaaat      60
   ccaggtattt ggagttatga aggtgttgct gcatctcata ttattttatc tggtttatta     120
   ttcttagctt cggtttggca ctgggtttac tgggatttag agttattccg tgacccaaga     180
   actggaaaaa ctgcattaga tttaccaaaa attttcggaa ttcacttatt cttatcaggt     240
   cttttatgtt ttggttttgg tgctttccac gtaacaggtt tatttggtcc tggtatttgg     300
   gtttcagatc cttatggatt aacaggaagt gttcaaccag ttgctccttc ttggggtgct     360
   gatgggtttg atcctttcaa ccctggtggt attgcagcgc accacattgc tgctggtatt     420
   ttaggtgttt tagcaggatt attccactta tgtgtacgtc cttctattcg tttatacttt     480
   ggtttatcaa tgggtagtat cgaaacagta ttatcaagta gtattgctgc tgttttctgg     540
   gctgctttcg ttgttgctgg aactatgtgg tatggttcag cagctactcc aattgaatta 600
   tttggtccta cacgttatca atgggaccaa ggtttcttcc aacaagaaat tcaaaaacga 660
   gttcaaacaa gtttagcagg tggttcttca ctttctgatg cttgggcgaa aattccagaa 720
   aaattagctt tctatgatta tattggaaac aaccctgcaa aaggtggtct tttccgtaca 780
   ggagctatga atagtggaga tggtattgct gttggatggt taggtcacgc agtatttaaa 840
   gatcaagatg gtcgtgaatt atacgtacgt cgtatgccta ctttctttga aacattccca 900
   gttttattaa ttgataaaga tggtgttgta cgtgctgacg ttcctttccg tcgtgctgaa 960
   tcaaaatata gtattgaaca agttggtgta tcagtaactt tctacggtgg tgaattagat 1020
   ggattaacat ttaatgatcc agcaactgtt aaaaaatatg ctcgtaaagc acaattaggt 1080
   gaaatttttg aatttgatcg ttcaacatta caatctgatg gtgtattccg tagtagtcca 1140
   cgtggttggt ttacttttgg tcacgtttgc tttgctttat tattcttctt tggacatatt 1200
   tggcatggtg cacgtacaat cttccgtgat gtatttgctg gtattgatga tgatctaaac 1260
   gaaagtttag aatttggtaa atacaaaaaa cttggtgata caagttctgt tcgtgaagct 1320
   ttctaattcg tttttttctc ttttttttct tttttctctt tggaaaaaga aaaaacatgt 1380
   ttattttgaa ttttttgttt agaactttac tgttcttttt ttattttaaa gtgtttctgt 1440
   ttttttttaa tacaaaaact tttttaaaat gaatttaaaa aacacaaaaa aagagttatt 1500
   gctattcaaa ataaacaaga gtttaaaaac aaagtttttt tctttagaaa aaaacttctt 1560
   catttttttt gaattgtttt tgaacttttt tcttctcttg cttttatcgt ttttttcttc 1620
   actttttgca aaaaagtgag aaaaaacagc aaagcaaaaa agtgaaaaaa agttcaaaaa 1680
   caattcaaaa aagacaaaac ctaaaaaaat atcacttgag atgggtctgg attttttcca 1740
   agcaaaagaa ttttgtattt tgttgaaagt ttttcataaa aatacaaatt tgcaattatt 1800
   attcttaaaa tcaaaatatt tgttaaccac atttcattct atggaagcat tagtttatac 1860
   ttttttatta atcggaacat taggaattat ctttttcgca attttcttta gagaaccacc 1920
   tcgtatggta aaataacgta agatctccta ggaaaatgaa ttttaaaaat tattttgtgc 1980
   aatttgatgt ttgcacgatt tgaaaacatt tgatttcttt tgtcaaaaat tttttatttt 2040
   taaacttttt ttaagttttg taaaaaaaga aataaaaaat aaataaaaat aaaaaaaatg 2100
   aaacttaaaa acataagtcg cttaaaattt ttttttattt tttccttttc tttttgttaa 2160
   ataaaataaa aagaaaagga aaaaataaaa aaaagaaaaa aacttaaagt ttaattttga 2220
   aagatgcttt tttttctaga ttaatgtttg tgacggtggt tgtgtccttt atcatcatca 2280
   tctttataat caaaacgttc aagctctgga aaaggtaaat ggccgtgatg tacatgcata 2340
   gcaccaaact ctgcacaacg atgtaaagtt ggaatatttt taccaggatt aagtaaaccg 2400
   tctggatcaa aagcagcttt tacagcgtga aatgtagtaa tttcatcact attgaattga 2460
   gcacacattt gattgatttt ttcacggccg attccatgct caccactaat agaaccacct 2520
   acttctacac ataactctaa gatttttcca ccaagttctt cagcacgagc aaactcacca 2580
   ggttcgttag catcgaataa gattaatgga tgcatattac catcgccagc atgaaataca 2640
   ttagctacac gtaaatcgta ttgttgagaa agacgtgcaa taccttctaa aacacctggt 2700
   aatgcacgac gtggaattgt accatccata caataatagt ctggtgaaat acgacctact 2760
   gcaggaaaag catttttacg acctgcccaa aatcttacac gttcagcttc atcttgtgct 2820
   aaacgaacat cagtagcacc agctttaagt aatatgtcgt ttacacgctc acagtcttcc 2880
   tgaacatcac tctctactcc atctaattca caaagtaaaa ttgcttcagc atctactgga 2940
   taaccagcgt gtataaaatc ttcagctgca cgaattgata agttatccat catctctaat 3000
   cctcctggaa ttataccatt tgcaatgata tcgcctacag ctaatcctgc tttttcaact 3060
   gaatcaaaag atgctaataa aacacgagca acaggtggtt ttggaagtaa tttaacagtt 3120
   acttctgttg taacacctaa cattccttct gatcctgtga ataaagctaa taaatcaaaa 3180
   ccaggagaat ctaatgcatc actacctaaa gtaagagcct caccgtctaa agtttgtact 3240
   tcaattttta ataagttgtg tactgttaaa ccatatttta aacagtgtac gccaccagca 3300
   ttctctgcta cattaccacc aattgaacaa gcaatttgac tactaggatc tggagcgtaa 3360
   tataagttat gaggagcaac tgcttgtgaa attgctaaat tacgaacacc aggttgaaca 3420
   cgtgcacgac gaccaactgg gttaatatct aaaatttctt taaaacgtgc cataactaat 3480
   aatacacctt tttctaaagg aagtgcaccg ccacttaagc ctgtacctgc accacgtgta 3540
   acaactggaa cacgtaatct atgacatact gctaatatag cagtaacttg ttccatttgt 3600
   tttggtaaaa ctactaataa aggtcttgtt ctgtaagctg ataatccatc acattcgtat 3660
   ggaataattt cttcatctgt atgtaaaatc tctaagccag gtacgtgttc acgaagtgcc 3720
   attaaaactg aagtacgatc aacatctggt aaagcaccat ctaaacgttc ttcatataat 3780
   atactcatat ggaattgttc aaaaaaagtt aaaaactttt atctttctcc atttttctct 3840
   ttaaaatgac tttttgcttt tttctttttt ttttgcaatg caaaaaaaag aaaaaaacaa 3900
   atgagaaaaa tgaatttatg gaaatctacc tttaggtcta aatttaaact tctctatatt 3960
   tttttgtttt gtttaaattt ttttttcttc tctgaaatag aattatatca taaaattaaa 4020
   acaaaaaaat tttttgtatc ttttttttga tcaaaaaaaa agctctgcac ttttcttttt 4080
   tcttttttaa tggaagaagt aaaaaacatt tcttccatta aaaaaaatta tccttgacgt 4140
   tgtttatgtt taggatttgt acaaataacc attactgtac cttttcgacg aattaaacga 4200
   catttagtac aaattttttt aactgaagaa cgaactttca taaaatgaat tttaaaaatt 4260
   attttgtgca atttgatgtt tgcacgattt gaaaacattt gatttctttt gtgctagcac 4320
   tagaggccgg ccactagtct cgagcccgtg ctggttgtca ttgcctctgg ataatttttc 4380
   tcgaactatg cctgcgcgtt gataccaatc caatggatct acaggcagaa cggcctctag 4440
   cggttttttt tacttttgct ttttttgctt ttgttcaaag aaaaaaaaat acaaaataaa 4500
   aaaaactaaa atgaaaaaac aaagaattct aaaattcata aaaaaaatta aaacccaatt 4560
   ttttttttgg aaacttttcc aaataataaa aaaatcaaaa aaaaattttt ctagtatttt 4620
   tttcatattt tgaaactttt tttgagttta taaaaaaata gaaaaaacaa atagatgaaa 4680
   atttagaaaa attataaacc aataaaaatg aagttttgcg tagaaaaaaa atttagttta 4740
   cttgttcccc aagagcaagt ggtaactttg aaaaaaatat ttaaacttaa aaatttgcta 4800
   aagttttgaa tttatgttaa aatttaaaaa aaataaaaat ttttaaacta tttttttatg 4860
   ttaaaaaaat agtttttatt attttctata atatagttta gttttttatt tttttcaatt 4920
   tctttttttt tttcaaagaa aaaagttttc cacggataga tttttatagg atcgacaaaa 4980
   tgttctatga acttttcata atggagaaaa aaatcactgg atataccacc gttgatatat 5040
   cccaatggca tcgtaaagaa cattttgagg catttcagtc agttgctcaa tgtacctata 5100
   accagaccgt tcagctggat attacggcct ttttaaagac cgtaaagaaa aataagcaca 5160
   agttttatcc ggcctttatt cacattcttg cccgcctgat gaatgctcat ccggagttcc 5220
   gtatggcaat gaaagacggt gagctggtga tatgggatag tgttcaccct tgttacaccg 5280
   ttttccatga gcaaactgaa acgttttcat cgctctggag tgaataccac gacgattccg 5340
   gcagtttcta cacatatatt cgcaagatgt ggcgtgttac ggtgaaaacc tggcctattt 5400
   ccctaaaggg tttattgaga atatgttttt cgtcagcgcc aatccctggg tgagtttcac 5460
   cagttttgat ttaaacgtgg ccaatatgga caacttcttc gcccccgttt tcactatggg 5520
   caaatattat acgcaaggcg acaaggtgct gatgccgctg gcgattcagg ttcatcatgc 5580
   cgtttgtgat ggcttccatg tcggcagaat gcttaatgaa ttacaacagt actgcgatga 5640
   gtggcagggc ggggcgtaac ctagatattt gagaatttgt atttaaaact gaaaaatttt 5700
   tgaacgaact cttttcaaaa atattaaact ttcttgagat gatttagtgt tatctcaaga 5760
   aagtttgttc ttttattttt taaaattttt aaaaatttta ttttctttta aacaggaaaa 5820
   taaataaaga aaaaagtgaa ttaaaaaaaa gctgggactt tcaaagtgac caatttttta 5880
   ctttaaagtt tttttttatt caataaaaaa atactaaaaa aatatgaaag tattacttaa 5940
   aatttcttaa aaaaaaaaga atgccttttt tcaaaaaaaa gtttaaaaaa aataaagttt 6000
   ttacgtattg tttaaaactt tttttgaaaa aagcattctt ttttcattta aagagttatc 6060
   ttttttatct cgtgcaagtt ttggaaattc atttttgtta aataactttg actttttatt 6120
   tcttaaattt ttggcttttc attttttttg gtttacaaat aaaactagag gccggccctg 6180
   cagttaatta aggatccact agtatttaaa ttcctgatgc ggtattttct ccttacgcat 6240
   ctgtgcggta tttcacaccg catatcgacc ggtcgaggag aacttctagt atatctacat 6300
   acctaatatt attgccttat taaaaatgga atcccaacaa ttacatcaaa atccacattc 6360
   tcttcaaaat caattgtcct gtacttcctt gttcatgtgt gttcaaaaac gttatattta 6420
   taggataatt atactctatt tctcaacaag taattggttg tttggccgag cggtctaagg 6480
   cgcctgattc aagaaatatc ttgaccgcag ttaactgtgg gaatactcag gtatcgtaag 6540
   atgcaagagt tcgaatctct tagcaaccat tatttttttc ctcaacataa cgagaacaca 6600
   caggggcgct atcgcacaga atcaaattcg atgactggaa attttttgtt aatttcagag 6660
   gtcgcctgac gcatatacct ttttcaactg aaaaattggg agaaaaagga aaggtgagag 6720
   cgccggaacc ggcttttcat atagaataga gaagcgttca tgactaaatg cttgcatcac 6780
   aatacttgaa gttgacaata ttatttaagg acctattgtt ttttccaata ggtggttagc 6840
   aatcgtctta ctttctaact tttcttacct tttacatttc agcaatatat atatatatat 6900
   ttcaaggata taccattcta atgtctgccc ctaagaagat cgtcgttttg ccaggtgacc 6960
   acgttggtca agaaatcaca gccgaagcca ttaaggttct taaagctatt tctgatgttc 7020
   gttccaatgt caagttcgat ttcgaaaatc atttaattgg tggtgctgct atcgatgcta 7080
   caggtgttcc acttccagat gaggcgctgg aagcctccaa gaaggctgat gccgttttgt 7140
   taggtgctgt gggtggtcct aaatggggta ccggtagtgt tagacctgaa caaggtttac 7200
   taaaaatccg taaagaactt caattgtacg ccaacttaag accatgtaac tttgcatccg 7260
   actctctttt agacttatct ccaatcaagc cacaatttgc taaaggtact gacttcgttg 7320
   ttgtcagaga attagtggga ggtatttact ttggtaagag aaaggaagac gatggtgatg 7380
   gtgtcgcttg ggatagtgaa caatacaccg ttccagaagt gcaaagaatc acaagaatgg 7440
   ccgctttcat ggccctacaa catgagccac cattgcctat ttggtccttg gataaagcta 7500
   atgttttggc ctcttcaaga ttatggagaa aaactgtgga ggaaaccatc aagaacgaat 7560
   tccctacatt gaaggttcaa catcaattga ttgattctgc cgccatgatc ctagttaaga 7620
   acccaaccca cctaaatggt attataatca ccagcaacat gtttggtgat atcatctccg 7680
   atgaagcctc cgttatccca ggttccttgg gtttgttgcc atctgcgtcc ttggcctctt 7740
   tgccagacaa gaacaccgca tttggtttgt acgaaccatg ccacggttct gctccagatt 7800
   tgccaaagaa taaggtcaac cctatcgcca ctatcttgtc tgctgcaatg atgttgaaat 7860
   tgtcattgaa cttgcctgaa gaaggtaagg ccattgaaga tgcagttaaa aaggttttgg 7920
   atgcaggtat cagaactggt gatttaggtg gttccaacag taccaccgaa gtcggtgatg 7980
   ctgtcgccga agaagttaag aaaatccttg cttaaaaaga ttctcttttt ttatgatatt 8040
   tgtacataaa ctttataaat gaaattcata atagaaacga cacgaaatta caaaatggaa 8100
   tatgttcata gggtagacga aactatatac gcaatctaca tacatttatc aagaaggaga 8160
   aaaaggagga tgtaaaggaa tacaggtaag caaattgata ctaatggctc aacgtgataa 8220
   ggaaaaagaa ttgcacttta acattaatat tgacaaggag gagggcacca cacaaaaagt 8280
   taggtgtaac agaaaatcat gaaactatga ttcctaaggt aagtcggttt aagacaatgg 8340
   gaaaagttag atgcctagag tattgattat cgagcaaata tcttctcatc tgtgatctcc 8400
   tagtgctagc taaagaagtt gttagatttt atattctatt ataaaatata aataatattt 8460
   tttaggccca taactcagtt ggtagagtga ttgccttaca agcaataggt catcggttca 8520
   agtccggttg ggcctataaa tacttttaaa aagtgtcaat ttaaccgctt gctccataca 8580
   ctttttataa cttttttgct ttgctgcttt ttttaaaaaa aagcataaaa gtagtaaaaa 8640
   atcatttgtc atgagcaaaa tattaaaaaa ccattcttta aataaaattt tttttattat 8700
   tctgtttaaa aaaatttttg tttgtaaaaa aaaagaaata aataaaacca aaataaaaaa 8760
   attttttctt ttttttaaaa taaattttgt taaaaatttt attttaagtt caataaaagt 8820
   tttttaattt tttaattttt gataaagtaa agttacttgg aaaattggtt tatacagaaa 8880
   aaattataaa ttatttattc atatgttacg tgaatgtgat tatagtcaag ctcttttaga 8940
   acaagttaat caagctatct ctgacaaaac tccattagtt attcaaggct caaactcaaa 9000
   agcattctta ggacgtccag taacaggcca aactttagac gttcgttgtc atcgtggtat 9060
   tgtaaattat gatccaactg agcttgtaat tactgcacgt gttggtacac ctttagtaac 9120
   aattgaagct gctttagaaa gtgctggtca aatgttacca tgtgaaccac ctcattacgg 9180
   tgaagaagct acttggggtg gtatggtagc ttgtggttta gctggtccac gtagaccttg 9240
   gagtggttct gttcgtgatt ttgtattagg tactcgtata attacaggtg ctggtaaaca 9300
   tttacgtttc ggtggtgaag ttatgaaaaa tgtagctggt tatgatttat cacgtttaat 9360
   ggtaggtagt tacggttgct taggcgtttt aacagaaatt tcaatgaaag ttttaccacg 9420
   tccaagagct tctttatcat tacgtcgtga aatatcatta caagaagcaa tgtcagaaat 9480
   tgcagaatgg caattacaac ctttaccaat aagtggttta tgttattttg ataatgcttt 9540
   atggatcaga ttagaaggag gcgaaggtag tgttaaagct gctcgtgaat tattaggtgg 9600
   tgaggaagta gcaggtcagt tttggcaaca attacgtgaa caacagcttc catttttctc 9660
   attaccaggt acattatggc gtattagttt accatctgat gcaccaatga tggacttacc 9720
   aggagaacaa cttattgatt ggggaggtgc tcttcgttgg ttaaaatcaa ctgctgaaga 9780
   taatcaaatc catcgtattg cacgtaatgc tggcggtcac gcaactcgtt tctcagcagg 9840
   tgatggtggt ttcgcacctt tatctgctcc attattccgt tatcatcaac aacttaaaca 9900
   acaattagat ccttgtggtg tttttaatcc aggacgtatg tacgctgagt tagattataa 9960
   agatgatgat gataaaggac acaaccaccg tcacaaacat tagtctagaa atttccattt 10020
   ttttcatttt tttttagaaa agttgtattt ttcttgatga aatgaaaatt tcaaaaaaga 10080
   aaaatacaat ttttctctac ttttttttga ttctttactt ttttttgaat tttttttgtg 10140
   cttcgttttt gaaaaaaact ttttatttga aaaattttgt tgaattaaaa aaaatcaact 10200
   caatacattt tttttgaact tctttttact tttttgcttt gttgcttttc ttttcatttt 10260
   tatcattttt tgcttcgctg ctttttatca ttttttgctt tgcaaaaaat gaagaagaaa 10320
   aaaagcgtaa aatgaaaaag aaaaaaagtt tcaaagaaaa aaaagcgaaa aagcaagaga 10380
   agataaaaaa caagaacaaa aaaattgttc aaaaacacaa tagaaaattc tttaaaaatt 10440
   ttttgatttt ttatagaatt tgtgagaaat agtaaaaaaa gtcaaaaaat cacaacatta 10500
   taaatagaaa aaatcttgtt ttgtaaaaat ttataatttt ttatattatt tcaattttaa 10560
   ataagatctt ttggagctga aaaaatatga gaaatagttt ggacaaacta gtctcgagcc 10620
   cgtatgatat tctaaggcgt tacgctgatg aatattctac agagttgcca taggcgttga 10680
   acgctacacg gacgatacga atttttgaat tagataaatg agtgttctca attttttttt 10740
   ctttgcattt tttgtttgtg ttgatttaca aaaacaatag aaaaaagaaa acaatatttt 10800
   ctttctaaaa aaaaacaaaa ttgatgaaaa atagacatga acaaaaaatt ttgaaagttg 10860
   acttttttaa aaaatttttg gtataataca aaaaaagaat ttttggaaag gtggcagagt 10920
   ggttgaatgc tctggttttg aaaaccagcg tggctttacg gtcaccgggg gttcgaatcc 10980
   ctccctttcc gataatatat acaaaaattt ttaaagtttt ttgtttattt tgtatagata 11040
   aaaaatctgc aataaaaatt tcgtttttta tttattcaaa aattctgttt ttttgaaaag 11100
   aaaataaaaa aaatgccaaa agtgagtttt ttattcaaat attagaaaaa gtttttgaaa 11160
   aatttaaaaa aatagaaaaa atttttttat ttttttcata atttaaaaaa ttatgttata 11220
   atttaaatta caaataggtt ttattaaaaa atttttacgt acagatgaat tctataaaat 11280
   tattttggag atcaccatat gcaaacacag ttaacagaag aaatgcgtca aaatgctcgt 11340
   gctttagaag cagacagtat cttacgtgct tgtgtacatt gtggcttttg tactgctaca 11400
   tgccctacat accaacttct tggtgatgaa ttagatggac caagaggtcg tatttattta 11460
   ataaaacaag tattagaagg taatgaagtt actttaaaaa ctcaagaaca cttagaccgt 11520
   tgtttaacat gccgtaattg tgaaactact tgtccaagtg gagtacgtta tcataattta 11580
   cttgatatag gtcgtgatat cgtagaacaa aaagttaaac gtcctttacc agaacgtatt 11640
   ttacgtgaag gacttcgtca agtagttcca agaccagcag ttttccgtgc tttaactcaa 11700
   gtaggtttag ttttacgtcc atttttacct gaacaagtac gtgctaaatt acctgcagaa 11760
   actgttaaag caaaaccacg tcctccttta cgtcataaac gtcgtgtttt aatgttagaa 11820
   ggttgcgctc aaccaacatt atctccaaat acaaatgcag caactgctcg tgtattagat 11880
   cgtttaggta tttcagttat gccagcaaat gaagcaggtt gttgtggtgc tgttgattat 11940
   cacttaaatg ctcaagaaaa aggtttagct agagctcgta acaacattga cgcttggtgg 12000
   ccagcaatcg aagcaggtgc tgaagctatt ttacaaactg catcaggttg cggtgcattt 12060
   gttaaagaat atggccaaat gttaaaaaac gacgctttat atgctgataa agcacgtcaa 12120
   gtaagtgaac ttgctgttga cttagtagaa ttattacgtg aagaacctct tgaaaaactt 12180
   gctattcgtg gtgataaaaa acttgctttc cactgtccat gtactttaca acacgctcaa 12240
   aaacttaatg gtgaagtaga aaaagttctt ttaagattag gttttacttt aacagatgtt 12300
   cctgattcac acttatgttg tggttcagct ggtacatacg ctcttacaca cccagactta 12360
   gctcgtcaat tacgtgacaa caaaatgaat gcacttgaaa gtggaaaacc agaaatgatt 12420
   gttacagcta atattggctg ccaaactcac ttagcttctg ctggtcgtac aagtgttcgt 12480
   cattggattg aaattgtaga acaagcatta gaaaaagaag attataaaga tgatgatgat 12540
   aaaggacaca accaccgtca caaacattaa tctagatttt attttttatg aaaaactcag 12600
   gcttaattta ggcttgagtt tttcattctt tttgaagctc tgaaatttta aaatttctag 12660
   tcttctttaa tgtttttaaa ttttaaaaaa taaatttctt ctctgctgtg tttttctttt 12720
   tttttgaaaa aacaaagaaa aaaaattttt ttgttttctt ctttgttttt ttatttcttt 12780
   ttgttttgtt tattttttag tttcagaatc tttgattcaa aaaaaaattt agtccgatta 12840
   ctccatagga gcaagcagta aaaaataaaa actgtaataa aaaataaaac aaaaatttta 12900
   tttctttttg ttttgcttga acttttcaaa aaaaaattga aaaattcaag caaaacaaaa 12960
   agaaacaaat aaaaaattta tgaattttct actttttcag gagttgaaat ttctccttta 13020
   cttaaaacat attttgctaa aaaaagcgct tgtgttgctt tttttgctac tttttgtttc 13080
   caagcatttt ttcgaatatt tttttttgat tttgatgtgc gtttttgtta acctaaaatc 13140
   ttgaaaagat ttactctttt caaattttta tgtttttatt ttttttattc ataaaaaaaa 13200
   acaatacata aaaataaagt atttcggctt caaaaaattt tatacaaaaa gttttttgat 13260
   taaaaactca gaaaaaataa aaaaacaaag tatgaatttt ttgaaaaatt catacctttt 13320
   atttttttgt aatttttagc ctttcaaaaa atttttgaag gcattttttt tttaatcctc 13380
   atgttcttca aaaggatctc tcaatttttt tgaaggaggt ccaaaactca catagattga 13440
   atatcctgtt gcacttaata atagaaacca taaaaaaaag gtaaagaaaa aagcaggact 13500
   gtccataatt ctttcatgtt ttttgttcaa atttattctc caataattat attacgacaa 13560
   aaagtaaaaa aaatcaaaat ttattcaaaa aaatggctac tggaacaact tcaaaagcta 13620
   aatcaagctt atctgatgca cttcaagaac caggtatcgt aactccttta ggaactttat 13680
   taagaccgtt aaactctgaa tcaggaaaag tattacctgg atggggaaca actgttttaa 13740
   tgggtgtttt cattgtactt tttgctgtat tcttattaat tattttagaa atttataaca 13800
   gttctttatt attagataat gttactatga gttgggaaac tttagcttct taattcaata 13860
   gaatagtttt attgcttttt ttatttttta ttttatcaaa aatttttttt gcaaaaataa 13920
   agaataaata aaattcaaaa aaattataga attagataaa attagtttca agttgaacta 13980
   agttgtcaat aaactttcaa atttgttttc tttttactgt tcattaagag caataaaaaa 14040
   aacttttggt cttggcaatc ttttaaaaaa gtcagaatca attctatttt aagaatccta 14100
   tggaatctat gtatttaatt ttagcaaaat taccagaagc ttatgcacct tttgatccta 14160
   ttgtagatgt tttaccaatt attcctattt tcttcttatt attagccttt gtatggcaag 14220
   catctgtaag ttttagataa aaaatttaaa agtttttttt gatacttttg taaaaaatat 14280
   caaaaaaaac ttttaaattt ttttcaattt tcattagcaa ctttagcttt aatattagct 14340
   aaagttgctc tcaaaaatat aatttttttt tgacttttta tttttttatt ttgtttcttt 14400
   tttaaaagtt acaacataaa gaaaatgaaa atagaaaatt tgtgaaacat aaaaaaaaag 14460
   aatgaaattt ttatgttcgt tttttgtttt atcttttcca actaaagtcg gcctctagct 14520
   agaggccggc caaatttttt tccaaaattc tataaaaaat caaaaaatta aaaaaaaaag 14580
   aaaaaacttt gttttgtgca aaacaaaaat cttgaattca aaacaaaata aagaattcaa 14640
   aaagattttt tttaagcaaa aggtaaaatg gaaaaaaatg tttttaaaaa aattttttct 14700
   tttttttaaa gctttgcttt tttcatgaaa aaaacaaagc tttaaaaaaa agaaaaaatt 14760
   ttaaagcaaa aaaaagaatt aaacacgtct tttttttgga ggacgacatc cattatgtgg 14820
   aattcctgtt ttttctcgaa taacatttac tttaattcca gctttaaaaa tttcacgaat 14880
   agctgtttcg cgaccttgtc ctggaccagt tactaaaatt tttgcttcat ttaatgcaaa 14940
   ttcacgtgat ttttttgcca caacttcagc agcttttttt gctgcaaatg ttgttgcttt 15000
   tctttttcca cggaaaccac aagctccagc agaactccaa caaaggactt caccacgaag 15060
   atttgctaat gtaataatag tattatgatg tccagcttga atataaacaa ttcctcgata 15120
   tgtacgtttt ttaatttttg taggtgatac ttttctagtt tgtctagcca tatgtaaaaa 15180
   tttaactata aatttctttt atatttttaa atcatttgat ttactatcta aaaaaaataa 15240
   gattttgaat ctttggaaag aaccattatt tggaaagatt tatttgttca attcttttgt 15300
   attttttttc aaaaaaattt tttagaattt tttatctaat tttttaatgt tttgttcaaa 15360
   cataaaaaat tttcttttca aaaaaggaaa aaaaatttct tttttttgaa aaagtaaaaa 15420
   taaaaaaagc tgtagctttt ttgttgaatc aaaaaaaaat aagaaattgt cctattttta 15480
   tgacaaaaga ttcaaaaaaa tgaaataaaa aagaaaaatt caaactttca aacaatgtat 15540
   tttgtatttt tgggccgagt cggattcgaa ccaacgtagg cgtaaccagc ggatttacaa 15600
   tccgccccca ttaaccactc gggcatcggc ccatgctttt ttttgactca tttgatcatt 15660
   tattttgcat gaattatact aaagattata ttaacaaaaa atttttgaaa tttcaatttt 15720
   ttttaattaa aaactcttct atttttttaa aattctttgt tttgaatttt ttttttcaat 15780
   ttaaaaaaaa aacatattaa aaaatatttt taaaaaattt tttgtattga aaacttacaa 15840
   taattttata aatttttttg aaaaattttg ttttttttat tctattgaaa tgaacaaaac 15900
   aaattttttt agtttttttt gtttttttgc tttgctgctt cttttgtttt tatcaataaa 15960
   taaatgaaaa tgaaaaacaa aaataaacaa tttgtgtttt tagagttcta aaatcaagaa 16020
   aaaaatactt cccctttaaa gaggaagtat tttaaaaaaa aattatagtt tgtcaatagt 16080
   ttcaaattca aatttgattt ctttccaaac ttcacaagca gcagctaatt ctggagacca 16140
   tttacaagct gagcggataa catcaccacc ttcacgagct aaatcacgac cttcgttacg    16200
   agcttgagta caagcttcta aagcaacacg gttagcaaca gcaccaggag cgttacccca    16260
   agggtgtcct aaagtacctc caccgaattg taaacaagcg tcatcaccaa agatttcaac    16320
   taaagctggc atgtgccata cgtgaatacc accagaagca actggcatag taccacccat    16380
   agaacaccag tcttgagtga agtaaatacc acggcta                             16417
<210> 115
   <211> 1553
   <212> DNA
   <213> Artificial Sequence
<220>
   <223> codon optimized sequence comprising CC-90
<400> 115
   atgagtatat tatatgaaga acgtttagat ggtgctttac cagatgttga tcgtacttca      60
   gttttaatgg cacttcgtga acacgtacct ggcttagaga ttttacatac agatgaagaa     120
   attattccat acgaatgtga tggattatca gcttacagaa caagaccttt attagtagtt     180
   ttaccaaaac aaatggaaca agttactgct atattagcag tatgtcatag attacgtgtt     240
   ccagttgtta cacgtggtgc aggtacaggc ttaagtggcg gtgcacttcc tttagaaaaa     300
   ggtgtattat tagttatggc acgttttaaa gaaattttag atattaaccc agttggtcgt     360
   cgtgcacgtg ttcaacctgg tgttcgtaat ttagcaattt cacaagcagt tgctcctcat     420
   aacttatatt acgctccaga tcctagtagt caaattgctt gttcaattgg tggtaatgta     480
   gcagagaatg ctggtggcgt acactgttta aaatatggtt taacagtaca caacttatta     540
   aaaattgaag tacaaacttt agacggtgag gctcttactt taggtagtga tgcattagat     600
   tctcctggtt ttgatttatt agctttattc acaggatcag aaggaatgtt aggtgttaca     660
   acagaagtaa ctgttaaatt acttccaaaa ccacctgttg ctcgtgtttt attagcatct     720
   tttgattcag ttgaaaaagc aggattagct gtaggcgata tcattgcaaa tggtataatt     780
   ccaggaggat tagagatgat ggataactta tcaattcgtg cagctgaaga ttttatacac     840
   gctggttatc cagtagatgc tgaagcaatt ttactttgtg aattagatgg agtagagagt     900
   gatgttcagg aagactgtga gcgtgtaaac gacatattac ttaaagctgg tgctactgat     960
   gttcgtttag cacaagatga agctgaacgt gtaagatttt gggcaggtcg taaaaatgct    1020
   tttcctgcag taggtcgtat ttcaccagac tattattgta tggatggtac aattccacgt    1080
   cgtgcattac caggtgtttt agaaggtatt gcacgtcttt ctcaacaata cgatttacgt    1140
   gtagctaatg tatttcatgc tggcgatggt aatatgcatc cattaatctt attcgatgct    1200
   aacgaacctg gtgagtttgc tcgtgctgaa gaacttggtg gaaaaatctt agagttatgt    1260
   gtagaagtag gtggttctat tagtggtgag catggaatcg gccgtgaaaa aatcaatcaa    1320
   atgtgtgctc aattcaatag tgatgaaatt actacatttc acgctgtaaa agctgctttt    1380
   gatccagacg gtttacttaa tcctggtaaa aatattccaa ctttacatcg ttgtgcagag    1440
   tttggtgcta tgcatgtaca tcacggccat ttaccttttc cagagcttga acgttttgat     1500
   tataaagatg atgatgataa aggacacaac caccgtcaca aacattaatc tag           1553
<210> 116
   <211> 1102
   <212> DNA
   <213> Artificial Sequence
<220>
   <223> codon optimized sequence comprising CC-91
<400> 116
   atgttacgtg aatgtgatta tagtcaagct cttttagaac aagttaatca agctatctct      60
   gacaaaactc cattagttat tcaaggctca aactcaaaag cattcttagg acgtccagta     120
   acaggccaaa ctttagacgt tcgttgtcat cgtggtattg taaattatga tccaactgag     180
   cttgtaatta ctgcacgtgt tggtacacct ttagtaacaa ttgaagctgc tttagaaagt     240
   gctggtcaaa tgttaccatg tgaaccacct cattacggtg aagaagctac ttggggtggt     300
   atggtagctt gtggtttagc tggtccacgt agaccttgga gtggttctgt tcgtgatttt     360
   gtattaggta ctcgtataat tacaggtgct ggtaaacatt tacgtttcgg tggtgaagtt     420
   atgaaaaatg tagctggtta tgatttatca cgtttaatgg taggtagtta cggttgctta     480
   ggcgttttaa cagaaatttc aatgaaagtt ttaccacgtc caagagcttc tttatcatta     540
   cgtcgtgaaa tatcattaca agaagcaatg tcagaaattg cagaatggca attacaacct     600
   ttaccaataa gtggtttatg ttattttgat aatgctttat ggatcagatt agaaggaggc     660
   gaaggtagtg ttaaagctgc tcgtgaatta ttaggtggtg aggaagtagc aggtcagttt     720
   tggcaacaat tacgtgaaca acagcttcca tttttctcat taccaggtac attatggcgt     780
   attagtttac catctgatgc accaatgatg gacttaccag gagaacaact tattgattgg     840
   ggaggtgctc ttcgttggtt aaaatcaact gctgaagata atcaaatcca tcgtattgca     900
   cgtaatgctg gcggtcacgc aactcgtttc tcagcaggtg atggtggttt cgcaccttta     960
   tctgctccat tattccgtta tcatcaacaa cttaaacaac aattagatcc ttgtggtgtt    1020
   tttaatccag gacgtatgta cgctgagtta gattataaag atgatgatga taaaggacac    1080
   aaccaccgtc acaaacatta gt                                             1102
<210> 117
   <211> 1272
   <212> DNA
   <213> Artificial Sequence
<220>
   <223> codon optimized sequence comprising CC-92
<400> 117
   atgcaaacac agttaacaga agaaatgcgt caaaatgctc gtgctttaga agcagacagt 60
   atcttacgtg cttgtgtaca ttgtggcttt tgtactgcta catgccctac ataccaactt 120
   cttggtgatg aattagatgg accaagaggt cgtatttatt taataaaaca agtattagaa 180
   ggtaatgaag ttactttaaa aactcaagaa cacttagacc gttgtttaac atgccgtaat 240
   tgtgaaacta cttgtccaag tggagtacgt tatcataatt tacttgatat aggtcgtgat 300
   atcgtagaac aaaaagttaa acgtccttta ccagaacgta ttttacgtga aggacttcgt 360
   caagtagttc caagaccagc agttttccgt gctttaactc aagtaggttt agttttacgt 420
   ccatttttac ctgaacaagt acgtgctaaa ttacctgcag aaactgttaa agcaaaacca 480
   cgtcctcctt tacgtcataa acgtcgtgtt ttaatgttag aaggttgcgc tcaaccaaca 540
   ttatctccaa atacaaatgc agcaactgct cgtgtattag atcgtttagg tatttcagtt 600
   atgccagcaa atgaagcagg ttgttgtggt gctgttgatt atcacttaaa tgctcaagaa 660
   aaaggtttag ctagagctcg taacaacatt gacgcttggt ggccagcaat cgaagcaggt 720
   gctgaagcta ttttacaaac tgcatcaggt tgcggtgcat ttgttaaaga atatggccaa 780
   atgttaaaaa acgacgcttt atatgctgat aaagcacgtc aagtaagtga acttgctgtt 840
   gacttagtag aattattacg tgaagaacct cttgaaaaac ttgctattcg tggtgataaa 900
   aaacttgctt tccactgtcc atgtacttta caacacgctc aaaaacttaa tggtgaagta 960
   gaaaaagttc ttttaagatt aggttttact ttaacagatg ttcctgattc acacttatgt 1020
   tgtggttcag ctggtacata cgctcttaca cacccagact tagctcgtca attacgtgac 1080
   aacaaaatga atgcacttga aagtggaaaa ccagaaatga ttgttacagc taatattggc 1140
   tgccaaactc acttagcttc tgctggtcgt acaagtgttc gtcattggat tgaaattgta 1200
   gaacaagcat tagaaaaaga agattataaa gatgatgatg ataaaggaca caaccaccgt 1260
   cacaaacatt aa 1272
<210> 118
   <211> 1179
   <212> DNA
   <213> Artificial Sequence
<220>
   <223> codon optimized sequence comprising HIS3
<400> 118
   aattcccgtt ttaagagctt ggtgagcgct aggagtcact gccaggtatc gtttgaacac      60
   ggcattagtc agggaagtca taacacagtc ctttcccgca attttctttt tctattactc     120
   ttggcctcct ctagtacact ctatattttt ttatgcctcg gtaatgattt tcattttttt     180
   ttttccacct agcggatgac tctttttttt tcttagcgat tggcattatc acataatgaa     240
   ttatacatta tataaagtaa tgtgatttct tcgaagaata tactaaaaaa tgagcaggca     300
   agataaacga aggcaaagat gacagagcag aaagccctag taaagcgtat tacaaatgaa     360
   accaagattc agattgcgat ctctttaaag ggtggtcccc tagcgataga gcactcgatc     420
   ttcccagaaa aagaggcaga agcagtagca gaacaggcca cacaatcgca agtgattaac     480
   gtccacacag gtatagggtt tctggaccat atgatacatg ctctggccaa gcattccggc     540
   tggtcgctaa tcgttgagtg cattggtgac ttacacatag acgaccatca caccactgaa     600
   gactgcggga ttgctctcgg tcaagctttt aaagaggccc taggggccgt gcgtggagta     660
   aaaaggtttg gatcaggatt tgcgcctttg gatgaggcac tttccagagc ggtggtagat     720
   ctttcgaaca ggccgtacgc agttgtcgaa cttggtttgc aaagggagaa agtaggagat     780
   ctctcttgcg agatgatccc gcattttctt gaaagctttg cagaggctag cagaattacc     840
   ctccacgttg attgtctgcg aggcaagaat gatcatcacc gtagtgagag tgcgttcaag     900
   gctcttgcgg ttgccataag agaagccacc tcgcccaatg gtaccaacga tgttccctcc     960
   accaaaggtg ttcttatgta gtgacaccga ttatttaaag ctgcagcata cgatatatat    1020
   acatgtgtat atatgtatac ctatgaatgt cagtaagtat gtatacgaac agtatgatac    1080
   tgaagatgac aaggtaatgc atcattctat acgtgtcatt ctgaacgagg cgcgctttcc    1140
   ttttttcttt ttgctttttc tttttttttc tcttgaact                               1179
<210> 119
   <211> 4879
   <212> DNA
   <213> Artificial Sequence
<220>
   <223> codon optimized sequence comprising LYS2
<400> 119
   agcagttgct ttctcctatg ggaagagctt tctaagtctg aagaagtaaa cagttctttg      60
   ctatttcaca cttcctggtt gatggtcact tgctgcctga aatatatata tatgtatgac     120
   atatgtactt gttttctttt ttgtgccttt gttacgtcta tattcattga aactgattat     180
   tcgattttct tcttgctgac cgcttctaga ggcatcgcac agttttagcg aggaaaactc     240
   ttcaatagtt ttgccagcgg aattccactt gcaattacat aaaaaattcc ggcggttttt     300
   cgcgtgtgac tcaatgtcga aatacctgcc taatgaacat gaacatcgcc caaatgtatt     360
   tgaagacccg ctgggagaag ttcaagatat ataagtaaca agcagccaat agtataaaaa     420
   aaaatctgag tttattacct ttcctggaat ttcagtgaaa aactgctaat tatagagaga     480
   tatcacagag ttactcacta atgactaacg aaaaggtctg gatagagaag ttggataatc     540
   caactctttc agtgttacca catgactttt tacgcccaca acaagaacct tatacgaaac     600
   aagctacata ttcgttacag ctacctcagc tcgatgtgcc tcatgatagt ttttctaaca     660
   aatacgctgt cgctttgagt gtatgggctg cattgatata tagagtaacc ggtgacgatg     720
   atattgttct ttatattgcg aataacaaaa tcttaagatt caatattcaa ccaacgtggt     780
   catttaatga gctgtattct acaattaaca atgagttgaa caagctcaat tctattgagg 840
   ccaatttttc ctttgacgag ctagctgaaa aaattcaaag ttgccaagat ctggaaagga 900
   cccctcagtt gttccgtttg gcctttttgg aaaaccaaga tttcaaatta gacgagttca 960
   agcatcattt agtggacttt gctttgaatt tggataccag taataatgcg catgttttga 1020
   acttaattta taacagctta ctgtattcga atgaaagagt aaccattgtt gcggaccaat 1080
   ttactcaata tttgactgct gcgctaagcg atccatccaa ttgcataact aaaatctctc 1140
   tgatcaccgc atcatccaag gatagtttac ctgatccaac taagaacttg ggctggtgcg 1200
   atttcgtggg gtgtattcac gacattttcc aggacaatgc tgaagccttc ccagagagaa 1260
   cctgtgttgt ggagactcca acactaaatt ccgacaagtc ccgttctttc acttatcgcg 1320
   acatcaaccg cacttctaac atagttgccc attatttgat taaaacaggt atcaaaagag 1380
   gtgatgtagt gatgatctat tcttctaggg gtgtggattt gatggtatgt gtgatgggtg 1440
   tcttgaaagc cggcgcaacc ttttcagtta tcgaccctgc atatccccca gccagacaaa 1500
   ccatttactt aggtgttgct aaaccacgtg ggttgattgt tattagagct gctggacaat 1560
   tggatcaact agtagaagat tacatcaatg atgaattgga gattgtttca agaatcaatt 1620
   ccatcgctat tcaagaaaat ggtaccattg aaggtggcaa attggacaat ggcgaggatg 1680
   ttttggctcc atatgatcac tacaaagaca ccagaacagg tgttgtagtt ggaccagatt 1740
   ccaacccaac cctatctttc acatctggtt ccgaaggtat tcctaagggt gttcttggta 1800
   gacatttttc cttggcttat tatttcaatt ggatgtccaa aaggttcaac ttaacagaaa 1860
   atgataaatt cacaatgctg agcggtattg cacatgatcc aattcaaaga gatatgttta 1920
   caccattatt tttaggtgcc caattgtatg tccctactca agatgatatt ggtacaccgg 1980
   gccgtttagc ggaatggatg agtaagtatg gttgcacagt tacccattta acacctgcca 2040
   tgggtcaatt acttactgcc caagctacta caccattccc taagttacat catgcgttct 2100
   ttgtgggtga cattttaaca aaacgtgatt gtctgaggtt acaaaccttg gcagaaaatt 2160
   gccgtattgt taatatgtac ggtaccactg aaacacagcg tgcagtttct tatttcgaag 2220
   ttaaatcaaa aaatgacgat ccaaactttt tgaaaaaatt gaaagatgtc atgcctgctg 2280
   gtaaaggtat gttgaacgtt cagctactag ttgttaacag gaacgatcgt actcaaatat 2340
   gtggtattgg cgaaataggt gagatttatg ttcgtgcagg tggtttggcc gaaggttata 2400
   gaggattacc agaattgaat aaagaaaaat ttgtgaacaa ctggtttgtt gaaaaagatc 2460
   actggaatta tttggataag gataatggtg aaccttggag acaattctgg ttaggtccaa 2520
   gagatagatt gtacagaacg ggtgatttag gtcgttatct accaaacggt gactgtgaat 2580
   gttgcggtag ggctgatgat caagttaaaa ttcgtgggtt cagaatcgaa ttaggagaaa 2640
   tagatacgca catttcccaa catccattgg taagagaaaa cattacttta gttcgcaaaa 2700
   atgccgacaa tgagccaaca ttgatcacat ttatggtccc aagatttgac aagccagatg 2760
   acttgtctaa gttccaaagt gatgttccaa aggaggttga aactgaccct atagttaagg 2820
   gcttaatcgg ttaccatctt ttatccaagg acatcaggac tttcttaaag aaaagattgg 2880
   ctagctatgc tatgccttcc ttgattgtgg ttatggataa actaccattg aatccaaatg 2940
   gtaaagttga taagcctaaa cttcaattcc caactcccaa gcaattaaat ttggtagctg 3000
   aaaatacagt ttctgaaact gacgactctc agtttaccaa tgttgagcgc gaggttagag 3060
   acttatggtt aagtatatta cctaccaagc cagcatctgt atcaccagat gattcgtttt 3120
   tcgatttagg tggtcattct atcttggcta ccaaaatgat ttttacctta aagaaaaagc 3180
   tgcaagttga tttaccattg ggcacaattt tcaagtatcc aacgataaag gcctttgccg 3240
   cggaaattga cagaattaaa tcatcgggtg gatcatctca aggtgaggtc gtcgaaaatg 3300
   tcactgcaaa ttatgcggaa gacgccaaga aattggttga gacgctacca agttcgtacc 3360
   cctctcgaga atattttgtt gaacctaata gtgccgaagg aaaaacaaca attaatgtgt 3420
   ttgttaccgg tgtcacagga tttctgggct cctacatcct tgcagatttg ttaggacgtt 3480
   ctccaaagaa ctacagtttc aaagtgtttg cccacgtcag ggccaaggat gaagaagctg 3540
   catttgcaag attacaaaag gcaggtatca cctatggtac ttggaacgaa aaatttgcct 3600
   caaatattaa agttgtatta ggcgatttat ctaaaagcca atttggtctt tcagatgaga 3660
   agtggatgga tttggcaaac acagttgata taattatcca taatggtgcg ttagttcact 3720
   gggtttatcc atatgccaaa ttgagggatc caaatgttat ttcaactatc aatgttatga 3780
   gcttagccgc cgtcggcaag ccaaagttct ttgactttgt ttcctccact tctactcttg 3840
   acactgaata ctactttaat ttgtcagata aacttgttag cgaagggaag ccaggcattt 3900
   tagaatcaga cgatttaatg aactctgcaa gcgggctcac tggtggatat ggtcagtcca 3960
   aatgggctgc tgagtacatc attagacgtg caggtgaaag gggcctacgt gggtgtattg 4020
   tcagaccagg ttacgtaaca ggtgcctctg ccaatggttc ttcaaacaca gatgatttct 4080
   tattgagatt tttgaaaggt tcagtccaat taggtaagat tccagatatc gaaaattccg 4140
   tgaatatggt tccagtagat catgttgctc gtgttgttgt tgctacgtct ttgaatcctc 4200
   ccaaagaaaa tgaattggcc gttgctcaag taacgggtca cccaagaata ttattcaaag 4260
   actacttgta tactttacac gattatggtt acgatgtcga aatcgaaagc tattctaaat 4320
   ggaagaaatc attggaggcg tctgttattg acaggaatga agaaaatgcg ttgtatcctt 4380
   tgctacacat ggtcttagac aacttacctg aaagtaccaa agctccggaa ctagacgata 4440
   ggaacgccgt ggcatcttta aagaaagaca ccgcatggac aggtgttgat tggtctaatg 4500
   gaataggtgt tactccagaa gaggttggta tatatattgc atttttaaac aaggttggat 4560
   ttttacctcc accaactcat aatgacaaac ttccactgcc aagtatagaa ctaactcaag 4620
   cgcaaataag tctagttgct tcaggtgctg gtgctcgtgg aagctccgca gcagcttaag 4680
   gttgagcatt acgtatgata tgtccatgta caataattaa atatgaatta ggagaaagac 4740
   ttagcttctt ttcgggtgat gtcacttaaa aactccgaga ataatatata ataagagaat 4800
   aaaatattag ttattgaata agaactgtaa atcagctggc gttagtctgc taatggcagc 4860
   ttcatcttgg tttattgta 4879
<210> 120
   <211> 24607
   <212> DNA
   <213> Artificial Sequence
<220>
   <223> codon optimized sequence comprising IS57-IS116-IS62-IS61
<400> 120
   cgtttaggtg taacacaatc ttggggtgga tggacaatta gcggtgaaac agcaacaaat      60
   ccaggtattt ggagttatga aggtgttgct gcatctcata ttattttatc tggtttatta     120
   ttcttagctt cggtttggca ctgggtttac tgggatttag agttattccg tgacccaaga     180
   actggaaaaa ctgcattaga tttaccaaaa attttcggaa ttcacttatt cttatcaggt     240
   cttttatgtt ttggttttgg tgctttccac gtaacaggtt tatttggtcc tggtatttgg     300
   gtttcagatc cttatggatt aacaggaagt gttcaaccag ttgctccttc ttggggtgct     360
   gatgggtttg atcctttcaa ccctggtggt attgcagcgc accacattgc tgctggtatt     420
   ttaggtgttt tagcaggatt attccactta tgtgtacgtc cttctattcg tttatacttt     480
   ggtttatcaa tgggtagtat cgaaacagta ttatcaagta gtattgctgc tgttttctgg     540
   gctgctttcg ttgttgctgg aactatgtgg tatggttcag cagctactcc aattgaatta     600
   tttggtccta cacgttatca atgggaccaa ggtttcttcc aacaagaaat tcaaaaacga     660
   gttcaaacaa gtttagcagg tggttcttca ctttctgatg cttgggcgaa aattccagaa     720
   aaattagctt tctatgatta tattggaaac aaccctgcaa aaggtggtct tttccgtaca     780
   ggagctatga atagtggaga tggtattgct gttggatggt taggtcacgc agtatttaaa     840
   gatcaagatg gtcgtgaatt atacgtacgt cgtatgccta ctttctttga aacattccca     900
   gttttattaa ttgataaaga tggtgttgta cgtgctgacg ttcctttccg tcgtgctgaa     960
   tcaaaatata gtattgaaca agttggtgta tcagtaactt tctacggtgg tgaattagat    1020
   ggattaacat ttaatgatcc agcaactgtt aaaaaatatg ctcgtaaagc acaattaggt    1080
   gaaatttttg aatttgatcg ttcaacatta caatctgatg gtgtattccg tagtagtcca    1140
   cgtggttggt ttacttttgg tcacgtttgc tttgctttat tattcttctt tggacatatt    1200
   tggcatggtg cacgtacaat cttccgtgat gtatttgctg gtattgatga tgatctaaac    1260
   gaaagtttag aatttggtaa atacaaaaaa cttggtgata caagttctgt tcgtgaagct    1320
   ttctaattcg tttttttctc ttttttttct tttttctctt tggaaaaaga aaaaacatgt    1380
   ttattttgaa tttttgtttt agaactttac tgttcttttt ttattttaaa gtgtttctgt    1440
   ttttttttaa tacaaaaact tttttaaaat gaatttaaaa aacacaaaaa aagagttatt 1500
   gctattcaaa ataaacaaga gtttaaaaac aaagtttttt tctttagaaa aaaacttctt 1560
   catttttttt gaattgtttt tgaacttttt tcttctcttg cttttatcgt ttttttcttc 1620
   actttttgca aaaaagtgag aaaaaacagc aaagcaaaaa agtgaaaaaa agttcaaaaa 1680
   caattcaaaa aagacaaaac ctaaaaaaat atcacttgag atgggtctgg attttttcca 1740
   agcaaaagaa ttttgtattt tgttgaaagt ttttcataaa aatacaaatt tgcaattatt 1800
   attcttaaaa tcaaaatatt tgttaaccac atttcattct atggaagcat tagtttatac 1860
   ttttttatta atcggaacat taggaattat ctttttcgca attttcttta gagaaccacc 1920
   tcgtatggta aaataagggc tcgagactag tttgtccaaa ctatttctca tattttttca 1980
   gctccaaaag atcttattta aaattgaaat aatataaaaa attataaatt tttacaaaac 2040
   aagatttttt ctatttataa tgttgtgatt ttttgacttt ttttactatt tctcacaaat 2100
   tctataaaaa atcaaaaaat ttttaaagaa ttttctattg tgtttttgaa caattttttt 2160
   gttcttgttt tttatcttct cttgcttttt cgcttttttt tctttgaaac tttttttctt 2220
   tttcatttta cgcttttttt cttcttcatt ttttgcaaag caaaaaatga taaaaagcag 2280
   cgaagcaaaa aatgataaaa atgaaaagaa aagcaacaaa gcaaaaaagt aaaaagaagt 2340
   tcaaaaaaaa tgtattgagt tgattttttt taattcaaca aaatttttca aataaaaagt 2400
   ttttttcaaa aacgaagcac aaaaaaaatt caaaaaaaag taaagaatca aaaaaaagta 2460
   gagaaaaatt gtatttttct tttttgaaat tttcatttca tcaagaaaaa tacaactttt 2520
   ctaaaaaaaa atgaaaaaaa tggaaatttc tagattaacc ggtgtgttta tgacgatgat 2580
   tgtgaccttg aaagtataag ttctcaccac ttttatcatc atcatctttg taatcaccgg 2640
   taccagcatg aactggacga gctccacttg ataattgaac atttgcagca tactcacgag 2700
   cccataagtc ataatgtaca atttcttcaa gacttggaga agttactaat tcattacggt 2760
   gtttatcaca tgttaattca actactttga agatgtctaa gtatgaaatt ttctcgtcaa 2820
   tgaacatttc tacagctttc tcattagcag cagataaaac accagtcata gtaccacctg 2880
   cacgaccagc tgcataagca agatccattg atggatattt tacgttgtca ggttttttga 2940
   atgtaagaga tcctaattta cataaatcta aacgtggcca agtaacttca ctgcaaggaa 3000
   cacggtctgg ccatgacatt gtatataaaa taggtaaacg catgtcaggc cagcctaatt 3060
   gagctaatac tgaagaatct tgtgtttcaa tcatactatg aataattgat tgtggatgaa 3120
   taacaatttc aatatcgtcg tattcagcac cgaaaagata atgtgcttca ataacttcta 3180
   aacctttgtt gaataaagtt gcactatcta cagtaatttt tttgcccata ttccagtttg 3240
   ggtgttttaa tgcatcagct acttttacct ctttaagttt ttctacaggc caatcacgaa 3300
   atgcaccacc tgaagctgtt aatatgattt ttcttaatgc accttcaggt aagccttgaa 3360
   tacattgaaa aattgctgaa tgttcactgt ctgctggaag aatttttaca ttgtgtttgt 3420
   ttgcaagtgg taatacgaat ggaccacctg cgattaatgt ttctttattt gctaaagcaa 3480
   tatctttacc agcctcaata gctgcaactg taggttttaa tccagcacaa ccaacaatac 3540
   ctgtaactac tgtaactgct tctggatgac gagcaacttc aattacacct tgttctcctg 3600
   gaataatctc taatttgtag tctaaatctg ctaatgcttc ttttaattca ttaattaaag 3660
   attcattacg aactgcaact aaagcaggtt taaaacgacg tacctgatca gctaataaag 3720
   taacgttaga accagcagct aatgcaacta cacgaaattt atctggattt tctgcaacaa 3780
   tgtctaaagt ttgagtaccg attgaaccag ttgatcctac aatactaata ggtttaggac 3840
   catcccatga ttgtctagga gcttctggaa cagcacgacc tggccaagca ggtggtggtt 3900
   gttgctgttg ctgtactttt acagagcatt ttactccttt accaaaacca cgaccttgat 3960
   tacgacgacg taaagaaaaa ccaccagata atttaggaat tggattgaaa cgagaagtat 4020
   ctaaaaatga aattgctttt gattctgctg gagataatga atttaatgtt ggtaccatat 4080
   gaataaataa tttataattt tttctgtata aaccaatttt ccaagtaact ttactttatc 4140
   aaaaattaaa aaattaaaaa acttttattg aacttaaaat aaaattttta acaaaattta 4200
   ttttaaaaaa aagaaaaaat ttttttattt tggttttatt tatttctttt tttttacaaa 4260
   caaaaatttt tttaaacaga ataataaaaa aaattttatt taaagaatgg ttttttaata 4320
   ttttgctcat gacaaatgat tttttactac ttttatgctt ttttttaaaa aaagcagcaa 4380
   agcaaaaaag ttataaaaag tgtatggagc aagcggttaa attgacactt tttaaaagta 4440
   tttataggcc caaccggact tgaaccgatg acctattgct tgtaaggcaa tcactctacc 4500
   aactgagtta tgggcctaaa aaatattatt tatattttat aatagaatat aaaatctaac 4560
   aacttcttta gctagcacta ggagaaaata gcctcgcgga gccatgtgcc atactcgtct 4620
   gcggagcact ctggtaatgc atatggtcca caggacattc gtcgcttccg ggtatgcgct 4680
   ctatgaattc ccgttttaag agcttggtga gcgctaggag tcactgccag gtatcgtttg 4740
   aacacggcat tagtcaggga agtcataaca cagtcctttc ccgcaatttt ctttttctat 4800
   tactcttggc ctcctctagt acactctata tttttttatg cctcggtaat gattttcatt 4860
   tttttttttc cacctagcgg atgactcttt ttttttctta gcgattggca ttatcacata 4920
   atgaattata cattatataa agtaatgtga tttcttcgaa gaatatacta aaaaatgagc 4980
   aggcaagata aacgaaggca aagatgacag agcagaaagc cctagtaaag cgtattacaa 5040
   atgaaaccaa gattcagatt gcgatctctt taaagggtgg tcccctagcg atagagcact 5100
   cgatcttccc agaaaaagag gcagaagcag tagcagaaca ggccacacaa tcgcaagtga 5160
   ttaacgtcca cacaggtata gggtttctgg accatatgat acatgctctg gccaagcatt 5220
   ccggctggtc gctaatcgtt gagtgcattg gtgacttaca catagacgac catcacacca 5280
   ctgaagactg cgggattgct ctcggtcaag cttttaaaga ggccctaggg gccgtgcgtg 5340
   gagtaaaaag gtttggatca ggatttgcgc ctttggatga ggcactttcc agagcggtgg 5400
   tagatctttc gaacaggccg tacgcagttg tcgaacttgg tttgcaaagg gagaaagtag 5460
   gagatctctc ttgcgagatg atcccgcatt ttcttgaaag ctttgcagag gctagcagaa 5520
   ttaccctcca cgttgattgt ctgcgaggca agaatgatca tcaccgtagt gagagtgcgt 5580
   tcaaggctct tgcggttgcc ataagagaag ccacctcgcc caatggtacc aacgatgttc 5640
   cctccaccaa aggtgttctt atgtagtgac accgattatt taaagctgca gcatacgata 5700
   tatatacatg tgtatatatg tatacctatg aatgtcagta agtatgtata cgaacagtat 5760
   gatactgaag atgacaaggt aatgcatcat tctatacgtg tcattctgaa cgaggcgcgc 5820
   tttccttttt tctttttgct ttttcttttt ttttctcttg aactaccact caatttgacc 5880
   tagtcctcaa acgttctgct aaaccgtgtc aatcagtgtc tgcttcctga gtgaaacccg 5940
   taagatctcc taggaaaatg aattttaaaa attattttgt gcaatttgat gtttgcacga 6000
   tttgaaaaca tttgatttct tttgtcaaaa attttttatt tttaaacttt ttttaagttt 6060
   tgtaaaaaaa gaaataaaaa ataaataaaa ataaaaaaaa tgaaacttaa aaacataagt 6120
   cgcttaaaat ttttttttat tttttccttt tctttttgtt aaataaaata aaaagaaaag 6180
   gaaaaaataa aaaaaagaaa aaaacttaaa gtttaatttt gaaagatgct tttttttcta 6240
   gattaaccgg ttgtattttc ttgatgaatt gtacgtgtta aataaaattc agctaaagct 6300
   aaatcttctg gacgtgtaac tttaatgtta tcagcacgac cttcaactaa ttgtggatga 6360
   aaaccacagt attctaaagc tgatgcttca tctgtaattg tagcaccttc atttaaagca 6420
   cgtgttaaac aatcatgtaa taattcacgt ggaaaaaatt gtggtgttaa agcgtgccat 6480
   aaaccattac gatcaactgt atgagcaata gcatttttac ctggttcagc acgtttcatt 6540
   gtatcacgaa ctggagcagc taaaatacca cctgtacgtg atgtttctga taaagctaat 6600
   aaacgagcta aatcatcttg atgtaaacat ggacgagcag catcatgaac taaaacccat 6660
   tgagcatcac cagcagcttt taaaccagct aaaactgaat cagcacgttc atcaccacca 6720
   tcaacaactg taatttgtgg atgattagct aatggtaatt gagcaaaacg tgaatcacct 6780
   ggtgaaatag caataacaac acgtttaaca cgtggatgag ctaataaagc atgaactgaa 6840
   tgttctaaaa ttgtttgatt accaattgat aagtattgtt ttggacattc tgtttgcata 6900
   cgacgaccaa aaccagcagc aggaacaaca gcacaaacat ctaaatgtgt tgtagcacct 6960
   ttgtcgtcat cgtctttata atccatatgg aattgttcaa aaaaagttaa aaacttttat 7020
   ctttctccat ttttctcttt aaaatgactt tttgcttttt tctttttttt ttgcaatgca 7080
   aaaaaaagaa aaaaacaaat gagaaaaatg aatttatgga aatctacctt taggtctaaa 7140
   tttaaacttc tctatatttt tttgttttgt ttaaattttt ttttcttctc tgaaatagaa 7200
   ttatatcata aaattaaaac aaaaaaattt tttgtatctt ttttttgatc aaaaaaaaag 7260
   ctctgcactt ttcttttttc ttttttaatg gaagaagtaa aaaacatttc ttccattaaa 7320
   aaaaattatc cttgacgttg tttatgttta ggatttgtac aaataaccat tactgtacct 7380
   tttcgacgaa ttaaacgaca tttagtacaa atttttttaa ctgaagaacg aactttcata 7440
   aaatgaattt taaaaattat tttgtgcaat ttgatgtttg cacgatttga aaacatttga 7500
   tttcttttgt gctagcacta gaggccggcc actagtctcg agcccgtgct ggttgtcatt 7560
   gcctctggat aatttttctc gaactatgcc tgcgcgttga taccaatcca atggatctac 7620
   aggcagaacg gcctctagcg gtttttttta cttttgcttt ttttgctttt gttcaaagaa 7680
   aaaaaaatac aaaataaaaa aaactaaaat gaaaaaacaa agaattctaa aattcataaa 7740
   aaaaattaaa acccaatttt ttttttggaa acttttccaa ataataaaaa aatcaaaaaa 7800
   aaatttttct agtatttttt tcatattttg aaactttttt tgagtttata aaaaaataga 7860
   aaaaacaaat agatgaaaat ttagaaaaat tataaaccaa taaaaatgaa gttttgcgta 7920
   gaaaaaaaat ttagtttact tgttccccaa gagcaagtgg taactttgaa aaaaatattt 7980
   aaacttaaaa atttgctaaa gttttgaatt tatgttaaaa tttaaaaaaa ataaaaattt 8040
   ttaaactatt tttttatgtt aaaaaaatag tttttattat tttctataat atagtttagt 8100
   tttttatttt tttcaatttc tttttttttt tcaaagaaaa aagttttcca cggatagatt 8160
   tttataggat cgacaaaatg ttctatgaac ttttcataat ggagaaaaaa atcactggat 8220
   ataccaccgt tgatatatcc caatggcatc gtaaagaaca ttttgaggca tttcagtcag 8280
   ttgctcaatg tacctataac cagaccgttc agctggatat tacggccttt ttaaagaccg 8340
   taaagaaaaa taagcacaag ttttatccgg cctttattca cattcttgcc cgcctgatga 8400
   atgctcatcc ggagttccgt atggcaatga aagacggtga gctggtgata tgggatagtg 8460
   ttcacccttg ttacaccgtt ttccatgagc aaactgaaac gttttcatcg ctctggagtg 8520
   aataccacga cgattccggc agtttctaca catatattcg caagatgtgg cgtgttacgg 8580
   tgaaaacctg gcctatttcc ctaaagggtt tattgagaat atgtttttcg tcagcgccaa 8640
   tccctgggtg agtttcacca gttttgattt aaacgtggcc aatatggaca acttcttcgc 8700
   ccccgttttc actatgggca aatattatac gcaaggcgac aaggtgctga tgccgctggc 8760
   gattcaggtt catcatgccg tttgtgatgg cttccatgtc ggcagaatgc ttaatgaatt 8820
   acaacagtac tgcgatgagt ggcagggcgg ggcgtaacct agatatttga gaatttgtat 8880
   ttaaaactga aaaatttttg aacgaactct tttcaaaaat attaaacttt cttgagatga 8940
   tttagtgtta tctcaagaaa gtttgttctt ttatttttta aaatttttaa aaattttatt 9000
   ttcttttaaa caggaaaata aataaagaaa aaagtgaatt aaaaaaaagc tgggactttc 9060
   aaagtgacca attttttact ttaaagtttt tttttattca ataaaaaaat actaaaaaaa 9120
   tatgaaagta ttacttaaaa tttcttaaaa aaaaaagaat gccttttttc aaaaaaaagt 9180
   ttaaaaaaaa taaagttttt acgtattgtt taaaactttt tttgaaaaaa gcattctttt 9240
   ttcatttaaa gagttatctt ttttatctcg tgcaagtttt ggaaattcat ttttgttaaa 9300
   taactttgac tttttatttc ttaaattttt ggcttttcat tttttttggt ttacaaataa 9360
   aactagaggc cggccctgca gttaattaag gatccactag tatttaaatt cctgatgcgg 9420
   tattttctcc ttacgcatct gtgcggtatt tcacaccgca tatcgaccgg tcgaggagaa 9480
   cttctagtat atctacatac ctaatattat tgccttatta aaaatggaat cccaacaatt 9540
   acatcaaaat ccacattctc ttcaaaatca attgtcctgt acttccttgt tcatgtgtgt 9600
   tcaaaaacgt tatatttata ggataattat actctatttc tcaacaagta attggttgtt 9660
   tggccgagcg gtctaaggcg cctgattcaa gaaatatctt gaccgcagtt aactgtggga 9720
   atactcaggt atcgtaagat gcaagagttc gaatctctta gcaaccatta tttttttcct 9780
   caacataacg agaacacaca ggggcgctat cgcacagaat caaattcgat gactggaaat 9840
   tttttgttaa tttcagaggt cgcctgacgc atataccttt ttcaactgaa aaattgggag 9900
   aaaaaggaaa ggtgagagcg ccggaaccgg cttttcatat agaatagaga agcgttcatg 9960
   actaaatgct tgcatcacaa tacttgaagt tgacaatatt atttaaggac ctattgtttt 10020
   ttccaatagg tggttagcaa tcgtcttact ttctaacttt tcttaccttt tacatttcag 10080
   caatatatat atatatattt caaggatata ccattctaat gtctgcccct aagaagatcg 10140
   tcgttttgcc aggtgaccac gttggtcaag aaatcacagc cgaagccatt aaggttctta 10200
   aagctatttc tgatgttcgt tccaatgtca agttcgattt cgaaaatcat ttaattggtg 10260
   gtgctgctat cgatgctaca ggtgttccac ttccagatga ggcgctggaa gcctccaaga 10320
   aggctgatgc cgttttgtta ggtgctgtgg gtggtcctaa atggggtacc ggtagtgtta 10380
   gacctgaaca aggtttacta aaaatccgta aagaacttca attgtacgcc aacttaagac 10440
   catgtaactt tgcatccgac tctcttttag acttatctcc aatcaagcca caatttgcta 10500
   aaggtactga cttcgttgtt gtcagagaat tagtgggagg tatttacttt ggtaagagaa 10560
   aggaagacga tggtgatggt gtcgcttggg atagtgaaca atacaccgtt ccagaagtgc 10620
   aaagaatcac aagaatggcc gctttcatgg ccctacaaca tgagccacca ttgcctattt 10680
   ggtccttgga taaagctaat gttttggcct cttcaagatt atggagaaaa actgtggagg 10740
   aaaccatcaa gaacgaattc cctacattga aggttcaaca tcaattgatt gattctgccg 10800
   ccatgatcct agttaagaac ccaacccacc taaatggtat tataatcacc agcaacatgt 10860
   ttggtgatat catctccgat gaagcctccg ttatcccagg ttccttgggt ttgttgccat 10920
   ctgcgtcctt ggcctctttg ccagacaaga acaccgcatt tggtttgtac gaaccatgcc 10980
   acggttctgc tccagatttg ccaaagaata aggtcaaccc tatcgccact atcttgtctg 11040
   ctgcaatgat gttgaaattg tcattgaact tgcctgaaga aggtaaggcc attgaagatg 11100
   cagttaaaaa ggttttggat gcaggtatca gaactggtga tttaggtggt tccaacagta 11160
   ccaccgaagt cggtgatgct gtcgccgaag aagttaagaa aatccttgct taaaaagatt 11220
   ctcttttttt atgatatttg tacataaact ttataaatga aattcataat agaaacgaca 11280
   cgaaattaca aaatggaata tgttcatagg gtagacgaaa ctatatacgc aatctacata 11340
   catttatcaa gaaggagaaa aaggaggatg taaaggaata caggtaagca aattgatact 11400
   aatggctcaa cgtgataagg aaaaagaatt gcactttaac attaatattg acaaggagga 11460
   gggcaccaca caaaaagtta ggtgtaacag aaaatcatga aactatgatt cctaaggtaa 11520
   gtcggtttaa gacaatggga aaagttagat gcctagagta ttgattatcg agcaaatatc 11580
   ttctcatctg tgatctccta gtgctagcta aagaagttgt tagattttat attctattat 11640
   aaaatataaa taatattttt taggcccata actcagttgg tagagtgatt gccttacaag 11700
   caataggtca tcggttcaag tccggttggg cctataaata cttttaaaaa gtgtcaattt 11760
   aaccgcttgc tccatacact ttttataact tttttgcttt gctgcttttt ttaaaaaaaa 11820
   gcataaaagt agtaaaaaat catttgtcat gagcaaaata ttaaaaaacc attctttaaa 11880
   taaaattttt tttattattc tgtttaaaaa aatttttgtt tgtaaaaaaa aagaaataaa 11940
   taaaaccaaa ataaaaaaat tttttctttt ttttaaaata aattttgtta aaaattttat 12000
   tttaagttca ataaaagttt tttaattttt taatttttga taaagtaaag ttacttggaa 12060
   aattggttta tacagaaaaa attataaatt atttattcat atggattata aagatgatga 12120
   cgacaaaggt atgcacaagt tcacaggtgt taacgctaaa ttccagcaac cagcattaag 12180
   aaatttatct ccagtggtag ttgagcgcga acgtgaggaa tttgtaggat tctttccaca 12240
   aattgttcgt gacttaactg aagatggtat tggtcatcca gaagtaggtg acgctgtagc 12300
   tcgtcttaaa gaagtattac aatacaacgc acctggtggt aaatgcaata gaggtttaac 12360
   agttgttgca gcttaccgtg aactttctgg accaggtcaa aaagacgctg aaagtcttcg 12420
   ttgtgcttta gcagtaggat ggtgtattga attattccaa gcctttttct tagttgctga 12480
   cgatataatg gaccagtcat taactagacg tggtcaatta tgttggtaca agaaagaagg 12540
   tgttggttta gatgcaataa atgattcttt tcttttagaa agctctgtgt atcgcgttct 12600
   taaaaagtat tgccgtcaac gtccatatta tgtacattta ttagagcttt ttcttcaaac 12660
   agcttaccaa acagaattag gacaaatgtt agatttaatc actgctcctg tatctaaggt 12720
   agatttaagc catttctcag aagaacgtta caaagctatt gttaagtata aaactgcttt 12780
   ctattcattc tatttaccag ttgcagcagc tatgtatatg gttggtatag attctaaaga 12840
   agaacatgaa aacgcaaaag ctattttact tgagatgggt gaatacttcc aaattcaaga 12900
   tgattattta gattgttttg gcgatcctgc tttaacaggt aaagtaggta ctgatattca 12960
   agataacaaa tgttcatggt tagttgtgca atgcttacaa agagtaacac cagaacaacg 13020
   tcaactttta gaagataatt acggtcgtaa agaaccagaa aaagttgcta aagttaaaga 13080
   attatatgag gctgtaggta tgagagccgc ctttcaacaa tacgaagaaa gtagttaccg 13140
   tcgtcttcaa gagttaattg agaaacattc taatcgttta ccaaaagaaa ttttcttagg 13200
   tttagctcag aaaatataca aacgtcaaaa atcaggtcca agatcttaat ctagaaattt 13260
   ccattttttt catttttttt tagaaaagtt gtatttttct tgatgaaatg aaaatttcaa 13320
   aaaagaaaaa tacaattttt ctctactttt ttttgattct ttactttttt ttgaattttt 13380
   tttgtgcttc gtttttgaaa aaaacttttt atttgaaaaa ttttgttgaa ttaaaaaaaa 13440
   tcaactcaat acattttttt tgaacttctt tttacttttt tgctttgttg cttttctttt 13500
   catttttatc attttttgct tcgctgcttt ttatcatttt ttgctttgca aaaaatgaag 13560
   aagaaaaaaa gcgtaaaatg aaaaagaaaa aaagtttcaa agaaaaaaaa gcgaaaaagc 13620
   aagagaagat aaaaaacaag aacaaaaaaa ttgttcaaaa acacaataga aaattcttta 13680
   aaaatttttt gattttttat agaatttgtg agaaatagta aaaaaagtca aaaaatcaca 13740
   acattataaa tagaaaaaat cttgttttgt aaaaatttat aattttttat attatttcaa 13800
   ttttaaataa gatcttttgg agctgaaaaa atatgagaaa tagtttggac aaactagtct 13860
   cgagcccgta tgatattcta aggcgttacg ctgatgaata ttctacagag ttgccatagg 13920
   cgttgaacgc tacacggacg atacgaaagc agttgctttc tcctatggga agagctttct 13980
   aagtctgaag aagtaaacag ttctttgcta tttcacactt cctggttgat ggtcacttgc 14040
   tgcctgaaat atatatatat gtatgacata tgtacttgtt ttcttttttg tgcctttgtt 14100
   acgtctatat tcattgaaac tgattattcg attttcttct tgctgaccgc ttctagaggc 14160
   atcgcacagt tttagcgagg aaaactcttc aatagttttg ccagcggaat tccacttgca 14220
   attacataaa aaattccggc ggtttttcgc gtgtgactca atgtcgaaat acctgcctaa 14280
   tgaacatgaa catcgcccaa atgtatttga agacccgctg ggagaagttc aagatatata 14340
   agtaacaagc agccaatagt ataaaaaaaa atctgagttt attacctttc ctggaatttc 14400
   agtgaaaaac tgctaattat agagagatat cacagagtta ctcactaatg actaacgaaa 14460
   aggtctggat agagaagttg gataatccaa ctctttcagt gttaccacat gactttttac 14520
   gcccacaaca agaaccttat acgaaacaag ctacatattc gttacagcta cctcagctcg 14580
   atgtgcctca tgatagtttt tctaacaaat acgctgtcgc tttgagtgta tgggctgcat 14640
   tgatatatag agtaaccggt gacgatgata ttgttcttta tattgcgaat aacaaaatct 14700
   taagattcaa tattcaacca acgtggtcat ttaatgagct gtattctaca attaacaatg 14760
   agttgaacaa gctcaattct attgaggcca atttttcctt tgacgagcta gctgaaaaaa 14820
   ttcaaagttg ccaagatctg gaaaggaccc ctcagttgtt ccgtttggcc tttttggaaa 14880
   accaagattt caaattagac gagttcaagc atcatttagt ggactttgct ttgaatttgg 14940
   ataccagtaa taatgcgcat gttttgaact taatttataa cagcttactg tattcgaatg 15000
   aaagagtaac cattgttgcg gaccaattta ctcaatattt gactgctgcg ctaagcgatc 15060
   catccaattg cataactaaa atctctctga tcaccgcatc atccaaggat agtttacctg 15120
   atccaactaa gaacttgggc tggtgcgatt tcgtggggtg tattcacgac attttccagg 15180
   acaatgctga agccttccca gagagaacct gtgttgtgga gactccaaca ctaaattccg 15240
   acaagtcccg ttctttcact tatcgcgaca tcaaccgcac ttctaacata gttgcccatt 15300
   atttgattaa aacaggtatc aaaagaggtg atgtagtgat gatctattct tctaggggtg 15360
   tggatttgat ggtatgtgtg atgggtgtct tgaaagccgg cgcaaccttt tcagttatcg 15420
   accctgcata tcccccagcc agacaaacca tttacttagg tgttgctaaa ccacgtgggt 15480
   tgattgttat tagagctgct ggacaattgg atcaactagt agaagattac atcaatgatg 15540
   aattggagat tgtttcaaga atcaattcca tcgctattca agaaaatggt accattgaag 15600
   gtggcaaatt ggacaatggc gaggatgttt tggctccata tgatcactac aaagacacca 15660
   gaacaggtgt tgtagttgga ccagattcca acccaaccct atctttcaca tctggttccg 15720
   aaggtattcc taagggtgtt cttggtagac atttttcctt ggcttattat ttcaattgga 15780
   tgtccaaaag gttcaactta acagaaaatg ataaattcac aatgctgagc ggtattgcac 15840
   atgatccaat tcaaagagat atgtttacac cattattttt aggtgcccaa ttgtatgtcc 15900
   ctactcaaga tgatattggt acaccgggcc gtttagcgga atggatgagt aagtatggtt 15960
   gcacagttac ccatttaaca cctgccatgg gtcaattact tactgcccaa gctactacac 16020
   cattccctaa gttacatcat gcgttctttg tgggtgacat tttaacaaaa cgtgattgtc 16080
   tgaggttaca aaccttggca gaaaattgcc gtattgttaa tatgtacggt accactgaaa 16140
   cacagcgtgc agtttcttat ttcgaagtta aatcaaaaaa tgacgatcca aactttttga 16200
   aaaaattgaa agatgtcatg cctgctggta aaggtatgtt gaacgttcag ctactagttg 16260
   ttaacaggaa cgatcgtact caaatatgtg gtattggcga aataggtgag atttatgttc 16320
   gtgcaggtgg tttggccgaa ggttatagag gattaccaga attgaataaa gaaaaatttg 16380
   tgaacaactg gtttgttgaa aaagatcact ggaattattt ggataaggat aatggtgaac 16440
   cttggagaca attctggtta ggtccaagag atagattgta cagaacgggt gatttaggtc 16500
   gttatctacc aaacggtgac tgtgaatgtt gcggtagggc tgatgatcaa gttaaaattc 16560
   gtgggttcag aatcgaatta ggagaaatag atacgcacat ttcccaacat ccattggtaa 16620
   gagaaaacat tactttagtt cgcaaaaatg ccgacaatga gccaacattg atcacattta 16680
   tggtcccaag atttgacaag ccagatgact tgtctaagtt ccaaagtgat gttccaaagg 16740
   aggttgaaac tgaccctata gttaagggct taatcggtta ccatctttta tccaaggaca 16800
   tcaggacttt cttaaagaaa agattggcta gctatgctat gccttccttg attgtggtta 16860
   tggataaact accattgaat ccaaatggta aagttgataa gcctaaactt caattcccaa 16920
   ctcccaagca attaaatttg gtagctgaaa atacagtttc tgaaactgac gactctcagt 16980
   ttaccaatgt tgagcgcgag gttagagact tatggttaag tatattacct accaagccag 17040
   catctgtatc accagatgat tcgtttttcg atttaggtgg tcattctatc ttggctacca 17100
   aaatgatttt taccttaaag aaaaagctgc aagttgattt accattgggc acaattttca 17160
   agtatccaac gataaaggcc tttgccgcgg aaattgacag aattaaatca tcgggtggat 17220
   catctcaagg tgaggtcgtc gaaaatgtca ctgcaaatta tgcggaagac gccaagaaat 17280
   tggttgagac gctaccaagt tcgtacccct ctcgagaata ttttgttgaa cctaatagtg 17340
   ccgaaggaaa aacaacaatt aatgtgtttg ttaccggtgt cacaggattt ctgggctcct 17400
   acatccttgc agatttgtta ggacgttctc caaagaacta cagtttcaaa gtgtttgccc 17460
   acgtcagggc caaggatgaa gaagctgcat ttgcaagatt acaaaaggca ggtatcacct 17520
   atggtacttg gaacgaaaaa tttgcctcaa atattaaagt tgtattaggc gatttatcta 17580
   aaagccaatt tggtctttca gatgagaagt ggatggattt ggcaaacaca gttgatataa 17640
   ttatccataa tggtgcgtta gttcactggg tttatccata tgccaaattg agggatccaa 17700
   atgttatttc aactatcaat gttatgagct tagccgccgt cggcaagcca aagttctttg 17760
   actttgtttc ctccacttct actcttgaca ctgaatacta ctttaatttg tcagataaac 17820
   ttgttagcga agggaagcca ggcattttag aatcagacga tttaatgaac tctgcaagcg 17880
   ggctcactgg tggatatggt cagtccaaat gggctgctga gtacatcatt agacgtgcag 17940
   gtgaaagggg cctacgtggg tgtattgtca gaccaggtta cgtaacaggt gcctctgcca 18000
   atggttcttc aaacacagat gatttcttat tgagattttt gaaaggttca gtccaattag 18060
   gtaagattcc agatatcgaa aattccgtga atatggttcc agtagatcat gttgctcgtg 18120
   ttgttgttgc tacgtctttg aatcctccca aagaaaatga attggccgtt gctcaagtaa 18180
   cgggtcaccc aagaatatta ttcaaagact acttgtatac tttacacgat tatggttacg 18240
   atgtcgaaat cgaaagctat tctaaatgga agaaatcatt ggaggcgtct gttattgaca 18300
   ggaatgaaga aaatgcgttg tatcctttgc tacacatggt cttagacaac ttacctgaaa 18360
   gtaccaaagc tccggaacta gacgatagga acgccgtggc atctttaaag aaagacaccg 18420
   catggacagg tgttgattgg tctaatggaa taggtgttac tccagaagag gttggtatat 18480
   atattgcatt tttaaacaag gttggatttt tacctccacc aactcataat gacaaacttc 18540
   cactgccaag tatagaacta actcaagcgc aaataagtct agttgcttca ggtgctggtg 18600
   ctcgtggaag ctccgcagca gcttaaggtt gagcattacg tatgatatgt ccatgtacaa 18660
   taattaaata tgaattagga gaaagactta gcttcttttc gggtgatgtc acttaaaaac 18720
   tccgagaata atatataata agagaataaa atattagtta ttgaataaga actgtaaatc 18780
   agctggcgtt agtctgctaa tggcagcttc atcttggttt attgtacttt caacttctag 18840
   aggagaaaag tattgacatg agcgctcccg gcacaacggc caaagaagtc tccaatttct 18900
   tatttctttt tgaattagat aaatgagtgt tctcaatttt tttttctttg cattttttgt 18960
   ttgtgttgat ttacaaaaac aatagaaaaa agaaaacaat attttctttc taaaaaaaaa 19020
   caaaattgat gaaaaataga catgaacaaa aaattttgaa agttgacttt tttaaaaaat 19080
   ttttggtata atacaaaaaa agaatttttg gaaaggtggc agagtggttg aatgctctgg 19140
   ttttgaaaac cagcgtggct ttacggtcac cgggggttcg aatccctccc tttccgataa 19200
   tatatacaaa aatttttaaa gttttttgtt tattttgtat agataaaaaa tctgcaataa 19260
   aaatttcgtt ttttatttat tcaaaaattc tgtttttttg aaaagaaaat aaaaaaaatg 19320
   ccaaaagtga gttttttatt caaatattag aaaaagtttt tgaaaaattt aaaaaaatag 19380
   aaaaaatttt tttatttttt tcataattta aaaaattatg ttataattta aattacaaat 19440
   aggttttatt aaaaaatttt tacgtacaga tgaattctat aaaattattt tggagatcac 19500
   catatggtac caacatcaat tcttaatact gtatcaacta ttcacagttc tcgtgtaact 19560
   tctgttgatc gtgttggtgt tttaagttta agaaattctg attcagttga atttacacgt 19620
   cgtcgtagtg gatttagtac tcttatttac gaatcacctg gtagacgttt tgtagtacgt 19680
   gcagctgaaa ctgatacaga taaagtaaaa tctcaaactc ctgataaagc tcctgcaggt 19740
   ggttcttcaa ttaaccaact tttaggaata aaaggtgcta gtcaagaaac aaacaaatgg 19800
   aaaatacgtt tacaacttac taaaccagta acatggccac ctttagtatg gggtgtagtt 19860
   tgtggtgctg ctgcatcagg caacttccat tggacaccag aagatgtagc taaaagtatt 19920
   ttatgtatga tgatgtctgg tccttgttta acaggttata cacaaactat taatgattgg 19980
   tatgacagag acattgatgc aatcaatgaa ccttaccgtc ctataccaag tggtgctatt 20040
   tcagaaccag aagttattac acaagtttgg gttcttttac ttggtggtct tggaattgct 20100
   ggtatcttag atgtatgggc tggtcataca acacctacag tattctatct tgctttagga 20160
   ggtagtttac tttcttacat ttactcagct ccacctttaa aacttaaaca aaatggttgg 20220
   gttggtaatt tcgctttagg tgcttcatat atatcattac cttggtgggc aggtcaagct 20280
   ctttttggca cattaacacc agacgttgtt gtacttactc ttctttacag tatagctggc 20340
   ttaggtatcg caattgtaaa tgactttaaa tcagttgaag gtgatagagc acttggttta 20400
   cagagtttac cagttgcttt tggaacagaa actgctaaat ggatatgtgt tggcgctatt 20460
   gacataacac aattaagtgt agcaggttac ttacttgcta gtggtaaacc atattacgct 20520
   ttagctttag tagctttaat cattcctcaa attgtttttc agttcaaata ctttcttaaa 20580
   gatcctgtaa aatacgacgt aaaatatcaa gcatctgctc aaccattttt agttttaggt 20640
   attttcgtaa ctgctcttgc aagtcaacac ggtaccggtg attataaaga cgatgatgac 20700
   aaatcaggtg aaaacttata ctttcaaggt cacaatcatc gtcacaaaca caccggttaa 20760
   tctagatttt attttttatg aaaaactcag gcttaattta ggcttgagtt tttcattctt 20820
   tttgaagctc tgaaatttta aaatttctag tcttctttaa tgtttttaaa ttttaaaaaa 20880
   taaatttctt ctctgctgtg tttttctttt tttttgaaaa aacaaagaaa aaaaattttt 20940
   ttgttttctt ctttgttttt ttatttcttt ttgttttgtt tattttttag tttcagaatc 21000
   tttgattcaa aaaaaaattt agtccgatta ctccatagga gcaagcagta aaaaataaaa 21060
   actgtaataa aaaataaaac aaaaatttta tttctttttg ttttgcttga acttttcaaa 21120
   aaaaaattga aaaattcaag caaaacaaaa agaaacaaat aaaaaattta tgaattttct 21180
   actttttcag gagttgaaat ttctccttta cttaaaacat attttgctaa aaaaagcgct 21240
   tgtgttgctt tttttgctac tttttgtttc caagcatttt ttcgaatatt tttttttgat 21300
   tttgatgtgc gtttttgtta acctaaaatc ttgaaaagat ttactctttt caaattttta 21360
   tgtttttatt ttttttattc ataaaaaaaa acaatacata aaaataaagt atttcggctt 21420
   caaaaaattt tatacaaaaa gttttttgat taaaaactca gaaaaaataa aaaaacaaag 21480
   tatgaatttt ttgaaaaatt catacctttt atttttttgt aatttttagc ctttcaaaaa 21540
   atttttgaag gcattttttt tttaatcctc atgttcttca aaaggatctc tcaatttttt 21600
   tgaaggaggt ccaaaactca catagattga atatcctgtt gcacttaata atagaaacca 21660
   taaaaaaaag gtaaagaaaa aagcaggact gtccataatt ctttcatgtt ttttgttcaa 21720
   atttattctc caataattat attacgacaa aaagtaaaaa aaatcaaaat ttattcaaaa 21780
   aaatggctac tggaacaact tcaaaagcta aatcaagctt atctgatgca cttcaagaac 21840
   caggtatcgt aactccttta ggaactttat taagaccgtt aaactctgaa tcaggaaaag 21900
   tattacctgg atggggaaca actgttttaa tgggtgtttt cattgtactt tttgctgtat 21960
   tcttattaat tattttagaa atttataaca gttctttatt attagataat gttactatga 22020
   gttgggaaac tttagcttct taattcaata gaatagtttt attgcttttt ttatttttta 22080
   ttttatcaaa aatttttttt gcaaaaataa agaataaata aaattcaaaa aaattataga 22140
   attagataaa attagtttca agttgaacta agttgtcaat aaactttcaa atttgttttc 22200
   tttttactgt tcattaagag caataaaaaa aacttttggt cttggcaatc ttttaaaaaa 22260
   gtcagaatca attctatttt aagaatccta tggaatctat gtatttaatt ttagcaaaat 22320
   taccagaagc ttatgcacct tttgatccta ttgtagatgt tttaccaatt attcctattt 22380
   tcttcttatt attagccttt gtatggcaag catctgtaag ttttagataa aaaatttaaa 22440
   agtttttttt gatacttttg taaaaaatat caaaaaaaac ttttaaattt ttttcaattt 22500
   tcattagcaa ctttagcttt aatattagct aaagttgctc tcaaaaatat aatttttttt 22560
   tgacttttta tttttttatt ttgtttcttt tttaaaagtt acaacataaa gaaaatgaaa 22620
   atagaaaatt tgtgaaacat aaaaaaaaag aatgaaattt ttatgttcgt tttttgtttt 22680
   atcttttcca actaaagtcg gcctctagct agaggccggc caaatttttt tccaaaattc 22740
   tataaaaaat caaaaaatta aaaaaaaaag aaaaaacttt gttttgtgca aaacaaaaat 22800
   cttgaattca aaacaaaata aagaattcaa aaagattttt tttaagcaaa aggtaaaatg 22860
   gaaaaaaatg tttttaaaaa aattttttct tttttttaaa gctttgcttt tttcatgaaa 22920
   aaaacaaagc tttaaaaaaa agaaaaaatt ttaaagcaaa aaaaagaatt aaacacgtct 22980
   tttttttgga ggacgacatc cattatgtgg aattcctgtt ttttctcgaa taacatttac 23040
   tttaattcca gctttaaaaa tttcacgaat agctgtttcg cgaccttgtc ctggaccagt 23100
   tactaaaatt tttgcttcat ttaatgcaaa ttcacgtgat ttttttgcca caacttcagc 23160
   agcttttttt gctgcaaatg ttgttgcttt tctttttcca cggaaaccac aagctccagc 23220
   agaactccaa caaaggactt caccacgaag atttgctaat gtaataatag tattatgatg 23280
   tccagcttga atataaacaa ttcctcgata tgtacgtttt ttaatttttg taggtgatac 23340
   ttttctagtt tgtctagcca tatgtaaaaa tttaactata aatttctttt atatttttaa 23400
   atcatttgat ttactatcta aaaaaaataa gattttgaat ctttggaaag aaccattatt 23460
   tggaaagatt tatttgttca attcttttgt attttttttc aaaaaaattt tttagaattt 23520
   tttatctaat tttttaatgt tttgttcaaa cataaaaaat tttcttttca aaaaaggaaa 23580
   aaaaatttct tttttttgaa aaagtaaaaa taaaaaaagc tgtagctttt ttgttgaatc 23640
   aaaaaaaaat aagaaattgt cctattttta tgacaaaaga ttcaaaaaaa tgaaataaaa 23700
   aagaaaaatt caaactttca aacaatgtat tttgtatttt tgggccgagt cggattcgaa 23760
   ccaacgtagg cgtaaccagc ggatttacaa tccgccccca ttaaccactc gggcatcggc 23820
   ccatgctttt ttttgactca tttgatcatt tattttgcat gaattatact aaagattata 23880
   ttaacaaaaa atttttgaaa tttcaatttt ttttaattaa aaactcttct atttttttaa 23940
   aattctttgt tttgaatttt ttttttcaat ttaaaaaaaa aacatattaa aaaatatttt 24000
   taaaaaattt tttgtattga aaacttacaa taattttata aatttttttg aaaaattttg 24060
   ttttttttat tctattgaaa tgaacaaaac aaattttttt agtttttttt gtttttttgc 24120
   tttgctgctt cttttgtttt tatcaataaa taaatgaaaa tgaaaaacaa aaataaacaa 24180
   tttgtgtttt tagagttcta aaatcaagaa aaaaatactt cccctttaaa gaggaagtat 24240
   tttaaaaaaa aattatagtt tgtcaatagt ttcaaattca aatttgattt ctttccaaac 24300
   ttcacaagca gcagctaatt ctggagacca tttacaagct gagcggataa catcaccacc 24360
   ttcacgagct aaatcacgac cttcgttacg agcttgagta caagcttcta aagcaacacg 24420
   gttagcaaca gcaccaggag cgttacccca agggtgtcct aaagtacctc caccgaattg 24480
   taaacaagcg tcatcaccaa agatttcaac taaagctggc atgtgccata cgtgaatacc 24540
   accagaagca actggcatag taccacccat agaacaccag tcttgagtga agtaaatacc 24600
   acggcta 24607
<210> 121
   <211> 1529
   <212> DNA
   <213> Artificial Sequence
<220>
   <223> codon optimized sequence comprising IS57
<400> 121
   atggtaccaa cattaaattc attatctcca gcagaatcaa aagcaatttc atttttagat      60
   acttctcgtt tcaatccaat tcctaaatta tctggtggtt tttctttacg tcgtcgtaat     120
   caaggtcgtg gttttggtaa aggagtaaaa tgctctgtaa aagtacagca acagcaacaa     180
   ccaccacctg cttggccagg tcgtgctgtt ccagaagctc ctagacaatc atgggatggt     240
   cctaaaccta ttagtattgt aggatcaact ggttcaatcg gtactcaaac tttagacatt     300
   gttgcagaaa atccagataa atttcgtgta gttgcattag ctgctggttc taacgttact     360
   ttattagctg atcaggtacg tcgttttaaa cctgctttag ttgcagttcg taatgaatct     420
   ttaattaatg aattaaaaga agcattagca gatttagact acaaattaga gattattcca     480
   ggagaacaag gtgtaattga agttgctcgt catccagaag cagttacagt agttacaggt     540
   attgttggtt gtgctggatt aaaacctaca gttgcagcta ttgaggctgg taaagatatt     600
   gctttagcaa ataaagaaac attaatcgca ggtggtccat tcgtattacc acttgcaaac     660
   aaacacaatg taaaaattct tccagcagac agtgaacatt cagcaatttt tcaatgtatt     720
   caaggcttac ctgaaggtgc attaagaaaa atcatattaa cagcttcagg tggtgcattt     780
   cgtgattggc ctgtagaaaa acttaaagag gtaaaagtag ctgatgcatt aaaacaccca     840
   aactggaata tgggcaaaaa aattactgta gatagtgcaa ctttattcaa caaaggttta     900
   gaagttattg aagcacatta tcttttcggt gctgaatacg acgatattga aattgttatt     960
   catccacaat caattattca tagtatgatt gaaacacaag attcttcagt attagctcaa    1020
   ttaggctggc ctgacatgcg tttacctatt ttatatacaa tgtcatggcc agaccgtgtt    1080
   ccttgcagtg aagttacttg gccacgttta gatttatgta aattaggatc tcttacattc    1140
   aaaaaacctg acaacgtaaa atatccatca atggatcttg cttatgcagc tggtcgtgca    1200
   ggtggtacta tgactggtgt tttatctgct gctaatgaga aagctgtaga aatgttcatt    1260
   gacgagaaaa tttcatactt agacatcttc aaagtagttg aattaacatg tgataaacac    1320
   cgtaatgaat tagtaacttc tccaagtctt gaagaaattg tacattatga cttatgggct    1380
   cgtgagtatg ctgcaaatgt tcaattatca agtggagctc gtccagttca tgctggtacc    1440
   ggtgattaca aagatgatga tgataaaagt ggtgagaact tatactttca aggtcacaat    1500
   catcgtcata aacacaccgg ttaatctag                                      1529
<210> 122
   <211> 749
   <212> DNA
   <213> Artificial Sequence
<220>
   <223> codon optimized sequence comprising IS116
<400> 122
   atggattata aagacgatga cgacaaaggt gctacaacac atttagatgt ttgtgctgtt      60
   gttcctgctg ctggttttgg tcgtcgtatg caaacagaat gtccaaaaca atacttatca     120
   attggtaatc aaacaatttt agaacattca gttcatgctt tattagctca tccacgtgtt     180
   aaacgtgttg ttattgctat ttcaccaggt gattcacgtt ttgctcaatt accattagct     240
   aatcatccac aaattacagt tgttgatggt ggtgatgaac gtgctgattc agttttagct     300
   ggtttaaaag ctgctggtga tgctcaatgg gttttagttc atgatgctgc tcgtccatgt     360
   ttacatcaag atgatttagc tcgtttatta gctttatcag aaacatcacg tacaggtggt     420
   attttagctg ctccagttcg tgatacaatg aaacgtgctg aaccaggtaa aaatgctatt     480
   gctcatacag ttgatcgtaa tggtttatgg cacgctttaa caccacaatt ttttccacgt     540
   gaattattac atgattgttt aacacgtgct ttaaatgaag gtgctacaat tacagatgaa     600
   gcatcagctt tagaatactg tggttttcat ccacaattag ttgaaggtcg tgctgataac     660
   attaaagtta cacgtccaga agatttagct ttagctgaat tttatttaac acgtacaatt     720
   catcaagaaa atacaaccgg ttaatctag                                      749
<210> 123
   <211> 1151
   <212> DNA
   <213> Artificial Sequence
<220>
   <223> codon optimized sequence comprising IS62
<400> 123
   atatggatta taaagatgat gacgacaaag gtatgcacaa gttcacaggt gttaacgcta      60
   aattccagca accagcatta agaaatttat ctccagtggt agttgagcgc gaacgtgagg     120
   aatttgtagg attctttcca caaattgttc gtgacttaac tgaagatggt attggtcatc     180
   cagaagtagg tgacgctgta gctcgtctta aagaagtatt acaatacaac gcacctggtg     240
   gtaaatgcaa tagaggttta acagttgttg cagcttaccg tgaactttct ggaccaggtc     300
   aaaaagacgc tgaaagtctt cgttgtgctt tagcagtagg atggtgtatt gaattattcc     360
   aagccttttt cttagttgct gacgatataa tggaccagtc attaactaga cgtggtcaat     420
   tatgttggta caagaaagaa ggtgttggtt tagatgcaat aaatgattct tttcttttag     480
   aaagctctgt gtatcgcgtt cttaaaaagt attgccgtca acgtccatat tatgtacatt     540
   tattagagct ttttcttcaa acagcttacc aaacagaatt aggacaaatg ttagatttaa      600
   tcactgctcc tgtatctaag gtagatttaa gccatttctc agaagaacgt tacaaagcta     660
   ttgttaagta taaaactgct ttctattcat tctatttacc agttgcagca gctatgtata     720
   tggttggtat agattctaaa gaagaacatg aaaacgcaaa agctatttta cttgagatgg     780
   gtgaatactt ccaaattcaa gatgattatt tagattgttt tggcgatcct gctttaacag     840
   gtaaagtagg tactgatatt caagataaca aatgttcatg gttagttgtg caatgcttac     900
   aaagagtaac accagaacaa cgtcaacttt tagaagataa ttacggtcgt aaagaaccag     960
   aaaaagttgc taaagttaaa gaattatatg aggctgtagg tatgagagcc gcctttcaac    1020
   aatacgaaga aagtagttac cgtcgtcttc aagagttaat tgagaaacat tctaatcgtt    1080
   taccaaaaga aattttctta ggtttagctc agaaaatata caaacgtcaa aaatcaggtc    1140
   caagatctta a                                                          1151
<210> 124
   <211> 1257
   <212> DNA
   <213> Artificial Sequence
<220>
   <223> codon optimized sequence comprising IS61
<400> 124
   atggtaccaa catcaattct taatactgta tcaactattc acagttctcg tgtaacttct      60
   gttgatcgtg ttggtgtttt aagtttaaga aattctgatt cagttgaatt tacacgtcgt     120
   cgtagtggat ttagtactct tatttacgaa tcacctggta gacgttttgt agtacgtgca     180
   gctgaaactg atacagataa agtaaaatct caaactcctg ataaagctcc tgcaggtggt     240
   tcttcaatta accaactttt aggaataaaa ggtgctagtc aagaaacaaa caaatggaaa     300
   atacgtttac aacttactaa accagtaaca tggccacctt tagtatgggg tgtagtttgt     360
   ggtgctgctg catcaggcaa cttccattgg acaccagaag atgtagctaa aagtatttta     420
   tgtatgatga tgtctggtcc ttgtttaaca ggttatacac aaactattaa tgattggtat     480
   gacagagaca ttgatgcaat caatgaacct taccgtccta taccaagtgg tgctatttca     540
   gaaccagaag ttattacaca agtttgggtt cttttacttg gtggtcttgg aattgctggt     600
   atcttagatg tatgggctgg tcatacaaca cctacagtat tctatcttgc tttaggaggt     660
   agtttacttt cttacattta ctcagctcca cctttaaaac ttaaacaaaa tggttgggtt     720
   ggtaatttcg ctttaggtgc ttcatatata tcattacctt ggtgggcagg tcaagctctt     780
   tttggcacat taacaccaga cgttgttgta cttactcttc tttacagtat agctggctta     840
   ggtatcgcaa ttgtaaatga ctttaaatca gttgaaggtg atagagcact tggtttacag     900
   agtttaccag ttgcttttgg aacagaaact gctaaatgga tatgtgttgg cgctattgac     960
   ataacacaat taagtgtagc aggttactta cttgctagtg gtaaaccata ttacgcttta    1020
   gctttagtag ctttaatcat tcctcaaatt gtttttcagt tcaaatactt tcttaaagat    1080
   cctgtaaaat acgacgtaaa atatcaagca tctgctcaac catttttagt tttaggtatt   1140
   ttcgtaactg ctcttgcaag tcaacacggt accggtgatt ataaagacga tgatgacaaa    1200
   tcaggtgaaa acttatactt tcaaggtcac aatcatcgtc acaaacacac cggttaa       1257
<210> 125
   <211> 5240
   <212> DNA
   <213> Scenedesmus dimorphus
<400> 125
   cgtttaggtg taacacaatc ttggggtgga tggacaatta gcggtgaaac agcaacaaat      60
   ccaggtattt ggagttatga aggtgttgct gcatctcata ttattttatc tggtttatta     120
   ttcttagctt cggtttggca ctgggtttac tgggatttag agttattccg tgacccaaga     180
   actggaaaaa ctgcattaga tttaccaaaa attttcggaa ttcacttatt cttatcaggt     240
   cttttatgtt ttggttttgg tgctttccac gtaacaggtt tatttggtcc tggtatttgg     300
   gtttcagatc cttatggatt aacaggaagt gttcaaccag ttgctccttc ttggggtgct     360
   gatgggtttg atcctttcaa ccctggtggt attgcagcgc accacattgc tgctggtatt     420
   ttaggtgttt tagcaggatt attccactta tgtgtacgtc cttctattcg tttatacttt     480
   ggtttatcaa tgggtagtat cgaaacagta ttatcaagta gtattgctgc tgttttctgg     540
   gctgctttcg ttgttgctgg aactatgtgg tatggttcag cagctactcc aattgaatta     600
   tttggtccta cacgttatca atgggaccaa ggtttcttcc aacaagaaat tcaaaaacga     660
   gttcaaacaa gtttagcagg tggttcttca ctttctgatg cttggtcgaa aattccagaa     720
   aaattagctt tctatgatta tattggaaac aaccctgcaa aaggtggtct tttccgtaca     780
   ggagctatga atagtggaga tggtattgct gttggatggt taggtcacgc agtatttaaa     840
   gatcaagatg gtcgtgaatt atacgtacgt cgtatgccta ctttctttga aacattccca     900
   gttttattaa ttgataaaga tggtgttgta cgtgctgacg ttcctttccg tcgtgctgaa     960
   tcaaaatata gtattgaaca agttggtgta tcagtaactt tctacggtgg tgaattagat    1020
   ggattaacat ttaatgatcc agcaactgtt aaaaaatatg ctcgtaaagc acaattaggt    1080
   gaaatttttg aatttgatcg ttcaacatta caatctgatg gtgtattccg tagtagtcca    1140
   cgtggttggt ttacttttgg tcacgtttgc tttgctttat tattcttctt tggacatatt    1200
   tggcatggtg cacgtacaat cttccgtgat gtatttgctg gtattgatga tgatctaaac    1260
   gaaagtttag aatttggtaa atacaaaaaa cttggtgata caagttctgt tcgtgaagct    1320
   ttctaattcg tttttttctc ttttttttct tttttctctt tggaaaaaga aaaaacatgt    1380
   ttattttgaa ttttttgttt agaactttac tgttcttttt ttattttaaa gtgtttttgt    1440
   ttttttttaa tacaaaaact tttttaaaat gaatttaaaa aacacaaaaa aaagagttat    1500
   tgctattcaa aataaacaag agtttaaaaa caaagttttt ttctaaagaa aaaaacttct    1560
   tcattttttt tgaattgttt ttaaactttt ttcttctctt gcttttagag tttttttctt    1620
   cactttttgc aaaaaagtga gaaaaaacag caaagcaaaa aagtgaaaaa aagttcaaaa    1680
   acaattcaaa aaagacaaaa cctaaaaaaa tatcacttga gatgggtctg gattttttcc    1740
   aagcaaaaga attttgtatt ttgttgaaag tttttcataa aaatacaaat ttgcaattat 1800
   tattcttaaa atcaaaatat ttgttaacca catttcattc tatggaagca ttagtttata 1860
   cttttttatt aatcggaaca ttaggaatta tctttttcgc aattttcttt agagaaccac 1920
   ctcgtatggt aaaataattg aaattttgct ttttttttat ggataaaaga aatgatttca 1980
   attcatttct tttatccatt tttttgaaaa tagtttttct caatttttta ttttttttgt 2040
   tttttctcta aaaatcaaaa attcaatttt gagaaaaaat tttatacaaa aagttttttg 2100
   attaaaaact cagaaaaaag aaaaaaacaa agtatgaatt ttttgaaaaa ttcatacctt 2160
   ttattttttt tgtaattttt agcctttcaa aaaatttttg aaggcatttt ttttttaatc 2220
   ctcatgttct tcaaaaggat ctctcaattt ttttgaagga ggtccaaaac tcacatagat 2280
   tgaatatcct gttgcactta ataatagaaa ccataaaaaa aaggtaaaga aaaaagcagg 2340
   actgtccata attctttcat gttttttgtt caaatttatt ctccaataat tatattacga 2400
   cagaaagtaa aaaaaatcaa aatttattca aaaaaatggc tactggaaca acttcaaaag 2460
   ctaaatcaag cttatctgat gcacttcaag aaccaggtat cgtaactcct ttaggaactt 2520
   tattaagacc gttaaactct gaatcaggaa aagtattacc tggatgggga acaactgttt 2580
   taatgggtgt tttcattgta ctttttgctg tattcttatt aattatttta gaaatttata 2640
   acagttcttt attattagat gatgttacta tgagttggga aactttagct tcttaattca 2700
   atagaatagt tttattgctt tttttatttt ttattttatc aaaaattttt tttgcaaaaa 2760
   taaagaataa ataaaattca aaaaaattat agaattagat aaaattagtt tcaagttgaa 2820
   ctaagttgtc aataaacttt caaatttgtt ttctttttac tgttcattaa gagcaataaa 2880
   aaaaactttt ggtcttggca atcttttaaa aaagtcagaa tcaattctat tttaagaatc 2940
   ctatggaatc tatgtattta attttagcaa aattaccaga agcttatgca ccttttgatc 3000
   ctattgtaga tgttttacca attattccta ttttcttctt attattagcc tttgtatggc 3060
   aagcatctgt aagttttaga taaaaaattg aaaagttttt tttgatactt ttgtaaaaaa 3120
   tatcaaaaaa aacttttcaa tttttttcaa ttttcattcg caactttagc tttaatatta 3180
   gctaaagttg ctctcaaaaa tataattttt ttttgacttt ttattttttt attttgtttc 3240
   ttttttaaaa gttacaacat aaagaaaatg aaaatagaaa atttgtgaaa cataaaaaaa 3300
   aagaatgaaa tttttatgtt cgttttttgt tttatctttt ccaactaaag taaatttttt 3360
   ttccaaaatt ctataaaaaa tcaaaaaatt aaaaaaaaag aaaaaacttt gttttgtgca 3420
   aaacaaaaat cttgaattca aaacaaaata aagaattcaa aaagattttt tttaagcaaa 3480
   aggtaaaatg gaaaaaaatg ttttttgctt taaaattttt tctttttttt aaagctttgc 3540
   ttttttcatg aaaaaaacaa agctttaaaa aaaagaaaaa attttaaagc aaaaaaaaga 3600
   attaaacacg tctttttttt ggaggacgac atccattatg tggaattcct gttttttctc 3660
   gaataacatt tactttaatt ccagctttaa aaatttcacg aatagctgtt tcgcgccctt 3720
   gtcctggacc agttactaaa atttttgctt catttaatgc aaattcacgt gatttttttg 3780
   ccacaacttc agcagctttt tttgctgcaa atgttgttgc ttttcttttt ccacggaaac 3840
   cacaagctcc agcagaactc caacaaagga cttcaccacg aagatttgct aatgtaataa 3900
   tagtattatg atgtccagct tgaatataaa caattcctcg atatgtacgt tttttaattt 3960
   ttgtaggtga tacttttcta gtttgtctag ccatatgtaa aaatttaact ataaatttct 4020
   tttatatttt taaatcattt gatttactat ctaaaaaaaa taaaattttg aatctttgga 4080
   aagaaccatt atttggaaag atttatttgt tcaattcttt tgtatttttt ttcaaaaaaa 4140
   ttttttagaa ttttttatct aattttttaa tgttttgttc aaacataaaa aattttcttt 4200
   tcaaaaaaga aaaaaaaatt tctttttttt gaaaaaggaa aaataaaaaa agctgtagct 4260
   tttttgttga atcaaaaaaa aataagaaat tgtcctattt ttatgacaaa agattcaaaa 4320
   aaatgaaata aaaaagaaaa attcaaactt tcaaacaatg tattttgtat ttttgggccg 4380
   agtcggattc gaaccaacgt aggcgtaacc agcggattta caatccgccc ccattaacca 4440
   ctcgggcatc ggcccatgct tttttttgac tcatttaatc atttattttg catgaattat 4500
   actaaagatt atattaacaa aaaatttttg aaatttcaat tttttttaat taaaaactct 4560
   tctatttttt aaaattcttt gttttgaatt tttttttcaa tttaaaaaaa aaaaacatat 4620
   taaaaaatat ttttaaaaaa ttttttgtat tgaaaactta caataatttt ataaattttt 4680
   ttgaaaaatt ttgttttttt tattctattg aaatgaacaa aacaaatttt tttagttttt 4740
   tttgtttttt tgttttgctg cttcttttgt ttttatcaat aaataaatga aaatgaaaaa 4800
   caaaaataaa caatttatgt ttttagagtt ctaaaatcaa gaaaaaaata cttccccttt 4860
   aaagaggaag tattttaaaa aaaaattata gtttgtcaat agtttcaaat tcaaatttga 4920
   tttctttcca aacttcacaa gcagcagcta attctggaga ccatttacaa gctgagcgga 4980
   taacatcacc accttcacga gctaaatcac gaccttcgtt acgagcttga gtacaagctt 5040
   ctaaagcaac acggttagca acagcaccag gagcgttacc ccaagggtgt cctaaagtac 5100
   ctccaccgaa ttgtaaacaa gcgtcatcac caaagatttc aactaaagct ggcatgtgcc 5160
   atacgtgaat accaccagaa gcaactggca tagtaccacc catagaacac cagtcttgag 5220
   tgaagtaaat accacggcta 5240
<210> 126
   <211> 5240
   <212> DNA
   <213> Scenedesmus dimorphus
<400> 126
   cgtttaggtg taacacaatc ttggggtgga tggacaatta gcggtgaaac agcaacaaat      60
   ccaggtattt ggagttatga aggtgttgct gcatctcata ttattttatc tggtttatta     120
   ttcttagctt cggtttggca ctgggtttac tgggatttag agttattccg tgacccaaga     180
   actggaaaaa ctgcattaga tttaccaaaa attttcggaa ttcacttatt cttatcaggt 240
   cttttatgtt ttggttttgg tgctttccac gtaacaggtt tatttggtcc tggtatttgg 300
   gtttcagatc cttatggatt aacaggaagt gttcaaccag ttgctccttc ttggggtgct 360
   gatgggtttg atcctttcaa ccctggtggt attgcagcgc accacattgc tgctggtatt 420
   ttaggtgttt tagcaggatt attccactta tgtgtacgtc cttctattcg tttatacttt 480
   ggtttatcaa tgggtagtat cgaaacagta ttatcaagta gtattgctgc tgttttctgg 540
   gctgctttcg ttgttgctgg aactatgtgg tatggttcag cagctactcc aattgaatta 600
   tttggtccta cacgttatca atgggaccaa ggtttcttcc aacaagaaat tcaaaaacga 660
   gttcaaacaa gtttagcagg tggttcttca ctttctgatg cttggtcgaa aattccagaa 720
   aaattagctt tctatgatta tattggaaac aaccctgcaa aaggtggtct tttccgtaca 780
   ggagctatga atagtggaga tggtattgct gttggatggt taggtcacgc agtatttaaa 840
   gatcaagatg gtcgtgaatt atacgtacgt cgtatgccta ctttctttga aacattccca 900
   gttttattaa ttgataaaga tggtgttgta cgtgctgacg ttcctttccg tcgtgctgaa 960
   tcaaaatata gtattgaaca agttggtgta tcagtaactt tctacggtgg tgaattagat 1020
   ggattaacat ttaatgatcc agcaactgtt aaaaaatatg ctcgtaaagc acaattaggt 1080
   gaaatttttg aatttgatcg ttcaacatta caatctgatg gtgtattccg tagtagtcca 1140
   cgtggttggt ttacttttgg tcacgtttgc tttgctttat tattcttctt tggacatatt 1200
   tggcatggtg cacgtacaat cttccgtgat gtatttgctg gtattgatga tgatctaaac 1260
   gaaagtttag aatttggtaa atacaaaaaa cttggtgata caagttctgt tcgtgaagct 1320
   ttctaattcg tttttttctc ttttttttct tttttctctt tggaaaaaga aaaaacatgt 1380
   ttattttgaa ttttttgttt agaactttac tgttcttttt ttattttaaa gtgtttttgt 1440
   ttttttttaa tacaaaaact tttttaaaat gaatttaaaa aacacaaaaa aaagagttat 1500
   tgctattcaa aataaacaag agtttaaaaa caaagttttt ttctaaagaa aaaaacttct 1560
   tcattttttt tgaattgttt ttaaactttt ttcttctctt gcttttagag tttttttctt 1620
   cactttttgc aaaaaagtga gaaaaaacag caaagcaaaa aagtgaaaaa aagttcaaaa 1680
   acaattcaaa aaagacaaaa cctaaaaaaa tatcacttga gatgggtctg gattttttcc 1740
   aagcaaaaga attttgtatt ttgttgaaag tttttcataa aaatacaaat ttgcaattat 1800
   tattcttaaa atcaaaatat ttgttaacca catttcattc tatggaagca ttagtttata 1860
   cttttttatt aatcggaaca ttaggaatta tctttttcgc aattttcttt agagaaccac 1920
   ctcgtatggt aaaataattg aaattttgct ttttttttat ggataaaaga aatgatttca 1980
   attcatttct tttatccatt tttttgaaaa tagtttttct caatttttta ttttttttgt 2040
   tttttctcta aaaatcaaaa attcaatttt gagaaaaaat tttatacaaa aagttttttg 2100
   attaaaaact cagaaaaaag aaaaaaacaa agtatgaatt ttttgaaaaa ttcatacctt 2160
   ttattttttt tgtaattttt agcctttcaa aaaatttttg aaggcatttt ttttttaatc 2220
   ctcatgttct tcaaaaggat ctctcaattt ttttgaagga ggtccaaaac tcacatagat 2280
   tgaatatcct gttgcactta ataatagaaa ccataaaaaa aaggtaaaga aaaaagcagg 2340
   actgtccata attctttcat gttttttgtt caaatttatt ctccaataat tatattacga 2400
   cagaaagtaa aaaaaatcaa aatttattca aaaaaatggc tactggaaca acttcaaaag 2460
   ctaaatcaag cttatctgat gcacttcaag aaccaggtat cgtaactcct ttaggaactt 2520
   tattaagacc gttaaactct gaatcaggaa aagtattacc tggatgggga acaactgttt 2580
   taatgggtgt tttcattgta ctttttgctg tattcttatt aattatttta gaaatttata 2640
   acagttcttt attattagat gatgttacta tgagttggga aactttagct tcttaattca 2700
   atagaatagt tttattgctt tttttatttt ttattttatc aaaaattttt tttgcaaaaa 2760
   taaagaataa ataaaattca aaaaaattat agaattagat aaaattagtt tcaagttgaa 2820
   ctaagttgtc aataaacttt caaatttgtt ttctttttac tgttcattaa gagcaataaa 2880
   aaaaactttt ggtcttggca atcttttaaa aaagtcagaa tcaattctat tttaagaatc 2940
   ctatggaatc tatgtattta attttagcaa aattaccaga agcttatgca ccttttgatc 3000
   ctattgtaga tgttttacca attattccta ttttcttctt attattagcc tttgtatggc 3060
   aagcatctgt aagttttaga taaaaaattg aaaagttttt tttgatactt ttgtaaaaaa 3120
   tatcaaaaaa aacttttcaa tttttttcaa ttttcattcg caactttagc tttaatatta 3180
   gctaaagttg ctctcaaaaa tataattttt ttttgacttt ttattttttt attttgtttc 3240
   ttttttaaaa gttacaacat aaagaaaatg aaaatagaaa atttgtgaaa cataaaaaaa 3300
   aagaatgaaa tttttatgtt cgttttttgt tttatctttt ccaactaaag taaatttttt 3360
   ttccaaaatt ctataaaaaa tcaaaaaatt aaaaaaaaag aaaaaacttt gttttgtgca 3420
   aaacaaaaat cttgaattca aaacaaaata aagaattcaa aaagattttt tttaagcaaa 3480
   aggtaaaatg gaaaaaaatg ttttttgctt taaaattttt tctttttttt aaagctttgc 3540
   ttttttcatg aaaaaaacaa agctttaaaa aaaagaaaaa attttaaagc aaaaaaaaga 3600
   attaaacacg tctttttttt ggaggacgac atccattatg tggaattcct gttttttctc 3660
   gaataacatt tactttaatt ccagctttaa aaatttcacg aatagctgtt tcgcgccctt 3720
   gtcctggacc agttactaaa atttttgctt catttaatgc aaattcacgt gatttttttg 3780
   ccacaacttc agcagctttt tttgctgcaa atgttgttgc ttttcttttt ccacggaaac 3840
   cacaagctcc agcagaactc caacaaagga cttcaccacg aagatttgct aatgtaataa 3900
   tagtattatg atgtccagct tgaatataaa caattcctcg atatgtacgt tttttaattt 3960
   ttgtaggtga tacttttcta gtttgtctag ccatatgtaa aaatttaact ataaatttct 4020
   tttatatttt taaatcattt gatttactat ctaaaaaaaa taaaattttg aatctttgga 4080
   aagaaccatt atttggaaag atttatttgt tcaattcttt tgtatttttt ttcaaaaaaa 4140
   ttttttagaa ttttttatct aattttttaa tgttttgttc aaacataaaa aattttcttt 4200
   tcaaaaaaga aaaaaaaatt tctttttttt gaaaaaggaa aaataaaaaa agctgtagct 4260
   tttttgttga atcaaaaaaa aataagaaat tgtcctattt ttatgacaaa agattcaaaa 4320
   aaatgaaata aaaaagaaaa attcaaactt tcaaacaatg tattttgtat ttttgggccg 4380
   agtcggattc gaaccaacgt aggcgtaacc agcggattta caatccgccc ccattaacca 4440
   ctcgggcatc ggcccatgct tttttttgac tcatttaatc atttattttg catgaattat 4500
   actaaagatt atattaacaa aaaatttttg aaatttcaat tttttttaat taaaaactct 4560
   tctatttttt aaaattcttt gttttgaatt tttttttcaa tttaaaaaaa aaaaacatat 4620
   taaaaaatat ttttaaaaaa ttttttgtat tgaaaactta caataatttt ataaattttt 4680
   ttgaaaaatt ttgttttttt tattctattg aaatgaacaa aacaaatttt tttagttttt 4740
   tttgtttttt tgttttgctg cttcttttgt ttttatcaat aaataaatga aaatgaaaaa 4800
   caaaaataaa caatttatgt ttttagagtt ctaaaatcaa gaaaaaaata cttccccttt 4860
   aaagaggaag tattttaaaa aaaaattata gtttgtcaat agtttcaaat tcaaatttga 4920
   tttctttcca aacttcacaa gcagcagcta attctggaga ccatttacaa gctgagcgga 4980
   taacatcacc accttcacga gctaaatcac gaccttcgtt acgagcttga gtacaagctt 5040
   ctaaagcaac acggttagca acagcaccag gagcgttacc ccaagggtgt cctaaagtac 5100
   ctccaccgaa ttgtaaacaa gcgtcatcac caaagatttc aactaaagct ggcatgtgcc 5160
   atacgtgaat accaccagaa gcaactggca tagtaccacc catagaacac cagtcttgag 5220
   tgaagtaaat accacggcta 5240
<210> 127
   <211> 876
   <212> DNA
   <213> Scenedesmus dimorphus
<400> 127
   ttgaactatc aagtttaggt tttaaaatct ttatttattt actttatttt ttaatttgaa 60
   aactctgcga gctttgcgag cactgatttc aaaatcttag tttcaagtaa aacttatttt 120
   caatctttat ttatttgtat tttcaaacta aaagtttgaa ttatctaatt tgaaacttta 180
   tgagctttac aaagactggt ttcaaatttt ttctttgttt agtttgtttt ttcaaactat 240
   tagtttaaac tatctaattt gaattataag tttgtatttt caaactcttt atttgttaac 300
   tttgtttttt agtttgaaat tctatgattt tggacattag aaggctttgc cttaatgatt 360
   ttccccgagc ccctcttggg attctttttt tttattttcc tttcaggact tattataata 420
   tatcaaaaat aatttttttg tcaatttttt ttaatatttt aaaaattttt taaattaaaa 480
   ataaattttt ttttattttt agattttaat ttttatttgt aagtttaata tttctaaaaa 540
   atttgaattt aagaattttt taatttgatg aaaaaaattg tttttgaatt tttttttttt 600
   actttaatta acttttttaa aaaatgatta aaaatttgaa gtttttaaaa aactatcttt 660
   ttttttgtaa aaatggtatt attttgtgta aaatacaaca aaaataacaa ttttcacctt 720
   attttaagtt taatttttca atgcaaaatt tattttcaac aaaatgaaaa atattttttt 780
   agaatatatt ttatggcggg cgtagccaag tggtaaggca atggattgtg actccatcat 840
   tcgcgggttc gaaccccgtc gttcgcccat aataaa 876
<210> 128
   <211> 1724
   <212> DNA
   <213> Artificial Sequence
<220>
   <223> codon optimized sequence comprising rb1L-CAT-psbE
<400> 128
   cggttttttt tacttttgct ttttttgctt ttgttcaaag aaaaaaaaat acaaaataaa        60
   aaaaactaaa atgaaaaaac aaagaattct aaaattcata aaaaaaatta aaacccaatt       120
   ttttttttgg aaacttttcc aaataataaa aaaatcaaaa aaaaattttt ctagtatttt       180
   tttcatattt tgaaactttt tttgagttta taaaaaaata gaaaaaacaa atagatgaaa       240
   atttagaaaa attataaacc aataaaaatg aagttttgcg tagaaaaaaa atttagttta       300
   cttgttcccc aagagcaagt ggtaactttg aaaaaaatat ttaaacttaa aaatttgcta       360
   aagttttgaa tttatgttaa aatttaaaaa aaataaaaat ttttaaacta tttttttatg       420
   ttaaaaaaat agtttttatt attttctata atatagttta gttttttatt tttttcaatt      480
   tctttttttt tttcaaagaa aaaagttttc cacggataga tttttatagg atcgacaaaa       540
   tgttctatga acttttcata atggagaaaa aaatcactgg atataccacc gttgatatat       600
   cccaatggca tcgtaaagaa cattttgagg catttcagtc agttgctcaa tgtacctata       660
   accagaccgt tcagctggat attacggcct ttttaaagac cgtaaagaaa aataagcaca       720
   agttttatcc ggcctttatt cacattcttg cccgcctgat gaatgctcat ccggagttcc       780
   gtatggcaat gaaagacggt gagctggtga tatgggatag tgttcaccct tgttacaccg       840
   ttttccatga gcaaactgaa acgttttcat cgctctggag tgaataccac gacgattccg       900
   gcagtttcta cacatatatt cgcaagatgt ggcgtgttac ggtgaaaacc tggcctattt       960
   ccctaaaggg tttattgaga atatgttttt cgtcagcgcc aatccctggg tgagtttcac      1020
   cagttttgat ttaaacgtgg ccaatatgga caacttcttc gcccccgttt tcactatggg      1080
   caaatattat acgcaaggcg acaaggtgct gatgccgctg gcgattcagg ttcatcatgc      1140
   cgtttgtgat ggcttccatg tcggcagaat gcttaatgaa ttacaacagt actgcgatga      1200
   gtggcagggc ggggcgtaac ctagatattt gagaatttgt atttaaaact gaaaaatttt      1260
   tgaacgaact cttttcaaaa atattaaact ttcttgagat gatttagtgt tatctcaaga      1320
   aagtttgttc ttttattttt taaaattttt aaaaatttta ttttctttta aacaggaaaa      1380
   taaataaaga aaaaagtgaa ttaaaaaaaa gctgggactt tcaaagtgac caatttttta      1440
   ctttaaagtt tttttttatt caataaaaaa atactaaaaa aatatgaaag tattacttaa      1500
   aatttcttaa aaaaaaaaga atgccttttt tcaaaaaaaa gtttaaaaaa aataaagttt      1560
   ttacgtattg tttaaaactt tttttgaaaa aagcattctt ttttcattta aagagttatc      1620
   ttttttatct cgtgcaagtt ttggaaattc atttttgtta aataactttg actttttatt      1680
   tcttaaattt ttggcttttc attttttttg gtttacaaat aaaa                      1724
<210> 129
   <211> 25
   <212> DNA
   <213> Artificial Sequence
<220>
   <223> PCR primer
<400> 129
   atggghytmc cwtggtaycg tgthc 25
<210> 130
   <211> 25
   <212> DNA
   <213> Artificial Sequence
<220>
   <223> PCR primer
<400> 130
   ccratgtggc grcaaggdat gttyg 25
<210> 131
   <211> 25
   <212> DNA
   <213> Artificial Sequence
<220>
   <223> PCR primer
<400> 131
   tttgwarrat rathavdarr aawrg 25
<210> 132
   <211> 24
   <212> DNA
   <213> Artificial Sequence
<220>
   <223> PCR primer
<400> 132
   kccwggdrmh actttwccdk mttc 24
<210> 133
   <211> 2805
   <212> DNA
   <213> Dunaliella aertiolecta
<400> 133
   tctgtgcatt taatgcacac agctctagta gctggttggg ctggtgctat gacattattt      60
   gaaattgcag tttttgatcc atcagatcca gtattaaacc ctatgtggcg tcaagggatg     120
   ttcgttcttc ctttccttac acgtttaggt gtaacacaat catggggtgg ttggacaatt     180
   agtggtgaaa catcttcaaa cccaggtatc tggagttatg aaggtgctgc agcttcgcac     240
   attgttcttt caggtttatt attccttgct tcagtttggc actgggttta ctgggattta     300
   gaattattcc gtgatccacg tacaggtaaa actgcattag atttaccaaa aatttttggt     360
   attcatcttt tcttagcagg acttctttgt tttggttttg gtgctttcca cgtaacaggt     420
   gtttttggac ctggtatttg ggtatcagat ccatatggat taacaggtag tgtacaacca     480
   gtagctcctt cttggggtgc tgaaggtttt gacccttaca acccaggtgg tgtaccagct     540
   caccatattg ctgctggtat tttaggtgta ttagcaggtt tattccacct ttgtgttcgt     600
   ccatcaattc gtttatattt tggtttatca atgggttcta ttgaatcagt tttatcaagt     660
   agtattgcag ccgtattctg ggcagctttc gtagtagcag gtactatgtg gtatggttct     720
   gcagcaactc caattgaatt atttggtcca acacgttacc aatgggatca aggtttcttc     780
   caacaagaaa ttcaaaaacg agtagcacaa agtacatctg aaaggtttat ctgtttcgta     840
   gcacaaagta catctgaagg tttatctgtt tcagaagctt gggcaaaaat tcctgaaaaa     900
   ttagctttct atgattacat tggtaataac ccagctaaag gtggattatt ccgtacaggt     960
   gctatgaaca gtggtgatgg tatcgctgta ggttggttag gacacgctag ttttaaagat    1020
   caagaaggtc gtgaactttt tgttcgtcgt atgcctactt tctttgaaac tttccctgtt    1080
   gttttaattg ataaagacgg tgttgttcgt gctgacgtac cattccgtaa agctgaatca    1140
   aaatactcaa ttgaacaagt tggtgtttca gttacattct atggtggtga attaaatggt    1200
   ttaacattta ctgacccttc aactgttaaa aaatatgcac gtaaagctca attaggtgaa    1260
   atctttgaat ttgaccgttc gactttacaa tctgacggtg tattccgtag tagcccacgt    1320
   ggttggttca cttttggaca cttatctttt gccttattat tcttctttgg tcatatttgg    1380
   catggttcaa gaactatttt ccgtgacgtt ttcgctggta ttgatgaaga cattaatgat    1440
   caattagaat tcggtaaata taagaaactt ggtgatactt catctgttcg tgaagctttc    1500
   taatcactat attaagtttt attccaatat tctaccaaga atatttagaa attcttgttt    1560
   ttaaattcat atagaaaaaa tctattagaa tttccgtcga gaaattctaa tagatttttt    1620
   ctctttttgg cggactaaac acttttcttt gtttttttgt tttccgacta gaaaatagaa    1680
   gattcattta acaaagttca catattttgt aatgtgattt ttttgtaatg tgatttttct    1740
   tatctttatt agattttcta tcttaccgta gattttaata cggtataatt gttttttttt    1800
   ttttagatgg gcttagattt tctctgagca aaagaatttg agttagtgta aacaaatttg    1860
   atcaaagttt atttcataaa aagaattttt ttttataaat acggaagaaa atatacgagc    1920
   taaattttat gttcttccgt tttattttta taaaatgtta atattttatt tttgttaaaa    1980
   aaatctaaga taatattttt tttaactcct tattggaatt aaattttatc ttttataact    2040
   ttaggtaagt cgttagatat tttaaattta tcttctgacc gcttcgttta ttttcttttc    2100
   gtaattcgga agaaaattct ttttgtttta cactaatgta aaatttggta ttatgttcct    2160
   atttgaaaag actagtcttt tcaataaatt tattaaacac ctttcatgga agctttagtt    2220
   tacactttct tattaattgg aacgttaggt attatctttt tctctatctt ttttagagag    2280
   cctccacgta ttgcaaaata gtaatagaat aattttttat taaaattact tgtcggacca    2340
   aacaataaag ttgttttttc cgatacgaaa atctacgaga aattctatat ttatcgtaaa    2400
   tacgtttcaa atgaagtatt ataagggttg gatttcatta acattaatta gtgaagtcca    2460
   acccttagaa tacttaaaat ttttacttaa ctaagattat taatttaatc ttcatgttct    2520
   tcaaaaggat ctctcaattt tcttgaagga ggtccaaaac taacataaac tgaataacct    2580
   gtagcactta ataaaagaaa ccataaaaag aaggtaaaga aaaaagccgg actttccata    2640
   aatttaaaat ttttgacaag acaaaattat tctccatata tattatacaa tattacagaa    2700
   aggaaaaaaa caaaaagatt ttattaacta tcaattatgg ctacaggaaa aaatagtaca    2760
   caaacttcaa catcacaaga accaggaatt gttacaccat tagaa                    2805
<210> 134
   <211> 3561
   <212> DNA
   <213> Dunaliella
<400> 134
   catttaatgc acactgctct agtagcaggt tgggctggtt caatgacatt attcgaaatc     60
   gccgtatttg acccttcaga ccctgtatta aaccctatgt ggcgtcaagg gatgttcgta    120
   cttcctttct taacacgtct aggtgtaaca caatcatggg gtggttggac aattactggt    180
   gaatcagcat caaacccagg tatctggagt tatgaaggtg ctgcagcttc tcacattgtt    240
   ctttcaggtt tattattcct tgcatcagtt tggcactggg tttactggga tttagaatta    300
   ttccgtgacc cacgtacagg taaaacagca ttagatttac caaaaatttt tggtattcac    360
   ctattcttat caggacttct ttgttttggt tttggtgctt tccatactac aggtgttttt    420
   ggacctggta tttgggtatc agatccatat ggtttaacag gtagtgtaca accagtagct    480
   ccttcttggg gtgcagaagg ttttgatcct tacaacccag gtggtgtacc tgctcaccat    540
   attgcagcag gtattttagg tgtattagct ggtttattcc acctatgtgt tcgtccatct    600
   attcgtttat actttggttt atcaatgggt tctattgaat cagttttatc aagtagtatt    660
   gcagcagtat tctgggcagc attcgttgtt tcaggtacta tgtggtacgg ttcagcaaca    720
   actccaattg aattatttgg tccaactcgt taccaatggg atcaaggttt cttccaacaa 780
   gaaattcaaa aacgagtagc acaaagtaca tctgacggtt tatctctttc tgaagcttgg 840
   tcaaaaattc ctgaaaaatt agctttctat gattacattg gtaacaaccc tgctaaaggt 900
   ggtttattcc gtacaggtgc tatgaatagt ggtgatggta ttgccgtagg ttggttaggc 960
   cacgctagtt tcaaagatca agaaggacgt gaactttttg ttcgtcgtat gcctactttc 1020
   ttcgaaactt tccctgttgt tttaattgat aaagatggta ttgttcgtgc tgacgttcca 1080
   ttccgtaaag cggaatctaa atactctatt gaacaagtag gtgtttcagt tacattctac 1140
   ggtggtgaat taaatggttt aacatttact gatccttcta cagttaaaaa atacgctcgt 1200
   aaagctcaat taggtgaaat cttcgaattt gaccgttcta ctttacaatc tgatggtgta 1260
   ttccgtagta gtccacgtgg ttggtttact ttcggacact tatcttttgc tttattattc 1320
   ttctttggtc acatttggca tggttcaaga actattttcc gtgatgtttt cgctggtatt 1380
   gacgaagata ttaatgacca attagaattc ggtaaataca agaaacttgg tgatacttca 1440
   tctgttcgtg aagctttcta atcaattttt tgatttaatc atttcgccac agagaacttt 1500
   tacaaaaata attttataaa actctctgaa attatttctc cacactttga ccttggtctc 1560
   tagttcgtct ggagaccaag gtcaaagtaa agttcttgta tgatatttct agaagaaaaa 1620
   atttgatttt cttcgataaa tactcatata aaaataaaaa atcgaaggga agaaaacttc 1680
   ttcgaagttt tcttttttta agataccgcc agtttaaaat ctaacaattt tattcctaag 1740
   gagaaaattc ctataggaat tttctctgcc gcgtttcgaa gaaacgcgta ggacttctta 1800
   gaagtccgcc tattaaattt ttaaaagaaa atttttattg aatgtttttc ttagacaaaa 1860
   gaaaaaacat tttttcgtgt aggatttaaa agaaattcaa gatttctatc ctagtcttaa 1920
   tttaagaaaa agaatatttt ttctaaggat ttacaaaatt attattcaat tgttgattta 1980
   aaatcttaaa attcgaaaat atttttgatt tttaaagaaa gagattaaaa aaaaaaaaaa 2040
   cgattttcta aatttaactt tagattctat ttttacttac ctaactaata aagtttttta 2100
   gaaatttaaa taaaaaattt tatgtggttt ttttgacgaa aaccctaaat cttgaacact 2160
   ttgtaaaata tcttttaaag attttttaaa cttctatatg taaaatttta ttttatttat 2220
   aaaagttttt tctccttaaa agttttattt atacttttaa agtattattt ttcgataaaa 2280
   aaaaacaaat atttcaataa aatataatta tattttataa aataaataaa agatgggctt 2340
   agaatttctc tgagcaaaag aatttaaact aatgtgaaaa acgtaagtta attttataaa 2400
   aaaaatctaa ttttgtaaat tatatgcgtt ttttacatta agtttaaatt tggttttatg 2460
   ttcctatttg aaaagcaaca gcttttcaat aaatttatta aacatttcgc atggaagctt 2520
   tagtttacac tttcttatta gttggaacat taggtattat tttcttctct attttcttta 2580
   gagaacctcc acgtattgct aaataataaa aaaacatttc tcaacgtaaa ttacaaataa 2640
   tttaataatt actttgggat ttgtgtaaaa ctcgcctaag aaatctgttg tatctttaat     2700
   cttcaggctt tgcacggttt aaattttcgt aggtcgcctc actgaggcga cctcgtgcct     2760
   ctcaaaaggt gctcaaagaa aaaaaaaaaa aaaaaagaaa gacaaaaaac ctttggtttt     2820
   ttgtacctta gaaaaaaact tttaattttt tttcctacta aaaattattt taggcggtgt     2880
   cgcgaagcaa cataggaaaa aaaattccgc tagggaagaa aaatttcctt aggaaatttt     2940
   ctattttttt aattctagtt ttttttgtcg gaaaaaaacc ttaggttttt ttcctaaggg     3000
   tctttttgcc gaaggcgaaa agcccgcctt attataaaca gtttattaat aaggttgaat     3060
   ttaactataa attttttaaa aaatgattat ttttgttaaa tcctaataat tttatgtatg     3120
   aaattttcat tgttagtaaa tcaattattt attaacatga aaaatttgaa gtttaaaaaa     3180
   ttctaaagga ttttttaaat cttctatatt tctcgaagaa aatcaaagat tttctccgag     3240
   aaatacagat atatcttgaa aatcaaagat tttcaagata ctttatcgtg aataaagatt     3300
   attttttaat cttcatgttc ttcaaaagga tctctcaatt ttcttgaagg tggtccaaaa     3360
   ctaatataaa ctgaataacc tgtagcactt aataaaagaa accataaaaa gaaggtaaag     3420
   aaaaaagccg gactttccat aaatttaaaa tttttgacaa gacaaaatta ttctccataa     3480
   attattatac aatattacag aaaggaaaaa atataaagat ttaatttatt ttttattatg     3540
   gctacaggta aaaataatac a                                               3561
<210> 135
   <211> 2730
   <212> DNA
   <213> N. abundans
<220>
   <221> misc_feature
   <222> (1477)..(1488)
   <223> N is A, C, T or G
<400> 135
   gctgtacatt taatgcatac ttcattagtt tctggttggg ccggttcaat ggctttttat      60
   gagcttgctg tttttgatcc ttctgatcca gttttaaatc caatgtggcg tcaaggtatg     120
   tttgttttac cttttatgac acgtttaggt atcactcaat cctggggtgg ttggactatc     180
   agtggtgaaa cggcttcaaa tccgggtatc tggagttatg agggtgtagc cgcagctcac     240
   atcgttttat caggtttact ctttgctgcg tctatctggc actgggttta ttgggatctt     300
   gaactttttc gtgatccaag aacttcaaat ccagctttag atcttccaaa aatttttggt      360
   atccatttat ttttatctgg tgttctttgt tttggttttg gagctttcca cgtaacaggt      420
   atttttggtc ctggtatttg ggtttctgat ccttatggaa ttacaggaac agttcaagca     480
   gttgcgcctt cttgggatgc tacagggttt gatccctata atccgggtgg aatttcagca     540
   catcatattg ctgccggcat tttaggtgta ttagctggtt tattccacct ttgtgttcgt     600
   ccgccacaac gattatacaa tggtctccgt atggggaata ttgaaacagt actttctagc 660
   agtattgcag cagttttttg ggcagctttt gttgtttctg gtactatgtg gtatggttcg 720
   gcggcaacac caattgaact ttttggtcct actcgttatc aatgggattt aggcttcttc 780
   caacaagaaa ttgaacgtcg tgtacaaaca agtctttctg agggcaaatc tgcttcgcaa 840
   gcgtgggcag aaattccaga aaaattagct ttttacgatt acattggaaa taatccagca 900
   aaaggtggtc ttttccgtgc gggtgctatg aacagtggag atggtattgc agtgggctgg 960
   ttaggtcatg ctgttttcaa agataaacaa ggtaacgaac tttttgtacg tcgtatgcca 1020
   actttctttg aaaccttccc tgtcgttctt gtagataaag atggtgttgt tcgtgcagat 1080
   gttcctttcc gtcgttctga atcaaagtac agtatcgaac aagttggtgt ttctgtaact 1140
   ttctatggtg gagagttaga tagtgtaact tttaatgatc cagcaactgt gaaaaaatat 1200
   gctcgacgtg ctcaattagg agaaattttc gaatttgatc gtgcaactct tcaatcagac 1260
   ggtgttttcc gtacaagtcc tcgtggttgg tttacatttg ctcatttatg ttttgctctt 1320
   cttttcttct ttggtcatat ttggcatggt gctcgcacaa tcttccgaga tgtatttgct 1380
   ggtatcgatg cagacttaga tgaacaagta gagtttggtg cattcttaaa acttggtgat 1440
   acttctactc gtcgtcaatc ggtttaaagg ttcgggnnnn nnnnnnnnag ttagtacaaa 1500
   actactattt ttggaaaagg aaataccttt cacaaactag actccaaggt ttctattcca 1560
   gaagcggaga aaagaaatag aaccagtgag ttttaaaact cactggttct taaaggctcg 1620
   tgagatagat tttttgaaaa ttttatctaa tctacaaaaa ttttttgaaa ctaactaagc 1680
   cagtttcatt tttttagaaa aaatgaagaa agaagtatcc actcaaatta tccaacagaa 1740
   caaaaaaaga agcttaatcc tcatgttctt caaatggatc acgaagttgt tgtgatggtg 1800
   gaccaaaacc tacgtaaata gaataaccgg taatacttaa taataaacac cataaaaaaa 1860
   tggtaaagaa aaaagctggg ctttccatac tgatttaatt tttagttact taatactgag 1920
   acaaaacgtt tttaaaaaca gagttgttcc acaaaaagcc cttttgggcc taaatggaat 1980
   tggtaccaat tttcttttcg gaaggaaata cattgaccgc ttatttgtat attttcccga 2040
   aattttataa aaagtttagt cttcggccca aaatagactc aaaacctaat tttttagtcg 2100
   aacacgaaaa taagatcttt acatttccca aaaataaaat ttttgggaac aacaagtttt 2160
   tgggacctaa aatcttttag tttaatttta gcaaaaacct tgttttcttt ctttaagtaa 2220
   aacacatttt gtaattaaat gagaattaga tgaattgaaa tcattaaaaa gtcaattagg 2280
   ttcgagttta ttttttttgt agaaaaattt agctaaaagt tttttctaat ttaaaagttc 2340
   ctatataata gatttagaaa aaagtcttga aatttttgaa agccaattac aaaaaaaatt 2400
   tggggtcttt ctgttttttg ttaaacaaac aaatggaacg gttactcagt cctaaaaatc 2460
   ttagttttga gaacaaaaac attgttttta atttttattt ttgcctcttt aatgtcaaat 2520
   agaagttaat tcatactagt ttggttcatc tcggcccttt cctttagaca agatgttgag 2580
   gcccaaactg tgtttttgag cgcactaaaa attttgttaa taaatttttt gtttttcggt 2640
   tttcttttta ttacagaaag taaaaaaaaa attatatcat tgtaaaaact atggcaactg 2700
   gaactacatc aaaagtaaaa tctgacgaca 2730
<210> 136
   <211> 2996
   <212> DNA
   <213> Chlamydomonas vulgaris
<400> 136
   tacgatcggg tcgtttattg ctgtgcattt aatgcatact tctttagttt ctggttgggc 60
   tggttcaatg gctttttatg aacttgctgt ttttgatcct tctgatccag ttttaaaccc 120
   aatgtggcgt caagggatgt ttgttcttcc ttttatgaca cgtttaggca tcactcaatc 180
   ttggggtggt tggactatca gtggtgaaac agcatcaaat ccaggtattt ggagttatga 240
   gggggttgct gctgctcaca tcattttatc tggattactt tttgctgctt ctatttggca 300
   ctgggtttat tgggaccttg agcttttccg tgacccacgt acgtcaaatc cagcattaga 360
   ccttccaaaa atttttggta ttcacttatt tttatcaggt cttctttgct ttggttttgg 420
   agctttccat gtaactggat tatttggtcc tggtatttgg gtttcagatc cttatggtat 480
   tacaggaact gttcaagcag ttgctccttc ttgggatgct acaggatttg atccttacaa 540
   cccaggtgga atttcagcac atcacattgc tgcaggtatt ttaggcgtct tagctggttt 600
   attccacctt tgtgttcgtc caccacaaag attatacaat ggacttcgca tgggtaacat 660
   tgaaacagtt ctttctagca gtattgcagc agttttctgg gcagcatttg ttgtatcggg 720
   aactatgtgg tatggttctg ctgcaacacc aattgaactt tttggtccaa ctcgttacca 780
   atgggattta ggtttcttcc aacaagaaat tgaacgtcgt gtacaaacaa gtcttgctga 840
   aggaaaatca gcttcacaag cttgggcaga aattccagaa aaattagctt tttacgatta 900
   tattggaaac aacccagcaa aaggtggtct tttccgtgcc ggtgctatga atagtggcga 960
   tggtattgca gtaggttggt taggccatgc tgttttcaaa gataaacaag gtaatgaact 1020
   ttttgttcgt cgtatgccaa ctttctttga aacattccct gtagttcttc ttgacaaaga 1080
   cggtgttgtt cgtgcagacg tgcctttccg tcgttctgaa tctaaataca gtattgaaca 1140
   agtgggtgtt tctgttacat tctacggtgg tgaattagat ggtgtaacat ttagtgatcc 1200
   agcaacagtg aaaaaatatg ctcgacgcgc gcaattagga gaaattttcg aatttgatcg 1260
   tgccactcta caatctgatg gggttttccg tacaagccct cgtggttggt ttacttttgc 1320
   tcatttatgt tttgcactcc ttttcttctt tggtcatatt tggcacggtg ctcgtacaat 1380
   cttccgagat gtatttgcag gtattgatgc agatttagac gaacaagtag aatttggtgc 1440
   attcttaaaa cttggtgata cttcaactcg tcgtcaatca gtataattca ttttttcttt 1500
   tacttcctct ctcaaatttt tcaaatttgg gagaatttac caaaactgaa ataatttgca 1560
   agcctctatc atcttaaaaa tattgtttgt aagaaaaaaa agaggtcggt tatttgttct 1620
   ccaaactttt tttcgaagtt tttagagaaa aaagtttggt cccaaaaacg tagtttttgg 1680
   ggaaaggaag tattttttcg ttttcttccc tttttctgta ttttttttac ctttgctttc 1740
   caacaaaaca gtattgttgg agaaatcgaa ttagtcccaa aatttaaatt ttgggactag 1800
   gtattagcat gaaaaaaaat gatagaaaaa ggtaaacagt gcacttttct cccaaactaa 1860
   atgatttttc atctagttgg ttactaacaa acagaacttt tttctatgga agcgttagtt 1920
   tatacttttt tattagttgg aacattaggt attatctttt ttgccatttt ctttagagaa 1980
   ccaccacgta ttgtaaaata aaagtaccat ttttggtttt cgttgaaaaa aacttttgat 2040
   atttttcatt tatttcaaaa gttataaaat ttggaataaa gggttaattt tcagaccaga 2100
   ttttttccca aaaactttgt ttttgggatt gggagaatta ttactgtttt agaaacacca 2160
   aagcttattc taaaacaata atagttttag caccaaggaa aattcacatt cccgaaaatt 2220
   cgaatggtct taatatttac tgactttgtg agttttaaaa ctcacaaagt tatttaaacg 2280
   aattaaaaag ggttaatata tttccttctt atttctattt agtagtttaa tgtaaaacag 2340
   tagtgaagaa ctagtttggg aatttatttt aatctttttc ttattaaagg acttttgtga 2400
   atagttctaa atttagttgg tcacttttga aaaaaagtaa taataattaa agaaaactaa 2460
   tgaaatttga tttactagag cattaatctt catgttcttc aaaaggatca cgaagttttt 2520
   gtgacggtgg cccaaagcca acatagattg aataaccagt aatacttaat aaaagacacc 2580
   ataaaaaaat ggtaaagaaa aaagctggac tttccatagg taaaaattac aaaactaaga 2640
   aatttcttta gaagataaaa ctttttttaa ctgtttagtt tttatgaaaa cagaaaaaaa 2700
   ttatcttcga tctattcttg ttttcatttt accaaaaaat aagcaaaaag ttatgttaaa 2760
   gtattctact ttatagaaaa gaactttttt acttttgtaa aatgaaaata tcttttttgt 2820
   attctaaaac ttttgagaaa cttaaactta ctatataata aagataagag ggagaaaaaa 2880
   gcttaaaagc tttttctctt ttttacagaa agtaaaaaaa attcttttat ttcaaaaatt 2940
   atggcaactg gaactacatc aaaagtaaaa tcagaagata ctggaattca actcca 2996
<210> 137
   <211> 1982
   <212> DNA
   <213> Tetraselmis suecica
<220>
   <221> misc_feature
   <222> (1050)..(1052)
   <223> N is A, C, T or G
<400> 137
   gtcgtttatt gcggtgcatt taatgcacac atcattagtt tctggttggg ctggttcaat 60
   ggccttttat gaacttgctg tttttgatcc atcagatcca gttttaaacc caatgtggcg 120
   tcaaggtatg tttgttttac ctttcatgac tcgtttaggt atcacccaat cttggggtgg 180
   ttggacaatt agtggtgaaa cggcttcaaa cccaggtatc tggagttatg aaggtgttgc 240
   tgcggctcac atcgttttat ctggagctct ttttggggca gctatttggc attgggtttt 300
   ttgggattta gaattattcc gtgacccaag aacaggtaac cctgcattag atttaccaaa 360
   aatttttggt attcacttat tcttatcagg gttattatgt tttggttttg gagcattcca 420
   tgtaacaggt ttatttggac ctgggatttg ggtttctgat ccttatggat taacaggaag 480
   tgttcaacca gtatctccat catggggagc cgatgggttt gatccataca atccgggtgg 540
   tattgcatct caccatattg ccgcaggtat tttaggtatt attgctggtt tattccattt 600
   atgtgttcgt cctccacaac gtttatataa tggtcttcgt atggggaaca ttgaaacagt 660
   tctttctagt agtattgcgg ctgttttctg ggcagctttt gttgttgcag gaacaatgtg 720
   gtatggttgt gcggcaacac caattgaatt atttggccca actcgttacc aatgggatca 780
   agggtatttc caagaggaaa ttacaaaacg tgtagaaaaa tctttatctg aagggcaatc 840
   tttatcagaa gcttggtctc aaattcctga aaaattagct ttctatgatt acattggaaa 900
   caacccagct aagggtgggt ttattccgta ctggggctat gaacagtggc gatggtattg 960
   ctgttgggtt ggttagtcat gcagttttcc cagatttaga tggtattgag tttatcagtt 1020
   tcgtcgtatg cccacgttct tgaacttttn nnagttaaat ttacggatcc cagcaacccg 1080
   ttaagaaata tgctcgtcgt gctcaattta ggagaaaatt ttgaaatttg accgtgccac 1140
   attacaaatc agatggtgtt ttccgaagca gtccacgtgg ttggtttact ttgggcattt 1200
   atcatttgct ttattatttt tctttggtca tatttggcac ggagctcgta caatcttccg 1260
   tgatgttttt gcagggattg atccagattt agatgagcaa gtagaatttg gggcattcca 1320
   aaaattagga gatacaacga ctcgtcgtca atctgtttag tttttcattt tgaattcatt 1380
   cctcggattc aattatatcc gcttaaatca attattcttt taaaaattta ttatggaagc 1440
   tcttgtttac acatttttac ttgtaggaac tttaggtatc atcttttttg caatcttttt 1500
   tagagaacca cctcgtattg caaaataaat agtttaactt caaacttatt atcaaaattg 1560
   ttgtgaattg gggattaacc caattcacaa caattcaaat attaatcaaa aatagtttgg 1620
   ttaatcttca tgctcttcaa acggatcacg aagatccttt gaaggtggac caaacccaac 1680
   ataaactgaa taccctgtaa tacttaaaag taaacaccat aagaaaacag taaaaaaaaa 1740
   agcaggactt tccataagta aattttttta atcttttaag ttaaattcaa tagcaataac 1800
   taacttattg aatttataaa actctattat tgttatatca taagtgaaag aacttttgac 1860
   tgaaaaattc aagaaagtaa aaaataattc ctacgtttat atattatggc aacaggaaca 1920
   tcaaaagcat ctaaaaatac acctgtaaat acagcaatgg cgacacaatt agaacgctat 1980
   aa 1982
<210> 138
   <211> 24
   <212> DNA
   <213> Artificial Sequence
<220>
   <223> PCR primer
<400> 138
   ccacctcgta tggtaaaata attg 24
<210> 139
   <211> 24
   <212> DNA
   <213> Artificial Sequence
<220>
   <223> PCR primer
<400> 139
   gaaagaatta tggacagtcc tgct 24
<210> 140
   <211> 21
   <212> DNA
   <213> Artificial Sequence
<220>
   <223> PCR primer
<400> 140
   gaaggaggtc caaaactcac a 21
<210> 141
   <211> 20
   <212> DNA
   <213> Artificial Sequence
<220>
   <223> PCR primer
<400> 141
   cctggttctt gaagtgcatc 20
<210> 142
   <211> 24
   <212> DNA
   <213> Artificial Sequence
<220>
   <223> PCr primer
<400> 142
   tgagttggga aactttagct tctt 24
<210> 143
   <211> 20
   <212> DNA
   <213> Artificial Sequence
<220>
   <223> PCR primer
<400> 143
   aaaagattgc caagaccaaa 20
<210> 144
   <211> 25
   <212> DNA
   <213> Artificial Sequence
<220>
   <223> PCR primer
<400> 144
   aaaaagaatg aaatttttat gttcg 25
<210> 145
   <211> 20
   <212> DNA
   <213> Artificial Sequence
<220>
   <223> PCR primer
<400> 145
   atggatgtcg tcctccaaaa 20
<210> 146
   <211> 762
   <212> DNA
   <213> Artificial Sequence
<220>
   <223> codon optimized sequence comprising BD11
<400> 146
   atggtaccag tatctttcac aagtctttta gcagcatctc caccttcacg tgcaagttgc 60
   cgtccagctg ctgaagtgga atcagttgca gtagaaaaac gtcaaacaat tcaaccaggt 120
   acaggttaca ataacggtta cttttattct tactggaatg atggacacgg tggtgttaca 180
   tatactaatg gacctggtgg tcaatttagt gtaaattgga gtaactcagg caattttgtt 240
   ggaggaaaag gttggcaacc tggtacaaag aataaggtaa tcaatttctc tggtagttac 300
   aaccctaatg gtaattctta tttaagtgta tacggttgga gccgtaaccc attaattgaa 360
   tattatattg tagagaactt tggtacatac aacccttcaa caggtgctac taaattaggt 420
   gaagttactt cagatggatc agtttatgat atttatcgta ctcaacgcgt aaatcaacca 480
   tctataattg gaactgccac tttctaccaa tactggagtg taagacgtaa tcatcgttca 540
   agtggtagtg ttaatacagc aaaccacttt aatgcatggg ctcaacaagg tttaacatta 600
   ggtacaatgg actatcaaat tgtagctgtt gaaggttatt tttcatcagg tagtgcttct 660
   atcactgtta gcggtaccgg tgattacaaa gatgatgacg ataaaagtgg tgaaaacctt 720
   tattttcaag gccataatca ccgtcacaaa cacactggtt aa 762
<210> 147
   <211> 1332
   <212> DNA
   <213> Artificial Sequence
<220>
   <223> codon optimized sequence comprising IS99
<400> 147
   atgacagtat acacagcttc agtaacagct ccagtaaaca ttgctacatt aaaatactgg      60
   ggtaaacgtg acacaaaatt aaacttacca acaaactcat caatttcagt aacattatca     120
   caagacgact tacgtacatt aacatcagct gctacagctc cagaattcga acgtgacaca     180
   ttatggttaa acggtgaacc acactcaatt gacaacgaac gtacacaaaa ctgtttacgt     240
   gacttacgtc aattacgtaa agaaatggaa tcaaaagacg cttcattacc aacattatca     300
   caatggaaat tacacattgt atcagaaaac aacttcccaa cagctgctgg tttagcttca     360
   tcagctgctg gtttcgctgc tttagtatca gctattgcta aattatacca attaccacaa     420
   tcaacatcag aaatttcacg tattgctcgt aaaggttcag gttcagcttg tcgttcatta     480
   ttcggtggtt acgtagcttg ggaaatgggt aaagctgaag acggtcacga ctcaatggct     540
   gtacaaattg ctgactcatc agactggcca caaatgaaag cttgtgtatt agtagtatca     600
   gacattaaaa aagacgtatc atcaacacaa ggtatgcaat taacagtagc tacatcagaa     660
   ttattcaaag aacgtattga acacgtagta ccaaaacgtt tcgaagtaat gcgtaaagct     720
   attgtagaaa aagacttcgc tacattcgct aaagaaacaa tgatggactc aaactcattc     780
   cacgctacat gtttagactc attcccacca attttctaca tgaacgacac atcaaaacgt     840
   attatttcat ggtgtcacac aattaaccaa ttctacggtg aaacaattgt agcttacaca     900
   ttcgacgctg gtccaaacgc tgtattatac tacttagctg aaaacgaatc aaaattattc     960
   gctttcattt acaaattatt cggttcagta ccaggttggg acaaaaaatt cacaacagaa    1020
   caattagaag ctttcaacca ccaattcgaa tcatcaaact tcacagctcg tgaattagac    1080
   ttagaattac aaaaagacgt agctcgtgta attttaacac aagtaggttc aggtccacaa    1140
   gaaacaaacg aatcattaat tgacgctaaa acaggtttac caaaagaaac cggttaccca    1200
   tacgacgtac ctgactatgc ttacccttac gacgtaccag actatgctta tccatacgac    1260
   gtaccagact acgctgaaaa cttatacttc caaggtcacc accaccacca ccatcaccac    1320
   ccaccaggtt aa                                                         1332
<210> 148
   <211> 660
   <212> DNA
   <213> Artificial Sequence
<220>
   <223> codon optimized sequence comprising CAT
<400> 148
   atggagaaaa aaatcactgg atataccacc gttgatatat cccaatggca tcgtaaagaa 60
   cattttgagg catttcagtc agttgctcaa tgtacctata accagaccgt tcagctggat 120
   attacggcct ttttaaagac cgtaaagaaa aataagcaca agttttatcc ggcctttatt 180
   cacattcttg cccgcctgat gaatgctcat ccggaattcc gtatggcaat gaaagacggt 240
   gagctggtga tatgggatag tgttcaccct tgttacaccg ttttccatga gcaaactgaa 300
   acgttttcat cgctctggag tgaataccac gacgatttcc ggcagtttct acacatatat 360
   tcgcaagatg tggcgtgtta cggtgaaaac ctggcctatt tccctaaagg gtttattgag 420
   aatatgtttt tcgtctcagc caatccctgg gtgagtttca ccagttttga tttaaacgtg 480
   gccaatatgg acaacttctt cgcccccgtt ttcaccatgg gcaaatatta tacgcaaggc 540
   gacaaggtgc tgatgccgct ggcgattcag gttcatcatg ccgtttgtga tggcttccat 600
   gtcggcagaa tgcttaatga attacaacag tactgcgatg agtggcaggg cggggcgtaa 660
<210> 149
   <211> 90
   <212> DNA
   <213> Artificial Sequence
<220>
   <223> PCR primer
<400> 149
   agtttttctc aattttttat tttttttgtt ttttctctaa aaatcaaaaa ttcaattttg 60
   agaaaacgta agatctccta ggaaaatgaa 90
<210> 150
   <211> 90
   <212> DNA
   <213> Artificial Sequence
<220>
   <223> PCR primer
<400> 150
   tcggtttaag acaatgggaa aagttagatg cctagagtat tgattatcga gcaaatatct 60
   tctcatctgt gacgggctcg agactagtgg 90
<210> 151
   <211> 90
   <212> DNA
   <213> Artificial Sequence
<220>
   <223> PCR primer
<400> 151
   tggattggta tcaacgcgca ggcatagttc gagaaaaatt atccagaggc aatgacaacc 60
   agcatctcct agtgctagct aaagaagttg 90
<210> 152
   <211> 90
   <212> DNA
   <213> Artificial Sequence
<220>
   <223> PCR primer
<400> 152
   tcaaaaaatt catactttgt ttttttattt tttctgagtt tttaatcaaa aaactttttg 60
   tataaaattg ggctcgagac tagtttgtcc 90
<210> 153
   <211> 22
   <212> DNA
   <213> Artificial Sequence
<220>
   <223> PCR primer
<400> 153
   tcttactgga atgatggaca cg 22
<210> 154
   <211> 22
   <212> DNA
   <213> Artificial Sequence
<220>
   <223> PCR primer
<400> 154
   gtgtttgtga cggtgattat gg 22
<210> 155
   <211> 22
   <212> DNA
   <213> Artificial Sequence
<220>
   <223> PCR primer
<400> 155
   tgtggacctg aacctacttg tg 22
<210> 156
   <211> 22
   <212> DNA
   <213> Artificial Sequence
<220>
   <223> PCR primer
<400> 156
   gaaatgggta aagctgaaga cg 22
<210> 157
   <211> 20
   <212> DNA
   <213> Artificial Sequence
<220>
   <223> PCR primer
<400> 157
   cttccaaaac cacctgttgc 20
<210> 158
   <211> 20
   <212> DNA
   <213> Artificial Sequence
<220>
   <223> PCR primer
<400> 158
   accgtctgga tcaaaagcag 20
<210> 159
   <211> 20
   <212> DNA
   <213> Artificial Sequence
<220>
   <223> PCR primer
<400> 159
   ttggagtggt tctgttcgtg 20
<210> 160
   <211> 21
   <212> DNA
   <213> Artificial Sequence
<220>
   <223> PCR primer
<400> 160
   cagcgtacat acgtcctgga t 21
<210> 161
   <211> 20
   <212> DNA
   <213> Artificial Sequence
<220>
   <223> PCR primer
<400> 161
   gttgcgctca accaacatta 20
<210> 162
   <211> 20
   <212> DNA
   <213> Artificial Sequence
<220>
   <223> PCR primer
<400> 162
   gtgacggtgg ttgtgtcctt 20
<210> 163
   <211> 20
   <212> DNA
   <213> Artificial Sequence
<220>
   <223> PCR primer
<400> 163
   cctgcaggtg gttcttcaat 20
<210> 164
   <211> 20
   <212> DNA
   <213> Artificial Sequence
<220>
   <223> PCR primer
<400> 164
   atgtcaatag cgccaacaca 20
<210> 165
   <211> 25
   <212> DNA
   <213> Artificial Sequence
<220>
   <223> PCR primer
<400> 165
   tggattataa agatgatgac gacaa 25
<210> 166
   <211> 22
   <212> DNA
   <213> Artificial Sequence
<220>
   <223> PCR primer
<400> 166
   gctgctgcaa ctggtaaata ga 22
<210> 167
   <211> 20
   <212> DNA
   <213> Artificial Sequence
<220>
   <223> PCR primer
<400> 167
   tccagcagaa tcaaaagcaa 20
<210> 168
   <211> 20
   <212> DNA
   <213> Artificial Sequence
<220>
   <223> PCR primer
<400> 168
   gcaccttcag gtaagccttg 20
<210> 169
   <211> 21
   <212> DNA
   <213> Artificial Sequence
<220>
   <223> PCR primer
<400> 169
   aagacgatga cgacaaaggt g 21
<210> 170
   <211> 21
   <212> DNA
   <213> Artificial Sequence
<220>
   <223> PCR primer
<400> 170
   tgttatcagc acgaccttca a 21

## Description

### BACKGROUND

Algae are unicellular organisms, producing oxygen by photosynthesis. One group, the microalgae, are useful for biotechnology applications for many reasons, including their high growth rate and tolerance to varying environmental conditions. The use of microalgae in a variety of industrial processes for commercially important products is known and/or has been suggested. For example, microalgae have uses in the production of nutritional supplements, pharmaceuticals, natural dyes, a food source for fish and crustaceans, biological control of agricultural pests, production of oxygen and removal of nitrogen, phosphorus and toxic substances in sewage treatment, and pollution controls, such as biodegradation of plastics or uptake of carbon dioxide.

Microalgae, like other organisms, contain lipids and fatty acids as membrane components, storage products, metabolites and sources of energy. Some algal strains, diatoms, and cyanobacteria have been found to contain proportionally high levels of lipids (over 30%). Microalgal strains with high oil or lipid content are of great interest in the search for a sustainable feedstock for the production of biofuels.

Some wild-type algae are suitable for use in various industrial applications. However, it is recognized that by modification of algae to improve particular characteristics useful for the aforementioned applications, the relevant processes are more likely to be commercially viable. To this end, algal strains can be developed which have improved characteristics over wild-type strains. Such developments have been made by traditional techniques of screening and mutation and selection. Further, recombinant DNA technologies have been widely suggested for algae. Such approaches may increase the economic validity of production of commercially valuable products.

One area in which algae have received increasing attention is the production of fuel products. Fuel products, such as oil, petrochemicals, and other substances useful for the production of petrochemicals are increasingly in demand. Much of today's fuel products are generated from fossil fuels, which are not considered renewable energy sources, as they are the result of organic material being covered by successive layers of sediment over the course of millions of years. There is also a growing desire to lessen dependence on imported crude oil. Public awareness regarding pollution and environmental hazards has also increased. As a result, there has been a growing interest and need for alternative methods to produce fuel products. Thus, there exists a pressing need for alternative methods to develop fuel products that are renewable, sustainable, and less harmful to the environment. One potential source of alternative production of fuel and fuel precursors is genetically modified organisms, such as bacteria and plants, including algae. To date, algae have yet to be successfully developed as a commercially viable platform for biofuel production, due mainly to the high cost of harvesting and processing of algae for recovery of the biofuel. Thus, a need exists to develop host organisms such as algae (for example, Scenedesmus sp. , Chlamydomonas sp., and Dunaliella sp.) and bacteria for which such costs are reduced. One way of genetically modifying an organism is to transform the organism with a nucleic acid that encodes for a protein, wherein expression of the protein results, for example, in the increased production of a product, or in the production of a product that the organism does not usually make.

### SUMMARY

The invention concerns a method of transforming a chloroplast genome of a *Scenedesmus sp.* with at least one exogenous nucleotide sequence, comprising: i) obtaining the exogenous nucleotide sequence flanked by two sequences that are homogenous to the chloroplast genome, wherein the exogenous nucleotide sequence comprises a nucleic acid sequence encoding a protein and a further nucleic acid sequence encoding a selectable marker; ii) binding the exogenous nucleotide sequence onto a particle; and iii) shooting the exogenous nucleotide sequence into the *Scenedesmus sp.* by particle bombardment carried out by a biolistic device with a helium pressure of at least 300psi,, wherein the chloroplast genome is transformed with the exogenous nucleotide sequence. Preferred embodiments are defined in the appended claims.

### BRIEF DESCRIPTION OF THE DRAWINGS

These and other features, aspects, and advantages of the present disclosure will become better understood with regard to the following description, appended claims and accompanying figures where:
**Figure** 1 shows a graphical representation of the mutated psbA fragment and 3'UTR vector used to engineer DCMU^{r} into *S. dimorphus.*
**Figure 2** shows amplification and digestion of DNA from psbA 264A DCMU^{r} transformants (3 and 4) and *S. dimorphus* wildtype (WT). U = uncut DNA and C = cut DNA (digested with Xba1).
**Figure** 3 shows a psbA S264 transformant that is DCMU^{r} and atrazine^{r}.
**Figure** 4 shows a graphical representation of p04-38.
**Figure 5** shows a graphical representation of p04-21.
**Figure 6** shows PCR amplification of DNA from *S. dimorphus* CAM^{r} transformants.
**Figure 7** shows a graphical representation of vector p04-31 used to transform *S. dimorphus.*
**Figure 8** shows a multiscreen of BD11 clones.
**Figure 9** shows a Western of SE0070 expressing BD11 (subclones of parent 2 and 4).
**Figure 10** shows endoxylanase activity in clarified lysates from *S. dimorphus* transfonnants containing the endoxylanase gene (parent 2 and 4).
**Figure 11** shows a graphical representation of p04-28.
**Figure 12** shows verification of homoplasmicity in several lines of S. *dimorphus* engineered with FPP-synthase.
**Figure 13** shows an anti-flag Western with farnesyl disphosphate (FPP) synthase protein expression in 7 transformants.
**Figure 14A** shows an overlay of the Total Ion Chromatogram (TIC) for wild type negative control (untransformed Scenedesmus dimorphus), the engineered strain (S. dimorphus transformed with FPP synthase (avian), and a positive control of FPP. The y axis is abundance. The x axis is time.
**Figure 14B** shows the TIC of the FPP positive control. The retention time of FPP is 11.441 minutes. The y axis is abundance. The x axis is time.
**Figure 14C** shows the mass spectrum of the FPP positive control at 11.441 minutes. The y axis is abundance. The x axis is m/z (mass to charge ratio).
**Figure 14D** shows the TIC of the engineered strain (S. dimorphus transformed with FPP synthase (avian) incubated with IPP and DMAPP. The retention time of the product (FPP) is 11.441 minutes. The y axis is abundance. The x axis is time.
**Figure 14E** shows the mass spectrum of the engineered strain (S. dimorphus transformed with FPP synthase (avian) at 11.441 minutes. The y axis is abundance. The x axis is m/z (mass to charge ratio).
**Figure 14F** shows the TIC of the untransformed wild type S. dimorphus strain incubated with IPP and DMAPP. The y axis is abundance. The x axis is time.
**Figure 14G** shows the mass spectrum of the untransformed wild type S. dimorphus strain at 11.441 minutes. The y axis is abundance. The x axis is m/z (mass to charge ratio).
**Figure 15A** shows an overlay of the Total Ion Chromatogram (TIC) for wild type negative control (untransformed Scenedesmus dimorphus) incubated with IPP and DMAPP, the engineered strain (S. dimorphus transformed with IS09 (FPP synthase)) incubated with IPP and DMAPP, and a positive control of FPP. All three enzymatic reactions were incubated with amorphadiene synthase to form amorpha-4,11-diene in a coupled enzyme assay. The y axis is abundance. The x axis is time.
**Figure 15B** shows the TIC of the FPP positive control incubated with amorphadiene synthase. The retention time of amorphadiene is 9.917 minutes. The y axis is abundance. The x axis is time.
**Figure 15C** shows the mass spectrum of the amorphadiene positive control at 9.917 minutes. The y axis is abundance. The x axis is m/z (mass to charge ratio).
**Figure 15D** shows the TIC of the engineered strain (S. dimorphus transformed with IS09 (FPP synthase), incubated with IPP and DMAPP and amorphadiene synthase. The retention time of the product of the reaction (amorphadiene) is 9.917 minutes. The y axis is abundance. The x axis is time.
**Figure 15E** shows the mass spectrum of the product produced by the engineered strain (S. dimorphus transformed with IS09 (FPP synthase)) when incubated with IPP, DMAPP, and amorphadiene synthase. The retention time of the product (amorphadiene) is 9.917 minutes. The y axis is abundance. The x axis is m/z (mass to charge ratio).
**Figure 15F** shows the TIC of the untransfonned wild type S. dimorphus strain incubated with IPP and DMAPP and amorphadiene synthase. The y axis is abundance. The x axis is time.
**Figure 15G** shows the mass spectrum (at 9.917 minutes) of the enzymatic reaction with untransformed wild type S. dimorphus strain incubated with IPP, DMAPP, and amorphadiene synthase. The y axis is abundance. The x axis is m/z (mass to charge ratio).
**Figure 16** shows a graphical representation of p04-196.
**Figures 17A** and **17B** show a comparison of SIM monitored GC/MS chromatogram of *S. dimorphus* transformed with IS-88 (17A) and wild-type *S. dimorphus* (17B). Chromatograms were monitored with ions m/z=229, 135, and 122 (diagnostic for fusicoccadiene). The retention time of the peak in (17A) (7.617 min) matches that of purified fusicoccadiene.
**Figures 18A** and **18B** show a mass spectra at the retention time of the fusicoccadiene peak (t=7.617 min) for *S. dimorphus*-IS88 (A) and wild type *S. dimorphus* (B). The mass spectrum of *S. dimorphus*-IS88 matches well with the known spectrum of fusicoccadiene. The mass spectrum of wild-type shows only background ions. **Figure 18C** shows the mass spectrum of purified fusicoccadiene.
**Figure 19** shows a graphical representation of p04-118.
**Figure 20** shows an anti-flag Western blot *of S. dimorphus* engineered with a gene encoding phytase (FD6).
**Figure 21** shows a graphical representation of p04-162.
**Figure 22** shows that the EreB gene (SEQ ID NO: 25) is amplified from DNA derived from several potential transformants but not from DNA derived from wild type *S. dimorphus.* Controls: W = no DNA; + = plasmid DNA; and D and O are *S. dimorphus* DNA.
**Figure 23** shows a graphical representation of p04-161.
**Figure 24** shows codA plates.
**Figure 25** shows a graphical representation of p04-267.
**Figure 26** shows an anti-flag Western blot of *S*. *dimorphus* engineered with FPP synthase (Is09) and bisabolene synthase (Is 11) genes showing expression of both proteins.
**Figure 27** shows a graphical representation of p04-116.
**Figure 28** shows that endoxylanase is produced as a single peptide (not a fusion with CAT) in engineered *S. dimorphus* cells.
**Figure 29** shows endoxylanase activity in engineered *S. dimorphus* (operon 1_1, 2_1, 2_2, 2_3). + is *S. dimorphus* engineered with *psbD* driving xylanase and "wt" is wild type.
**Figure 30A** and **Figure 30B** show that endoxylanase and CAT are transcribed as a single transcript. Figure 30A shows the primer design and Figure 30B is an agarose gel showing amplification of cDNA from 4 of the 5 transformants corresponding to the endoxylanase-CAT transcript. Regarding Figure 30A, SEQ ID NO: 60 is a nucleic acid sequence of the top strand of RBS1 and SEQ ID NO: 171 is a nucleic acid sequence of the bottom strand of RBS1, as described in Example 13.

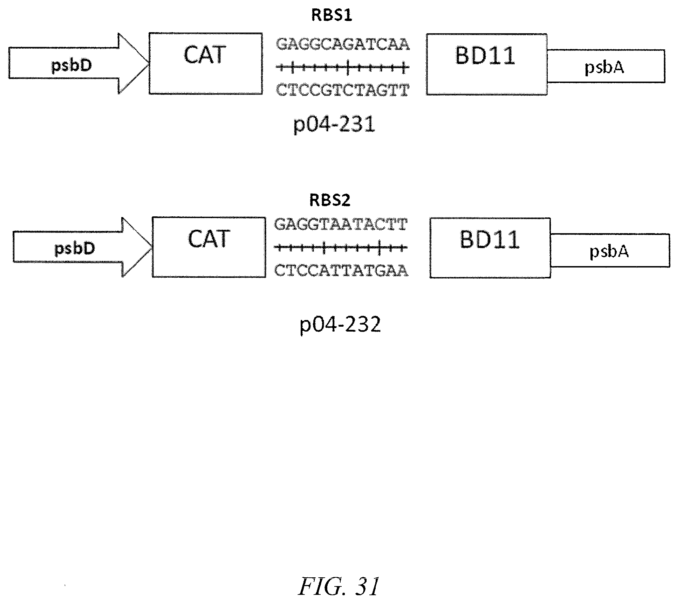

**Figure 31** shows a graphical representation of transforming DNA with different RBS sequences. In both cases, the *psbD* promoter and the *psbA*3'UTR from *S. dimorphus* are used to regulate CAT-RBS-BD11 expression. BD11 encodes the endoxylanse gene from *T. reesei.* These cassettes were subcloned into vector p04-166 between region Homology A and homology region B. Regarding the top half of Figure 31, SEQ ID NO: 60 is a nucleic acid sequence of the top strand of RBS1 and SEQ ID NO: 171 is a nucleic acid sequence of the bottom strand of RBS1, as described in Example 13. Regarding the bottom half of Figure 31, SEQ ID NO: 61 is a nucleic acid sequence of the top strand of RBS2 and SEQ ID NO: 172 is a nucleic acid sequence of the bottom strand of RBS2, as described in Example 13.
**Figure 32A** shows xylanase activity of p04-231 from TAP plates. **Figure 32B** shows xylanase activity of p04-232 from TAP plates. Endoxylanase activity was detected in cells engineered with RBS1 linking CAT and endoxylanase (p04-231) but not with RBS2 (p04-232).
**Figure 33** shows a graphical representation of p04-142.
**Figure 34** shows verification of homoplasmicity in clone 52, an engineered *S. dimorphus* line containing a CAT cassette in the region between psbT and psbN.
**Figure 35** shows a graphical representation of the transforming DNA (**A**) and loopout product **(B)** that results from recombination at the identical D2 (psbD) promoter segments.
**Figure 36** shows failure to amplify a CAT fragment in a multiplex PCR *of S. dimorphus.*
**Figure 37** shows a graphical representation of p04-291 and p04-294. BAD1 and BAD4 are the betaine aldehyde dehydrogenase genes from spinach and sugar beet, respectively.
**Figure 38** shows an anti-HA western blot showing expression of betaine aldehyde dehydrogenase from spinach (291 clones 1, 2, 3, BAD1) or from sugar beet (294 4-1, 5-1, 6-1, 7-1, BAD4) in *S. dimorphus.*
**Figure 39** shows a graphical representation of p45-5 and p45-6.
**Figure 40** shows an agarose gel of EreB amplification from *D*. *tertiolecta* transformants in lanes 4, 5, 6.
**Figure 41** shows a graphical representation of p45-12.
**Figure 42** shows an agarose gel of EreB amplification *from D. tertiolecta* transformant 12-3.
**Figure 43** shows that Xylanase protein (BD11) is detected in *D. tertiolecta* transformant 12-3 via an anti-flag Western blot.
**Figure 44** shows that Xylanase activity is detected in *D. tertiolecta* transformant 12-3. Positive control is *S. dimorphus* engineered with endoxylanase.
**Figure 45** shows vector gutless pUC (2,436 bp).
**Figure 46** shows vector p04-35 (4,304 bp).
**Figure 47** shows vector pSS-007 (6,132 bp).
**Figure 48** shows vector pSS-013 (7,970 bp).
**Figure 49** shows vector pSS-023 (10.322 kb).
**Figure 50** shows Gene Vector 1 (5,774 bp).
**Figure 51** shows Gene Vector 2 (10.198 kb).
**Figure 52** shows Gene Vector 3 (7,111 bp).
**Figure 53** shows vector pRS414 (4,784 bp).
**Figure 54** shows vector pBeloBAC11 (7,507 bp).
**Figure 55** shows vector pLW001 (10.049 kb).
**Figure 56** shows vector pLW092 (13.737 kb).
**Figure 57** shows vector pBeloBAC-TRP (10.524 kb).
**Figure 58** shows vector pLW100 (18.847 kb).
**Figure 59** shows vector p04-198.
**Figure 60** shows vector pSS-035 (6,491 bp).
**Figure 61** shows vector pSS-023 CC93 CC94 (15.083 kb).
**Figure 62** shows vector pSS-023 CC93 CC97 (15.077 kb).
**Figure 63** shows vector pLW100 CC90 CC91 CC92 (26.319 kb).
**Figure 64** shows vector pLW100 four gene assembly (34.509 kb).
**Figure 65** shows pSS-023 restriction digest mapping with *NdeI, PacI, PstI, ScaI, SnaBI,* and *SpeI.*
**Figure 66** shows pLW001 restriction digest mapping with *EcoRV, NotI, PmlI, PvuI*, and *SnaBI.*
**Figures 67A**-**E** show pLW092 restriction digest mapping with *PacI*(c), *PstI*(e), *SeaI*(b), and *XhoI* (d), and uncut (a).
**Figure 68** shows pLW100 restriction digest mapping with *EcoRV, NdeI, NotI, PacI, PstI, ScaI* and *XhoI.*
**Figure 69** shows pSS-035 restriction digest mapping with *EcoPI, EcoRV, KpnI, NotI, PvuII,* and *ScaI.*
**Figure 70** shows plasmid DNA comprising four two-gene contigs digested with NdeI.
**Figure 71** shows plasmid DNA comprising two three-gene contigs digested with NdeI.
**Figure 72** shows plasmid DNA comprising four four-gene contigs digested with NdeI.
**Figure 73** shows PCR amplification of the conserved *psbB-psbT-psbH-psbN* gene cluster from S. *dimorphus.*
**Figure 74** shows PCR amplification of the conserved *psbB-psbT-psbH-psbN* gene cluster from a strain of genus *Dunaliella*; an unknown species.
**Figure 75** shows PCR amplification of the conserved *psbB-psbT-psbH-psbN* gene cluster *from N. abudans*.
**Figure 76** shows vector p04-128.
**Figure 77** shows vector p04-129.
**Figure 78** shows vector p04-130.
**Figure 79** shows vector p04-131.
**Figure 80** shows vector p04-142.
**Figure 81** shows vector p04-143.
**Figure 82** shows vector p04-144.
**Figure 83** shows vector p04-145.
**Figure 84** shows a homoplasmicity PCR screen for clones from *S. dimorphus* that have a resistance cassette between either *psbT* and *psbN* (p04-142) or between *psbN* and *psbH* (p04-143).
**Figure 85** shows a homoplasmicity PCR screen for clones from *S. dimorphus* that have a resistance cassette between either *psbH* and *psbK* (p04-144) or 3' of *psbK* (p04-145).
**Figure 86** is a nucleotide alignment of the *psbB* gene from four different algae species. From top to bottom: Chlamy psbB (SEQ ID NO: 173), Chlorella psbB (SEQ ID NO: 174), Porphyra psbB (SEQ ID NO: 175), and Scenedesmus psbB (SEQ ID NO: 176). The Majority nucleic acid sequence is also shown (SEQ ID NO: 177).
**Figure 87** is a nucleotide alignment of the *psbH* region from four different algae species. From top to bottom: Chlamy psbH(SEQ ID NO: 178), Chlorella psbH (SEQ ID NO: 179), Porphyra psbH (SEQ ID NO: 180), and Scenedesmus psbH (SEQ ID NO: 181). The Majority nucleic acid sequence is also shown (SEQ ID NO: 182)
**Figure 88** is an alignment of the genome region from the *psbB* gene to the *psbH* gene of four different algae species.
**Figure 89** is vector p04-151.
**Figure 90A-D** shows restriction enzyme mapping results.
**Figure 91** shows vector pLW106.
**Figure 92A and B** depict 4 clones that screen PCR positive for both BD11 and IS99.
**Figure 93A-C** depict 4 clones that screen PCR positive for CC90, CC91, and CC92.
**Figure 94A and B** depict 2 clones that screen PCR positive for IS61, IS62, IS57 and IS116.

### DETAILED DESCRIPTION

The following detailed description is provided to aid those skilled in the art in practicing the present disclosure. Even so, this detailed description should not be construed to unduly limit the present disclosure as modifications and variations in the embodiments discussed herein can be made by those of ordinary skill in the art without departing from the scope of the present disclosure.

As used in this specification and the appended claims, the singular forms "a", "an" and "the" include plural reference unless the context clearly dictates otherwise.

### Endogenous

An endogenous nucleic acid, nucleotide, polypeptide, or protein as described herein is defined in relationship to the host organism. An endogenous nucleic acid, nucleotide, polypeptide, or protein is one that naturally occurs in the host organism.

### Exogenous

An exogenous nucleic acid, nucleotide, polypeptide, or protein as described herein is defined in relationship to the host organism. An exogenous nucleic acid, nucleotide, polypeptide, or protein is one that does not naturally occur in the host organism or is a different location in the host organism.

Examples of genes, nucleic acids, proteins, and polypeptides that can be used in the examples disclosed herein include, but are not limited to:
SEQ ID NO: 1 is a PCR primer.
SEQ ID NO: 2 is a PCR primer.
SEQ ID NO: 3 is a PCR primer.
SEQ ID NO: 4 is a PCR primer.
SEQ ID NO: 5 is a PCR primer.
SEQ ID NO: 6 is a PCR primer.
SEQ ID NO: 7 is a PCR primer.
SEQ ID NO: 8 is a PCR primer.
SEQ ID NO: 9 is a PCR primer.
SEQ ID NO: 10 is a PCR primer.
SEQ ID NO: 11 is a PCR primer.
SEQ ID NO: 12 is a PCR primer.
SEQ ID NO: 13 is a PCR primer.
SEQ ID NO: 14 is a PCR primer.
SEQ ID NO: 15 is a PCR primer (#4682).
SEQ ID NO: 16 is a PCR primer (#4982).
SEQ ID NO: 17 is a PCR primer.
SEQ ID NO: 18 is a PCRprimer.
SEQ ID NO: 19 is a PCR primer.
SEQ ID NO: 20 is a nucleotide sequence of an artificial FLAG epitope tag linked to a MAT epitope tag by a TEV protease site.
SEQ ID NO: 21 is a gene encoding an endoxylanase from *T. reesei* codon optimized for chloroplast expression in C. *reinhardtii.*
SEQ ID NO: 22 is a nucleotide sequence of an artificial TEV protease site linked to a FLAG epitope tag.
SEQ ID NO: 23 is a gene encoding an FPP synthase from G. *gallus* codon optimized for chloroplast expression in C. *reinhardtii.*
SEQ ID NO: 24 is a nucleotide sequence of an artificial streptavidin epitope tag.
SEQ ID NO: 25 is a gene encoding a fusicoccadiene synthase from P. *amygdali* codon optimized according to the most frequent codons in the C. *reinhardtii* chloroplast.
SEQ ID NO: 26 is a gene encoding a phytase from *E. coli* codon optimized for chloroplast expression in C. *reinhardtii.*
SEQ ID NO: 27 is a nucleotide sequence of an artificial FLAG epitope tag linked to a MAT epitope tag by a TEV protease site.
SEQ ID NO: 28 is a modified chloramphenicol acetyltransferase gene from *E. coli* with the nucleotide at position 64 changed from an A to a G, the nucleotides at positions 436, 437, and 438 were changed from TCA to AGC, and the nucleotide at position 516 was changed from a C to a T.
SEQ ID NO: 29 is a modified erythromycin esterase gene from *E. coli* with the nucleotide at position 153 changed from a C to a T, the nucleotide at position 195 changed from a T to a C, the nucleotide at position 198 changed from a A to a C, the nucleotide at position 603 changed from a T to a A, the nucleotide at position 1194 changed from a C to a T, and the nucleotide at position 1203 changed from a T to an A.
SEQ ID NO: 30 is a fragment of genomic DNA from *S. dimorphus* that encodes a region containing a portion of the 3' end of the *psbA* gene and some untranslated region, with nucleotide 1913 of the fragment mutated from a T to a G for the S264A mutation, and nucleotides 1928 to 1930 mutated from CGT to AGA to generate a silent *XbaI* restriction site.
SEQ ID NO: 31 is a gene encoding a cytosine deaminase from *E. coli* codon optimized for expression in the chloroplast of C. *reinhardtii.*
SEQ ID NO: 32 is a gene encoding a betaine aldehyde dehydrogenase from *S. oleracea* codon optimized according to the tRNA usage of the chloroplast of C. *reinhardtii.*
SEQ ID NO: 33 is a nucleotide sequence of an artificial 3xHA tag linked to a 6xHIS tag by a TEV protease site.
SEQ ID NO: 34 is a gene encoding a betaine aldehyde dehydrogenase from *B. vulgaris* codon optimized for expression in the chloroplast of C. *reinhardtii.*
SEQ ID NO: 35 is a gene encoding an E-alpha-bisabolene synthase *from A. grandis* codon optimized for expression in the chloroplast of C. *reinhardtii.*
SEQ ID NO: 36 is a modified nucleotide sequence that is the reverse complement of SEQ ID NO: 37 with extra nucleotides on the 5' and 3'ends; nucleotides 1-43 are extra on the 3' end and nucleotides 532-541 are extra on the 5' end.
SEQ ID NO: 37 is a nucleotide sequence of the endogenous promoter from the *psbA* gene of *S. dimorphus* that was cloned into an integration vector.
SEQ ID NO: 38 is a modified nucleotide sequence that is the reverse complement of SEQ ID NO: 39 with extra nucleotides on the 5' end and a nucleotide insertion; nucleotides 535-716 are extra 5' sequence and nucleotides 176-188 are the insertion.
SEQ ID NO: 39 is a nucleotide sequence of the endogenous promoter for the *psbB* gene of *S. dimorphus* that was cloned into integration vectors.
SEQ ID NO: 40 is a sequence of the endogenous promoter for the *psbD* gene of *S*. *dimorphus* that was cloned into integration vectors.
SEQ ID NO: 41 is a modified nucleotide sequence that is the reverse complement of SEQ ID NO: 42 with extra sequence on the 5'; nucleotides 537-464 are extra sequences on the 5' end, nucleotide 308 is changed from a C to a T, nucleotide 310 is changed from a C to a T, and nucleotide 259 is changed from an A to a G.
SEQ ID NO: 42 is a nucleotide sequence of the endogenous promoter for the *tufA* gene of *S. dimorphus* that was cloned into integration vectors.
SEQ ID NO: 43 is a modified nucleotide sequence that is the reverse of SEQ ID NO: 44 with extra sequences on the 5' end; nucleotides 550-557 are extra sequences on the 5' end.
SEQ ID NO: 44 is a nucleotide sequence for the endogenous promoter of the *rpoA* of *S. dimorphus.*
SEQ ID NO: 45 is a nucleotide sequence for the endogenous promote of the *cemA* gene in *S. dimorphus* that was cloned into integration vectors.
SEQ ID NO: 46 is a modified nucleotide sequence that is the reverse complement of SEQ ID NO: 47 with an insertion at nucleotides 233-266.
SEQ ID NO: 47 is a nucleotide sequence for the endogenous promoter of the *ftsH* gene in *S. dimorphus* that was cloned into integration vectors.
SEQ ID NO: 48 is a modified nucleotide sequence of SEQ ID NO: 49 that has extra sequences on the 5' end; nucleotides 1-19 are extra sequences, nucleotide 404 has been changed from an A to a T.
SEQ ID NO: 49 is a nucleotide sequence for the endogenous promoter of the *rbcL* gene in *S. dimorphus* that was cloned into integration vectors.
SEQ ID NO: 50 is a modified nucleotide sequence of SEQ ID NO: 51 that has 24 nucleotides truncated on the 5' end; the nucleotide at position 2 is changed from a G to a C, position 5 is changed from an A to a G, at positions 199 and 200 two T's are inserted, and at position 472 it is changed from an A to a G.
SEQ ID NO: 51 is the nucleotide sequence of the endogenous promoter for the *chlB* gene from S. *dimorphus* that was cloned into integration vectors.
SEQ ID NO: 52 is a modified nucleotide sequence of SEQ ID NO: 53 where nucleotides 1-3 are extra sequence, the nucleotide at position 442 is a G insertion, and the R at position 482 is a result of poor sequencing.
SEQ ID NO: 53 is a nucleotide sequence for the endogenous promoter of the *petA* gene in *S. dimorphus* that was cloned into integration vectors.
SEQ ID NO: 54 is a modified nucleotide sequence that is the reverse complement of SEQ ID NO: 55 where nucleotides 3, 8, 18, 21, 49, 57, and 82 are insertions, nucleotides 484-503 are extra on the 5' end, nucleotide 26 is changed from a C to a T, and nucleotide 30 is changed from an A to a C.
SEQ ID NO: 55 is the nucleotide sequence of the endogenous promoter for the *petB* gene from S. *dimorphus* that was cloned into integration vectors.
SEQ ID NO: 56 is the modified nucleotide sequence of SEQ ID NO: 57 that has 3 nucleotides truncated on the 5' end.
SEQ ID NO: 57 is the nucleotide sequence of the endogenous terminator region for the *rbcL* gene from *S. dimorphus* that was cloned into integration vectors.
SEQ ID NO: 58 is the nucleotide sequence of the endogenous terminator region for *the psbA* gene from *S. dimorphus* that was cloned into integration vectors.
SEQ ID NO: 59 is the nucleotide sequence of the endogenous terminator region for the *psaB* gene from *S. dimorphus* that was cloned into integration vectors.
SEQ ID NO: 60 is a nucleic acid linker sequence (RBS1).
SEQ ID NO: 61 is a nucleic acid linker sequence (RBS2).
SEQ ID NO: 62 is the nucleotide sequence of the endogenous promoter region for the *psbD* gene from *D. tertiolecta* that was cloned into integration vectors.
SEQ ID NO: 63 is the nucleotide sequence of the endogenous promoter region for the *tufA* gene from *D. tertiolecta* that was cloned into integration vectors.
SEQ ID NO: 64 is the nucleotide sequence of the endogenous terminator region for the *rbcL* gene from *D. tertiolecta* that was cloned into integration vectors.
SEQ ID NO: 65 is the nucleotide sequence of the endogenous terminator region for the *psbA* gene from a *Dunaliella* isolate of unknown species that was cloned into integration vectors.
SEQ ID NO: 66 is PCR primer 1.
SEQ ID NO: 67 is PCR primer 2.
SEQ ID NO: 68 is PCR primer 3.
SEQ ID NO: 69 is PCR primer 4.
SEQ ID NO: 70 is PCR primer 5.
SEQ ID NO: 71 is PCR primer 6.
SEQ ID NO: 72 is PCR primer 7.
SEQ ID NO: 73 is PCR primer 8.
SEQ ID NO: 74 is PCR primer 9.
SEQ ID NO: 75 is PCR primer 10.
SEQ ID NO: 76 is PCR primer 11.
SEQ ID NO: 77 is PCR primer 12.
SEQ ID NO: 78 is PCRprimer 13.
SEQ ID NO: 79 is PCR primer 14.
SEQ ID NO: 80 is PCR primer 15.
SEQ ID NO: 81 is PCR primer 16.
SEQ ID NO: 82 is PCR primer 17.
SEQ SEQ ID NO: 83 is PCR primer 18.
SEQ ID NO: 84 is PCR primer 19.
SEQ ID NO: 85 is PCR primer 20.
SEQ ID NO: 86 is PCR primer 21.
SEQ ID NO: 87 is PCR primer 22.
SEQ ID NO: 88 is PCR primer 23.
SEQ ID NO: 89 is PCR primer 24.
SEQ ID NO: 90 is PCR primer 25.
SEQ ID NO: 91 is PCR primer 26.
SEQ ID NO: 92 is PCR primer 27.
SEQ ID NO: 93 is PCR primer 28.
SEQ ID NO: 94 is PCR primer 29.
SEQ ID NO: 95 is PCR primer 30.
SEQ ID NO: 96 is PCR primer31.
SEQ ID NO: 97 is PCR primer 32.
SEQ ID NO: 98 is PCR primer 33.
SEQ ID NO: 99 is PCRprimer 34.
SEQ ID NO: 100 is PCR primer 35.
SEQ ID NO: 101 is PCR primer 36.
SEQ ID NO: 102 is PCR primer 37.
SEQ ID NO: 103 comprises a nucleic acid sequence encoding for URA3.
SEQ ID NO: 104 comprises a nucleic acid sequence encoding for ADE2.
SEQ ID NO: 105 comprises a nucleic acid sequence encoding for URA3-ADE2.
SEQ ID NO: 106 is a nucleic acid linker sequence with engineered restriction sites.
SEQ ID NO: 107 comprises a nucleic acid sequence encoding for TRP1-ARS1-CEN4 (from pYAC4).
SEQ ID NO: 108 comprises a nucleic acid sequence encoding for LEU2.
SEQ ID NO: 109 comprises a nucleic acid sequence encoding for CC-93.
SEQ ID NO: 110 comprises a nucleic acid sequence encoding for CC-94.
SEQ ID NO: 111 comprises the contig sequence (CC93-CC94) that was inserted into pSS-023.
SEQ ID NO: 112 comprises the contig sequence (CC93-CC97) that was inserted into pSS-023.
SEQ ID NO: 113 comprises a nucleic acid sequence encoding for CC-97.
SEQ ID NO: 114 comprises the contig sequence (CC90-CC91-CC92) that was inserted into pLW100.
SEQ ID NO: 115 comprises a nucleic acid sequence encoding for CC-90.
SEQ ID NO: 116 comprises a nucleic acid sequence encoding for CC-91.
SEQ ID NO: 117 comprises a nucleic acid sequence encoding for CC-92.
SEQ ID NO: 118 comprises a nucleic acid sequence encoding for HIS3.
SEQ ID NO: 119 comprises a nucleic acid sequence encoding for LYS2.
SEQ ID NO: 120 comprises the contig sequence (IS57-IS116-IS62-IS61) that was inserted into pLW100.
SEQ ID NO: 121 comprises a nucleic acid sequence encoding for IS57.
SEQ ID NO: 122 comprises a nucleic acid sequence encoding for IS116.
SEQ ID NO: 123 comprises a nucleic acid sequence encoding for IS62.
SEQ ID NO: 124 comprises a nucleic acid sequence encoding for IS61.
SEQ ID NO: 125 is a 5,240 base pair sequence from *Scenedesmus obliquus.*
SEQ ID NO: 126 is the A3 homology region.
SEQ ID NO: 127 is the B3 homology region.
SEQ ID NO: 128 comprises a sequence encoding for rblcL-CAT-psbE.
SEQ ID NO: 129 is a degenerate PCR primer.
SEQ ID NO: 130 is a degenerate PCR primer.
SEQ ID NO: 131 is a degenerate PCR primer.
SEQ ID NO: 132 is a degenerate PCR primer.
SEQ ID NO: 133 is genomic sequence of the region encoding *the psbB, psbT, psbN, andpsbHgenes* from *D. tertiolecta.*
SEQ ID NO: 134 is genomic sequence of the region encoding the *psbB, psbT, psbN,* and *psbH* genes from a *Dunaliella* of unknown species.
SEQ ID NO: 135 is a partial genomic sequence of the region encoding the *psbB*, *psbT, psbN,* and *psbH* genes from *N. abudans*; the stretch ofN's represents a gap in the sequence.
SEQ ID NO: 136 is genomic sequence of the region encoding the *psbB, psbT, psbN,* and *psbH* genes from an isolate of C. *vulgaris*.
SEQ ID NO: 137 is genomic sequence of the region encoding the *psbB, psbT, psbN,* and *psbH* genes from *T. suecica.*
SEQ ID NO: 138 is PCR primer (#4682).
SEQ ID NO: 139 is PCR primer (#4982).
SEQ ID NO: 140 is PCR primer 4684.
SEQ ID NO: 141 is PCR primer 4685.
SEQ ID NO: 142 is PCR primer 4686
SEQ ID NO: 143 is PCR primer 4687.
SEQ ID NO: 144 is PCR primer 4688.
SEQ ID NO: 145 is PCR primer 4689.
SEQ ID NO: 146 comprise a nucleotide sequence encoding BD11.
SEQ ID NO: 147 comprise a nucleotide sequence encoding IS99.
SEQ ID NO: 148 comprise a nucleotide sequence encoding CAT.
SEQ ID NO: 149 to SEQ ID NO: 170 are PCR primers.

The present disclosure relates to methods of transforming various species of algae, for example, algae from the genus *Scenedesmus* and from the genus *Dunaliella,* vectors and nucleic acid constructs useful in conducting such transformations, and recombinant *Scenedesmus* and *Dunaliella* organisms produced using the vectors and methods disclosed herein. In one embodiment, the *Scenedesmus sp.* utilized is *Scenedesmus dimorphus. Scenedesmus sp.* are members of the Chlorophyceans a diverse assemblage of green algae. *Scenedesmus* is a genus consisting of unicells or flat coenobial colonies of 2, 4, 8 or 16 linearly arranged cells. Cells contain a single plastid with pyrenoid and uninucleate. *Scenedesmus sp.* are common inhabitants of the plankton of freshwaters and brackish waters and occasionally form dense populations. In one example, the organism utilized is from the genus Dunaliella. In another example, the Dunaliella sp. is D. tertiolecta.

One embodiment, the disclosure provides vectors useful in the transformation of *Scenedesmus sp.,* for example, *Scenedesmus dimorphus* or *Scenedesmus obliquus*. In another, example, the disclosure provides vectors useful in the transformation of *Dunaliella sp.,* for example, *Dunaliella tertiolecta.* An expression cassette can be constructed in an appropriate vector. In some instances, the cassette is designed to express one or more protein-coding sequences in a host cell. Such vectors can be constructed using standard techniques known in the art. In a typical expression cassette, the promoter or regulatory element is positioned on the 5' or upstream side of a coding sequence whose expression is desired. In other cassettes, a coding sequence may be flanked by sequences which allow for expression upon insertion into a target genome (e.g., nuclear or plastid). For example, a nucleic acid encoding an enzyme involved in the synthesis of a compound of interest, for example an isoprenoid, such that expression of the enzyme is controlled by a naturally occurring regulatory element. Any regulatory element which provides expression under appropriate conditions such that the mRNA or protein product is expressed to a level sufficient to produce useful amount of the desired compound can be used.

One or more additional protein coding sequences can be operatively fused downstream or 3' of a promoter. Coding sequences for single proteins can be used, as well as coding sequences for fusions of two or more proteins. Coding sequences may also contain additional elements that would allow the expressed proteins to be targeted to the cell surface and either be anchored on the cell surface or be secreted to the environment. A selectable marker is also employed in the design of the vector for efficient selection of algae transformed by the vector. Both a selectable marker and another sequence which one desires to introduce may be introduced fused to and downstream of a single promoter. Alternatively, two protein coding sequences can be introduced, each under the control of a promoter.

One approach to construction of a genetically manipulated strain of *Scenedesmus* or *Dunaliella* involves transformation with a nucleic acid which encodes a gene of interest, typically an enzyme capable of converting a precursor into a fuel product or precursor of a fuel product (e.g., an isoprenoid or fatty acid), a biomass degrading enzyme, or an enzyme for the improvement of a characteristic of a feedstuff. In some embodiments, a transformation may introduce nucleic acids into any plastid of the host alga cell (e.g., chloroplast). In other embodiments, a transformation may introduce nucleic acids into the nuclear genome of the host cell. In still other embodiments, a transformation introduces nucleic acids into both the nuclear genome and a plastid. In some instances, the nucleic acids encoding proteins of interest (e.g., transporters or enzymes) are codon-biased for the intended site of insertion (e.g., nuclear codon-biased for insertion in the nucleus, chloroplast codon-biased for insertion in the chloroplast).

To construct the vector, the upstream DNA sequences of a gene expressed under control of a suitable promoter may be restriction mapped and areas important for the expression of the protein characterized. The exact location of the start codon of the gene is determined and, making use of this information and the restriction map, a vector may be designed for expression of an endogenous or exogenous protein by removing the region responsible for encoding the gene's protein but leaving the upstream region found to contain the genetic material responsible for control of the gene's expression. A synthetic oligonucleotide is typically inserted in the location where the protein sequence once was, such that any additional gene could be cloned in using restriction endonuclease sites in the synthetic oligonucleotide (i.e., a multicloning site). An unrelated gene (or coding sequence) inserted at this site would then be under the control of an extant start codon and upstream regulatory region that will drive expression of the foreign (i.e., not normally there) protein encoded by this gene. Once the gene for the foreign protein is put into a cloning vector, it can be introduced into the host organism using any of several methods, some of which might be particular to the host organism. Variations on these methods are amply described in the general literature.

The term "exogenous" is used herein in a comparative sense to indicate that a nucleotide sequence (or polypeptide) being referred to is from a source other than a reference source and is different from the sequence of the reference, or is linked to a second nucleotide sequence (or polypeptide) with which it is not normally associated, or is modified such that it is in a form that is not normally associated with a reference material. For example, a polynucleotide encoding an enzyme is exogenous with respect to a nucleotide sequence of a chloroplast, where the polynucleotide is not normally found in the chloroplast (e.g., a mutated polynucleotide encoding a chloroplast sequence or a nuclear sequence). As another example, a polynucleotide encoding an enzyme is exogenous with respect to a host organism where the polynucleotide comprises operatively linked sequences (e.g., promoters, homologous recombination sites, selectable markers, and/or termination sequences), that are not normally found in the reference organism.

Polynucleotides encoding enzymes and other proteins useful in the present disclosure may be isolated and/or synthesized by any means known in the art, including, but not limited to cloning, sub-cloning, and PCR. A vector herein may encode polypeptide(s) having a role in the mevalonate pathway, such as, for example, thiolase, HMG-CoA synthase, HMG-CoA reductase, mevalonate kinase, phosphemevalonate kinase, and mevalonate-5-pyrophosphate decarboxylase. In other embodiments, the polypeptides are enzymes in the non-mevalonate pathway, such as DOXP synthase, DOXP reductase, 4-diphosphocytidyl-2-C-methyl-D-erythritol synthase, 4-diphophocytidyl-2-C-methyl-D-erythritol kinase, 2-C-methyl-D-erythritol 2,4,-cyclodiphosphate synthase, HMB-PP synthase, HMB-PP reductase, or DOXP reductoisomerase.

One example is directed to a vector comprising a nucleic acid encoding an enzyme capable of modulating a fusicoccadiene biosynthetic pathway. Such a vector may further comprise a promoter for expression of the nucleic acid in algae. Nucleic acid(s) included in such vectors may contain a codon biased form of a gene, optimized for expression in a host organism of choice. In one example, the fusicoccadiene produced is fusicocca-2,10(14)-diene. Another aspect of the present disclosure is directed to a vector comprising a nucleic acid encoding an enzyme that produces a fusicoccadiene when the vector is integrated into a genome of an organism, such as photosynthetic algae, wherein the organism does not produce fusicoccadiene without the vector and wherein the fusicoccadiene is metabolically inactive in the organism.

Further provided herein is a method of producing a fuel product, comprising: a) transforming a *Scenedesmus sp* or *Dunaliella sp.,* wherein the transformation results in the production or increased production of a fusicoccadiene; b) collecting the fusicoccadiene from the organism; and c) using the fusicoccadiene to produce a fuel product.

The present disclosure also contemplates host cells making polypeptides that contribute to the secretion of fatty acids, lipids or oils, by transforming host cells (e.g., algal cells) and/or organisms comprising host cells with nucleic acids encoding one or more different transporters. In some examples, the host cells or organisms are also transformed with one or more enzymes that contribute to the production of fatty acids, lipids or oils are anabolic enzymes. Some examples of anabolic enzymes that contribute to the synthesis of fatty acids include, but are not limited to, acetyl-CoA carboxylase, ketoreductase, thioesterase, malonyltransferase, dehydratase, acyl-CoA ligase, ketoacylsynthase, enoylreductase and a desaturase. In some examples, the enzymes are catabolic or biodegrading enzymes. In some examples, a single enzyme is produced.

Some host cells may be transformed with multiple genes encoding one or more enzymes. For example, a single transformed cell may contain exogenous nucleic acids encoding enzymes that make up an entire synthesis pathway. One example of a pathway might include genes encoding an acetyl CoA carboxylase, a malonyltransferase, a ketoacylsynthase, and a thioesterase. Cells transformed with entire pathways and/or enzymes extracted from those cells, can synthesize complete fatty acids or intermediates of the fatty acid synthesis pathway. Constructs may contain multiple copies of the same gene, multiple genes encoding the same enzyme from different organisms, and/or multiple genes with one or more mutations in the coding sequence(s).

In some instances, the host cell will naturally produce the fatty acid, lipid, triglyceride or oil of interest. Thus, transformation of the host cell with a polynucleotide encoding a transport protein will allow for secretion or increased secretion of the molecule of interest from the cell. In other instances, the host cell is transformed with a polynucleotide encoding one or more enzymes necessary for the production of the molecule of interest. The enzymes produced by the modified cells result in the production of fatty acids, lipids, triglycerides or oils that may be collected from the cells and/or the surrounding environment (e.g., bioreactor, growth medium). In some examples, the collection of the fatty acids, lipids, triglycerides or oils is performed after the product is secreted from the cell via a cell membrane transporter.

Synthesis of fatty acids, lipids or oils can also be accomplished by engineering a cell to express an accessory molecule or modulation molecule. In certain examples, the accessory molecule is an enzyme that produces a substrate utilized by a fatty acid synthesizing enzyme. In some examples the accessory or modulation molecule contributes to the growth or nourishment of the biomass.

An additional aspect of the present disclosure provides a vector comprising a nucleic acid encoding a biomass degrading enzyme and a promoter configured for expression of the nucleic acids in a non-vascular photosynthetic organism, for example a *Scenedesmus sp.* and more particularly *S*. *dimorphus* or *Dunaliella sp..* Vectors of the present disclosure may contain nucleic acids encoding more than one biomass degrading enzyme and, in other instances, may contain nucleic acids encoding polypeptides which covalently link biomass degrading enzymes. Biomass degrading enzymes may include cellulolytic enzymes, hemicellulolytic enzymes and ligninolytic enzymes. More specifically, the biomass degrading enzymes maybe exo-β-glucanase, endo-β-glucanase, β-glucosidase, endoxylanase, or lignase. Nucleic acids encoding the biomass degrading enzymes may be derived from fungal or bacterial sources, for example, those encoding exo-β-glucanase in Trichoderma viride, exo-β-glucanase in Trichoderma reesei, exo-β-glucanase in Aspergillus aculeatus, endo-β-glucanase in Trichoderma reesei, endo-β-glucanase in Aspergillus niger, β- glucosidase in Trichoderma reesei, β-glucosidase in Aspergillus niger endoxylanase in Trichoderma reesei, and endoxylanase in Aspergillus niger. Other nucleic acids encoding biomass degrading enzymes may be endogenous to the organisms.

Also provided is a composition containing a plurality of vectors each of which encodes a different biomass degrading enzyme and a promoter for expression of said biomass degrading enzymes in a chloroplast. Such compositions may contain multiple copies of a particular vector encoding a particular enzyme. In some instances, the vectors will contain nucleic acids encoding cellulolytic, hemicellulolytic and/or ligninolytic enzymes. More specifically, the plurality of vectors may contain vectors capable of expressing exo-β-glucanase, endo-β-glucanase, β-glucosidase, endoxylanase and/or lignase. Some of the vectors of this example are capable of insertion into a chloroplast genome and such insertion can lead to disruption of the photosynthetic capability of the transformed chloroplast. Insertion of other vectors into a chloroplast genome does not disrupt photosynthetic capability of the transformed chloroplast. Some vectors provide for expression of biomass degrading enzymes which are sequestered in a transformed chloroplast.

Another vector encodes a plurality of distinct biomass degrading enzymes and a promoter for expression of the biomass degrading enzymes in a non- vascular photosynthetic organism. The biomass degrading enzymes may be one or more of cellulollytic, hemicellulolytic or ligninolytic enzymes. In some vectors, the plurality of distinct biomass degrading enzymes is two or more of exo-β-glucanase, endo- β-glucanase, β-glucosidase, lignase and endoxylanase. In some examples, the plurality of enzymes is operatively linked. In other examples, the plurality of enzymes is expressed as a functional protein complex. Insertion of some vectors into a host cell genome does not disrupt photosynthetic capability of the organism. Vectors encoding a plurality of distinct enzymes, may lead to production of enzymes which are sequestered in a chloroplast of a transformed organism. The present disclosure also provides an algal cell and in particular a *Scenedemus sp.* or *Dunaliella sp.* transformed with a vector encoding a plurality of distinct enzymes. For some examples, the organism may be grown in the absence of light and/or in the presence of an organic carbon source.

Yet another aspect provides a genetically modified chloroplast of a *Scenedemus sp.* or *Dunaliella sp*. producing one or more biomass degrading enzymes. Such enzymes may be cellulolytic, hemicellulolytic or ligninolytic enzymes, and more specifically, may be an exo-β-glucanase, an endo-β-glucanase, a β-glucosidase, an endoxylanase, a lignase and/or combinations thereof. The one or more enzymes are be sequestered in the chloroplast in some examples. The present disclosure also provides photosynthetic organisms containing the genetically modified chloroplasts of the present disclosure.

Yet another aspect provides a method for preparing a biomass-degrading enzyme. This method comprises the steps of (1) transforming a photosynthetic, non- vascular organism and in particular a *Scenedesmus sp.* or *Dunaliella sp.* to produce or increase production of said biomass-degrading enzyme and (2) collecting the biomass-degrading enzyme from said transformed organism. Transformation may be conducted with a composition containing a plurality of different vectors encoding different biomass degrading enzymes. Transformation may also be conducted with a vector encoding a plurality of distinct biomass degrading enzymes. Any or all of the enzymes may be operatively linked to each other. In some instances, a chloroplast is transformed. This method may have one or more additional steps, including: (a) harvesting transformed organisms; (b) drying transformed organisms; (c) harvesting enzymes from a cell medium; (d) mechanically disrupting transformed organisms; or (e) chemically disrupting transformed organisms. The method may also comprise further purification of an enzyme through performance liquid chromatography.

Still another method of the present disclosure allows for preparing a biofuel. One step of this method includes treating a biomass with one or more biomass degrading enzymes derived from a photosynthetic, nonvascular organism for a sufficient amount of time to degrade at least a portion of said biomass. The biofuel produced may be ethanol. The enzymes of this method may contain at least traces of said photosynthetic nonvascular organism from which they are derived. Additionally, the enzymes useful for some examples of this method include cellulolytic, hemicellulolytic and ligninolytic enzymes. Specific enzymes useful for some aspects of this method include exo-β-glucanase, endo-β-glucanase, β-glucosidase, endoxylanase, and/or lignase. Multiple types of biomass including agricultural waste, paper mill waste, corn stover, wheat stover, soy stover, switchgrass, duckweed, poplar trees, woodchips, sawdust, wet distiller grain, dray distiller grain, human waste, newspaper, recycled paper products, or human garbage may be treated with this method of the disclosure. Biomass may also be derived from a high-cellulose content organism, such as switchgrass or duckweed. The enzyme(s) used in this method may be liberated from the organism and this liberation may involve chemical or mechanical disruption of the cells of the organism. In an alternate example, the enzyme(s) are secreted from the organism and then collected from a culture medium. The treatment of the biomass may involve a fermentation process, which may utilize a microorganism other than the organism which produced the enzyme(s). In some instances the non-vascular photosynthetic organism may be added to a saccharification tank. This example may also comprise the step of collecting the biofuel. Collection may be performed by distillation. In some instances, the biofuel is mixed with another fuel.

An additional method provides for making at least one biomass degrading enzyme by transforming a chloroplast to make a biomass degrading enzyme. The biomass degrading enzyme may be a cellulolytic enzymes, a hemicellulolytic enzyme, or a ligninolytic enzyme, and specifically may be exo-β-glucanase, endo-β-glucanase, β-glucosidase, endoxylanase, or lignase. In some instances, the biomass degrading enzyme is sequestered in the transformed chloroplast. The method may further involve disrupting, via chemical or mechanical means, the transformed chloroplast to release the biomass degrading enzyme(s). In some instances, multiple enzymes will be produced by a transformed chloroplast. The biomass degrading enzymes may be of fungal or bacterial origin, for example, exo-β-glucanase, endo-β-glucanase, β-glucosidase, endoxylanase, lignase, or a combination thereof.

Some host cells may be transformed with multiple genes encoding one or more enzymes. For example, a single transformed cell may contain exogenous nucleic acids encoding an entire biodegradation pathway. One example of a pathway might include genes encoding an exo-β-glucanase (acts on the cellulose end chain), an endo-β-glucanase (acts on the interior portion of a cellulose chain), β-glucosidase (avoids reaction inhibitors by / degrades cellobiose), and endoxylanase (acts on hemicellulose cross liking). Such cells transformed with entire pathways and/or enzymes extracted from them, can degrade certain components of biomass. Constructs may contain multiple copies of the same gene, and/or multiple genes encoding the same enzyme from different organisms, and/or multiple genes with mutations in one or more parts of the coding sequences.

Alternately, biomass degradation pathways can be created by transforming host cells with the individual enzymes of the pathway and then combining the cells producing the individual enzymes. This approach allows for the combination of enzymes to more particularly match the biomass of interest by altering the relative ratios of the multiple transformed strains. For example, two times as many cells expressing the first enzyme of a pathway may be added to a mix where the first step of the reaction pathway is the limiting step.

Following transformation with enzyme-encoding constructs, the host cells and/or organisms are grown. The biomass degrading enzymes may be collected from the organisms/cells. Collection may be by any means known in the art, including, but not limited to concentrating cells, mechanical or chemical disruption of cells, and purification of enzymes from cell cultures and/or cell lysates. Cells and/or organisms can be grown and then the enzyme(s) collected by any means. One method of extracting the enzyme is by harvesting the host cell or a group of host cells and then drying the host cell(s). The enzyme(s) from the dried host cell(s) are then harvested by crushing the cells to expose the enzyme. The whole product of crushed cells is then used to degrade biomass. Many methods of extracting proteins from intact cells are well known in the art, and are also contemplated herein (e.g., introducing an exogenous nucleic acid construct in which an enzyme-encoding sequence is operably linked to a sequence encoding a secretion signal - excreted enzyme is isolated from the growth medium).

Extracting and utilizing the biomass-degrading enzyme can also be accomplished by expressing a vector containing nucleic acids that encode a biomass production-modulation molecule in the host cell. In this example, the host cell produces the biomass, and also produces a biomass-degrading enzyme. The biomass-degrading enzyme can then degrade the biomass produced by the host cell. In some instances, vector used for the production of a biomass-degrading enzyme may not be continuously active. Such vectors can comprise one or more inducible promoters and one or more biomass-degrading enzymes. Such promoters activate the production of biomass-degrading enzymes, for example, after the biomass has grown to sufficient density or reached certain maturity.

The present methods can also be performed by introducing a recombinant nucleic acid molecule into a chloroplast, wherein the recombinant nucleic acid molecule includes a first polynucleotide, which encodes at least one polypeptide (i.e., 1, 2, 3, 4, or more). In some examples, a polypeptide is operatively linked to a second, third, fourth, fifth, sixth, seventh, eighth, ninth, tenth and/or subsequent polypeptide. For example, several enzymes in a biodegradation pathway may be linked, either directly or indirectly, such that products produced by one enzyme in the pathway, once produced, are in close proximity to the next enzyme in the pathway.

Another aspect provides host organisms or cells disclosed herein (e.g. *Scenedesmus sp.* or *Dunaliella sp.*) that have been genetically modified or modified (e.g. by methods disclosed herein) for use as a feedstock. The compositions of genetically modified algae disclosed here can be used directly as a feedstock or can be added to a feedstock to generate a modified or improved feedstock. For example a composition can comprise a feedstock and a genetically modified algae. Genetic modification of an algae can comprise engineering an algae to express one or more enzymes. In some aspects the enzyme can be a biomass degrading enzyme and in some aspects the enzyme can be a biosynthetic enzyme. Genetically modified algae can also express both types of enzymes (e.g. a biomass degrading enzyme and a biosynthetic enzyme). The enzyme expressed can be one that is naturally expressed in the algae or not naturally expressed in the algae. In some aspects the enzyme produced is not naturally expressed in the algae. For example an enzyme (e.g. a biomass degrading enzyme) can be an exogenous enzyme. In another example a composition can comprise a feedstock and a genetically modified algae wherein the algae is modified to increase the expression of a naturally occurring enzyme (e.g. a biomass-degrading enzyme). In some aspects an enzyme can be secreted from a genetically modified algae or added to the feedstock as an independent ingredient.

Biomass degrading enzymes can improve the nutrient value of an existing feedstock by breaking down complex components of the feedstock (e.g. indigestible components) into components that can be absorbed and used by the animal. A biomass-degrading enzyme can be expressed and retained in the algae or secreted or expelled (i.e. produced ex vivo) from the algae. Genetically modified algae that provide the biomass degrading enzymes can also be utilized by the animal for the inherent nutrient value of the algae. For example a composition can comprise a feedstock, a genetically modified algae, and a biomass-degrading enzyme that is ex vivo to the genetically modified algae. In another example a genetically modified algae is modified to increase expression of a naturally occurring biomass-degrading enzyme.

The expression of certain exogenous biosynthetic enzymes in an algae can allow the biosynthesis of nutrient rich lipids, fatty acids and carbohydrates. Genetically modified algae that express such nutrient rich components can be added to an existing feedstock to supplement the nutritional value of the feedstock. In some aspects such genetically modified algae can comprise as much as 100% of the feedstock. Algae can be genetically modified to produce or increase production of one or more fatty acids, lipids or hydrocarbons. In one example a genetically modified algae comprise an exogenous nucleic acid encoding an enzyme in an isoprenoid biosynthesis pathway. In some aspects a genetically modified algae can have a higher content, or an altered content, or a different content of, for example, fatty acids, lipids or hydrocarbons (e.g. isoprenoids) than an unmodified algae of the same species. For example, the modified algae can produce more of a desired isoprenoid, and/or produce an isoprenoid that the algae does not normally produce, and/or produce isoprenoids that are normally produced but at different amounts than are produced in an unmodified algae.

Therefore in one aspect a composition can comprise a feedstock and a genetically modified algae wherein the algae has a higher lipid, fatty acid, or isoprenoid content relative to an unmodified algae of the same species. The biosynthetic enzymes can also be one found in a mevalonate pathway. For example the enzyme can be farnesyl pyrophosphate synthase, geranyl geranyl phosphate synthase, squalene synthase, thioesterase, or fatty acyl-CoA desaturase.

An improved feedstock can be comprised entirely or partially of a genetically modified algae. In some aspects a genetically modified algae can be added to a composition to generate an improved feedstock. The composition may not be considered a feedstock suitable for consumption by animals until after the addition of a genetically modified algae. In some aspects a genetically modified algae can be added to an existing feedstock to generate an improved feedstock. In some aspects a genetically modified algae can be added to an existing feedstock at a ratio of at least 1:20 (weight of algae/wt of feedstock). In some aspects an improved feedstock can comprises up to 10, 20, 30, 40, 50, 60, 70, 80, 90, 95, or 100 percent of a genetically modified algae. In some aspects a viable genetically modified algae can be added to a feedstock (e.g. as a seed culture) at a concentration of less than 5% (w/w) of the feedstock wherein the genetically modified algae multiplies to become up to 10, 20, 30, 40, 50, 60, 70, 80, 90 or 95% percent of the feedstock (w/w). A feedstock or improved feedstock can also comprise additional nutrients, ingredients or supplements (e.g. vitamins). An improved feedstock comprising a genetically modified algae can also comprise any normal ingredient of an animal feed including but not limited any vegetable, fruit, seed, root, flower, leaf, stem, stalk or plant product of any plant. An improved feedstock comprising a genetically modified algae can also comprise any animal parts or products (e.g. meat, bone, milk, excrement, skin). An improved feedstock comprising a genetically modified NVPO can also comprise any product or bi-product of a manufacturing process (e.g. sawdust or brewers waste). Additional non limiting examples of ingredients of a feedstock or an improved feedstock as disclosed herein include alfalfa, barley, blood meal, grass, legumes, silage, beet, bone meal, brewer grain, brewer's yeast, broom grass, carrot, cattle manure, clover, coffee, corn, corn glutten meal, distiller grains, poultry fat, grape, hominy feed, hop leaves, spent hops, molasses, oats, algae, peanuts, potato, poultry litter, poultry manure, rape meal, rye, safflower, sorghum, soybean, soy, sunflower meal, timothy hay, or triticale. Therefore in one aspect a composition can comprise a feedstock and a genetically modified NVPO wherein the feedstock comprises one or more of alfalfa, barley, blood meal, beet, bone meal, brewer grain, brewer's yeast, broom grass, carrot, cattle manure, clover, coffee, corn, corn glutten meal, distiller grains, poultry fat, grape, hominy feed, hop leaves, spent hops, molasses, oats, algae, peanuts, potato, poultry litter, poultry manure, rape meal, rye, safflower, sorghum, soybean, soy, sunflower meal, timothy hay, or triticale.

In some aspect a genetically modified algae can be used for a purpose (e.g. in producing a recombinant product or biofuel) and the remaining portion thereof can be used for an improved feedstock. Therefore an improved feedstock can comprise a portion of a genetically modified algae. For example a composition of an animal feed ingredient can comprise of whole and/or defatted algae (e.g. after removal of fatty acids, lipids or hydrocarbons, e.g. after hexane extraction) or a mixture of whole and defatted algae, which provides both the feed enzyme and the inherent nutritive value of the algae. In another example a genetically modified algae can be washed, dehydrated, centrifuged, filtered, defatted, lysed, dried, processed (e.g. extracted), or milled. The remaining portion thereof can be used as a feedstock, as an improved feedstock or as a supplement to improve a feedstock. For example a composition can comprise a feedstock and a portion of a genetically modified algae wherein the genetically modified algae is at least partially depleted of a lipid, fatty acid, isoprenoid, carotenoid, carbohydrate, or selected protein. The genetically modified algae can also be genetically modified to produce a biomass-degrading enzyme as disclosed herein.

Methods of generating, modifying, supplementing or improving a feedstock composition are also disclosed herein. The methods can comprise combining a genetically modified algae or a portion thereof with a feedstock to generate the improved feedstock. In one example the method comprises removing a lipid, fatty acid, isoprenoid, or carbohydrate from a genetically modified algae. The remaining genetically modified algae, or a portion thereof, can be combined with a feedstock to generate the improved feedstock composition. In one example of the method the modified algae does not express an exogenous phytase. The genetically modified algae or a portion thereof can comprise a nucleic acid (e.g. an exogenous nucleic acid). The nucleic acid can be a vector. In one example of the method, the nucleic acid encodes a biomass degrading enzyme. The biomass-degrading enzyme can be a galactanase, xylanase, protease, carbohydrase, lipase, reductase, oxidase, transglutaminase, or phytase. The biomass-degrading enzyme can be a carbohydrase wherein the carbohydrase is an α-amylase, β-amylase, endo-β-glucanase, endoxylanase, β-mannanase, α-galactosidase, or pullulanase. The biomass-degrading enzyme can be a protease wherein the protease is a subtilisin, bromelain, or fungal acid-stable protease. The biomass-degrading enzyme can be a phytase. In another example of the method the genetically modified NVPO further comprises an exogenous nucleic acid encoding an enzyme in an isoprenoid biosynthesis pathway. The enzyme in the isoprenoid biosynthesis pathway can be farnesyl pyrophosphate synthase, geranyl geranyl phosphate synthase, squalene synthase, thioesterase, or fatty acyl-CoA desaturase. The enzyme in the isoprenoid biosynthesis pathway can be in a mevalonate pathway. In yet another example of the method, the method can further comprise removing a lipid, fatty acid, or isoprenoid, from the genetically modified NVPO prior to combining with a feedstock to generate the improved feedstock.

Candidate genes for directing the expression of proteins (e.g. enzymes) in genetically modified algae for use in animal feeds can be obtained from a variety of organisms including eukaryotes, prokaryotes, or viruses. In some instances, an expressed enzyme is one member of a metabolic pathway (e.g. an isoprenoid biosynthesis pathway). Several enzymes may be introduced into the algae to produce increased levels of desired metabolites, or several enzymes may be introduced to produce a algae containing multiple useful feed enzyme activities (e.g. simultaneous production of xylanase, endo-β-glucanase, and phytase activities).

Feed enzymes can be expressed in host organisms (e.g. *Scenedesmus sp.*) and purified to a useful level. The purified enzymes can be added to animal feed in a manner similar to current practice. Feed enzymes can also be expressed in host organisms (e.g. algae), and the resulting host organisms can be added as a feed ingredient, adding both nutritive value and desired enzyme activity to the animal feed product. In this application, the genetically modified host organisms can be added to a feedstock alive, whole and non-viable or as a lysate wherein the host organisms are lysed by any suitable means (e.g. physical, chemical or thermal).

Many animal feeds can contain plant seeds, including soybeans, maize, wheat, and barley among others. Plant seeds can contain high levels of myo-inositol polyphosphate (phytic acid). This phytic acid is indigestible to non-ruminant animals, and so feeds with high levels of phytic acid may have low levels of bioavailable phosphorous. The phytic acid can also chelate many important nutritive minerals, such as calcium and magnesium. Incorporation of a phytase into the feed, which can act in the animals upper gut, can release both the chelated mineral nutrients and significant levels of bioavailable phosphorous. The net result is that less free phosphorous needs to be added to the animal feed product. In addition, phosphorous levels in the excreta can be reduced, which can reduce downstream phosphorous pollution.

Genetically modified algae that express phytases or similar enzymes can be added to a feedstock to improve the nutrient or digestible properties of the feed. Phytases contemplated for use herein can be from any organism (e.g. bacterial or fungal derived). Non limiting examples of types of phytases contemplated for use herein include 3-phytase (alternative name 1- phytase; a myo-inositol hexaphosphate 3-phosphohydrolase, EC 3.1.3.8), 4-phytase (alternative name 6-phytase, name based on 1 L-numbering system and not I D- numbering, EC 3.1.3.26), and 5-phytase (EC 3.1.3.72). Additional non limiting examples of phytases include microbial phytases, such as fungal, yeast or bacterial phytases such as disclosed in EP 684313, US 6139902, EP 420358, WO 97/35017, WO 98/28408, WO 98/28409, JP 1 1000164, WO98/13480, AU 724094, WO 97/33976, US 6110719, WO 2006/038062, WO 2006/038128, WO 2004/085638, WO 2006/037328, WO 2006/037327, WO 2006/043178, US 5830732 and under UniProt designations P34753, P34752, P34755, 000093, 031097, P42094, 066037 and P34754 (UniProt, (2008) http://www.uniprot.org/). Polypeptides having an amino acid sequence of at least 75% identity to an amino acid sequence (comprising the active site) of any one of the phytases disclosed above are also contemplated for use herein. In one example a composition can comprise a feedstock and a genetically modified algae. The genetically modified algae can be genetically modified to produce a biomass-degrading enzyme such as a phytase. In one aspect the phytase is a phytase of bacterial or fungal origin. In one aspect the biomass-degrading enzyme is an enzyme other than a phytase.

Many plant parts (e.g. seeds, fruits, stems, roots, leaves and flowers) from plants such as, for example, soybeans, wheat, and barley contain polysaccharides that are indigestible by some animals (e.g. non-ruminant animals). Non limiting examples of such carbohydrates include xylans, raffinose, stachyose, and glucans. The presence of indigestible carbohydrates in animal feed can reduce nutrient availability. Indigestible carbohydrates in poultry feed can result in sticky feces, which can increase disease levels. The presence of one or more carbohydrate degrading enzymes (e.g. α-amylase) in the animal feed can help break down polysaccharides, increase nutrient availability, increase the bioavailable energy content of the animal feed, and reduce health risks. Non limiting examples of carbohydrate degrading enzymes contemplated for use herein include amylases (e.g. α-amylase and β-amylase), β-mannanase, maltase, lactase, β-glucanase, endo-β-glucanase, glucose isomerase, endoxylanase, α-galactosidase, glucose oxidase, pullulanase, invertase and any carbohydrate digesting enzyme of bacterial, fungal, plant or animal origin. In one example a composition can comprise a feedstock and a genetically modified algae. The genetically modified algae can be genetically modified to produce a biomass-degrading enzyme such as a carbohydrase. In one aspect the carbohydrase can be an α-amylase, β-amylase, endo-β-glucanase, endoxylanase, β-mannanase, α-galactosidase, or pullulanase.

Many feedstocks contain plant parts (e.g. seeds) with anti-nutritive proteins (e.g. protease inhibitors, amylase inhibitors and others) that reduce the availability of nutrients in an animal feed. Addition of a broad spectrum protease (e.g. bromelain, subtilisin, or a fungal acid-stable protease) can break down these anti-nutritive proteins and increase the availability of nutrients in the animal's feed. Non limiting examples of proteases contemplated for use herein include endopeptidases and exopeptidases. Non limiting examples of proteases contemplated for use herein include serine proteases (e.g. subtilisin, chymotrypsins, glutamyl peptidases, dipeptidyl-peptidases, carboxypeptidases, dipeptidases, and aminopeptidases), cyteine proteases (e.g. papain, calpain-2, and papain-like peptidases and bromelain), aspartic peptidases (e.g. pepsins and pepsin A), glutamic proteases, threonine proteases, fungal acid proteases and acid stable proteases such as those disclosed in (US 6855548). In one example a composition can comprise a feedstock and a genetically modified algae. The genetically modified algae can be genetically modified to produce a biomass-degrading enzyme such as a protease. In one aspect the protease can be a subtilisin, bromelain or fungal acid-stable protease.

Non limiting examples of lipases contemplated for use herein include pancreatic lipase, lysosomal lipase, lysosomal acid lipase, acid cholesteryl ester hydrolase, hepatic lipase, lipoprotein lipase, gastric lipase, endothelial lipase, pancreatic lipase related protein 2, pancreatic lipase related protein 1, lingual lipase and phospholipases (e.g. phospholipase A1(EC 3.1.1.32), phospholipase A2, phospholipase B (lysophospholipase), phospholipase C and phospholipase D).

An improved feedstock can be generated by combining a feedstock with a algae that is genetically altered to produce an enzyme (e.g. a carbohydrase, protease or lipase). In some aspects the enzyme is produced ex vivo to the organism. In some aspects the enzyme is secreted. Enzymes produced ex vivo to the organisms can break down components of a feedstock prior to ingestion by an animal. Therefore an improved feedstock can be generated by combining a feedstock with an algae that is genetically altered to produce an enzyme (e.g. a carbohydrase, protease or lipase) and subjecting the mixture to a holding period. A holding period can allow the genetically altered algae to multiply and to secrete more enzyme into the feedstock. A holding period can be from several hours up to several days. In some aspects a holding period is for up to 1, 2, 3, 4, 5, 6, 7, 8, 9 or 10 days. In some aspects a holding period is for up to several days to several weeks. In some aspects a holding period is indefinite. An indefinite holding period allows intermittent removal and use of the improved feedstock and intermittent addition of the base feedstock

### Host Cells or Host Organisms

Biomass useful in the methods and systems described herein can be obtained from host cells or host organisms.

A host cell can contain a polynucleotide encoding a polypeptide of the present disclosure. In some embodiments, a host cell is part of a multicellular organism. In other embodiments, a host cell is cultured as a unicellular organism.

Host organisms can include any suitable host, for example, a microorganism. Microorganisms which are useful for the methods described herein include, for example, photosynthetic bacteria (e.g., cyanobacteria), non-photosynthetic bacteria (e.g., *E. coli*), yeast (e.g., *Saccharomyces cerevisiae*), and algae (e. g., microalgae such as *Chlamydomonas reinhardtii*).

Examples of host organisms that can be transformed with a polynucleotide of interest (for example, a polynucleotide that encodes a protein involved in the isoprenoid biosynthesis pathway) include vascular and non-vascular organisms. The organism can be prokaryotic or eukaryotic. The organism can be unicellular or multicellular. A host organism is an organism comprising a host cell. In other embodiments, the host organism is photosynthetic. A photosynthetic organism is one that naturally photosynthesizes (e.g., an alga) or that is genetically engineered or otherwise modified to be photosynthetic. In some instances, a photosynthetic organism may be transformed with a construct or vector of the disclosure which renders all or part of the photosynthetic apparatus inoperable.

By way of example, a non-vascular photosynthetic microalga species (for example, *C*. *reinhardtii*, *Nannochloropsis oceania*, *N. salina*, *D. salina*, *H. pluvalis*, *S. dimorphus*, *D. viridis*, *Chlorella sp*., and *D. tertiolecta*) can be genetically engineered to produce a polypeptide of interest, for example a fusicoccadiene synthase or an FPP synthase. Production of a fusicoccadiene synthase or an FPP synthase in these microalgae can be achieved by engineering the microalgae to express the fusicoccadiene synthase or FPP synthase in the algal chloroplast or nucleus.

In other embodiments the host organism is a vascular plant. Non-limiting examples of such plants include various monocots and dicots, including high oil seed plants such as high oil seed *Brassica* (e.g., *Brassica nigra*, *Brassica napus*, *Brassica hirta*, *Brassica rapa*, *Brassica campestris, Brassica carinata*, and *Brassica juncea*), soybean (*Glycine max*), castor bean (*Ricinus communis*), cotton, safflower (*Carthamus tinctorius*), sunflower (*Helianthus annuus*), flax (*Linum usitatissimum*), corn (*Zea mays*), coconut (*Cocos nucifera*), palm (*Elaeis guineensis*), oil nut trees such as olive (*Olea europaea*), sesame, and peanut (*Arachis hypogaea*), as well as *Arabidopsis*, tobacco, wheat, barley, oats, amaranth, potato, rice, tomato, and legumes (e.g., peas, beans, lentils, alfalfa, etc.).

The host cell can be prokaryotic. Examples of some prokaryotic organisms of the present disclosure include, but are not limited to, cyanobacteria (e.g., *Synechococcus*, *Synechocystis*, *Athrospira*, *Gleocapsa*, *Oscillatoria*, and, *Pseudoanabaena*). Suitable prokaryotic cells include, but are not limited to, any of a variety of laboratory strains of Escherichia coli, Lactobacillus sp., Salmonella sp., and Shigella sp. (for example, as described in Carrier et al. (1992) J. Immunol. 148:1176-1181; U.S. Pat. No. 6,447,784; and Sizemore et al. (1995) Science 270:299-302). Examples of Salmonella strains which can be employed in the present disclosure include, but are not limited to, Salmonella typhi and S. typhimurium. Suitable Shigella strains include, but are not limited to, Shigella flexneri, Shigella sonnei, and Shigella disenteriae. Typically, the laboratory strain is one that is non-pathogenic. Non-limiting examples of other suitable bacteria include, but are not limited to, Pseudomonas pudita, Pseudomonas aeruginosa, Pseudomonas mevalonii, Rhodobacter sphaeroides, Rhodobacter capsulatus, Rhodospirillum rubrum, and Rhodococcus sp.

In some examples, the host organism is eukaryotic (e.g. green algae, red algae, brown algae). In some examples, the algae is a green algae, for example, a Chlorophycean. The algae can be unicellular or multicellular. Suitable eukaryotic host cells include, but are not limited to, yeast cells, insect cells, plant cells, fungal cells, and algal cells. Suitable eukaryotic host cells include, but are not limited to, Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica, Pichia sp., Saccharomyces cerevisiae, Saccharomyces sp., Hansenula polymorpha, Kluyveromyces sp., Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Neurospora crassa, and Chlamydomonas reinhardtii. In other embodiments, the host cell is a microalga (e.g., *Chlamydomonas reinhardtii*, *Dunaliella salina*, *Haematococcus pluvialis*, *Nannochloropsis oceania, N. salina*, *Scenedesmus dimorphus*, *Chlorella spp*., *D. viridis*, or *D. tertiolecta*).

In some instances the organism is a rhodophyte, chlorophyte, heterokontophyte, tribophyte, glaucophyte, chlorarachniophyte, euglenoid, haptophyte, cryptomonad, dinoflagellum, or phytoplankton.

In some instances a host organism is vascular and photosynthetic. Examples of vascular plants include, but are not limited to, angiosperms, gymnosperms, rhyniophytes, or other tracheophytes.

In some instances a host organism is non-vascular and photosynthetic. As used herein, the term "non-vascular photosynthetic organism," refers to any macroscopic or microscopic organism, including, but not limited to, algae, cyanobacteria and photosynthetic bacteria, which does not have a vascular system such as that found in vascular plants. Examples of non-vascular photosynthetic organisms include bryophtyes, such as marchantiophytes or anthocerotophytes. In some instances the organism is a cyanobacteria. In some instances, the organism is algae (e.g., macroalgae or microalgae). The algae can be unicellular or multicellular algae. For example, the microalgae *Chlamydomonas reinhardtii* may be transformed with a vector, or a linearized portion thereof, encoding one or more proteins of interest (e.g., a protein involved in the isoprenoid biosynthesis pathway).

Methods for algal transformation are described in U.S. Provisional Patent Application No. 60/142,091. The methods of the present disclosure can be carried out using algae, for example, the microalga, C. *reinhardtii.* The use of microalgae to express a polypeptide or protein complex according to a method of the disclosure provides the advantage that large populations of the microalgae can be grown, including commercially (Cyanotech Corp.; Kailua-Kona HI), thus allowing for production and, if desired, isolation of large amounts of a desired product.

The vectors of the present disclosure may be capable of stable or transient transformation of multiple photosynthetic organisms, including, but not limited to, photosynthetic bacteria (including cyanobacteria), cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, and phytoplankton. Other vectors of the present disclosure are capable of stable or transient transformation of, for example, C. *reinhardtii*, *N. oceania*, *N. salina*, *D. salina*, *H. pluvalis*, *S. dimorphus*, *D. viridis*, or *D. tertiolecta.*

Examples of appropriate hosts, include but are not limited to: bacterial cells, such as E. coli, Streptomyces, Salmonella typhimurium; fungal cells, such as yeast; insect cells, such as Drosophila S2 and Spodoptera Sf9; animal cells, such as CHO, COS or Bowes melanoma; adenoviruses; and plant cells. The selection of an appropriate host is deemed to be within the scope of those skilled in the art.

Polynucleotides selected and isolated as described herein are introduced into a suitable host cell. A suitable host cell is any cell which is capable of promoting recombination and/or reductive reassortment. The selected polynucleotides can be, for example, in a vector which includes appropriate control sequences. The host cell can be, for example, a higher eukaryotic cell, such as a mammalian cell, or a lower eukaryotic cell, such as a yeast cell, or the host cell can be a prokaryotic cell, such as a bacterial cell. Introduction of a construct (vector) into the host cell can be effected by, for example, calcium phosphate transfection, DEAE-Dextran mediated transfection, or electroporation.

Recombinant polypeptides, including protein complexes, can be expressed in plants, allowing for the production of crops of such plants and, therefore, the ability to conveniently produce large amounts of a desired product. Accordingly, the methods of the disclosure can be practiced using any plant, including, for example, microalga and macroalgae, (such as marine algae and seaweeds), as well as plants that grow in soil.

In one embodiment, the host cell is a plant. The term "plant" is used broadly herein to refer to a eukaryotic organism containing plastids, such as chloroplasts, and includes any such organism at any stage of development, or to part of a plant, including a plant cutting, a plant cell, a plant cell culture, a plant organ, a plant seed, and a plantlet. A plant cell is the structural and physiological unit of the plant, comprising a protoplast and a cell wall. A plant cell can be in the form of an isolated single cell or a cultured cell, or can be part of higher organized unit, for example, a plant tissue, plant organ, or plant. Thus, a plant cell can be a protoplast, a gamete producing cell, or a cell or collection of cells that can regenerate into a whole plant. As such, a seed, which comprises multiple plant cells and is capable of regenerating into a whole plant, is considered plant cell for purposes of this disclosure. A plant tissue or plant organ can be a seed, protoplast, callus, or any other groups of plant cells that is organized into a structural or functional unit. Particularly useful parts of a plant include harvestable parts and parts useful for propagation of progeny plants. A harvestable part of a plant can be any useful part of a plant, for example, flowers, pollen, seedlings, tubers, leaves, stems, fruit, seeds, and roots. A part of a plant useful for propagation includes, for example, seeds, fruits, cuttings, seedlings, tubers, and rootstocks.

A method of the disclosure can generate a plant containing genomic DNA (for example, a nuclear and/or plastid genomic DNA) that is genetically modified to contain a stably integrated polynucleotide (for example, as described in Hager and Bock, Appl. Microbiol Biotechnol. 54:302-310, 2000). Accordingly, the present disclosure further provides a transgenic plant, e.g. *C*. *reinhardtii*, which comprises one or more chloroplasts containing a polynucleotide encoding one or more exogenous or endogenous polypeptides, including polypeptides that can allow for secretion of fuel products and/or fuel product precursors (e.g., isoprenoids, fatty acids, lipids, triglycerides). A photosynthetic organism of the present disclosure comprises at least one host cell that is modified to generate, for example, a fuel product or a fuel product precursor.

Some of the host organisms useful in the disclosed embodiments are, for example, are extremophiles, such as hyperthermophiles, psychrophiles, psychrotrophs, halophiles, barophiles and acidophiles. Some of the host organisms which may be used to practice the present disclosure are halophilic (e.g., *Dunaliella salina*, *D. viridis*, or *D. tertiolecta*)*.* For example, *D. salina* can grow in ocean water and salt lakes (for example, salinity from 30-300 parts per thousand) and high salinity media (e.g., artificial seawater medium, seawater nutrient agar, brackish water medium, and seawater medium). In some examples of the disclosure, a host cell expressing a protein of the present disclosure can be grown in a liquid environment which is, for example, 0.1, 0.2, 0.3, 0.4, 0.5, 0.6, 0.7, 0.8, 0.9, 1.0, 1.1, 1.2,1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 31., 3.2, 33, 3.4, 3.5, 3.6, 3.7, 3.8, 3.9, 4.0, 4.1, 4.2, 4.3 molar or higher concentrations of sodium chloride. One of skill in the art will recognize that other salts (sodium salts, calcium salts, potassium salts, or other salts) may also be present in the liquid environments.

Where a halophilic organism is utilized for the present disclosure, it may be transformed with any of the vectors described herein. For example, *D. salina* may be transformed with a vector which is capable of insertion into the chloroplast or nuclear genome and which contains nucleic acids which encode a protein (e.g., an FPP synthase or a fusicoccadiene synthase). Transformed halophilic organisms may then be grown in high-saline environments (e.g., salt lakes, salt ponds, and high-saline media) to produce the products (e.g., lipids) of interest. Isolation of the products may involve removing a transformed organism from a high-saline environment prior to extracting the product from the organism. In instances where the product is secreted into the surrounding environment, it may be necessary to desalinate the liquid environment prior to any further processing of the product.

The present disclosure further provides compositions comprising a genetically modified host cell. A composition comprises a genetically modified host cell; and will in some examples comprise one or more further components, which components are selected based in part on the intended use of the genetically modified host cell. Suitable components include, but are not limited to, salts; buffers; stabilizers; protease-inhibiting agents; cell membrane- and/or cell wall-preserving compounds, e.g., glycerol and dimethylsulfoxide; and nutritional media appropriate to the cell.

For the production of a protein, for example, an isoprenoid or isoprenoid precursor compound, a host cell can be, for example, one that produces, or has been genetically modified to produce, one or more enzymes in a prenyl transferase pathway and/or a mevalonate pathway and/or an isoprenoid biosynthetic pathway. In some examples, the host cell is one that produces a substrate of a prenyl transferase, isoprenoid synthase or mevalonate pathway enzyme.

In some examples, a genetically modified host cell is a host cell that comprises an endogenous mevalonate pathway and/or isoprenoid biosynthetic pathway and/or prenyl transferase pathway. In other examples, a genetically modified host cell is a host cell that does not normally produce mevalonate or IPP via a mevalonate pathway, or FPP, GPP or GGPP via a prenyl transferase pathway, but has been genetically modified with one or more polynucleotides comprising nucleotide sequences encoding one or more mevalonate pathway, isoprenoid synthase pathway or prenyl transferase pathway enzymes (for example, as described in U.S. Patent Publication No. 2004/005678; U.S. Patent Publication No. 2003/0148479; and Martin et al. (2003) Nat. Biotech. 21(7):796-802).

### Culturing of Cells or Organisms

An organism may be grown under conditions which permit photosynthesis, however, this is not a requirement (e.g., a host organism may be grown in the absence of light). In some instances, the host organism may be genetically modified in such a way that its photosynthetic capability is diminished or destroyed. In growth conditions where a host organism is not capable of photosynthesis (e.g., because of the absence of light and/or genetic modification), typically, the organism will be provided with the necessary nutrients to support growth in the absence of photosynthesis. For example, a culture medium in (or on) which an organism is grown, may be supplemented with any required nutrient, including an organic carbon source, nitrogen source, phosphorous source, vitamins, metals, lipids, nucleic acids, micronutrients, and/or an organism-specific requirement. Organic carbon sources include any source of carbon which the host organism is able to metabolize including, but not limited to, acetate, simple carbohydrates (e.g., glucose, sucrose, and lactose), complex carbohydrates (e.g., starch and glycogen), proteins, and lipids. One of skill in the art will recognize that not all organisms will be able to sufficiently metabolize a particular nutrient and that nutrient mixtures may need to be modified from one organism to another in order to provide the appropriate nutrient mix.

Optimal growth of organisms occurs usually at a temperature of about 20°C to about 25 °C, although some organisms can still grow at a temperature of up to about 35 °C. Active growth is typically performed in liquid culture. If the organisms are grown in a liquid medium and are shaken or mixed, the density of the cells can be anywhere from about 1 to 5 x 10⁸cells/ml at the stationary phase. For example, the density of the cells at the stationary phase for Chlamydomonas sp. can be about 1 to 5 x 10⁷cells/ml; the density of the cells at the stationary phase for Nannochloropsis sp. can be about 1 to 5 x 10⁸cells/ml; the density of the cells at the stationary phase for Scenedesmus sp. can be about 1 to 5 x 10⁷ cells/ml; and the density of the cells at the stationary phase for Chlorella sp. can be about 1 to 5 x 10⁸cells/ml. Exemplary cell densities at the stationary phase are as follows: Chlamydomonas sp. can be about 1 x 10⁷cells/ml; Nannochloropsis sp. can be about 1 x 10⁸cells/ml; Scenedesmus sp. can be about 1 x 10⁷cells/ml; and Chlorella sp. can be about 1 x 10⁸cells/ml. An exemplary growth rate may yield, for example, a two to four fold increase in cells per day, depending on the growth conditions. In addition, doubling times for organisms can be, for example, 5 hours to 30 hours. The organism can also be grown on solid media, for example, media containing about 1.5% agar, in plates or in slants.

One source of energy is fluorescent light that can be placed, for example, at a distance of about 1 inch to about two feet from the organism. Examples of types of fluorescent lights includes, for example, cool white and daylight. Bubbling with air or CO₂ improves the growth rate of the organism. Bubbling with CO₂ can be, for example, at 1 % to 5% CO₂. If the lights are turned on and off at regular intervals (for example, 12:12 or 14:10 hours of light:dark) the cells of some organisms will become synchronized.

Long term storage of organisms can be achieved by streaking them onto plates, sealing the plates with, for example, Parafilm™, and placing them in dim light at about 10 °C to about 18 °C. Alternatively, organisms may be grown as streaks or stabs into agar tubes, capped, and stored at about 10 °C to about 18 °C. Both methods allow for the storage of the organisms for several months.

For longer storage, the organisms can be grown in liquid culture to mid to late log phase and then supplemented with a penetrating cryoprotective agent like DMSO or MeOH, and stored at less than -130 °C. An exemplary range of DMSO concentrations that can be used is 5 to 8%. An exemplary range of MeOH concentrations that can be used is 3 to 9% .

Organisms can be grown on a defined minimal medium (for example, high salt medium (HSM), modified artificial sea water medium (MASM), or F/2 medium) with light as the sole energy source. In other instances, the organism can be grown in a medium (for example, tris acetate phosphate (TAP) medium), and supplemented with an organic carbon source.

Organisms, such as algae, can grow naturally in fresh water or marine water. Culture media for freshwater algae can be, for example, synthetic media, enriched media, soil water media, and solidified media, such as agar. Various culture media have been developed and used for the isolation and cultivation of fresh water algae and are described in Watanabe, M.W. (2005). Freshwater Culture Media. In R.A. Andersen (Ed.), Algal Culturing Techniques (pp. 13-20). Elsevier Academic Press. Culture media for marine algae can be, for example, artificial seawater media or natural seawater media. Guidelines for the preparation of media are described in Harrison, P.J. and Berges, J.A. (2005). Marine Culture Media. In R.A. Andersen (Ed.), Algal Culturing Techniques (pp. 21-33). Elsevier Academic Press.

Organisms may be grown in outdoor open water, such as ponds, the ocean, seas, rivers, waterbeds, marshes, shallow pools, lakes, aqueducts, and reservoirs. When grown in water, the organism can be contained in a halo-like object comprised of lego-like particles. The halo-like object encircles the organism and allows it to retain nutrients from the water beneath while keeping it in open sunlight.

In some instances, organisms can be grown in containers wherein each container comprises one or two organisms, or a plurality of organisms. The containers can be configured to float on water. For example, a container can be filled by a combination of air and water to make the container and the organism(s) in it buoyant. An organism that is adapted to grow in fresh water can thus be grown in salt water (i.e., the ocean) and vice versa. This mechanism allows for automatic death of the organism if there is any damage to the container.

Culturing techniques for algae are well know to one of skill in the art and are described, for example, in Freshwater Culture Media. In R.A. Andersen (Ed.), Algal Culturing Techniques. Elsevier Academic Press.

Because photosynthetic organisms, for example, algae, require sunlight, CO₂ and water for growth, they can be cultivated in, for example, open ponds and lakes. However, these open systems are more vulnerable to contamination than a closed system. One challenge with using an open system is that the organism of interest may not grow as quickly as a potential invader. This becomes a problem when another organism invades the liquid environment in which the organism of interest is growing, and the invading organism has a faster growth rate and takes over the system.

In addition, in open systems there is less control over water temperature, CO₂ concentration, and lighting conditions. The growing season of the organism is largely dependent on location and, aside from tropical areas, is limited to the warmer months of the year. In addition, in an open system, the number of different organisms that can be grown is limited to those that are able to survive in the chosen location. An open system, however, is cheaper to set up and/or maintain than a closed system.

Another approach to growing an organism is to use a semi-closed system, such as covering the pond or pool with a structure, for example, a "greenhouse-type" structure. While this can result in a smaller system, it addresses many of the problems associated with an open system. The advantages of a semi-closed system are that it can allow for a greater number of different organisms to be grown, it can allow for an organism to be dominant over an invading organism by allowing the organism of interest to out compete the invading organism for nutrients required for its growth, and it can extend the growing season for the organism. For example, if the system is heated, the organism can grow year round.

A variation of the pond system is an artificial pond, for example, a raceway pond. In these ponds, the organism, water, and nutrients circulate around a "racetrack." Paddlewheels provide constant motion to the liquid in the racetrack, allowing for the organism to be circulated back to the surface of the liquid at a chosen frequency. Paddlewheels also provide a source of agitation and oxygenate the system. These raceway ponds can be enclosed, for example, in a building or a greenhouse, or can be located outdoors.

Raceway ponds are usually kept shallow because the organism needs to be exposed to sunlight, and sunlight can only penetrate the pond water to a limited depth. The depth of a raceway pond can be, for example, about 4 to about 12 inches. In addition, the volume of liquid that can be contained in a raceway pond can be, for example, about 200 liters to about 600,000 liters.

The raceway ponds can be operated in a continuous manner, with, for example, CO₂ and nutrients being constantly fed to the ponds, while water containing the organism is removed at the other end.

If the raceway pond is placed outdoors, there are several different ways to address the invasion of an unwanted organism. For example, the pH or salinity of the liquid in which the desired organism is in can be such that the invading organism either slows down its growth or dies.

Also, chemicals can be added to the liquid, such as bleach, or a pesticide can be added to the liquid, such as glyphosate. In addition, the organism of interest can be genetically modified such that it is better suited to survive in the liquid environment. Any one or more of the above strategies can be used to address the invasion of an unwanted organism.

Alternatively, organisms, such as algae, can be grown in closed structures such as photobioreactors, where the environment is under stricter control than in open systems or semi-closed systems. A photobioreactor is a bioreactor which incorporates some type of light source to provide photonic energy input into the reactor. The term photobioreactor can refer to a system closed to the environment and having no direct exchange of gases and contaminants with the environment. A photobioreactor can be described as an enclosed, illuminated culture vessel designed for controlled biomass production of phototrophic liquid cell suspension cultures. Examples of photobioreactors include, for example, glass containers, plastic tubes, tanks, plastic sleeves, and bags. Examples of light sources that can be used to provide the energy required to sustain photosynthesis include, for example, fluorescent bulbs, LEDs, and natural sunlight. Because these systems are closed everything that the organism needs to grow (for example, carbon dioxide, nutrients, water, and light) must be introduced into the bioreactor.

Photobioreactors, despite the costs to set up and maintain them, have several advantages over open systems, they can, for example, prevent or minimize contamination, permit axenic organism cultivation of monocultures (a culture consisting of only one species of organism), offer better control over the culture conditions (for example, pH, light, carbon dioxide, and temperature), prevent water evaporation, lower carbon dioxide losses due to out gassing, and permit higher cell concentrations.

On the other hand, certain requirements of photobioreactors, such as cooling, mixing, control of oxygen accumulation and biofouling, make these systems more expensive to build and operate than open systems or semi-closed systems.

Photobioreactors can be set up to be continually harvested (as is with the majority of the larger volume cultivation systems), or harvested one batch at a time (for example, as with polyethlyene bag cultivation). A batch photobioreactor is set up with, for example, nutrients, an organism (for example, algae), and water, and the organism is allowed to grow until the batch is harvested. A continuous photobioreactor can be harvested, for example, either continually, daily, or at fixed time intervals.

High density photobioreactors are described in, for example, Lee, et al., Biotech. Bioengineering 44:1161-1167, 1994. Other types of bioreactors, such as those for sewage and waste water treatments, are described in, Sawayama, et al., Appl. Micro. Biotech., 41:729-731, 1994. Additional examples of photobioreactors are described in, U.S. Appl. Publ. No. 2005/0260553, U.S. Pat. No. 5,958,761, and U.S. Pat. No. 6,083,740. Also, organisms, such as algae may be mass-cultured for the removal of heavy metals (for example, as described in Wilkinson, Biotech. Letters, 11:861-864, 1989), hydrogen (for example, as described in U.S. Patent Application Publication No. 2003/0162273), and pharmaceutical compounds from a water, soil, or other source or sample. Organisms can also be cultured in conventional fermentation bioreactors, which include, but are not limited to, batch, fed-batch, cell recycle, and continuous fermentors. Additional methods of culturing organisms and variations of the methods described herein are known to one of skill in the art.

Organisms can also be grown near ethanol production plants or other facilities or regions (e.g., cities and highways) generating CO₂. As such, the methods herein contemplate business methods for selling carbon credits to ethanol plants or other facilities or regions generating CO₂ while making fuels or fuel products by growing one or more of the organisms described herein near the ethanol production plant, facility, or region.

The organism of interest, grown in any of the systems described herein, can be, for example, continually harvested, or harvested one batch at a time.

CO₂ can be delivered to any of the systems described herein, for example, by bubbling in CO₂ from under the surface of the liquid containing the organism. Also, sparges can be used to inject CO₂ into the liquid. Spargers are, for example, porous disc or tube assemblies that are also referred to as Bubblers, Carbonators, Aerators, Porous Stones and Diffusers.

Nutrients that can be used in the systems described herein include, for example, nitrogen (in the form of NO₃⁻ or NH₄⁺), phosphorus, and trace metals (Fe, Mg, K, Ca, Co, Cu, Mn, Mo, Zn, V, and B). The nutrients can come, for example, in a solid form or in a liquid form. If the nutrients are in a solid form they can be mixed with, for example, fresh or salt water prior to being delivered to the liquid containing the organism, or prior to being delivered to a photobioreactor.

Organisms can be grown in cultures, for example large scale cultures, where large scale cultures refers to growth of cultures in volumes of greater than about 6 liters, or greater than about 10 liters, or greater than about 20 liters. Large scale growth can also be growth of cultures in volumes of 50 liters or more, 100 liters or more, or 200 liters or more. Large scale growth can be growth of cultures in, for example, ponds, containers, vessels, or other areas, where the pond, container, vessel, or area that contains the culture is for example, at lease 5 square meters, at least 10 square meters, at least 200 square meters, at least 500 square meters, at least 1,500 square meters, at least 2,500 square meters, in area, or greater.

*Clamydomonas sp*., *Nannochloropsis sp*., *Scenedesmus sp*., and *Chlorella sp.* are exemplary algae that can be cultured as described herein and can grow under a wide array of conditions.

One organism that can be cultured as described herein is a commonly used laboratory species C. reinhardtii. Cells of this species are haploid, and can grow on a simple medium of inorganic salts, using photosynthesis to provide energy. This organism can also grow in total darkness if acetate is provided as a carbon source. C. reinhardtii can be readily grown at room temperature under standard fluorescent lights. In addition, the cells can be synchronized by placing them on a light-dark cycle. Other methods of culturing C. reinhardtii cells are known to one of skill in the art.

### Polynucleotides and Polypeptides

Also provided are isolated polynucleotides encoding a protein, for example, an FPP synthase, described herein. As used herein "isolated polynucleotide" means a polynucleotide that is free of one or both of the nucleotide sequences which flank the polynucleotide in the naturally-occurring genome of the organism from which the polynucleotide is derived. The term includes, for example, a polynucleotide or fragment thereof that is incorporated into a vector or expression cassette; into an autonomously replicating plasmid or virus; into the genomic DNA of a prokaryote or eukaryote; or that exists as a separate molecule independent of other polynucleotides. It also includes a recombinant polynucleotide that is part of a hybrid polynucleotide, for example, one encoding a polypeptide sequence.

The novel proteins of the present disclosure can be made by any method known in the art. The protein may be synthesized using either solid-phase peptide synthesis or by classical solution peptide synthesis also known as liquid-phase peptide synthesis. Using Val-Pro-Pro, Enalapril and Lisinopril as starting templates, several series of peptide analogs such as X-Pro-Pro, X-Ala-Pro, and X-Lys-Pro, wherein X represents any amino acid residue, may be synthesized using solid-phase or liquid-phase peptide synthesis. Methods for carrying out liquid phase synthesis of libraries of peptides and oligonucleotides coupled to a soluble oligomeric support have also been described. Bayer, Ernst and Mutter, Manfred, Nature 237:512-513 (1972); Bayer, Ernst, et al., J. Am. Chem. Soc. 96:7333-7336 (1974); Bonora, Gian Maria, et al., Nucleic Acids Res. 18:3155-3159 (1990). Liquid phase synthetic methods have the advantage over solid phase synthetic methods in that liquid phase synthesis methods do not require a structure present on a first reactant which is suitable for attaching the reactant to the solid phase. Also, liquid phase synthesis methods do not require avoiding chemical conditions which may cleave the bond between the solid phase and the first reactant (or intermediate product). In addition, reactions in a homogeneous solution may give better yields and more complete reactions than those obtained in heterogeneous solid phase/liquid phase systems such as those present in solid phase synthesis.

In oligomer-supported liquid phase synthesis the growing product is attached to a large soluble polymeric group. The product from each step of the synthesis can then be separated from unreacted reactants based on the large difference in size between the relatively large polymer-attached product and the unreacted reactants. This permits reactions to take place in homogeneous solutions, and eliminates tedious purification steps associated with traditional liquid phase synthesis. Oligomer-supported liquid phase synthesis has also been adapted to automatic liquid phase synthesis of peptides. Bayer, Ernst, et al., Peptides: Chemistry, Structure, Biology, 426-432.

For solid-phase peptide synthesis, the procedure entails the sequential assembly of the appropriate amino acids into a peptide of a desired sequence while the end of the growing peptide is linked to an insoluble support. Usually, the carboxyl terminus of the peptide is linked to a polymer from which it can be liberated upon treatment with a cleavage reagent. In a common method, an amino acid is bound to a resin particle, and the peptide generated in a stepwise manner by successive additions of protected amino acids to produce a chain of amino acids. Modifications of the technique described by Merrifield are commonly used. See, e.g., Merrifield, J. Am. Chem. Soc. 96: 2989-93 (1964). In an automated solid-phase method, peptides are synthesized by loading the carboxy-terminal amino acid onto an organic linker (e.g., PAM, 4-oxymethylphenylacetamidomethyl), which is covalently attached to an insoluble polystyrene resin cross-linked with divinyl benzene. The terminal amine may be protected by blocking with t-butyloxycarbonyl. Hydroxyl- and carboxyl- groups are commonly protected by blocking with O-benzyl groups. Synthesis is accomplished in an automated peptide synthesizer, such as that available from Applied Biosystems (Foster City, California). Following synthesis, the product may be removed from the resin. The blocking groups are removed by using hydrofluoric acid or trifluoromethyl sulfonic acid according to established methods. A routine synthesis may produce 0.5 mmole of peptide resin. Following cleavage and purification, a yield of approximately 60 to 70% is typically produced. Purification of the product peptides is accomplished by, for example, crystallizing the peptide from an organic solvent such as methyl-butyl ether, then dissolving in distilled water, and using dialysis (if the molecular weight of the subject peptide is greater than about 500 daltons) or reverse high pressure liquid chromatography (e.g., using a C¹⁸ column with 0.1 % trifluoroacetic acid and acetonitrile as solvents) if the molecular weight of the peptide is less than 500 daltons. Purified peptide may be lyophilized and stored in a dry state until use. Analysis of the resulting peptides may be accomplished using the common methods of analytical high pressure liquid chromatography (HPLC) and electrospray mass spectrometry (ES-MS).

In other cases, a protein, for example, a protein involved in the isoprenoid biosynthesis pathway or in fatty acid synthesis, is produced by recombinant methods. For production of any of the proteins described herein, host cells transformed with an expression vector containing the polynucleotide encoding such a protein can be used. The host cell can be a higher eukaryotic cell, such as a mammalian cell, or a lower eukaryotic cell such as a yeast or algal cell, or the host can be a prokaryotic cell such as a bacterial cell. Introduction of the expression vector into the host cell can be accomplished by a variety of methods including calcium phosphate transfection, DEAE-dextran mediated transfection, polybrene, protoplast fusion, liposomes, direct microinjection into the nuclei, scrape loading, biolistic transformation and electroporation. Large scale production of proteins from recombinant organisms is a well established process practiced on a commercial scale and well within the capabilities of one skilled in the art.

It should be recognized that the present disclosure is not limited to transgenic cells, organisms, and plastids containing a protein or proteins as disclosed herein, but also encompasses such cells, organisms, and plastids transformed with additional nucleotide sequences encoding enzymes involved in fatty acid synthesis. Thus, some examples involve the introduction of one or more sequences encoding proteins involved in fatty acid synthesis in addition to a protein disclosed herein. For example, several enzymes in a fatty acid production pathway may be linked, either directly or indirectly, such that products produced by one enzyme in the pathway, once produced, are in close proximity to the next enzyme in the pathway. These additional sequences may be contained in a single vector either operatively linked to a single promoter or linked to multiple promoters, e.g. one promoter for each sequence. Alternatively, the additional coding sequences may be contained in a plurality of additional vectors. When a plurality of vectors are used, they can be introduced into the host cell or organism simultaneously or sequentially.

Additional embodiments provide a plastid, and in particular a chloroplast, transformed with a polynucleotide encoding a protein of the present disclosure. The protein may be introduced into the genome of the plastid using any of the methods described herein or otherwise known in the art. The plastid may be contained in the organism in which it naturally occurs. Alternatively, the plastid may be an isolated plastid, that is, a plastid that has been removed from the cell in which it normally occurs. Methods for the isolation of plastids are known in the art and can be found, for example, in Maliga et al., Methods in Plant Molecular Biology, Cold Spring Harbor Laboratory Press, 1995; Gupta and Singh, J. Biosci., 21:819 (1996); and Camara et al., Plant Physiol., 73:94 (1983). The isolated plastid transformed with a protein of the present disclosure can be introduced into a host cell. The host cell can be one that naturally contains the plastid or one in which the plastid is not naturally found.

Also within the scope of the present disclosure are artificial plastid genomes, for example chloroplast genomes, that contain nucleotide sequences encoding any one or more of the proteins of the present disclosure. Methods for the assembly of artificial plastid genomes can be found in co-pending U.S. Patent Application serial number 12/287,230 filed October 6, 2008, published as U.S. Publication No. 2009/0123977 on May 14, 2009, and U.S. Patent Application serial number 12/384,893 filed April 8, 2009, published as U.S. Publication No. 2009/0269816 on October 29, 2009.

### Introduction of Polynucleotide into a Host Organism or Cell

To generate a genetically modified host cell, a polynucleotide, or a polynucleotide cloned into a vector, is introduced stably or transiently into a host cell, using established techniques, including, but not limited to, electroporation, calcium phosphate precipitation, DEAE-dextran mediated transfection, and liposome-mediated transfection. For transformation, a polynucleotide of the present disclosure will generally further include a selectable marker, e.g., any of several well-known selectable markers such as neomycin resistance, ampicillin resistance, tetracycline resistance, chloramphenicol resistance, and kanamycin resistance.

A polynucleotide or recombinant nucleic acid molecule described herein, can be introduced into a cell (e.g., alga cell) using any method known in the art. A polynucleotide can be introduced into a cell by a variety of methods, which are well known in the art and selected, in part, based on the particular host cell. For example, the polynucleotide can be introduced into a cell using a direct gene transfer method such as electroporation or microprojectile mediated (biolistic) transformation using a particle gun, or the "glass bead method," or by pollen-mediated transformation, liposome-mediated transformation, transformation using wounded or enzyme-degraded immature embryos, or wounded or enzyme-degraded embryogenic callus (for example, as described in Potrykus, Ann. Rev. Plant. Physiol. Plant Mol. Biol. 42:205-225, 1991).

As discussed above, microprojectile mediated transformation can be used to introduce a polynucleotide into a cell (for example, as described in Klein et al., Nature 327:70-73, 1987). This method utilizes microprojectiles such as gold or tungsten, which are coated with the desired polynucleotide by precipitation with calcium chloride, spermidine or polyethylene glycol. The microprojectile particles are accelerated at high speed into a cell using a device such as the BIOLISTIC PD-1000 particle gun (BioRad; Hercules Calif.); a Helios Gene Gun (Cat. #165-2431 and #165-2432; BioRad, U.S.A.); or an Accell Gene Gun (Auragen, U.S.A.). Methods for the transformation using biolistic methods are well known in the art (for example, as described in Christou, Trends in Plant Science 1:423-431, 1996). Microprojectile mediated transformation has been used, for example, to generate a variety of transgenic plant species, including cotton, tobacco, corn, hybrid poplar and papaya. Important cereal crops such as wheat, oat, barley, sorghum and rice also have been transformed using microprojectile mediated delivery (for example, as described in Duan et al., Nature Biotech. 14:494-498, 1996; and Shimamoto, Curr. Opin. Biotech. 5:158-162, 1994). The transformation of most dicotyledonous plants is possible with the methods described above. Transformation of monocotyledonous plants also can be transformed using, for example, biolistic methods as described above, protoplast transformation, electroporation of partially permeabilized cells, introduction of DNA using glass fibers, and the glass bead agitation method.

The basic techniques used for transformation and expression in photosynthetic microorganisms are similar to those commonly used for *E. coli, Saccharomyces cerevisiae* and other species. Transformation methods customized for a photosynthetic microorganisms, e.g., the chloroplast of a strain of algae, are known in the art. These methods have been described in a number of texts for standard molecular biological manipulation (see Packer & Glaser, 1988, "Cyanobacteria", Meth. Enzymol., Vol. 167; Weissbach & Weissbach, 1988, "Methods for plant molecular biology," Academic Press, New York, Sambrook, Fritsch & Maniatis, 1989, "Molecular Cloning: A laboratory manual," 2nd edition Cold Spring Harbor Laboratory Press, Cold Spring Harbor, N.Y.; and Clark M S, 1997, Plant Molecular Biology, Springer, N.Y.). These methods include, for example, biolistic devices (see, for example, Sanford, Trends In Biotech. (1988) 6: 299-302, U.S. Pat. No. 4,945,050; electroporation (Fromm et al., Proc. Nat'l. Acad. Sci. (USA) (1985) 82: 5824-5828); use of a laser beam, electroporation, microinjection or any other method capable of introducing DNA into a host cell.

Plastid transformation is a routine and well known method for introducing a polynucleotide into a plant cell chloroplast (see U.S. Pat. Nos. 5,451,513, 5,545,817, and 5,545,818; WO 95/16783; McBride et al., Proc. Natl. Acad. Sci., USA 91:7301-7305, 1994). In the invention, chloroplast transformation involves introducing regions of chloroplast DNA flanking a desired nucleotide sequence, allowing for homologous recombination of the exogenous DNA into the target chloroplast genome. In some instances one to 1.5 kb flanking nucleotide sequences of chloroplast genomic DNA may be used. Using this method, point mutations in the chloroplast 16S rRNA and rps12 genes, which confer resistance to spectinomycin and streptomycin, can be utilized as selectable markers for transformation (Svab et al., Proc. Natl. Acad. Sci., USA 87:8526-8530, 1990), and can result in stable homoplasmic transformants, at a frequency of approximately one per 100 bombardments of target leaves.

A further refinement in chloroplast transformation/expression technology that facilitates control over the timing and tissue pattern of expression of introduced DNA coding sequences in plant plastid genomes has been described in PCT International Publication WO 95/16783 and U.S. Patent 5,576,198. This method involves the introduction into plant cells of constructs for nuclear transformation that provide for the expression of a viral single subunit RNA polymerase and targeting of this polymerase into the plastids via fusion to a plastid transit peptide. Transformation of plastids with DNA constructs comprising a viral single subunit RNA polymerase-specific promoter specific to the RNA polymerase expressed from the nuclear expression constructs operably linked to DNA coding sequences of interest permits control of the plastid expression constructs in a tissue and/or developmental specific manner in plants comprising both the nuclear polymerase construct and the plastid expression constructs. Expression of the nuclear RNA polymerase coding sequence can be placed under the control of either a constitutive promoter, or a tissue-or developmental stage-specific promoter, thereby extending this control to the plastid expression construct responsive to the plastid-targeted, nuclear-encoded viral RNA polymerase.

When nuclear transformation is utilized, the protein can be modified for plastid targeting by employing plant cell nuclear transformation constructs wherein DNA coding sequences of interest are fused to any of the available transit peptide sequences capable of facilitating transport of the encoded enzymes into plant plastids, and driving expression by employing an appropriate promoter. Targeting of the protein can be achieved by fusing DNA encoding plastid, e.g., chloroplast, leucoplast, amyloplast, etc., transit peptide sequences to the 5' end of DNAs encoding the enzymes. The sequences that encode a transit peptide region can be obtained, for example, from plant nuclear-encoded plastid proteins, such as the small subunit (SSU) of ribulose bisphosphate carboxylase, EPSP synthase, plant fatty acid biosynthesis related genes including fatty acyl-ACP thioesterases, acyl carrier protein (ACP), stearoyl-ACP desaturase, β-ketoacyl-ACP synthase and acyl-ACP thioesterase, or LHCPII genes, etc. Plastid transit peptide sequences can also be obtained from nucleic acid sequences encoding carotenoid biosynthetic enzymes, such as GGPP synthase, phytoene synthase, and phytoene desaturase. Other transit peptide sequences are disclosed in Von Heijne et al. (1991) Plant Mol. Biol. Rep. 9: 104; Clark et al. (1989) J. Biol. Chem. 264: 17544; della-Cioppa et al. (1987) Plant Physiol. 84: 965; Romer et al. (1993) Biochem. Biophys. Res. Commun. 196: 1414; and Shah et al. (1986) Science 233: 478. Another transit peptide sequence is that of the intact ACCase from Chlamydomonas (genbank EDO96563, amino acids 1-33). The encoding sequence for a transit peptide effective in transport to plastids can include all or a portion of the encoding sequence for a particular transit peptide, and may also contain portions of the mature protein encoding sequence associated with a particular transit peptide. Numerous examples of transit peptides that can be used to deliver target proteins into plastids exist, and the particular transit peptide encoding sequences useful in the present disclosure are not critical as long as delivery into a plastid is obtained. Proteolytic processing within the plastid then produces the mature enzyme. This technique has proven successful with enzymes involved in polyhydroxyalkanoate biosynthesis (Nawrath et al. (1994) Proc. Natl. Acad. Sci. USA 91: 12760), and neomycin phosphotransferase II (NPT-II) and CP4 EPSPS (Padgette et al. (1995) Crop Sci. 35: 1451), for example.

Of interest are transit peptide sequences derived from enzymes known to be imported into the leucoplasts of seeds. Examples of enzymes containing useful transit peptides include those related to lipid biosynthesis (e.g., subunits of the plastid-targeted dicot acetyl-CoA carboxylase, biotin carboxylase, biotin carboxyl carrier protein, α-carboxy-transferase, and plastid-targeted monocot multifunctional acetyl-CoA carboxylase (Mw, 220,000); plastidic subunits of the fatty acid synthase complex (e.g., acyl carrier protein (ACP), malonyl-ACP synthase, KASI, KASII, and KASIII); steroyl-ACP desaturase; thioesterases (specific for short, medium, and long chain acyl ACP); plastid-targeted acyl transferases (e.g., glycerol-3-phosphate and acyl transferase); enzymes involved in the biosynthesis of aspartate family amino acids; phytoene synthase; gibberellic acid biosynthesis (e.g., *ent*-kaurene synthases 1 and 2); and carotenoid biosynthesis (e.g., lycopene synthase).

In some embodiments, an alga is transformed with a nucleic acid which encodes a protein of interest, for example, a prenyl transferase, an isoprenoid synthase, or an enzyme capable of converting a precursor into a fuel product or a precursor of a fuel product (e.g., an isoprenoid or fatty acid).

In one embodiment, a transformation may introduce a nucleic acid into a plastid of the host alga (e.g., chloroplast). In another embodiments a transformation may introduce a nucleic acid into the nuclear genome of the host alga. In still another embodiment, a transformation may introduce nucleic acids into both the nuclear genome and into a plastid.

Transformed cells can be plated on selective media following introduction of exogenous nucleic acids. This method may also comprise several steps for screening. A screen of primary transformants can be conducted to determine which clones have proper insertion of the exogenous nucleic acids. Clones which show the proper integration may be propagated and re-screened to ensure genetic stability. Such methodology ensures that the transformants contain the genes of interest. In many instances, such screening is performed by polymerase chain reaction (PCR); however, any other appropriate technique known in the art may be utilized. Many different methods of PCR are known in the art (e.g., nested PCR, real time PCR). For any given screen, one of skill in the art will recognize that PCR components may be varied to achieve optimal screening results. For example, magnesium concentration may need to be adjusted upwards when PCR is performed on disrupted alga cells to which (which chelates magnesium) is added to chelate toxic metals. Following the screening for clones with the proper integration of exogenous nucleic acids, clones can be screened for the presence of the encoded protein(s) and/or products. Protein expression screening can be performed by Western blot analysis and/or enzyme activity assays. Transporter and/or product screening may be performed by any method known in the art, for example ATP turnover assay, substrate transport assay, HPLC or gas chromatography.

The expression of the protein or enzyme can be accomplished by inserting a polynucleotide sequence (gene) encoding the protein or enzyme into the chloroplast or nuclear genome of a microalgae. The modified strain of microalgae can be made homoplasmic to ensure that the polynucleotide will be stably maintained in the chloroplast genome of all descendents. A microalga is homoplasmic for a gene when the inserted gene is present in all copies of the chloroplast genome, for example. It is apparent to one of skill in the art that a chloroplast may contain multiple copies of its genome, and therefore, the term "homoplasmic" or "homoplasmy' refers to the state where all copies of a particular locus of interest are substantially identical. Plastid expression, in which genes are inserted by homologous recombination into all of the several thousand copies of the circular plastid genome present in each plant cell, takes advantage of the enormous copy number advantage over nuclear-expressed genes to permit expression levels that can readily exceed 10% or more of the total soluble plant protein. The process of determining the plasmic state of an organism of the present disclosure involves screening transformants for the presence of exogenous nucleic acids and the absence of wild-type nucleic acids at a given locus of interest.

### Vectors

Construct, vector and plasmid are used interchangeably throughout the disclosure. Nucleic acids encoding the proteins described herein, can be contained in vectors, including cloning and expression vectors. A cloning vector is a self-replicating DNA molecule that serves to transfer a DNA segment into a host cell. Three common types of cloning vectors are bacterial plasmids, phages, and other viruses. An expression vector is a cloning vector designed so that a coding sequence inserted at a particular site will be transcribed and translated into a protein. Both cloning and expression vectors can contain nucleotide sequences that allow the vectors to replicate in one or more suitable host cells. In cloning vectors, this sequence is generally one that enables the vector to replicate independently of the host cell chromosomes, and also includes either origins of replication or autonomously replicating sequences.

In some embodiments, a polynucleotide of the present disclosure is cloned or inserted into an expression vector using cloning techniques know to one of skill in the art. The nucleotide sequences may be inserted into a vector by a variety of methods. In the most common method the sequences are inserted into an appropriate restriction endonuclease site(s) using procedures commonly known to those skilled in the art and detailed in, for example, Sambrook et al., Molecular Cloning, A Laboratory Manual, 2nd Ed., Cold Spring Harbor Press, (1989) and Ausubel et al., Short Protocols in Molecular Biology, 2nd Ed., John Wiley & Sons (1992).

Suitable expression vectors include, but are not limited to, baculovirus vectors, bacteriophage vectors, plasmids, phagemids, cosmids, fosmids, bacterial artificial chromosomes, viral vectors (e.g. viral vectors based on vaccinia virus, poliovirus, adenovirus, adeno-associated virus, SV40, and herpes simplex virus), PI-based artificial chromosomes, yeast plasmids, yeast artificial chromosomes, and any other vectors specific for specific hosts of interest (such as *E. coli* and yeast). Thus, for example, a polynucleotide encoding an FPP synthase, can be inserted into any one of a variety of expression vectors that are capable of expressing the enzyme. Such vectors can include, for example, chromosomal, nonchromosomal and synthetic DNA sequences.

Suitable expression vectors include chromosomal, non-chromosomal and synthetic DNA sequences, for example, SV 40 derivatives; bacterial plasmids; phage DNA; baculovirus; yeast plasmids; vectors derived from combinations of plasmids and phage DNA; and viral DNA such as vaccinia, adenovirus, fowl pox virus, and pseudorabies. In addition, any other vector that is replicable and viable in the host may be used. For example, vectors such as Ble2A, Arg7/2A, and SEnuc357 can be used for the expression of a protein.

Numerous suitable expression vectors are known to those of skill in the art. The following vectors are provided by way of example; for bacterial host cells: pQE vectors (Qiagen), pBluescript plasmids, pNH vectors, lambda-ZAP vectors (Stratagene), pTrc99a, pKK223-3, pDR540, and pRIT2T (Pharmacia); for eukaryotic host cells: pXT1, pSG5 (Stratagene), pSVK3, pBPV, pMSG, pET21a-d(+) vectors (Novagen), and pSVLSV40 (Pharmacia). However, any other plasmid or other vector may be used so long as it is compatible with the host cell.

The expression vector, or a linearized portion thereof, can encode one or more exogenous or endogenous nucleotide sequences. Examples of exogenous nucleotide sequences that can be transformed into a host include genes from bacteria, fungi, plants, photosynthetic bacteria or other algae. Examples of other types of nucleotide sequences that can be transformed into a host, include, but are not limited to, transporter genes, isoprenoid producing genes, genes which encode for proteins which produce isoprenoids with two phosphates (e.g., GPP synthase and/or FPP synthase), genes which encode for proteins which produce fatty acids, lipids, or triglycerides, for example, ACCases, endogenous promoters, and 5' UTRs from the psbA, atpA, or rbcL genes. In some instances, an exogenous sequence is flanked by two homologous sequences.

Homologous sequences are, for example, those that have at least 50%, at least 60%, at least 70%, at least 80%, at least 90%, at least 95%, at least 98%, or at least at least 99% sequence identity to a reference amino acid sequence, for example, the amino acid sequence found naturally in the host cell. The first and second sequences enable recombination of the exogenous or endogenous sequence into the genome of the host organism. The first and second homologous sequences can be at least 100, at least 200, at least 300, at least 400, at least 500, or at least 1000, or at least 1500 nucleotides in length.

The polynucleotide sequence may comprise nucleotide sequences that are codon biased for expression in the organism being transformed. The skilled artisan is well aware of the "codon-bias" exhibited by a specific host cell in usage of nucleotide codons to specify a given amino acid. Without being bound by theory, by using a host cell's preferred codons, the rate of translation may be greater. Therefore, when synthesizing a gene for improved expression in a host cell, it may be desirable to design the gene such that its frequency of codon usage approaches the frequency of preferred codon usage of the host cell. In some organisms, codon bias differs between the nuclear genome and organelle genomes, thus, codon optimization or biasing may be performed for the target genome (e.g., nuclear codon biased or chloroplast codon biased). In some embodiments, codon biasing occurs before mutagenesis to generate a polypeptide. In other examples, codon biasing occurs after mutagenesis to generate a polynucleotide. In yet other examples, codon biasing occurs before mutagenesis as well as after mutagenesis. Codon bias is described in detail herein.

In some examples, a vector comprises a polynucleotide operably linked to one or more control elements, such as a promoter and/or a transcription terminator. A nucleic acid sequence is operably linked when it is placed into a functional relationship with another nucleic acid sequence. For example, DNA for a presequence or secretory leader is operatively linked to DNA for a polypeptide if it is expressed as a preprotein which participates in the secretion of the polypeptide; a promoter is operably linked to a coding sequence if it affects the transcription of the sequence; or a ribosome binding site is operably linked to a coding sequence if it is positioned so as to facilitate translation. Generally, operably linked sequences are contiguous and, in the case of a secretory leader, contiguous and in reading phase. Linking is achieved by ligation at restriction enzyme sites. If suitable restriction sites are not available, then synthetic oligonucleotide adapters or linkers can be used as is known to those skilled in the art. Sambrook et al., Molecular Cloning, A Laboratory Manual, 2nd Ed., Cold Spring Harbor Press, (1989) and Ausubel et al., Short Protocols in Molecular Biology, 2nd Ed., John Wiley & Sons (1992).

A vector in some examples provides for amplification of the copy number of a polynucleotide. A vector can be, for example, an expression vector that provides for expression of an ACCase, a prenyl transferase, an isoprenoid synthase, or a mevalonate synthesis enzyme in a host cell, e.g., a prokaryotic host cell or a eukaryotic host cell.

A polynucleotide or polynucleotides can be contained in a vector or vectors. For example, where a second (or more) nucleic acid molecule is desired, the second nucleic acid molecule can be contained in a vector, which can, but need not be, the same vector as that containing the first nucleic acid molecule. For example, an algal host cell modified to express two endogenous or exogenous genes may be transformed with a single vector containing both sequences, or two vectors, each comprising one gene to be expressed. The vector can be any vector useful for introducing a polynucleotide into a genome and can include a nucleotide sequence of genomic DNA (e.g., nuclear or plastid) that is sufficient to undergo homologous recombination with genomic DNA, for example, a nucleotide sequence comprising about 400 to about 1500 or more substantially contiguous nucleotides of genomic DNA.

A regulatory or control element, as the term is used herein, broadly refers to a nucleotide sequence that regulates the transcription or translation of a polynucleotide or the localization of a polypeptide to which it is operatively linked. Examples include, but are not limited to, an RBS, a promoter, enhancer, transcription terminator, an initiation (start) codon, a splicing signal for intron excision and maintenance of a correct reading frame, a STOP codon, an amber or ochre codon, and an IRES. A regulatory element can include a promoter and transcriptional and translational stop signals. Elements may be provided with linkers for the purpose of introducing specific restriction sites facilitating ligation of the control sequences with the coding region of a nucleotide sequence encoding a polypeptide. Additionally, a sequence comprising a cell compartmentalization signal (i.e., a sequence that targets a polypeptide to the cytosol, nucleus, chloroplast membrane or cell membrane) can be attached to the polynucleotide encoding a protein of interest. Such signals are well known in the art and have been widely reported (see, e.g., U.S. Pat. No. 5,776,689).

Promoters are untranslated sequences located generally 100 to 1000 base pairs (bp) upstream from the start codon of a structural gene that regulate the transcription and translation of nucleic acid sequences under their control.

Promoters useful for the present disclosure may come from any source (e.g., viral, bacterial, fungal, protist, and animal). The promoters contemplated herein can be specific to photosynthetic organisms, non-vascular photosynthetic organisms, and vascular photosynthetic organisms (e.g., algae, flowering plants). In some instances, the nucleic acids above are inserted into a vector that comprises a promoter of a photosynthetic organism, e.g., algae. The promoter can be a constitutive promoter or an inducible promoter. A promoter typically includes necessary nucleic acid sequences near the start site of transcription, (e.g., a TATA element). Common promoters used in expression vectors include, but are not limited to, LTR or SV40 promoter, the E. coli lac or trp promoters, and the phage lambda PL promoter. Other promoters known to control the expression of genes in prokaryotic or eukaryotic cells can be used and are known to those skilled in the art. Expression vectors may also contain a ribosome binding site for translation initiation, and a transcription terminator. The vector may also contain sequences useful for the amplification of gene expression.

A "constitutive" promoter is a promoter that is active under most environmental and developmental conditions. An "inducible" promoter is a promoter that is active under controllable environmental or developmental conditions. Examples of inducible promoters/regulatory elements include, for example, a nitrate-inducible promoter (for example, as described in Bock et al, Plant MoI. Biol. 17:9 (1991)), or a light-inducible promoter, (for example, as described in Feinbaum et al, Mol Gen. Genet. 226:449 (1991); and Lam and Chua, Science 248:471 (1990)), or a heat responsive promoter (for example, as described in Muller et al., Gene 111: 165-73 (1992)).

In many examples, a polynucleotide of the present disclosure includes a nucleotide sequence encoding a protein or enzyme of the present disclosure, where the nucleotide sequence encoding the polypeptide is operably linked to an inducible promoter. Inducible promoters are well known in the art. Suitable inducible promoters include, but are not limited to, the pL of bacteriophage λ; Placo; Ptrp; Ptac (Ptrp-lac hybrid promoter); an isopropyl-beta-D-thiogalactopyranoside (IPTG)-inducible promoter, e.g., a lacZ promoter; a tetracycline-inducible promoter; an arabinose inducible promoter, e.g., P_{BAD} (for example, as described in Guzman et al. (1995) J. Bacteriol. 177:4121-4130); a xylose-inducible promoter, e.g., Pxyl (for example, as described in Kim et al. (1996) Gene 181:71-76); a GAL1 promoter; a tryptophan promoter; a lac promoter; an alcohol-inducible promoter, e.g., a methanol-inducible promoter, an ethanol-inducible promoter; a raffinose-inducible promoter; and a heat-inducible promoter, e.g., heat inducible lambda P_{L} promoter and a promoter controlled by a heat-sensitive repressor (e.g., C1857-repressed lambda-based expression vectors; for example, as described in Hoffmann et al. (1999) FEMS Microbiol Lett. 177(2):327-34).

In many examples, a polynucleotide of the present disclosure includes a nucleotide sequence encoding a protein or enzyme of the present disclosure, where the nucleotide sequence encoding the polypeptide is operably linked to a constitutive promoter. Suitable constitutive promoters for use in prokaryotic cells are known in the art and include, but are not limited to, a sigma70 promoter, and a consensus sigma70 promoter.

Suitable promoters for use in prokaryotic host cells include, but are not limited to, a bacteriophage T7 RNA polymerase promoter; a trp promoter; a lac operon promoter; a hybrid promoter, e.g., a lac/tac hybrid promoter, a tac/trc hybrid promoter, a trp/lac promoter, a T7/lac promoter; a trc promoter; a tac promoter; an araBAD promoter; in vivo regulated promoters, such as an ssaG promoter or a related promoter (for example, as described in U.S. Patent Publication No. 20040131637), a pagC promoter (for example, as described in Pulkkinen and Miller, J. Bacteriol., 1991: 173(1): 86-93; and Alpuche-Aranda et al., PNAS, 1992; 89(21): 10079-83), a nirB promoter (for example, as described in Harborne et al. (1992) Mol. Micro. 6:2805-2813; Dunstan et al. (1999) Infect. Immun. 67:5133-5141; McKelvie et al. (2004) Vaccine 22:3243-3255; and Chatfield et al. (1992) Biotechnol. 10:888-892); a sigma70 promoter, e.g., a consensus sigma70 promoter (for example, GenBank Accession Nos. AX798980, AX798961, and AX798183); a stationary phase promoter, e.g., a dps promoter, an spv promoter; a promoter derived from the pathogenicity island SPI-2 (for example, as described in WO96/17951); an actA promoter (for example, as described in Shetron-Rama et al. (2002) Infect. Immun. 70:1087-1096); an rpsM promoter (for example, as described in Valdivia and Falkow (1996). Mol. Microbiol. 22:367-378); a tet promoter (for example, as described in Hillen, W. and Wissmann, A. (1989) In Saenger, W. and Heinemann, U. (eds), Topics in Molecular and Structural Biology, Protein-Nucleic Acid Interaction. Macmillan, London, UK, Vol. 10, pp. 143-162); and an SP6 promoter (for example, as described in Melton et al. (1984) Nucl. Acids Res. 12:7035-7056).

In yeast, a number of vectors containing constitutive or inducible promoters may be used. For a review of such vectors see, Current Protocols in Molecular Biology, Vol. 2, 1988, Ed. Ausubel, et al., Greene Publish. Assoc. & Wiley Interscience, Ch. 13; Grant, et al., 1987, Expression and Secretion Vectors for Yeast, in Methods in Enzymology, Eds. Wu & Grossman, 31987, Acad. Press, N.Y., Vol. 153, pp. 516-544; Glover, 1986, DNA Cloning, Vol. II, IRL Press, Wash., D.C., Ch. 3; Bitter, 1987, Heterologous Gene Expression in Yeast, Methods in Enzymology, Eds. Berger & Kimmel, Acad. Press, N.Y., Vol. 152, pp. 673-684; and The Molecular Biology of the Yeast Saccharomyces, 1982, Eds. Strathern et al., Cold Spring Harbor Press, Vols. I and II. A constitutive yeast promoter such as ADH or LEU2 or an inducible promoter such as GAL may be used (for example, as described in Cloning in Yeast, Ch. 3, R. Rothstein In: DNA Cloning Vol. 11, A Practical Approach, Ed. DM Glover, 1986, IRL Press, Wash., D.C.). Alternatively, vectors may be used which promote integration of foreign DNA sequences into the yeast chromosome.

Non-limiting examples of suitable eukaryotic promoters include CMV immediate early, HSV thymidine kinase, early and late SV40, LTRs from retrovirus, and mouse metallothionein-I. Selection of the appropriate vector and promoter is well within the level of ordinary skill in the art. The expression vector may also contain a ribosome binding site for translation initiation and a transcription terminator. The expression vector may also include appropriate sequences for amplifying expression.

A vector utilized in the practice of the disclosure also can contain one or more additional nucleotide sequences that confer desirable characteristics on the vector, including, for example, sequences such as cloning sites that facilitate manipulation of the vector, regulatory elements that direct replication of the vector or transcription of nucleotide sequences contain therein, and sequences that encode a selectable marker. As such, the vector can contain, for example, one or more cloning sites such as a multiple cloning site, which can, but need not, be positioned such that a exogenous or endogenous polynucleotide can be inserted into the vector and operatively linked to a desired element.

The vector also can contain a prokaryote origin of replication (ori), for example, an E. coli ori or a cosmid ori, thus allowing passage of the vector into a prokaryote host cell, as well as into a plant chloroplast. Various bacterial and viral origins of replication are well known to those skilled in the art and include, but are not limited to the pBR322 plasmid origin, the 2u plasmid origin, and the SV40, polyoma, adenovirus, VSV, and BPV viral origins.

A regulatory or control element, as the term is used herein, broadly refers to a nucleotide sequence that regulates the transcription or translation of a polynucleotide or the localization of a polypeptide to which it is operatively linked. Examples include, but are not limited to, an RBS, a promoter, enhancer, transcription terminator, an initiation (start) codon, a splicing signal for intron excision and maintenance of a correct reading frame, a STOP codon, an amber or ochre codon, an IRES. Additionally, an element can be a cell compartmentalization signal (i.e., a sequence that targets a polypeptide to the cytosol, nucleus, chloroplast membrane or cell membrane). In some aspects of the present disclosure, a cell compartmentalization signal (e.g., a cell membrane targeting sequence) may be ligated to a gene and/or transcript, such that translation of the gene occurs in the chloroplast. In other aspects, a cell compartmentalization signal may be ligated to a gene such that, following translation of the gene, the protein is transported to the cell membrane. Cell compartmentalization signals are well known in the art and have been widely reported (see, e.g., U.S. Pat. No. 5,776,689).

A vector, or a linearized portion thereof, may include a nucleotide sequence encoding a reporter polypeptide or other selectable marker. The term "reporter" or "selectable marker" refers to a polynucleotide (or encoded polypeptide) that confers a detectable phenotype. A reporter generally encodes a detectable polypeptide, for example, a green fluorescent protein or an enzyme such as luciferase, which, when contacted with an appropriate agent (a particular wavelength of light or luciferin, respectively) generates a signal that can be detected by eye or using appropriate instrumentation (for example, as described in Giacomin, Plant Sci. 116:59-72, 1996; Scikantha, J. Bacteriol. 178:121, 1996; Gerdes, FEBS Lett. 389:44-47, 1996; and Jefferson, EMBO J. 6:3901-3907, 1997, fl-glucuronidase). A selectable marker generally is a molecule that, when present or expressed in a cell, provides a selective advantage (or disadvantage) to the cell containing the marker, for example, the ability to grow in the presence of an agent that otherwise would kill the cell.

A selectable marker can provide a means to obtain, for example, prokaryotic cells, eukaryotic cells, and/or plant cells that express the marker and, therefore, can be useful as a component of a vector of the disclosure. The selection gene or marker can encode for a protein necessary for the survival or growth of the host cell transformed with the vector. One class of selectable markers are native or modified genes which restore a biological or physiological function to a host cell (e.g., restores photosynthetic capability or restores a metabolic pathway). Other examples of selectable markers include, but are not limited to, those that confer antimetabolite resistance, for example, dihydrofolate reductase, which confers resistance to methotrexate (for example, as described in Reiss, Plant Physiol. (Life Sci. Adv.) 13:143-149, 1994); neomycin phosphotransferase, which confers resistance to the aminoglycosides neomycin, kanamycin and paromycin (for example, as described in Herrera-Estrella, EMBO J. 2:987-995, 1983), hygro, which confers resistance to hygromycin (for example, as described in Marsh, Gene 32:481-485, 1984), trpB, which allows cells to utilize indole in place of tryptophan; hisD, which allows cells to utilize histinol in place of histidine (for example, as described in Hartman, Proc. Natl. Acad. Sci., USA 85:8047, 1988); mannose-6-phosphate isomerase which allows cells to utilize mannose (for example, as described in PCT Publication Application No. WO 94/20627); ornithine decarboxylase, which confers resistance to the ornithine decarboxylase inhibitor, 2-(difluoromethyl)-DL-ornithine (DFMO; for example, as described in McConlogue, 1987, In: Current Communications in Molecular Biology, Cold Spring Harbor Laboratory ed.); and deaminase from *Aspergillus terreus*, which confers resistance to Blasticidin S (for example, as described in Tamura, Biosci. Biotechnol. Biochem. 59:2336-2338, 1995). Additional selectable markers include those that confer herbicide resistance, for example, phosphinothricin acetyltransferase gene, which confers resistance to phosphinothricin (for example, as described in White et al., Nucl. Acids Res. 18:1062, 1990; and Spencer et al., Theor. Appl. Genet. 79:625-631, 1990), a mutant EPSPV-synthase, which confers glyphosate resistance (for example, as described in Hinchee et al., BioTechnology 91:915-922, 1998), a mutant acetolactate synthase, which confers imidazolione or sulfonylurea resistance (for example, as described in Lee et al., EMBO J. 7:1241-1248, 1988), a mutant psbA, which confers resistance to atrazine (for example, as described in Smeda et al., Plant Physiol. 103:911 - 917, 1993), or a mutant protoporphyrinogen oxidase (for example, as described in U.S. Pat. No. 5,767,373), or other markers conferring resistance to an herbicide such as glufosinate. Selectable markers include polynucleotides that confer dihydrofolate reductase (DHFR) or neomycin resistance for eukaryotic cells; tetramycin or ampicillin resistance for prokaryotes such as *E. coli*; and bleomycin, gentamycin, glyphosate, hygromycin, kanamycin, methotrexate, phleomycin, phosphinotricin, spectinomycin, dtreptomycin, streptomycin, sulfonamide and sulfonylurea resistance in plants (for example, as described in Maliga et al., Methods in Plant Molecular Biology, Cold Spring Harbor Laboratory Press, 1995, page 39). Additional selectable markers include a mutation in dichlorophenyl dimethylurea (DCMU) that results in resistance to DCMU. Selectable markers also include chloramphenicol acetyltransferase (CAT) and tetracycline. The selection marker can have its own promoter or its expression can be driven by a promoter driving the expression of a polypeptide of interest.

Reporter genes greatly enhance the ability to monitor gene expression in a number of biological organisms. Reporter genes have been successfully used in chloroplasts of higher plants, and high levels of recombinant protein expression have been reported. In addition, reporter genes have been used in the chloroplast of *C*. *reinhardtii.* In chloroplasts of higher plants, β-glucuronidase (uidA, for example, as described in Staub and Maliga, EMBO J. 12:601-606, 1993), neomycin phosphotransferase (nptII, for example, as described in Carrer et al., Mol. Gen. Genet. 241:49-56, 1993), adenosyl-3-adenyltransf-erase (aadA, for example, as described in Svab and Maliga, Proc. Natl. Acad. Sci., USA 90:913-917, 1993), and the *Aequorea victoria* GFP (for example, as described in Sidorov et al., Plant J. 19:209-216, 1999) have been used as reporter genes (for example, as described in Heifetz, Biochemie 82:655-666, 2000). Each of these genes has attributes that make them useful reporters of chloroplast gene expression, such as ease of analysis, sensitivity, or the ability to examine expression in situ. Based upon these studies, other exogenous proteins have been expressed in the chloroplasts of higher plants such as *Bacillus thuringiensis* Cry toxins, conferring resistance to insect herbivores (for example, as described in Kota et al., Proc. Natl. Acad. Sci., USA 96:1840-1845, 1999), or human somatotropin (for example, as described in Staub et al., Nat. Biotechnol. 18:333-338, 2000), a potential biopharmaceutical. Several reporter genes have been expressed in the chloroplast of the eukaryotic green alga, C. *reinhardtii*, including aadA (for example, as described in Goldschmidt-Clermont, Nucl. Acids Res. 19:4083-4089 1991; and Zerges and Rochaix, Mol. Cell Biol. 14:5268-5277, 1994), uidA (for example, as described in Sakamoto et al., Proc. Natl. Acad. Sci., USA 90:477-501, 1993; and Ishikura et al., J. Biosci. Bioeng. 87:307-314 1999), Renilla luciferase (for example, as described in Minko et al., Mol. Gen. Genet. 262:421-425, 1999) and the amino glycoside phosphotransferase from *Acinetobacter baumanii*, aphA6 (for example, as described in Bateman and Purton, Mol. Gen. Genet 263:404-410, 2000). In one example the protein described herein is modified by the addition of an N-terminal strep tag epitope to add in the detection of protein expression.

In some instances, the vectors of the present disclosure will contain elements such as an *E. coli* or *S. cerevisiae* origin of replication. Such features, combined with appropriate selectable markers, allows for the vector to be "shuttled" between the target host cell and a bacterial and/or yeast cell. The ability to passage a shuttle vector of the disclosure in a secondary host may allow for more convenient manipulation of the features of the vector. For example, a reaction mixture containing the vector and inserted polynucleotide(s) of interest can be transformed into prokaryote host cells such as *E. coli,* amplified and collected using routine methods, and examined to identify vectors containing an insert or construct of interest. If desired, the vector can be further manipulated, for example, by performing site directed mutagenesis of the inserted polynucleotide, then again amplifying and selecting vectors having a mutated polynucleotide of interest. A shuttle vector then can be introduced into plant cell chloroplasts, wherein a polypeptide of interest can be expressed and, if desired, isolated according to a method of the disclosure.

Knowledge of the chloroplast or nuclear genome of the host organism, for example, C. *reinhardtii*, is useful in the construction of vectors for use in the disclosed examples. Chloroplast vectors and methods for selecting regions of a chloroplast genome for use as a vector are well known (see, for example, Bock, J. Mol. Biol. 312:425-438, 2001; Staub and Maliga, Plant Cell 4:39-45, 1992; and Kavanagh et al., Genetics 152:1111-1122, 1999, ). The entire chloroplast genome of C. *reinhardtii* is available to the public on the world wide web, at the URL "biology.duke.edu/chlamy_genome/- chloro.html" (see "view complete genome as text file" link and "maps of the chloroplast genome" link; J. Maul, J. W. Lilly, and D. B. Stern, unpublished results; revised Jan. 28, 2002; to be published as GenBank Acc. No. AF396929; and Maul, J. E., et al. (2002) The Plant Cell, Vol. 14 (2659-2679)). Generally, the nucleotide sequence of the chloroplast genomic DNA that is selected for use is not a portion of a gene, including a regulatory sequence or coding sequence. For example, the selected sequence is not a gene that if disrupted, due to the homologous recombination event, would produce a deleterious effect with respect to the chloroplast. For example, a deleterious effect on the replication of the chloroplast genome or to a plant cell containing the chloroplast. In this respect, the website containing the *C*. *reinhardtii* chloroplast genome sequence also provides maps showing coding and non-coding regions of the chloroplast genome, thus facilitating selection of a sequence useful for constructing a vector (also described in Maul, J. E., et al. (2002) The Plant Cell, Vol. 14 (2659-2679)). For example, the chloroplast vector, p322, is a clone extending from the Eco (Eco RI) site at about position 143.1 kb to the Xho (Xho I) site at about position 148.5 kb (see, world wide web, at the URL "biology.duke.edu/chlamy_genome/chloro.html", and clicking on "maps of the chloroplast genome" link, and "140-150 kb" link; also accessible directly on world wide web at URL "biology.duke.edu/chlam- y/chloro/chlorol40.html").

In addition, the entire nuclear genome of *C*. *reinhardtii* is described in Merchant, S. S., et al., Science (2007), 318(5848):245-250, thus facilitating one of skill in the art to select a sequence or sequences useful for constructing a vector.

For expression of the polypeptide in a host, an expression cassette or vector may be employed. The expression vector will provide a transcriptional and translational initiation region, which may be inducible or constitutive, where the coding region is operably linked under the transcriptional control of the transcriptional initiation region, and a transcriptional and translational termination region. These control regions may be native to the gene, or may be derived from an exogenous source. Expression vectors generally have convenient restriction sites located near the promoter sequence to provide for the insertion of nucleic acid sequences encoding exogenous or endogenous proteins. A selectable marker operative in the expression host may be present.

The nucleotide sequences may be inserted into a vector by a variety of methods. In the most common method the sequences are inserted into an appropriate restriction endonuclease site(s) using procedures commonly known to those skilled in the art and detailed in, for example, Sambrook et al., Molecular Cloning, A Laboratory Manual, 2nd Ed., Cold Spring Harbor Press, (1989) and Ausubel et al., Short Protocols in Molecular Biology, 2nd Ed., John Wiley & Sons (1992).

The description herein provides that host cells may be transformed with vectors. One of skill in the art will recognize that such transformation includes transformation with circular or linearized vectors, or linearized portions of a vector. Thus, a host cell comprising a vector may contain the entire vector in the cell (in either circular or linear form), or may contain a linearized portion of a vector of the present disclosure. In some instances 0.5 to 1.5 kb flanking nucleotide sequences of chloroplast genomic DNA may be used. In some instances 0.5 to 1.5 kb flanking nucleotide sequences of nuclear genomic DNA may be used, or 2.0 to 5.0 kb may be used.

### Compounds

The modified or transformed host organism disclosed herein is useful in the production of a desired compound, composition, or product. The present disclosure provides methods of producing, for example, an isoprenoid or isoprenoid precursor compound in a host cell. One such method involves, culturing a modified host cell in a suitable culture medium under conditions that promote synthesis of a product, for example, an isoprenoid compound or isoprenoid precursor compound, where the isoprenoid compound is generated by the expression of an enzyme of the present disclosure, wherein the enzyme uses a substrate present in the host cell. In some examples, a method further comprises isolating the isoprenoid compound from the cell and/or from the culture medium.

In some examples, the product (e.g. fuel molecule) is collected by harvesting the liquid medium. As some fuel molecules (e.g., monoterpenes) are immiscible in water, they would float to the surface of the liquid medium and could be extracted easily, for example by skimming. In other instances, the fuel molecules can be extracted from the liquid medium. In still other instances, the fuel molecules are volatile. In such instances, impermeable barriers can cover or otherwise surround the growth environment and can be extracted from the air within the barrier. For some fuel molecules, the product may be extracted from both the environment (e.g., liquid environment and/or air) and from the intact host cells. Typically, the organism would be harvested at an appropriate point and the product may then be extracted from the organism. The collection of cells may be by any means known in the art, including, but not limited to concentrating cells, mechanical or chemical disruption of cells, and purification of product(s) from cell cultures and/or cell lysates. Cells and/or organisms can be grown and then the product(s) collected by any means known to one of skill in the art. One method of extracting the product is by harvesting the host cell or a group of host cells and then drying the cell(s). The product(s) from the dried host cell(s) are then harvested by crushing the cells to expose the product. In some instances, the product may be produced without killing the organisms. Producing and/or expressing the product may not render the organism unviable.

In some examples, a genetically modified host cell is cultured in a suitable medium (e.g., Luria-Bertoni broth, optionally supplemented with one or more additional agents, such as an inducer (e.g., where the isoprenoid synthase is under the control of an inducible promoter); and the culture medium is overlaid with an organic solvent, e.g. dodecane, forming an organic layer. The compound produced by the genetically modified host partitions into the organic layer, from which it can then be purified. In some examples, where, for example, a prenyl transferase, isoprenoid synthase or mevalonate synthesis-encoding nucleotide sequence is operably linked to an inducible promoter, an inducer is added to the culture medium; and, after a suitable time, the compound is isolated from the organic layer overlaid on the culture medium.

In some examples, the compound or product, for example, an isoprenoid compound will be separated from other products which may be present in the organic layer. Separation of the compound from other products that may be present in the organic layer is readily achieved using, e.g., standard chromatographic techniques.

Methods of culturing the host cells, separating products, and isolating the desired product or products are known to one of skill in the art and are discussed further herein.

In some examples, the compound, for example, an isoprenoid or isoprenoid compound is produced in a genetically modified host cell at a level that is at least about 2-fold, at least about 5-fold, at least about 10-fold, at least about 25-fold, at least about 50-fold, at least about 100-fold, at least about 500-fold, at least about 1000-fold, at least about 2000-fold, at least about 3000-fold, at least about 4000-fold, at least about 5000-fold, or at least about 10,000-fold, or more, higher than the level of the isoprenoid or isoprenoid precursor compound produced in an unmodified host cell that produces the isoprenoid or isoprenoid precursor compound via the same biosynthetic pathway.

In some examples, the compound, for example, an isoprenoid compound is pure, e.g., at least about 40% pure, at least about 50% pure, at least about 60% pure, at least about 70% pure, at least about 80% pure, at least about 90% pure, at least about 95% pure, at least about 98%, or more than 98% pure. "Pure" in the context of an isoprenoid compound refers to an isoprenoid compound that is free from other isoprenoid compounds, portions of compounds, contaminants, and unwanted byproducts, for example.

Examples of products contemplated herein include hydrocarbon products and hydrocarbon derivative products. A hydrocarbon product is one that consists of only hydrogen molecules and carbon molecules. A hydrocarbon derivative product is a hydrocarbon product with one or more heteroatoms, wherein the heteroatom is any atom that is not hydrogen or carbon. Examples of heteroatoms include, but are not limited to, nitrogen, oxygen, sulfur, and phosphorus. Some products can be hydrocarbon-rich, wherein, for example, at least 50%, at least 60%, at least 70%, at least 80%, at least 90%, or at least 95% of the product by weight is made up of carbon and hydrogen.

In one example, the vector comprises one or more nucleic acid sequences involved in isoprenoid synthesis. The terms "isoprenoid," "isoprenoid compound," "terpene," "terpene compound," "terpenoid," and "terpenoid compound" are used interchangeably herein. Isoprenoid compounds include, but are not limited to, monoterpenes, sesquiterpenes, diterpenes, triterpenes, and polyterpenes.

One exemplary group of hydrocarbon products are isoprenoids. Isoprenoids (including terpenoids) are derived from isoprene subunits, but are modified, for example, by the addition of heteroatoms such as oxygen, by carbon skeleton rearrangement, and by alkylation. Isoprenoids generally have a number of carbon atoms which is evenly divisible by five, but this is not a requirement as "irregular" terpenoids are known to one of skill in the art. Carotenoids, such as carotenes and xanthophylls, are examples of isoprenoids that are useful products. A steroid is an example of a terpenoid. Examples of isoprenoids include, but are not limited to, hemiterpenes (C5), monoterpenes (C10), sesquiterpenes (C15), diterpenes (C20), triterpenes (C30), tetraterpenes (C40), polyterpenes (Cₙ, wherein "n" is equal to or greater than 45), and their derivatives. Other examples of isoprenoids include, but are not limited to, limonene, 1,8-cineole, α-pinene, camphene, (+)-sabinene, myrcene, abietadiene, taxadiene, farnesyl pyrophosphate, fusicoccadiene, amorphadiene, (E)-α-bisabolene, zingiberene, or diapophytoene, and their derivatives.

Products, for example fuel products, comprising hydrocarbons, may be precursors or products conventionally derived from crude oil, or petroleum, such as, but not limited to, liquid petroleum gas, naptha (ligroin), gasoline, kerosene, diesel, lubricating oil, heavy gas, coke, asphalt, tar, and waxes.

Useful products include, but are not limited to, terpenes and terpenoids as described above. An exemplary group ofterpenes are diterpenes (C20). Diterpenes are hydrocarbons that can be modified (e.g. oxidized, methyl groups removed, or cyclized); the carbon skeleton of a diterpene can be rearranged, to form, for example, terpenoids, such as fusicoccadiene. Fusicoccadiene may also be formed, for example, directly from the isoprene precursors, without being bound by the availability of diterpene or GGDP. Genetic modification of organisms, such as algae, by the methods described herein, can lead to the production of fusicoccadiene, for example, and other types of terpenes, such as limonene, for example. Genetic modification can also lead to the production of modified terpenes, such as methyl squalene or hydroxylated and/or conjugated terpenes such as paclitaxel.

Other useful products can be, for example, a product comprising a hydrocarbon obtained from an organism expressing a diterpene synthase. Such exemplary products include ent-kaurene, casbene, and fusicoccadiene, and may also include fuel additives.

In some examples, a product (such as a fuel product) contemplated herein comprises one or more carbons derived from an inorganic carbon source. In some examples, at least 10%, at least 20%, at least 30%, at least 40%, at least 50%, at least 60%, at least 70%, at least 80%, at least 90%, at least 95%, or at least 99% of the carbons of a product as described herein are derived from an inorganic carbon source. Examples of inorganic carbon sources include, but are not limited to, carbon dioxide, carbonate, bicarbonate, and carbonic acid. The product can be, for example, an organic molecule with carbons from an inorganic carbon source that were fixed during photosynthesis.

The products produced by the present disclosure may be naturally, or non-naturally (e.g., as a result of transformation) produced by the host cell(s) and/or organism(s) transformed. For example, products not naturally produced by algae may include non-native terpenes/terpenoids such as fusicoccadiene or limonene. A product naturally produced in algae may be a terpene such as a carotenoid (for example, beta-carotene). The host cell may be genetically modified, for example, by transformation of the cell with a sequence encoding a protein, wherein expression of the protein results in the secretion of a naturally or a non-naturally produced product (e.g. limonene) or products. The product may be a molecule not found in nature.

Examples of products include petrochemical products, precursors of petrochemical products, fuel products, petroleum products, precursors of petroleum products, and all other substances that may be useful in the petrochemical industry. The product may be used for generating substances, or materials, useful in the petrochemical industry. The products may be used in a combustor such as a boiler, kiln, dryer or furnace. Other examples of combustors are internal combustion engines such as vehicle engines or generators, including gasoline engines, diesel engines, jet engines, and other types of engines. In one example, a method herein comprises combusting a refined or "upgraded" composition. For example, combusting a refined composition can comprise inserting the refined composition into a combustion engine, such as an automobile engine or a jet engine. Products described herein may also be used to produce plastics, resins, fibers, elastomers, pharmaceuticals, neutraceuticals, lubricants, and gels, for example.

Useful products can also include isoprenoid precursors. Isoprenoid precursors are generated by one of two pathways; the mevalonate pathway or the methylerythritol phosphate (MEP) pathway. Both pathways generate dimethylallyl pyrophosphate (DMAPP) and isopentyl pyrophosphate (IPP), the common C5 precursor for isoprenoids. The DMAPP and IPP are condensed to form geranyl-diphosphate (GPP), or other precursors, such as farnesyl-diphosphate (FPP) or geranylgeranyl-diphosphate (GGPP), from which higher isoprenoids are formed.

Useful products can also include small alkanes (for example, 1 to approximately 4 carbons) such as methane, ethane, propane, or butane, which may be used for heating (such as in cooking) or making plastics. Products may also include molecules with a carbon backbone of approximately 5 to approximately 9 carbon atoms, such as naptha or ligroin, or their precursors. Other products may include molecules with a carbon background of about 5 to about 12 carbon atoms, or cycloalkanes used as gasoline or motor fuel. Molecules and aromatics of approximately 10 to approximately 18 carbons, such as kerosene, or its precursors, may also be useful as products. Other products include lubricating oil, heavy gas oil, or fuel oil, or their precursors, and can contain alkanes, cycloalkanes, or aromatics of approximately 12 to approximately 70 carbons. Products also include other residuals that can be derived from or found in crude oil, such as coke, asphalt, tar, and waxes, generally containing multiple rings with about 70 or more carbons, and their precursors.

Examples of products, which can include the isoprenoids of the present disclosure, are fuel products, fragrance products, and insecticide products. In some instances, a product may be used directly. In other instances, the product may be used as a "feedstock" to produce another product. For example, where the product is an isoprenoid, the isoprenoid may be hydrogenated and "cracked" to produce a shorter chain hydrocarbon (e.g., farnesene is hydrogenated to produce farnesene which is then cracked to produce propane, butane, octane, or other fuel products).

Modified organisms can be grown, in some examples in the presence of CO₂, to produce a desired polypeptide. In some examples, the products produced by the modified organism are isolated or collected. Collected products, such as terpenes and terpenoids, may then be further modified, for example, by refining and/or cracking to produce fuel molecules or components.

The various products may be further refined to a final product for an end user by a number of processes. Refining can, for example, occur by fractional distillation. For example, a mixture of products, such as a mix of different hydrocarbons with various chain lengths may be separated into various components by fractional distillation.

Refining may also include any one or more of the following steps, cracking, unifying, or altering the product. Large products, such as large hydrocarbons (e.g. ≥ C10), may be broken down into smaller fragments by cracking. Cracking may be performed by heat or high pressure, such as by steam, visbreaking, or coking. Products may also be refined by visbreaking, for example by thermally cracking large hydrocarbon molecules in the product by heating the product in a furnace. Refining may also include coking, wherein a heavy, almost pure carbon residue is produced. Cracking may also be performed by catalytic means to enhance the rate of the cracking reaction by using catalysts such as, but not limited to, zeolite, aluminum hydrosilicate, bauxite, or silica-alumina. Catalysis may be by fluid catalytic cracking, whereby a hot catalyst, such as zeolite, is used to catalyze cracking reactions. Catalysis may also be performed by hydrocracking, where lower temperatures are generally used in comparison to fluid catalytic cracking. Hydrocracking can occur in the presence of elevated partial pressure of hydrogen gas. Products may be refined by catalytic cracking to generate diesel, gasoline, and/or kerosene.

The products may also be refined by combining them in a unification step, for example by using catalysts, such as platinum or a platinum-rhenium mix. The unification process can produce hydrogen gas, a byproduct, which may be used in cracking.

The products may also be refined by altering, rearranging, or restructuring hydrocarbons into smaller molecules. There are a number of chemical reactions that occur in catalytic reforming processes which are known to one of ordinary skill in the arts. Catalytic reforming can be performed in the presence of a catalyst and a high partial pressure of hydrogen. One common process is alkylation. For example, propylene and butylene are mixed with a catalyst such as hydrofluoric acid or sulfuric acid, and the resulting products are high octane hydrocarbons, which can be used to reduce knocking in gasoline blends.

The products may also be blended or combined into mixtures to obtain an end product. For example, the products may be blended to form gasoline of various grades, gasoline with or without additives, lubricating oils of various weights and grades, kerosene of various grades, jet fuel, diesel fuel, heating oil, and chemicals for making plastics and other polymers. Compositions of the products described herein may be combined or blended with fuel products produced by other means.

Some products produced from the host cells of the disclosure, especially after refining, will be identical to existing petrochemicals, i.e. contain the same chemical structure. For instance, crude oil contains the isoprenoid pristane, which is thought to be a breakdown product of phytol, which is a component of chlorophyll. Some of the products may not be the same as existing petrochemicals. However, although a molecule may not exist in conventional petrochemicals or refining, it may still be useful in these industries. For example, a hydrocarbon could be produced that is in the boiling point range of gasoline, and that could be used as gasoline or an additive, even though the hydrocarbon does not normally occur in gasoline.

A product herein can be described by its Carbon Isotope Distribution (CID). At the molecular level, a CID is the statistical likelihood of a single carbon atom within a molecule to be one of the naturally occurring carbon isotopes (for example, ¹²C, ¹³C, or ¹⁴C). At the bulk level of a product, a CID may be the relative abundance of naturally occurring carbon isotopes (for example, ¹²C, ¹³C, or ¹⁴C) in a compound containing at least one carbon atom. It is noted that the CID of a fossil fuel may differ based on its source. For example, with CID(fos), the CID of carbon in a fossil fuel, such as petroleum, natural gas, and coal is distinguishable from the CID(atm), the CID of carbon in current atmospheric carbon dioxide. Additionally, the CID(photo-atm) refers to the CID of a carbon-based compound made by photosynthesis in recent history where the source of inorganic carbon was carbon dioxide in the atmosphere. Also, CID(photo-fos) refers to the CID of a carbon based compound made by photosynthesis in recent history where the source of substantially all of the inorganic carbon was carbon dioxide produced by the burning of fossil fuels (for example, coal, natural gas, and/or petroleum). The exact distribution is also a characteristic of 1) the type of photosynthetic organism that produced the molecule, and 2) the source of inorganic carbon. These isotope distributions can be used to define the composition of photosynthetically-derived fuel products. Carbon isotopes are unevenly distributed among and within different compounds and the isotopic distribution can reveal information about the physical, chemical, and metabolic processes involved in carbon transformation. The overall abundance of ¹³C relative to ¹²C in a photosynthetic organism is often less than the overall abundance of ¹³C relative to ¹²C in atmospheric carbon dioxide, indicating that carbon isotope discrimation occurs in the incorporation of carbon dioxide into photosynthetic biomass.

A product, either before or after refining, can be identical to an existing petrochemical. Some of the fuel products may not be the same as existing petrochemicals. In one example, a fuel product is similar to an existing petrochemical, except for the carbon isotope distribution. For example, it is believed that no fossil fuel petrochemicals have a δ¹³C distribution of less than -32%, whereas fuel products as described herein can have a δ¹³C distribution of less than -32%, less than -35%, less than -40%, less than -45%, less than -50%, less than -55%, or less than -60%. In another example, a fuel product or composition is similar but not the same as an existing fossil fuel petrochemical and has a δ¹³C distribution of less than -32%, less than -35%, less than -40%, less than -45%, less than -50%, less than -55%, or less than -60%.

A fuel product can be a composition comprising, for example, hydrogen and carbon molecules, wherein the hydrogen and carbon molecules are at least about 80% of the atomic weight of the composition, and wherein the δ¹³C distribution of the composition is less than about -32%. For some fuel products described herein, the hydrogen and carbon molecules are at least 90% of the atomic weight of the composition. For example, a biodiesel or fatty acid methyl ester (which has less than 90% hydrogen and carbon molecules by weight) may not be part of the composition. In still other compositions, the hydrogen and carbon molecules are at least 95 or at least 99% of the atomic weight of the composition. In yet other compositions, the hydrogen and carbon molecules are 100% of the atomic weight of the composition. In some examples, the composition is a liquid. In other examples, the composition is a fuel additive or a fuel product.

Also described herein is a fuel product comprising a composition comprising: hydrogen and carbon molecules, wherein the hydrogen and carbon molecules are at least 80% of the atomic weight of the composition, and wherein the δ¹³C distribution of the composition is less than -32%; and a fuel component. In some examples, the δ¹³C distribution of the composition is less than about -35%, less than about -40%, less than about -45%, less than about -50%, less than about -55%, or less than about -60%. In some examples, the fuel component of the composition is a blending fuel, for example, a fossil fuel, gasoline, diesel, ethanol, jet fuel, or any combination thereof. In still other examples, the blending fuel has a δ¹³C distribution of greater than -32%. For some fuel products described herein, the fuel component is a fuel additive which may be MTBE, an anti-oxidant, an antistatic agent, a corrosion inhibitor, or any combination thereof. A fuel product as described herein may be a product generated by blending a fuel product as described and a fuel component. In some examples, the fuel product has a δ¹³C distribution of greater than -32%. In other examples, the fuel product has a δ¹³C distribution of less than -32%. For example, an oil composition extracted from an organism can be blended with a fuel component prior to refining (for example, cracking) in order to generate a fuel product as described herein. A fuel component, can be a fossil fuel, or a mixing blend for generating a fuel product. For example, a mixture for fuel blending may be a hydrocarbon mixture that is suitable for blending with another hydrocarbon mixture to generate a fuel product. For example, a mixture of light alkanes may not have a certain octane number to be suitable for a type of fuel, however, it can be blended with a high octane mixture to generate a fuel product. In another example, a composition with a δ¹³C distribution of less than -32% is blended with a hydrocarbon mixture for fuel blending to create a fuel product. In some examples, the composition or fuel component alone are not suitable as a fuel product, however, when combined, they are useful as a fuel product. In other examples, either the composition or the fuel component or both individually are suitable as a fuel product. In yet another examples, the fuel component is an existing petroleum product, such as gasoline or jet fuel. In other examples, the fuel component is derived from a renewable resource, such as bioethanol, biodiesel, and biogasoline.

Oil compositions, derived from biomass obtained from a host cell, can be used for producing high-octane hydrocarbon products. Thus, one example describes a method of forming a fuel product, comprising: obtaining an upgraded oil composition, cracking the oil composition, and blending the resulting one or more light hydrocarbons, having 4 to 12 carbons and an Octane number of 80 or higher, with a hydrocarbon having an Octane number of 80 or less. The hydrocarbons having an Octane number of 80 or less are, for example, fossil fuels derived from refining crude oil.

The biomass feedstock obtained from a host organism can be modified or tagged such that the light hydrocarbon products can be identified or traced back to their original feedstock. For example, carbon isotopes can be introduced into a biomass hydrocarbon in the course of its biosynthesis. The tagged hydrocarbon feedstock can be subjected to the refining processes described herein to produce a light hydrocarbon product tagged with a carbon isotope. The isotopes allow for the identification of the tagged products, either alone or in combination with other untagged products, such that the tagged products can be traced back to their original biomass feedstocks.

### Table 1. Examples of Enzymes Involved in the Isoprenoid Pathway

The enzymes utilized may be encoded by nucleotide sequences derived from any organism, including bacteria, plants, fungi and animals. In some instances, the enzymes are isoprenoid producing enzymes. As used herein, an "isoprenoid producing enzyme" is a naturally or non-naturally occurring enzyme which produces or increases production of an isoprenoid. In some instances, an isoprenoid producing enzyme produces isoprenoids with two phosphate groups (e.g., GPP synthase, FPP synthase, DMAPP synthase). In other instances, isoprenoid producing enzymes produce isoprenoids with zero, one, three or more phosphates or may produce isoprenoids with other functional groups. Non-limiting examples of such enzymes and their sources are shown in **Table 1.**

| **Synthase** | **Source** | **NCBI protein ID** |
|---|---|---|
| Limonene | *M. spicata* | 2ONH_A |
| Cineole | *S. officinalis* | AAC26016 |
| Pinene | *A. grandis* | AAK83564 |
| Camphene | *A. grandis* | AAB70707 |
| Sabinene | *S. officinalis* | AAC26018 |
| Myrcene | *A. grandis* | AAB71084 |
| Abietadiene | *A. grandis* | Q38710 |
| Taxadiene | *T. brevifolia* | AAK83566 |
| FPP | *G. gallus* | P08836 |
| Amorphadiene | *A. annua* | AAF61439 |
| Bisabolene | *A. grandis* | 081086 |
| Diapophytoene | *S. aureus* | |
| Diapophytoene desaturase | *S. aureus* | |
| GPPS-LSU | *M. spicata* | AAF08793 |
| GPPS-SSU | *M. spicata* | AAF08792 |
| GPPS | *A. thaliana* | CAC16849 |
| GPPS | *C. reinhardtii* | EDP05515 |
| FPP | *E. coli* | NP_414955 |
| FPP | *A. thaliana* | NP_199588 |
| FPP | *A. thaliana* | NP_193452 |
| FPP | *C. reinhardtii* | EDP03194 |
| IPP isomerase | *E. coli* | NP_417365 |
| IPP isomerase | *H. pluvialis* | ABB80114 |
| Limonene | *L. angustifolia* | ABB73044 |
| Monoterpene | *S. lycopersicum* | AAX69064 |
| Terpinolene | *O. basilicum* | AAV63792 |
| Myrcene | *O. basilicum* | AAV63791 |
| Zingiberene | *O. basilicum* | AAV63788 |
| Myrcene | *Q. ilex* | CAC41012 |
| Myrcene | *P. abies* | AAS47696 |
| Myrcene, ocimene | *A. thaliana* | NP_179998 |
| Myrcene, ocimene | *A. thaliana* | NP_567511 |
| Sesquiterpene | Z. *mays*; B73 | AAS88571 |
| Sesquiterpene | *A. thaliana* | NP_199276 |
| Sesquiterpene | *A. thaliana* | NP_193064 |
| Sesquiterpene | *A. thaliana* | NP_193066 |
| Curcumene | *P. cablin* | AAS86319 |
| Farnesene | *M. domestica* | AAX19772 |
| Farnesene | *C. sativus* | AAU05951 |
| Farnesene | *C. junos* | AAK54279 |
| Farnesene | *P. abies* | AAS47697 |
| Bisabolene | *P. abies* | AAS47689 |
| Sesquiterpene | *A. thaliana* | NP_197784 |
| Sesquiterpene | *A. thaliana* | NP_175313 |
| GPP Chimera | | |
| GPPS-LSU+SSU fusion | | |
| Geranylgeranyl reductase | *A. thaliana* | NP_177587 |
| Geranylgeranyl reductase | *C. reinhardtii* | EDP09986 |
| Chlorophyllidohydrolase | *C. reinhardtii* | EDP01364 |
| Chlorophyllidohydrolase | *A. thaliana* | NP_564094 |
| Chlorophyllidohydrolase | *A. thaliana* | NP_199199 |
| Phosphatase | *S. cerevisiae* | AAB64930 |
| FPP A118W | *G. gallus* | |

### Codon Optimization

As discussed above, one or more codons of an encoding polynucleotide can be "biased" or "optimized" to reflect the codon usage of the host organism. For example, one or more codons of an encoding polynucleotide can be "biased" or "optimized" to reflect chloroplast codon usage (**Table 2**) or nuclear codon usage (**Table 3**). Most amino acids are encoded by two or more different (degenerate) codons, and it is well recognized that various organisms utilize certain codons in preference to others. "Biased" or codon "optimized" can be used interchangeably throughout the specification. Codon bias can be variously skewed in different plants, including, for example, in alga as compared to tobacco. Generally, the codon bias selected reflects codon usage of the plant (or organelle therein) which is being transformed with the nucleic acids of the present disclosure.

A polynucleotide that is biased for a particular codon usage can be synthesized de novo, or can be genetically modified using routine recombinant DNA techniques, for example, by a site directed mutagenesis method, to change one or more codons such that they are biased for chloroplast codon usage.

Such preferential codon usage, which is utilized in chloroplasts, is referred to herein as "chloroplast codon usage." **Table 2** (below) shows the chloroplast codon usage for *C*. *reinhardtii* (see U.S. Patent Application Publication No.: 2004/0014174, published January 22, 2004).

**Table 2**

| **Chloroplast Codon Usage in Chlamydomonas reinhardtii** | | | |
|---|---|---|---|
| UUU 34.1*(348**) | UCU 19.4(198) | UAU 23.7(242) | UGU 8.5(87) |
| UUC 14.2(145) | UCC 4.9(50) | UAC 10.4(106) | UGC 2.6(27) |
| UUA 72.8(742) | UCA 20.4(208) | UAA 2.7(28) | UGA 0.1(1) |
| UUG 5.6(57) | UCG 5.2(53) | UAG 0.7(7) | UGG 13.7(140) |
| CUU 14.8(151) | CCU 14.9(152) | CAU 11.1(113) | CGU 25.5(260) |
| CUC 1.0(10) | CCC 5.4(55) | CAC 8.4(86) | CGC 5.1(52) |
| CUA 6.8(69) | CCA 19.3(197) | CAA 34.8(355) | CGA 3.8(39) |
| CUG 7.2(73) | CCG 3.0(31) | CAG 5.4(55) | CGG 0.5(5) |
| AUU 44.6(455) | ACU 23.3(237) | AAU 44.0(449) | AGU 16.9(172) |
| AUC 9.7(99) | ACC 7.8(80) | AAC 19.7(201) | AGC 6.7(68) |
| AUA 8.2(84) | ACA 29.3(299) | AAA 61.5(627) | AGA 5.0(51) |
| AUG 23.3(238) | ACG 4.2(43) | AAG 11.0(112) | AGG 1.5(15) |
| GUU 27.5(280) | GCU 30.6(312) | GAU 23.8(243) | GGU 40.0(408) |
| GUC 4.6(47) | GCC 11.1(113) | GAC 11.6(118) | GGC 8.7(89) |
| GUA 26.4(269) | GCA 19.9(203) | GAA 40.3(411) | GGA 9.6(98) |
| GUG 7.1(72) | GCG 4.3(44) | GAG 6.9(70) | GGG 4.3(44) |

| | | | |
|---|---|---|---|
| * -Frequency of codon usage per 1,000 codons. ** - Number of times observed in 36 chloroplast coding sequences (10,193 codons). | | | |

The chloroplast codon bias can, but need not, be selected based on a particular organism in which a synthetic polynucleotide is to be expressed. The manipulation can be a change to a codon, for example, by a method such as site directed mutagenesis, by a method such as PCR using a primer that is mismatched for the nucleotide(s) to be changed such that the amplification product is biased to reflect chloroplast codon usage, or can be the de novo synthesis of polynucleotide sequence such that the change (bias) is introduced as a consequence of the synthesis procedure.

In addition to utilizing chloroplast codon bias as a means to provide efficient translation of a polypeptide, it will be recognized that an alternative means for obtaining efficient translation of a polypeptide in a chloroplast is to re-engineer the chloroplast genome (e.g., a C. *reinhardtii* chloroplast genome) for the expression of tRNAs not otherwise expressed in the chloroplast genome. Such an engineered algae expressing one or more exogenous tRNA molecules provides the advantage that it would obviate a requirement to modify every polynucleotide of interest that is to be introduced into and expressed from a chloroplast genome; instead, algae such as C. *reinhardtii* that comprise a genetically modified chloroplast genome can be provided and utilized for efficient translation of a polypeptide according to any method of the disclosure. Correlations between tRNA abundance and codon usage in highly expressed genes is well known (for example, as described in Franklin et al., Plant J. 30:733-744, 2002; Dong et al., J. Mol. Biol. 260:649-663, 1996; Duret, Trends Genet. 16:287-289, 2000; Goldman et. al., J. Mol. Biol. 245:467-473, 1995; and Komar et. al., Biol. Chem. 379:1295-1300, 1998). In E. *coli,* for example, re-engineering of strains to express underutilized tRNAs resulted in enhanced expression of genes which utilize these codons (see Novy et al., in Novations 12:1-3, 2001). Utilizing endogenous tRNA genes, site directed mutagenesis can be used to make a synthetic tRNA gene, which can be introduced into chloroplasts to complement rare or unused tRNA genes in a chloroplast genome, such as a C. *reinhardtii* chloroplast genome.

Generally, the chloroplast codon bias selected for purposes of the present disclosure, including, for example, in preparing a synthetic polynucleotide as disclosed herein reflects chloroplast codon usage of a plant chloroplast, and includes a codon bias that, with respect to the third position of a codon, is skewed towards A/T, for example, where the third position has greater than about 66% AT bias, or greater than about 70% AT bias. In one example, the chloroplast codon usage is biased to reflect alga chloroplast codon usage, for example, *C. reinhardtii,* which has about 74.6% AT bias in the third codon position. Preferred codon usage in the chloroplasts of algae has been described in US 2004/0014174.

**Table** 3 exemplifies codons that are preferentially used in algal nuclear genes. The nuclear codon bias can, but need not, be selected based on a particular organism in which a synthetic polynucleotide is to be expressed. The manipulation can be a change to a codon, for example, by a method such as site directed mutagenesis, by a method such as PCR using a primer that is mismatched for the nucleotide(s) to be changed such that the amplification product is biased to reflect nuclear codon usage, or can be the de novo synthesis of polynucleotide sequence such that the change (bias) is introduced as a consequence of the synthesis procedure.

In addition to utilizing nuclear codon bias as a means to provide efficient translation of a polypeptide, it will be recognized that an alternative means for obtaining efficient translation of a polypeptide in a nucleus is to re-engineer the nuclear genome (e.g., a C. *reinhardtii* nuclear genome) for the expression of tRNAs not otherwise expressed in the nuclear genome. Such an engineered algae expressing one or more exogenous tRNA molecules provides the advantage that it would obviate a requirement to modify every polynucleotide of interest that is to be introduced into and expressed from a nuclear genome; instead, algae such as C. *reinhardtii* that comprise a genetically modified nuclear genome can be provided and utilized for efficient translation of a polypeptide according to any method of the disclosure. Correlations between tRNA abundance and codon usage in highly expressed genes is well known (for example, as described in Franklin et al., Plant J. 30:733-744, 2002; Dong et al., J. Mol. Biol. 260:649-663, 1996; Duret, Trends Genet. 16:287-289, 2000; Goldman et. Al., J. Mol. Biol. 245:467-473, 1995; and Komar et. Al., Biol. Chem. 379:1295-1300, 1998). In *E*. *coli,* for example, re-engineering of strains to express underutilized tRNAs resulted in enhanced expression of genes which utilize these codons (see Novy et al., in Novations 12:1-3, 2001). Utilizing endogenous tRNA genes, site directed mutagenesis can be used to make a synthetic tRNA gene, which can be introduced into the nucleus to complement rare or unused tRNA genes in a nuclear genome, such as a C. *reinhardtii* nuclear genome.

Generally, the nuclear codon bias selected for purposes of the present disclosure, including, for example, in preparing a synthetic polynucleotide as disclosed herein, can reflect nuclear codon usage of an algal nucleus and includes a codon bias that results in the coding sequence containing greater than 60% G/C content.

**Table 3**

| fields: [triplet] [frequency: **per thousand]** ([number]) Coding GC 66.30% 1^{st} letter GC 64.80% 2^{nd} letter GC 47.90% 3^{rd} letter GC 86.21 % | | | |
|---|---|---|---|
| **Nuclear Codon Usage in Chlamydomonas reinhardtii** | | | |
| UUU 5.0 (2110) | UCU 4.7 (1992) | UAU 2.6 (1085) | UGU 1.4 (601) |
| UUC 27.1 (11411) | UCC 16.1 (6782) | UAC 22.8 (9579) | UGC 13.1 (5498) |
| UUA 0.6 (247) | UCA 3.2 (1348) | UAA 1.0 (441) | UGA 0.5 (227) |
| UUG 4.0 (1673) | UCG 16.1 (6763) | UAG 0.4 (183) | UGG 13.2 (5559) |
| CUU 4.4 (1869) | CCU 8.1 (3416) | CAU 2.2 (919) | CGU 4.9 (2071) |
| CUC 13.0 (5480) | CCC 29.5 (12409) | CAC 17.2 (7252) | CGC 34.9 (14676) |
| CUA 2.6 (1086) | CCA 5.1 (2124) | CAA 4.2 (1780) | CGA 2.0 (841) |
| CUG 65.2 (27420) | CCG 20.7 (8684) | CAG 36.3 (15283) | CGG 11.2 (4711) |
| AUU 8.0 (3360) | ACU 5.2 (2171) | AAU 2.8 (1157) | AGU 2.6 (1089) |
| AUC 26.6 (11200) | ACC 27.7 (11663) | AAC 28.5 (11977) | AGC 22.8 (9590) |
| AUA 1.1 (443) | ACA 4.1 (1713) | AAA 2.4 (1028) | AGA 0.7 (287) |
| 0AUG 25.7 (10796) | ACG 15.9 (6684) | AAG 43.3 (18212) | AGG 2.7 (1150) |
| GUU 5.1 (2158) | GCU 16.7 (7030) | GAU 6.7 (2805) | GGU 9.5 (3984) |
| GUC 15.4 (6496) | GCC 54.6 (22960) | GAC 41.7 (17519) | GGC 62.0 (26064) |
| GUA 2.0 (857) | GCA 10.6 (4467) | GAA 2.8 (1172) | GGA 5.0 (2084) |
| GUG 46.5 (19558) | GCG 44.4 (18688) | GAG 53.5 (22486) | GGG 9.7 (4087) |

### Table 4

**Table 4** lists the codon selected at each position for backtranslating the protein to a DNA sequence for synthesis. The selected codon is the sequence recognized by the tRNA encoded in the chloroplast genome when present; the stop codon (TAA) is the codon most frequently present in the chloroplast encoded genes. If an undesired restriction site is created, the next best choice according to the regular Chlamydomonas chloroplast usage table that eliminates the restriction site is selected.

**Table 4**

| Amino acid | Codon utilized |
|---|---|
| F | TTC |
| L | TTA |
| I | ATC |
| V | GTA |
| S | TCA |
| P | CCA |
| T | ACA |
| A | GCA |
| Y | TAC |
| H | CAC |
| Q | CAA |
| N | AAC |
| K | AAA |
| D | GAC |
| E | GAA |
| C | TGC |
| R | CGT |
| G | GGC |
| W | TGG |
| M | ATG |
| STOP | TAA |

### Percent Sequence Identity

One example of an algorithm that is suitable for determining percent sequence identity or sequence similarity between nucleic acid or polypeptide sequences is the BLAST algorithm, which is described, e.g., in Altschul et al., J. Mol. Biol. 215:403-410 (1990). Software for performing BLAST analysis is publicly available through the National Center for Biotechnology Information. The BLAST algorithm parameters W, T, and X determine the sensitivity and speed of the alignment. The BLASTN program (for nucleotide sequences) uses as defaults a word length (W) of 11, an expectation (E) of 10, a cutoff of 100, M=5, N=-4, and a comparison of both strands. For amino acid sequences, the BLASTP program uses as defaults a word length (W) of 3, an expectation (E) of 10, and the BLOSUM62 scoring matrix (as described, for example, in Henikoff & Henikoff (1989) Proc. Natl. Acad. Sci. USA, 89:10915). In addition to calculating percent sequence identity, the BLAST algorithm also can perform a statistical analysis of the similarity between two sequences (for example, as described in Karlin & Altschul, Proc. Nat'l. Acad. Sci. USA, 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, less than about 0.01, or less than about 0.001.

### Fatty Acids and Glycerol Lipids

The present disclosure describes host cells capable of making polypeptides that contribute to the accumulation and/or secretion of fatty acids, glycerol lipids, or oils, by transforming host cells (e.g., alga cells such as *C. reinhardtii, D. salvia, H. pluvalis,* and cyanobacterial cells) with nucleic acids encoding one or more different enzymes. Examples of such enzymes include acetyl-CoA carboxylase, ketoreductase, thioesterase, malonyltransferase, dehydratase, acyl-CoA ligase, ketoacylsynthase, enoylreductase, and desaturase. The enzymes can be, for example, catabolic or biodegrading enzymes.

In some instances, the host cell will naturally produce the fatty acid, glycerol lipid, triglyceride, or oil of interest. Therefore, transformation of the host cell with a polynucleotide encoding an enzyme, for example an ACCase, will allow for the increased activity of the enzyme and/or increased accumulation and/or secretion of a molecule of interest (e.g., a lipid) in the cell.

A change in the accumulation and/or secretion of a desired product, for example, fatty acids, glycerol lipids, or oils, by a transformed host cell can include, for example, a change in the total oil content over that normally present in the cell, or a change in the type of oil that is normally present in the cell.

A change in the accumulation and/or secretion of a desired product, for example, fatty acids, glycerol lipids, or oils, by a transformed host cell can include, for example, a change in the total lipid content over that normally present in the cell, or a change in the type of lipids that are normally present in the cell.

Increased malonyl CoA production is required for increased. Increased fatty acid biosynthesis is required for increased accumulation of fatty acid based lipids. An increase in fatty acid based lipids can be measured by methyl tert-butyl ether (MTBE) extraction.

Some host cells may be transformed with multiple genes encoding one or more enzymes. For example, a single transformed cell may contain exogenous nucleic acids encoding enzymes that make up an entire glycerolipid synthesis pathway. One example of a pathway might include genes encoding an acetyl CoA carboxylase, a malonyltransferase, a ketoacylsynthase, and a thioesterase. Cells transformed with an entire pathway and/or enzymes extracted from those cells, can synthesize, for example, complete fatty acids or intermediates of the fatty acid synthesis pathway. Constructs may contain, for example, multiple copies of the same gene, multiple genes encoding the same enzyme from different organisms, and/or multiple genes with one or more mutations in the coding sequence(s).

The enzyme(s) produced by the modified cells may result in the production of fatty acids, glycerol lipids, triglycerides, or oils that may be collected from the cells and/or the surrounding environment (e.g., bioreactor or growth medium). In some examples, the collection of the fatty acids, glycerol lipids, triglycerides, or oils is performed after the product is secreted from the cell via a cell membrane transporter.

Examples of candidate *Chlamydomonas* genes encoding enzymes of glycerolipid metabolism that can be used in the described examples are described in The Chlamydomonas Sourcebook Second Edition, Organellar and Metabolic Processes, Vol. 2, pp. 41-68, David B. Stern (Ed.), (2009), Elsevier Academic Press.

For example, enzymes involved in plastid, mitochondrial, and cytosolic pathways, along with plastidic and cytosolic isoforms of fatty acid desaturases, and triglyceride synthesis enzymes are described (and their accession numbers provided). An exemplary chart of some of the genes described is provided below:

| | | |
|---|---|---|
| Acyl-ACP thioesterase | FAT1 | EDP08596 |
| Long-chain acyl-CoA synthetase | LCS1 | EDO96800 |
| CDP-DAG: Inositol phosphotransferase | PIS1 | EDP06395 |
| Acyl-CoA: Diacylglycerol acyltransferase | DGA1 | EDO96893 |
| Phospholipid: Diacylglycerol acyltransferase | LRO1(LCA1) | EDP07444 |

Examples of the types of fatty acids and/or glycerol lipids that a host cell or organism can produce, are described below.

Lipids are a broad group of naturally occurring molecules which includes fats, waxes, sterols, fatsoluble vitamins (such as vitamins A, D, E and K), monoglycerides, diglycerides, phospholipids, and others. The main biological functions of lipids include energy storage, as structural components of cell membranes, and as important signaling molecules.

Lipids may be broadly defined as hydrophobic or amphiphilic small molecules; the amphiphilic nature of some lipids allows them to form structures such as vesicles, liposomes, or membranes in an aqueous environment. Biological lipids originate entirely or in part from two distinct types of biochemical subunits or "building blocks": ketoacyl and isoprene groups. Lipids may be divided into eight categories: fatty acyls, glycerolipids, glycerophospholipids, sphingolipids, saccharolipids and polyketides (derived from condensation of ketoacyl subunits); and sterol lipids and prenol lipids (derived from condensation of isoprene subunits). For this disclosure, saccharolipids will not be discussed.

Fats are a subgroup of lipids called triglycerides. Lipids also encompass molecules such as fatty acids and their derivatives (including tri-, di-, and monoglycerides and phospholipids), as well as other sterol-containing metabolites such as cholesterol. Humans and other mammals use various biosynthetic pathways to both break down and synthesize lipids.

### Fatty Acyls

Fatty acyls, a generic term for describing fatty acids, their conjugates and derivatives, are a diverse group of molecules synthesized by chain-elongation of an acetyl-CoA primer with malonyl-CoA or methylmalonyl-CoA groups in a process called fatty acid synthesis. A fatty acid is any of the aliphatic monocarboxylic acids that can be liberated by hydrolysis from naturally occurring fats and oils. They are made of a hydrocarbon chain that terminates with a carboxylic acid group; this arrangement confers the molecule with a polar, hydrophilic end, and a nonpolar, hydrophobic end that is insoluble in water. The fatty acid structure is one of the most fundamental categories of biological lipids, and is commonly used as a building block of more structurally complex lipids. The carbon chain, typically between four to 24 carbons long, may be saturated or unsaturated, and may be attached to functional groups containing oxygen, halogens, nitrogen and sulfur; branched fatty acids and hydroxyl fatty acids also occur, and very long chain acids of over 30 carbons are found in waxes. Where a double bond exists, there is the possibility of either a cis or trans geometric isomerism, which significantly affects the molecule's molecular configuration. Cis-double bonds cause the fatty acid chain to bend, an effect that is more pronounced the more double bonds there are in a chain. This in turn plays an important role in the structure and function of cell membranes. Most naturally occurring fatty acids are of the cis configuration, although the trans form does exist in some natural and partially hydrogenated fats and oils.

Examples of biologically important fatty acids are the eicosanoids, derived primarily from arachidonic acid and eicosapentaenoic acid, which include prostaglandins, leukotrienes, and thromboxanes. Other major lipid classes in the fatty acid category are the fatty esters and fatty amides. Fatty esters include important biochemical intermediates such as wax esters, fatty acid thioester coenzyme A derivatives, fatty acid thioester ACP derivatives and fatty acid carnitines. The fatty amides include N-acyl ethanolamines.

### Glycerolipids

Glycerolipids are composed mainly of mono-, di- and tri-substituted glycerols, the most well-known being the fatty acid esters of glycerol (triacylglycerols), also known as triglycerides. In these compounds, the three hydroxyl groups of glycerol are each esterified, usually by different fatty acids. Because they function as a food store, these lipids comprise the bulk of storage fat in animal tissues. The hydrolysis of the ester bonds of triacylglycerols and the release of glycerol and fatty acids from adipose tissue is called fat mobilization.

Additional subclasses of glycerolipids are represented by glycosylglycerols, which are characterized by the presence of one or more sugar residues attached to glycerol via a glycosidic linkage. An example of a structure in this category is the digalactosyldiacylglycerols found in plant membranes.

Exemplary *Chlamydomonas* glycerolipids include: DGDG, digalactosyldiacylglycerol; DGTS, diacylglyceryl-N, N, N-trimethylhomoserine; MGDG, monogalactosyldiacylglycerol; PtdEtn, phosphatidylethanolamine; PtdGro, phosphatidylglycerol; PtdIns, phosphatidylinositol; SQDG, sulfoquinovosyldiacylglycerol; and TAG, triacylglycerol.

### Glycerophospholipids

Glycerophospholipids are any derivative of glycerophosphoric acid that contains at least one *O*-acyl, *O*-alkyl, or *O*-alkenyl group attached to the glycerol residue. The common glycerophospholipids are named as derivatives of phosphatidic acid (phosphatidyl choline, phosphatidyl serine, and phosphatidyl ethanolamine).

Glycerophospholipids, also referred to as phospholipids, are ubiquitous in nature and are key components of the lipid bilayer of cells, as well as being involved in metabolism and cell signaling. Glycerophospholipids may be subdivided into distinct classes, based on the nature of the polar headgroup at the *sn-3* position of the glycerol backbone in eukaryotes and eubacteria, or the *sn-1* position in the case of archaebacteria.

Examples of glycerophospholipids found in biological membranes are phosphatidylcholine (also known as PC, GPCho or lecithin), phosphatidylethanolamine (PE or GPEtn) and phosphatidylserine (PS or GPSer). In addition to serving as a primary component of cellular membranes and binding sites for intra- and intercellular proteins, some glycerophospholipids in eukaryotic cells, such as phosphatidylinositols and phosphatidic acids are either precursors of, or are themselves, membrane-derived second messengers. Typically, one or both of these hydroxyl groups are acylated with long-chain fatty acids, but there are also alkyl-linked and 1Z-alkenyl-linked (plasmalogen) glycerophospholipids, as well as dialkylether variants in archaebacteria.

### Sphingolipids

Sphingolipids are any of class of lipids containing the long-chain amino diol, sphingosine, or a closely related base (i.e. a sphingoid). A fatty acid is bound in an amide linkage to the amino group and the terminal hydroxyl may be linked to a number of residues such as a phosphate ester or a carbohydrate. The predominant base in animals is sphingosine while in plants it is phytosphingosine.

The main classes are: (1) phosphosphigolipids (also known as sphingophospholipids), of which the main representative is sphingomyelin; and (2) glycosphingolipids, which contain at least one monosaccharide and a sphingoid, and include the cerebrosides and gangliosides. Sphingolipids play an important structural role in cell membranes and may be involved in the regulation of protein kinase C.

As mentioned above, sphingolipids are a complex family of compounds that share a common structural feature, a sphingoid base backbone, and are synthesized de novo from the amino acid serine and a long-chain fatty acyl CoA, that are then converted into ceramides, phosphosphingolipids, glycosphingolipids and other compounds. The major sphingoid base of mammals is commonly referred to as sphingosine. Ceramides (N-acyl-sphingoid bases) are a major subclass of sphingoid base derivatives with an amide-linked fatty acid. The fatty acids are typically saturated or mono-unsaturated with chain lengths from 16 to 26 carbon atoms.

The major phosphosphingolipids of mammals are sphingomyelins (ceramide phosphocholines), whereas insects contain mainly ceramide phosphoethanolamines, and fungi have phytoceramide phosphoinositols and mannose-containing headgroups. The glycosphingolipids are a diverse family of molecules composed of one or more sugar residues linked via a glycosidic bond to the sphingoid base. Examples of these are the simple and complex glycosphingolipids such as cerebrosides and gangliosides.

### Sterol Lipids

Sterol lipids, such as cholesterol and its derivatives, are an important component of membrane lipids, along with the glycerophospholipids and sphingomyelins. The steroids, all derived from the same fused four-ring core structure, have different biological roles as hormones and signaling molecules. The eighteen-carbon (C18) steroids include the estrogen family whereas the C19 steroids comprise the androgens such as testosterone and androsterone. The C21 subclass includes the progestogens as well as the glucocorticoids and mineralocorticoids. The secosteroids, comprising various forms of vitamin D, are characterized by cleavage of the B ring of the core structure. Other examples of sterols are the bile acids and their conjugates, which in mammals are oxidized derivatives of cholesterol and are synthesized in the liver. The plant equivalents are the phytosterols, such as β-sitosterol, stigmasterol, and brassicasterol; the latter compound is also used as a biomarker for algal growth. The predominant sterol in fungal cell membranes is ergosterol.

### Prenol Lipids

Prenol lipids are synthesized from the 5-carbon precursors isopentenyl diphosphate and dimethylallyl diphosphate that are produced mainly via the mevalonic acid (MVA) pathway. The simple isoprenoids (for example, linear alcohols and diphosphates) are formed by the successive addition of C5 units, and are classified according to the number of these terpene units. Structures containing greater than 40 carbons are known as polyterpenes. Carotenoids are important simple isoprenoids that function as antioxidants and as precursors of vitamin A. Another biologically important class of molecules is exemplified by the quinones and hydroquinones, which contain an isoprenoid tail attached to a quinonoid core of non-isoprenoid origin. Prokaryotes synthesize polyprenols (called bactoprenols) in which the terminal isoprenoid unit attached to oxygen remains unsaturated, whereas in animal polyprenols (dolichols) the terminal isoprenoid is reduced.

### Polyketides

Polyketides or sometimes acetogenin are any of a diverse group of natural products synthesized via linear poly-β-ketones, which are themselves formed by repetitive head-to-tail addition of acetyl (or substituted acetyl) units indirectly derived from acetate (or a substituted acetate) by a mechanism similar to that for fatty-acid biosynthesis but without the intermediate reductive steps. In many case, acetyl-CoA functions as the starter unit and malonyl-CoA as the extending unit. Various molecules other than acetyl-CoA may be used as starter, often with methoylmalonyl-CoA as the extending unit. The poly-β-ketones so formed may undergo a variety of further types of reactions, which include alkylation, cyclization, glycosylation, oxidation, and reduction. The classes of product formed - and their corresponding starter substances - comprise *inter alia*: coniine (of hemlock) and orsellinate (of lichens) - acetyl-CoA; flavanoids and stilbenes - cinnamoyl-CoA; tetracyclines - amide of malonyl-CoA; urushiols (of poison ivy) - palmitoleoyl-CoA; and erythonolides - propionyl-CoA and methyl-malonyl-CoA as extender.

Polyketides comprise a large number of secondary metabolites and natural products from animal, plant, bacterial, fungal and marine sources, and have great structural diversity. Many polyketides are cyclic molecules whose backbones are often further modified by glycosylation, methylation, hydroxylation, oxidation, and/or other processes. Many commonly used anti-microbial, anti-parasitic, and anti-cancer agents are polyketides or polyketide derivatives, such as erythromycins, tetracyclines, avermectins, and antitumor epothilones.

The following examples are intended to provide illustrations of the application of the present disclosure. The following examples are not intended to completely define or otherwise limit the scope of the disclosure. One of skill in the art will appreciate that many other methods known in the art may be substituted in lieu of the ones specifically described or referenced herein.

### EXAMPLES

### Example 1: Transfonnation and Screening Methods

In this example, a method for transformation of *Scenedesmus sp.* is described. Algae cells are grown to log phase (approximately 0.5-1.0 x 10⁷ cells/mL) in TAP medium (Gorman and Levine, Proc. Natl. Acad. Sci., USA 54:1665-1669, 1965) at 23°C under constant illumination of 50-100uE on a rotary shaker set at 100 rpm. Cells are harvested at 1000 x g for 5 min. The supernatant is decanted and cells are resuspended in TAP media at 10⁸ cells/mL. 5 x 10⁷ cells are spread on selective agar medium and transformed by particle bombardment with 550 nm or 1000nm diameter gold particles carrying the transforming DNA@ 375-500psi with the Helios Gene Gun (Bio-Rad) from a shot distance of 2-4cm. Desired algae clones are those that grow on selective media.

PCR is used to identify transformed algae strains. For PCR analysis, colony lysates are prepared by suspending algae cells (from agar plate or liquid culture) in lysis buffer (0.5% SDS, 100 mM NaCl, 10 mM EDTA, 75 mM Tris-HCl, pH 7.5) and heating to 98°C for 10 minutes, followed by cooling to near 23°C. Lysates are diluted 50-fold in 100mM Tris-HCl pH 7.5 and 2uL is used as template in a 25uL reaction. Alternatively, total genomic DNA preparations may be substituted for colony lysates. A PCR cocktail consisting of reaction buffer, dNTPs, PCR primer pair(s) (indicated in each example below), DNA polymerase, and water is prepared. Algal DNA is added to provide template for the reaction. Annealing temperature gradients are employed to determine optimal annealing temperature for specific primer pairs. In many cases, algae transformants are analyzed by PCR in two ways. First, primers are used that are specific for the transgene being introduced into the chloroplast genome. Desired algae transformants are those that give rise to PCR product(s) of expected size(s). Second, two sets of primer pairs are used to determine the degree to which the transforming DNA was integrated into the chloroplast genome (heteroplasmic vs. homoplasmic). The first pair of primers amplifies a region spanning the site of integration. The second pair of primers amplifies a constant, or control region that is not targeted by the transforming DNA, so should produce a product of expected size in all cases. This reaction confirms that the absence of a PCR product from the region spanning the site of integration did not result from cellular and/or other contaminants that inhibited the PCR reaction. Concentrations of the primer pairs are varied so that both amplicons are amplified in the same reaction. The number of cycles used is >30 to increase sensitivity. The most desired clones are those that yield a product for the constant region but not for the region spanning the site of integration. Once identified, clones are analyzed for changes in phenotype.

One of skill in the art will appreciate that many other transformation methods known in the art may be substituted in lieu of the ones specifically described or referenced herein.

### Example 2: Chloroplast Transformation of S. dimorphus using 3-(3,4-Dichlorophenyl)-1,1-dimethylurea (DCMU) selection

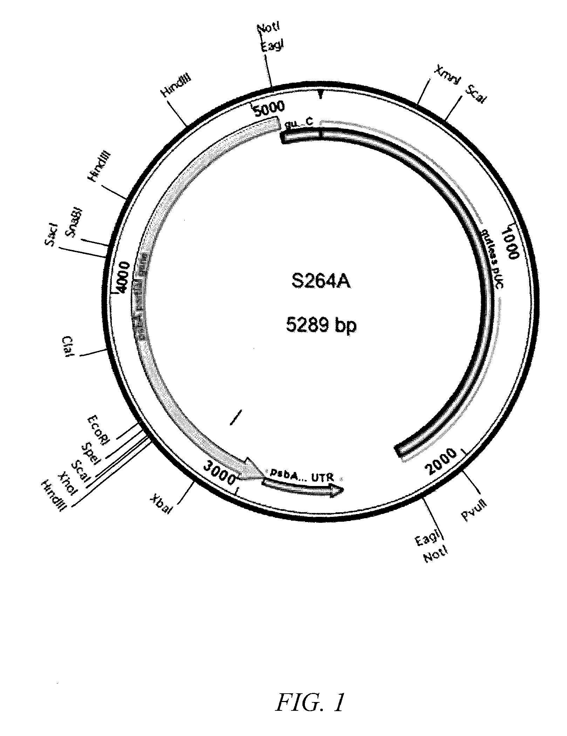

In this example, DCMU resistance was established as a selection method for transformation of *S. dimorphus.* Transforming DNA (SEQ ID NO: 30, S264A fragment) is shown graphically in **Figure 1****.** In this instance, a DNA fragment encompassing the 3' end of the gene encoding psbA and it's 3' UTR from *S. dimorphus* was amplified by PCR, subcloned into pUC18, and mutated via Quikchange PCR (Stratagene) to generate a S264A mutation along with a silent *Xba*I restriction site. Nucleotide 1913 of the fragment was mutated from a T to a G for the S264A mutation, and nucleotides 1928 to 1930 were mutated from CGT to AGA to generate the *silent Xba*I restriction site.

Transforming DNA was introduced into *S. dimorphus* via particle bombardment (as described in **EXAMPLE 1**) with DNA carried on 1000 nm gold particles, @ 375 psi and a shooting distance of 2cm. Transformants were selected by growth on HSM media + 0.5uM DCMU under constant light 100-200uE @ 23°Cfor approximately 3 weeks.

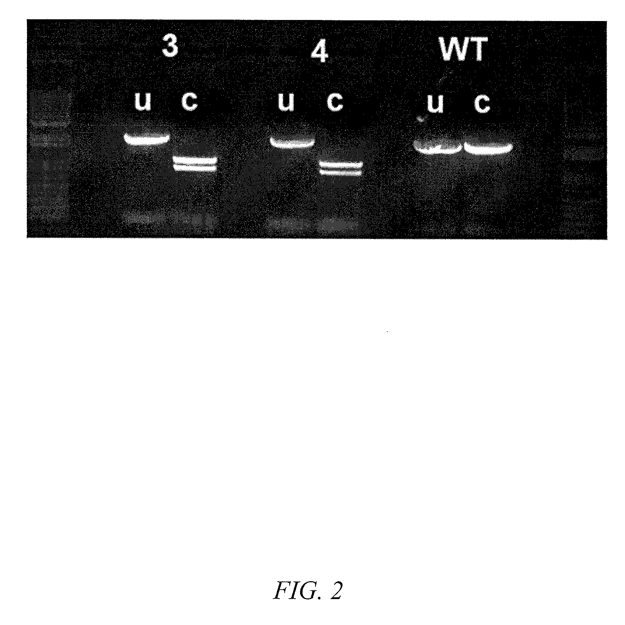

Transformants were verified by PCR screening (as described in **EXAMPLE 1**) using primers (SEQ ID NO: 17 and SEQ ID NO: 14) specific for a 2.1 kb region surrounding the bases changed for the S264A mutation. The PCR products were then digested with *Xba*I to distinguish transformants from spontaneous mutants that may arise as a result of plating cells onto media containing DCMU. **Figure 2** shows that DNA amplified from clones 3 and 4 is completely digested by *Xba*I (indicating that clones 3 and 4 are bonafide transformants while DNA amplified from wildtype cells (WT) is not. These data were confirmed by DNA sequencing of the PCR product.

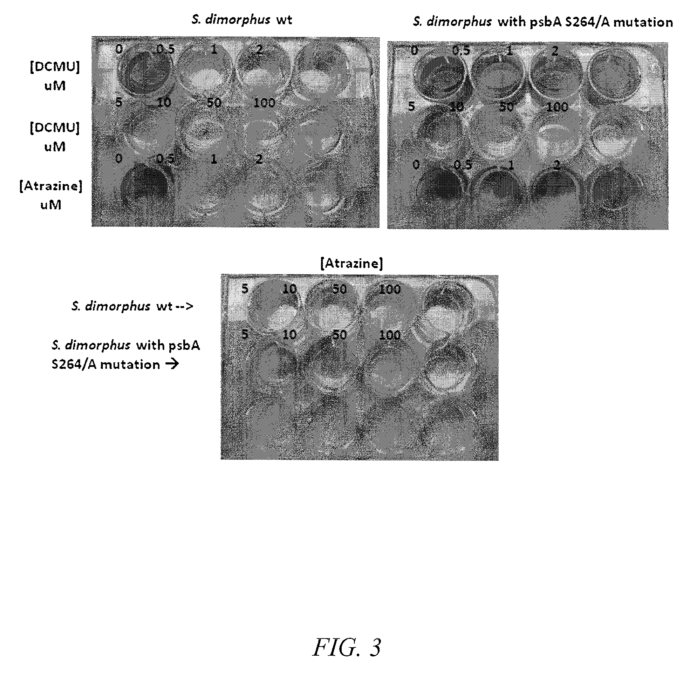

Transformants were grown to saturation in TAP media, diluted 1:100 in HSM + various concentrations of DCMU and grown under constant light 50-100uE with CO2 enrichment for 4 days. **Figure 3** shows that transformants with the psbA S264A mutation grow in up to 10uM DCMU or 10uM Atrazine whereas wild type S. dimporphus (wt) fails to grow in 0.5uM DCMU or 0.5uM Atrazine.

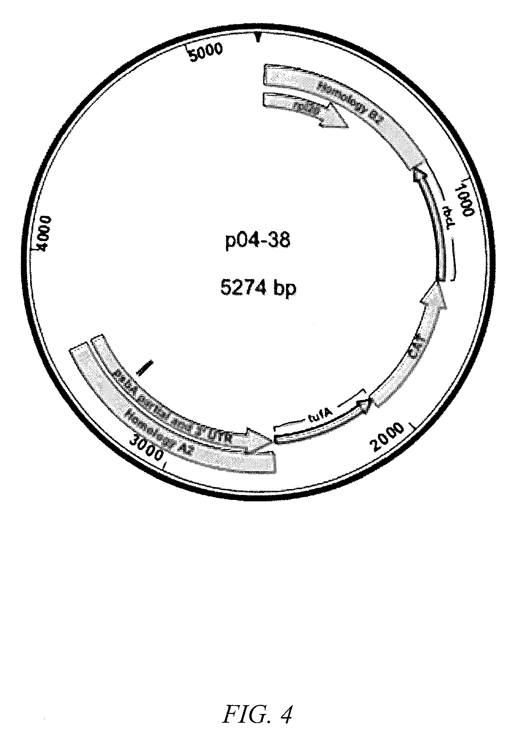

In order to determine if DCMU selection could result in incorporation of an expression cassette downstream of the psbA gene, A vector was constructed containing an expression cassette consisting of an endogenous promoter, a chloramphenicol acetyltransferase (CAT) gene, and an endogenous terminator cloned ∼500bp downstream of the S264A/XbaI mutated psbA gene fragment from *S. dimorphus* and including the rpl20 gene. Transforming DNA is shown graphically in **Figure 4****.** In this instance the DNA segment labeled "CAT" is the chloramphenicol acetyl transferase gene from *E*. *coli,* the segment labeled "tufA" is the promoter and 5' UTR sequence for the *tufA* gene from *S. dimorphus,* and the segment labeled "rbcL" is the 3' UTR for the *rbcL* gene from S. *dimorphus.* The selection marker cassette is targeted to the *S. dimorphus* chloroplast genome via the segments labeled "Homology A2" and "Homology B2" which are 1000 bp fragments homologous to sequences of DNA adjacent to nucleotide 065,353 and include an S264A/XbaI mutated partial psbA coding sequence, its 3'UTR, and the rpl20 coding sequence. This vector targets integration of the selection marker cassette approximately 400bp 3' of the stop codon of the *psbA* gene.

Transforming DNA was introduced into *S*. *dimorphus* via particle bombardment (as described in **EXAMPLE 1**) with DNA carried on 550 nm gold particles, @ 500 psi and a shooting distance of 4cm. Transformants were selected by growth on HSM media + 1uM DCMU under constant light 100-200uE @ RT for approximately 3 weeks.

To determine if the transformants were resistant to chloramphenicol (CAM), they were patched onto TAP agar medium containing 25ug/mL CAM. In all cases, the DCMU^{r} transformants were also resistant to CAM indicating that the CAT cassette was incorporated into the genome.

One of skill in the art will appreciate that many other methods known in the art may be substituted in lieu of the ones specifically described or referenced.

### Example 3: Use of Chloramphenical Acetyl Transferase as a Selection Marker in S. dimorphus

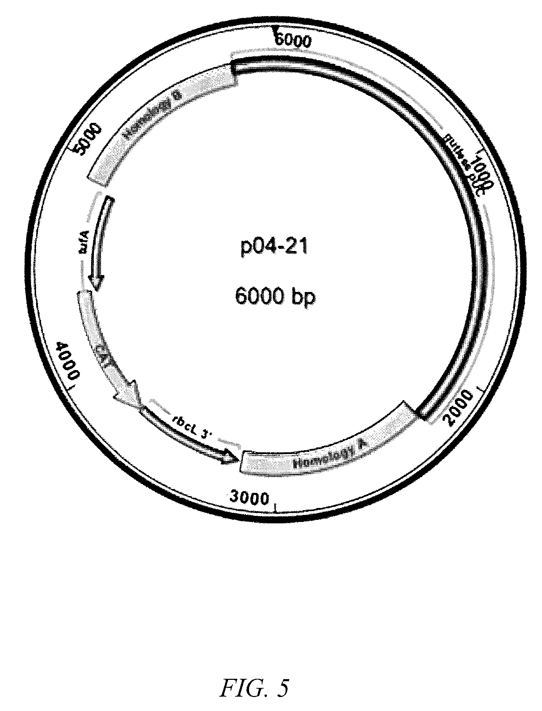

In this example, a nucleic acid encoding chloramphenicol acetyl transferase gene (CAT) from *E. coli* was introduced into *S. dimorphus.* Transforming DNA is shown graphically in **Figure 5****.** In this instance the DNA segment labeled "CAT" is the chloramphenicol acetyl transferase gene (SEQ ID NO: 28), the segment labeled "tufA" is the promoter and 5' UTR sequence for the *psbD* (SEQ ID NO: 40) or *tufA* gene (SEQ ID NO: 42)from *S*. *dimorphus,* and the segment labeled "rbcL 3' " is the 3' UTR for the *rbcL* gene from *S. dimorphus* (SEQ ID NO: 57). The selection marker cassette is targeted to the *S. dimorphus* chloroplast genome via the segments labeled "Homology A" and "Homology B" which are approximately 1000 bp fragments homologous to sequences of DNA adjacent to nucleotide 035,138 (Site 2; nucleotide locations according to the sequence available from NCBI for *S. obliquus*, NC_008101) on the 5' and 3' sides, respectively. All DNA segments were subcloned into pUC 18. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297,192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP agar medium + 25 µg/mL chloramphenicol (TAP-CAM) under constant light 50-100uE @ RT for approximately 2 weeks. Transformants were patched onto TAP-CAM agar medium, grown for 4 days under constant light.

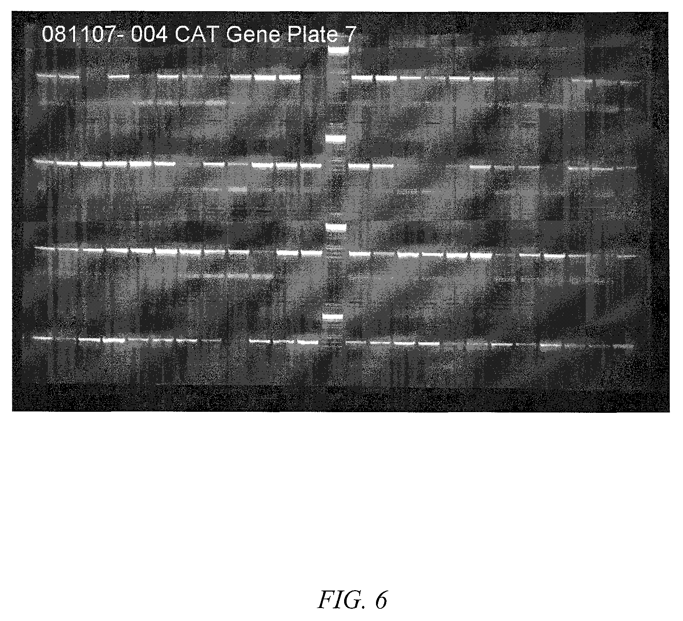

Cells from the patched transformants were analyzed by PCR screening (as described in **EXAMPLE 1**). The presence of the CAT selection marker was determined using primers that amplify the entire 660 bp gene (SEQ ID NO: 18 and SEQ ID NO: 19). **Figure 6** shows that a 660bp fragment (representing the CAT gene) is amplified from DNA of several transformants (all lanes except +, - and ladders) while it is not amplified from DNA of wild type cells (-). DNA ladder is a 1 kb ladder.

One of skill in the art will appreciate that many other methods known in the art may be substituted in lieu of the ones specifically described or referenced.

### Example 4: Production of Endoxylanase in S. dimorphus

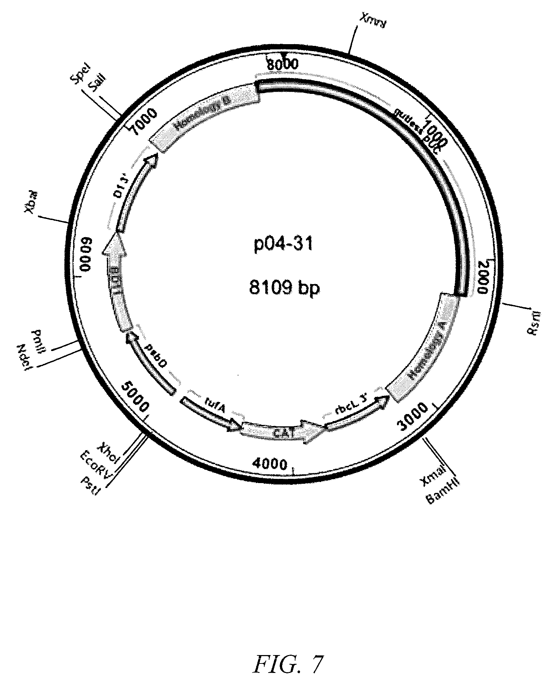

In this example a nucleic acid encoding endoxylanase from *T. reesei* was introduced into *S*. *dimorphus.* Transforming DNA (p04-31) is shown graphically **in** **Figure 7****.** In this instance the DNA segment labeled "BD11" is the endoxylanase encoding gene (SEQ ID NO: 21, BD11), the segment labeled "psbD" is the promoter and 5' UTR for the *psbD* gene from *S. dimorphus,* the segment labeled "D1 3'" is the 3' UTR for the *psbA* gene from S. *dimorphus,* and the segment labeled "CAT" is the chloramphenicol acetyl transferase gene (CAT) from *E*. *coli,* which is regulated by the promoter and 5' UTR sequence for the *tufA* gene from *S. dimorphus* and the 3' UTR sequence for the *rbcL* gene from *S. dimorphus.* The transgene expression cassette and selection marker are targeted to the *S. dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All DNA segments were subcloned into pUC 18 (gutless pUC). All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Transforming DNA was introduced into *S. dimorphus* via particle bombardment according to the method described in **EXAMPLE 1** with DNA carried on 550 nm gold particles@ 500psi and a shooting distance of 4cm. Transformants were selected by growth on TAP-CAM agar medium under constant light 50-100uE @ RT for approximately 2 weeks. Transformants were streaked onto TAP-CAM agar medium to ensure single colony isolation and grown for 4 days under constant light.

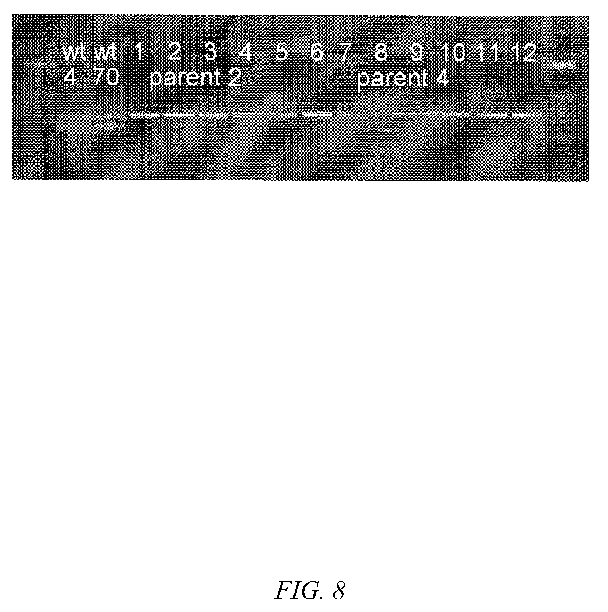

Transformants were analyzed by PCR screening (as described in **EXAMPLE 1**). The degree to which the transforming DNA was integrated into the chloroplast genome was determined using primers that amplify a 400 bp constant region (SEQ ID NO: 1 and SEQ ID NO: 2) and a 250 bp region spanning the integration site (SEQ ID NO: 3 and SEQ ID NO: 4). Integration occurs approximately 1000bp 5' of the start codon of the *psbA* gene. **Figure 8** shows that subclones from two independent transformants (parent 2 and 4) are homoplasmic, i.e., only the constant region (400bp product) was amplified, while in the control reactions (wt) both the constant region and the region spanning the integration site (250bp) were amplified.

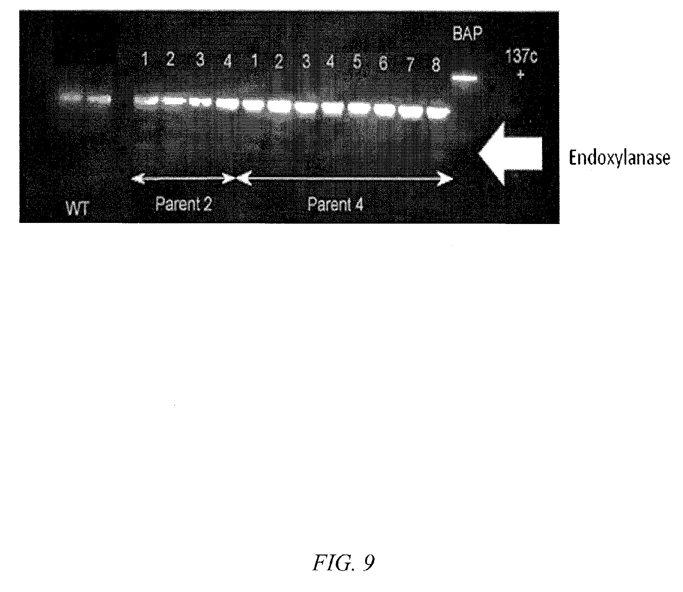

To ensure that the presence of the endoxylanase-encoding gene led to expression of the endoxylanase protein, a Western blot was performed. Briefly, approximately 1x10⁸ to 2x10⁸ algae cells were collected from TAP agar medium and resuspended in approximately 1mL BugBuster solution (Novagen) in a 1.5mL eppendorf tube. 1.0 mm Zirconia beads (BioSpec Products, Inc) were then added to fill the tube with minimal headspace, ∼500uL of beads. Cells were lysed in a bead beating apparatus (Mini Beadbeater™, BioSpec Products, Inc.) by shaking for 3-5 minutes three times. Cell lysates were clarified by centrifugation for 15 minutes at 20,000g and the supernatants were normalized for total soluble protein (Coomassie Plus Protein Assay Kit, Thermo Scientific). Samples were mixed 1:4 with loading buffer (XT Sample Buffer with β-mercaptoethanol, Bio-Rad), heated to 98°C for 5 min, cooled to 23°C, and proteins were separated by SDS-PAGE, followed by transfer to PVDF membrane. The membrane was blocked with Starting Block T20 Blocking Buffer (Thermo Scientific) for 15 min, incubated with horseradish peroxidase-linked anti-FLAG antibody (diluted 1:2,500 in Starting Block T20 Blocking Buffer) at 23°C for 2 hours, washed three times with TBST. Proteins were visualized with chemiluminescent detection. Results from multiple clones (**Figure 9****,** parent 2 and 4) show that expression of the endoxylanase gene in S. dimorphus cells resulted in production of the protein.

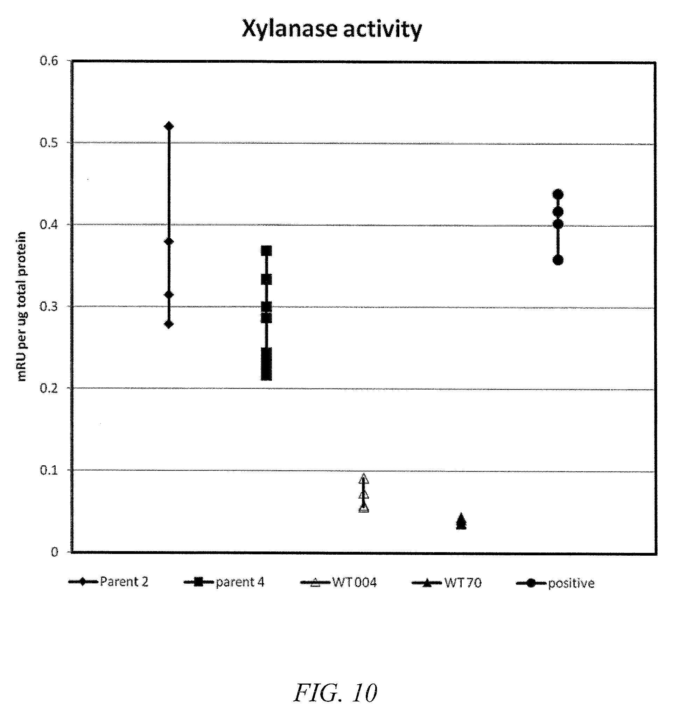

To determine if the endoxylanase produced by transformed algae cells was functional, endoxylanase activity was tested using an enzyme function assay. Briefly, algae cells were collected from TAP agar medium and suspended in BugBuster solution (Novagen). Cells were lysed by bead beating using zirconium beads. Cell lysates were clarified by centrifugation and the supernatants were normalized for total soluble protein (Coomassie Plus Protein Assay Kit, Thermo Scientific). 100 µL of each sample was mixed with 10 µL of 10X xylanase assay buffer (1M sodium acetate, pH=4.8) and 50 µL of the sample mixture was added to one well in a black 96-well plate. EnzCheck Ultra Xylanase substrate (Invitrogen) was dissolved at a concentration of 50ug/ml in 100mM sodium acetate pH 4.8, and 50 µL of substrate was added to each well of the microplate. The fluorescent signal was measured in a SpectraMax M2 microplate reader (Molecular Devices), with an excitation wavelength of 360 nm and an emission wavelength of 460 nm, without a cutoff filter and with the plate chamber set to 42 degrees Celsius. The fluorescence signal was measured for 15 minutes, and the enzyme velocity was calculated with Softmax Pro v5.2 (Molecular Devices). Enzyme velocities were recorded as RFU/minute. Enzyme specific activities were calculated as milliRFU per minute per µg of total soluble protein. **Figure 10** shows that endoxylanse activity is at least 4 fold higher in transformants than in wild type cells and similar in velocity to a positive control (Chlamydomonas expressing endoxylanse algae cells).

These data demonstrate that the chloroplast of *S*. *dimorphus* can be transformed with foreign DNA containing an expression cassette with a selectable marker and a separate expression cassette with a gene encoding an endoxylanase, and the expressed proteins are functional. One of skill in the art will appreciate that many other methods known in the art may be substituted in lieu of the ones specifically described or referenced.

### Example 5: Production of FPP Synthase in S. dimorphus

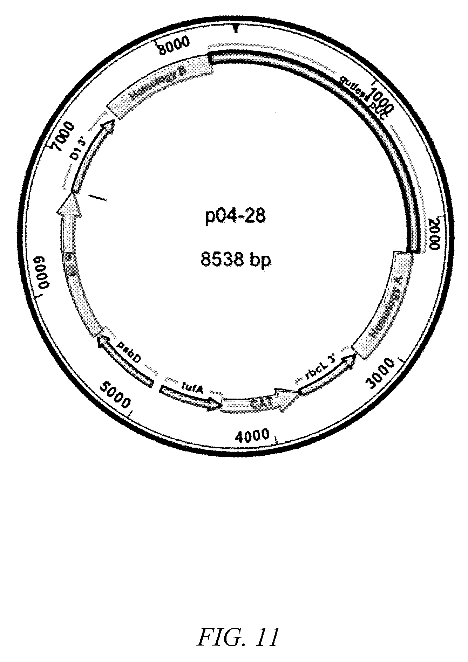

In this example a nucleic acid encoding FPP synthase from *G. gallus* was introduced into *S*. *dimorphus.* Transforming DNA is shown graphically in **Figure 11****.** In this instance the DNA segment labeled "Is09" is the FPP synthase encoding gene (SEQ ID NO: 23 Is09), the segment labeled "psbD" is the promoter and 5' UTR for the *psbD* gene from *S. dimorphus,* the segment labeled "D1 3'UTR" is the 3' UTR for the *psbA* gene from *S. dimorphus,* and the segment labeled "CAT" is the chloramphenicol acetyl transferase gene (CAT) from *E. coli,* which is regulated by the promoter and 5' UTR sequence for the *tufA* gene from *S. dimorphus* and the 3' UTR sequence for the *rbcL* gene from *S. dimorphus.* The transgene expression cassette and selection marker are targeted to the *S. dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All DNA segments were subcloned into pUC 18 (gutless pUC). All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Transforming DNA was introduced into *S. dimorphus* via particle bombardment according to the method described in **EXAMPLE 1** with DNA carried on 550 nm gold particles@ 500psi and a shooting distance of 4cm. Transformants were selected by growth on TAP-CAM agar medium under constant light 50-100uE @ RT for approximately 2 weeks. Transformants were streaked onto TAP-CAM agar medium to ensure single colony isolation and grown for 4 days under constant light.

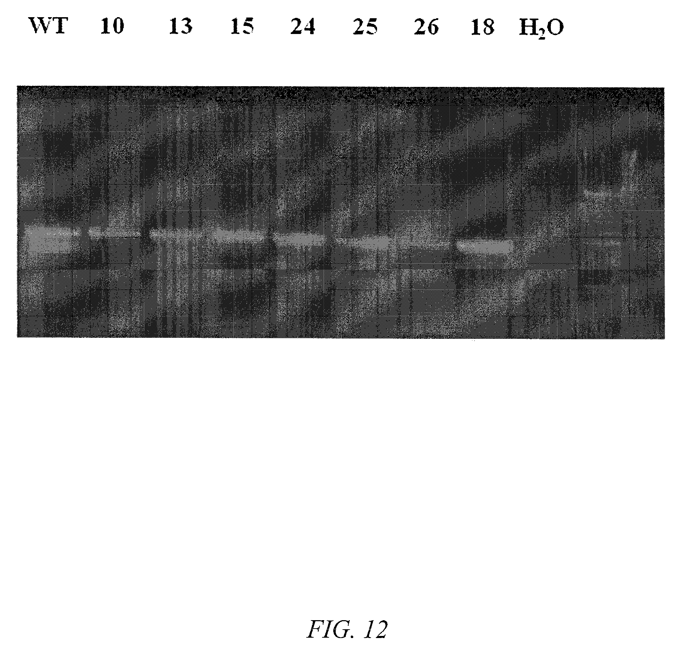

Transformants were analyzed by PCR screening (as described in **EXAMPLE 1**). The degree to which the transforming DNA was integrated into the chloroplast genome was determined using primers that amplify a 400 bp constant region (SEQ ID NO: 1 and SEQ ID NO: 2) and a 250 bp region spanning the integration site (SEQ ID NO: 3 and SEQ ID NO: 4). **Figure 12** shows that seven independent transformants are homoplasmic, i.e., only the constant region (400bp product) was amplified, while in the control reactions (WT) both the constant region and the region spanning the integration site (250bp) were amplified.

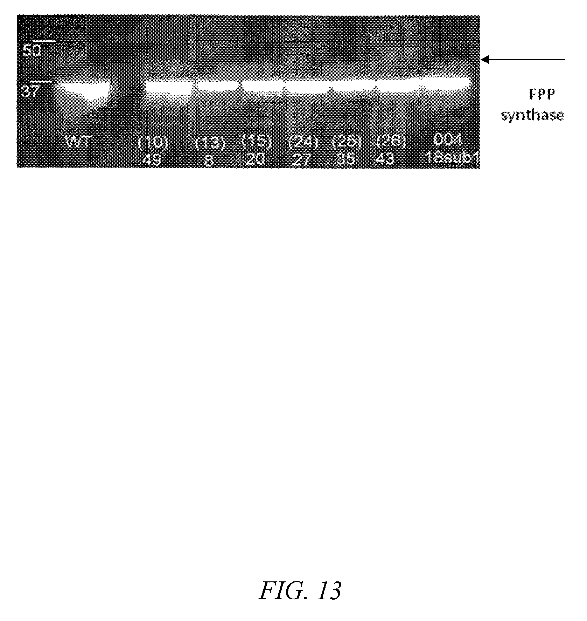

To ensure that the presence of the FPP synthase-encoding gene led to expression of the FPP synthase protein, a Western blot was performed (as described in **EXAMPLE 4).** Results from multiple clones **(****Figure 13****)** show that expression of the FPP synthase gene in *S. dimorphus* cells resulted in production of the protein.

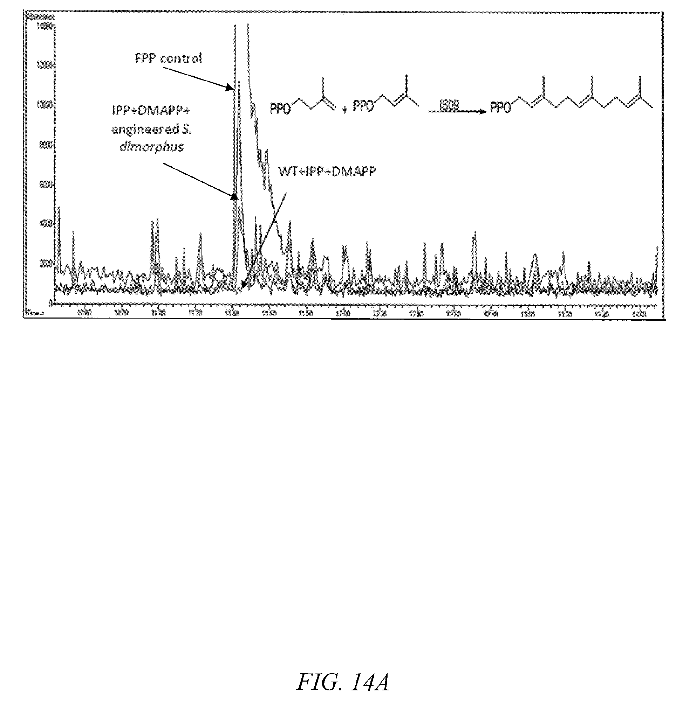

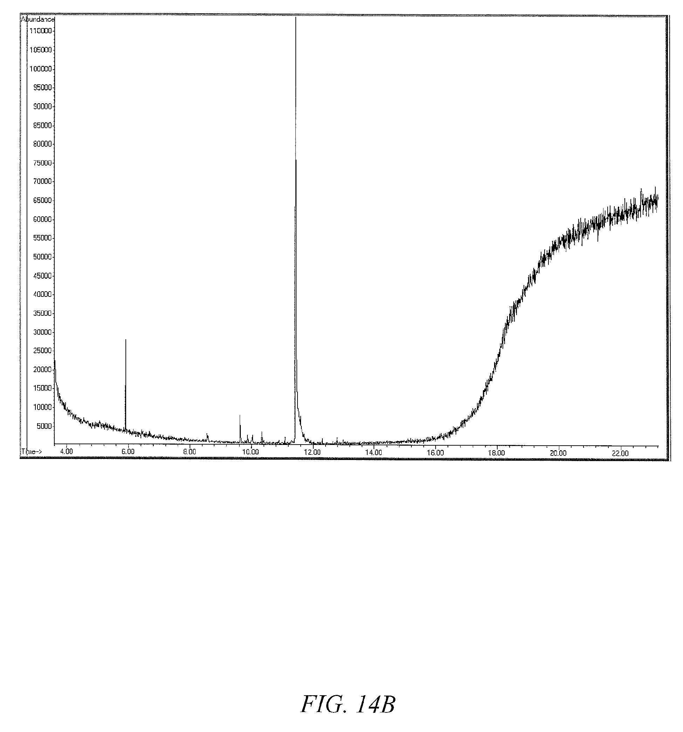

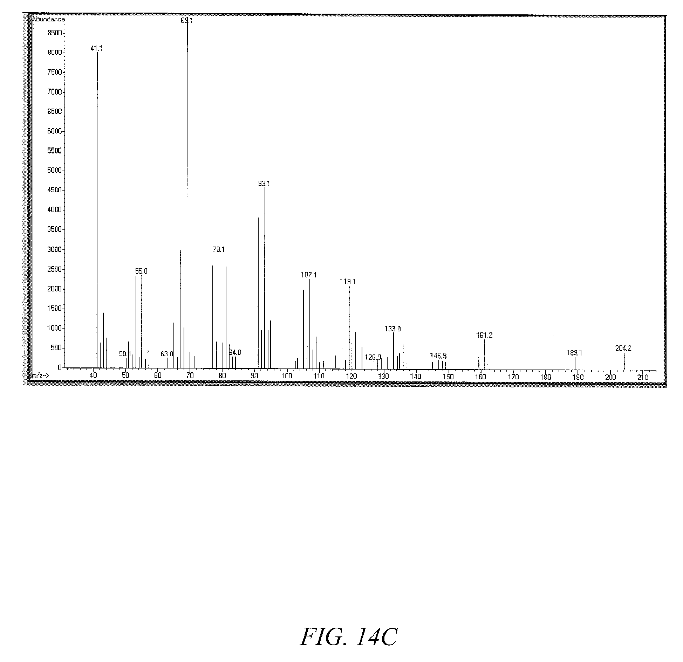

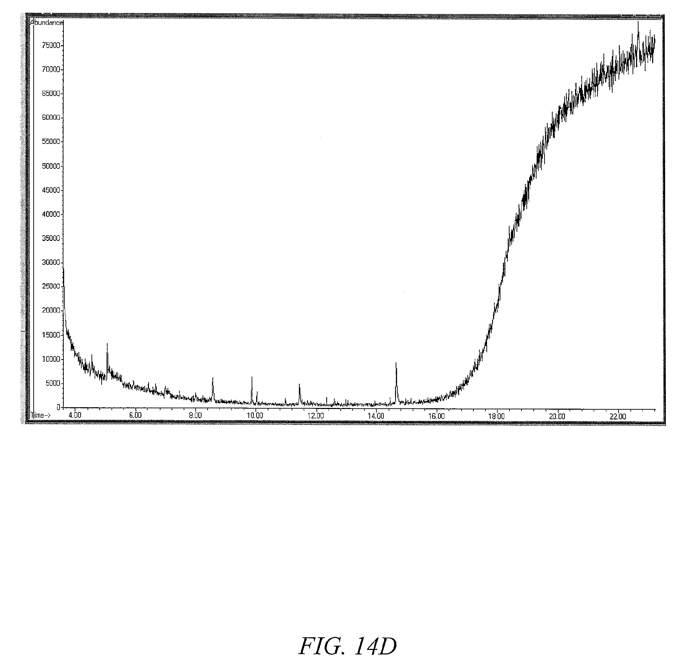

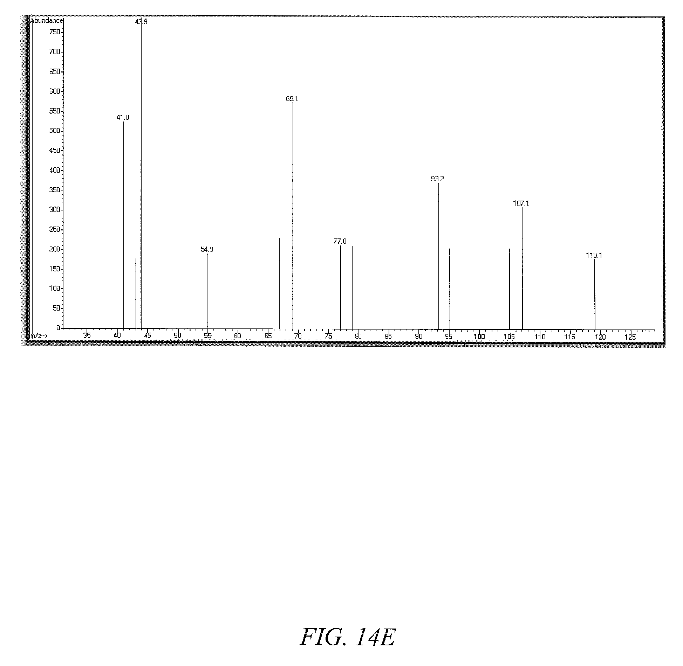

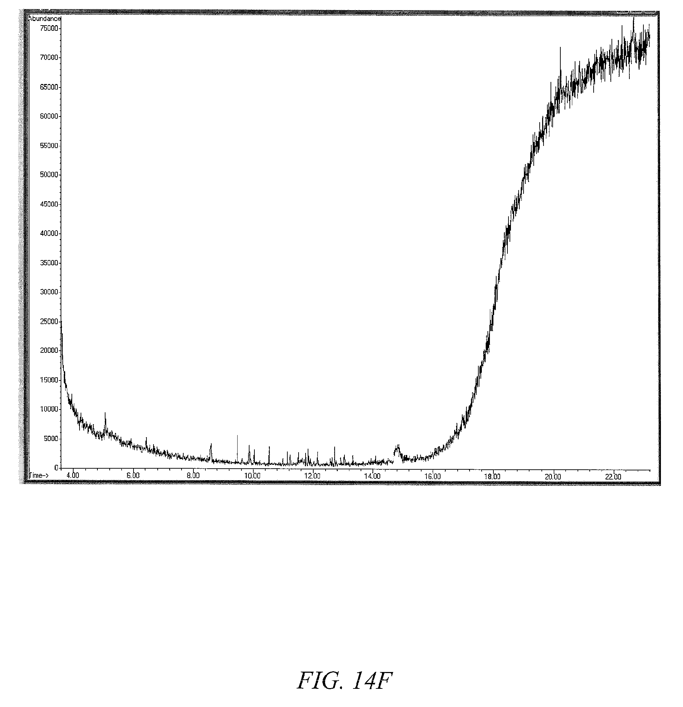

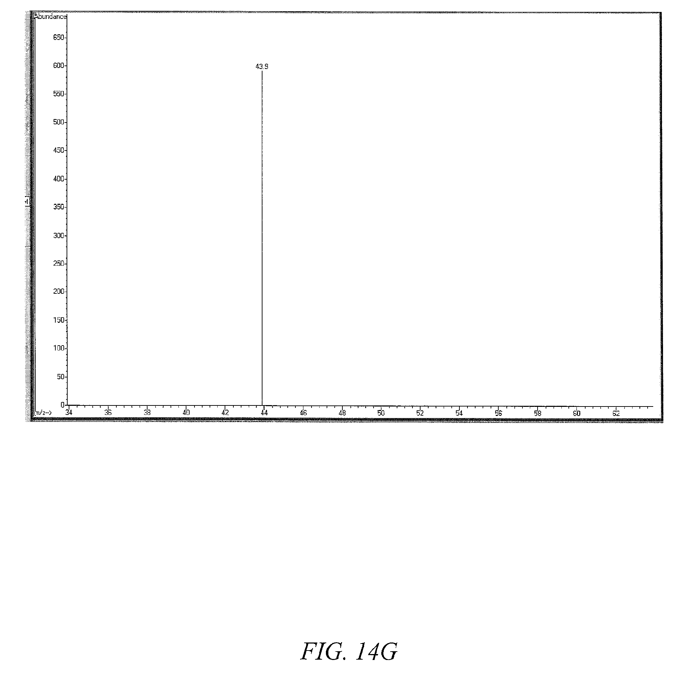

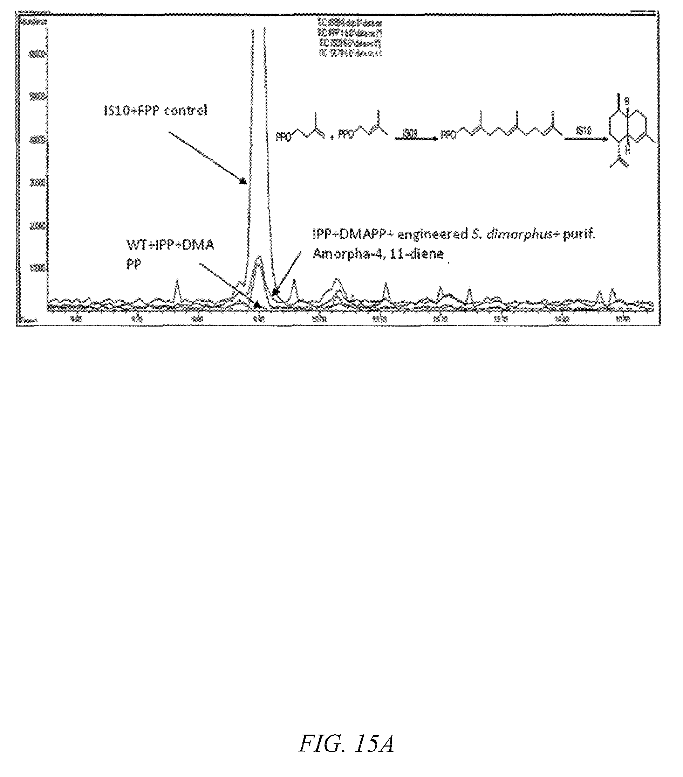

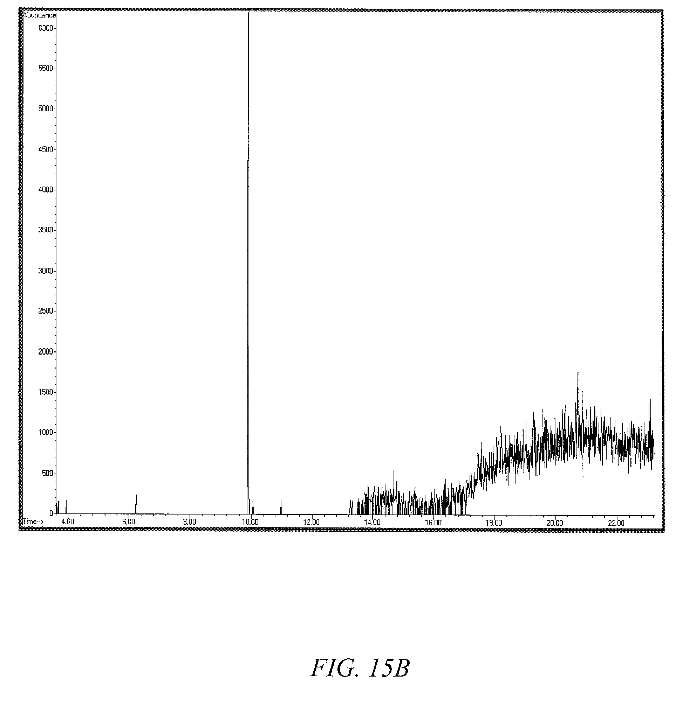

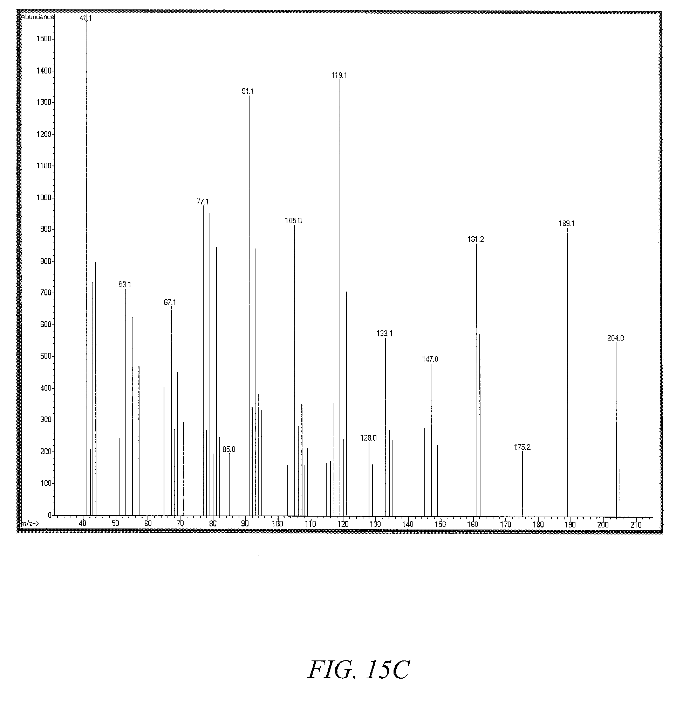

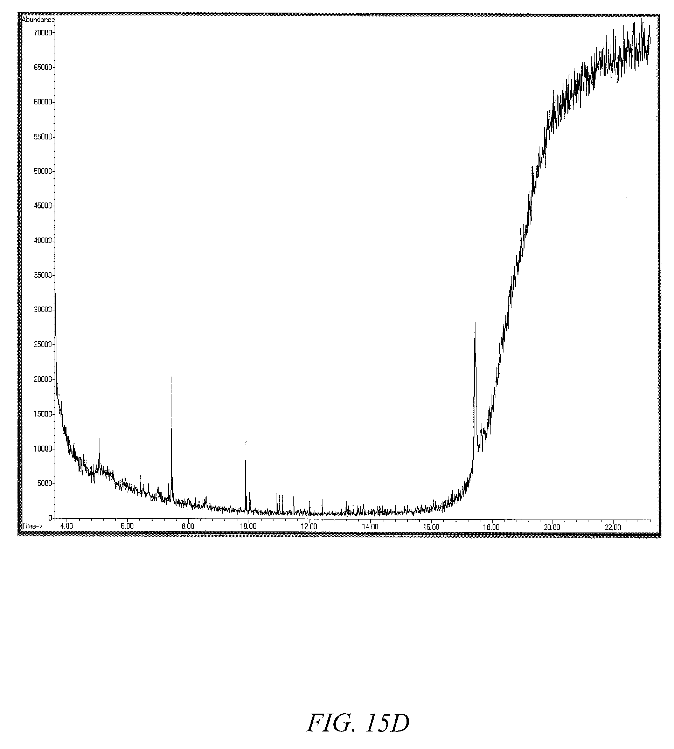

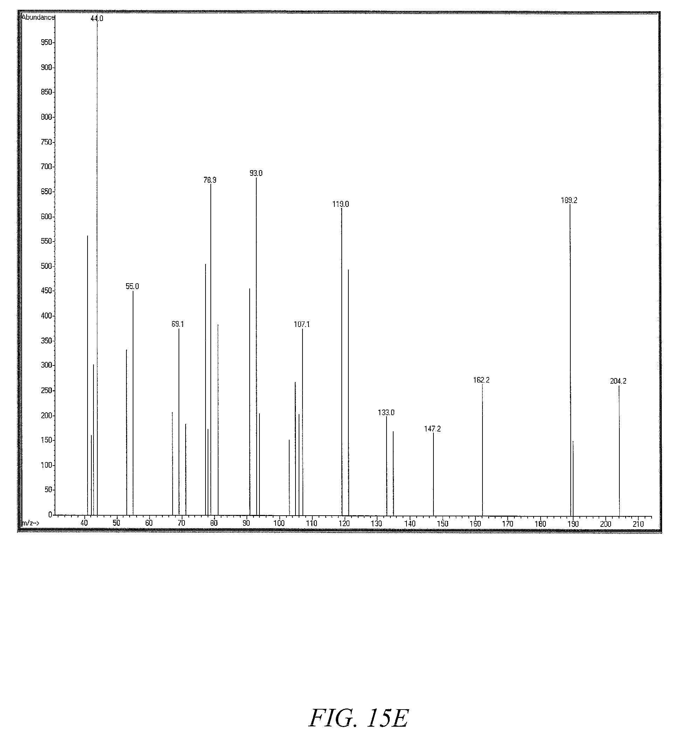

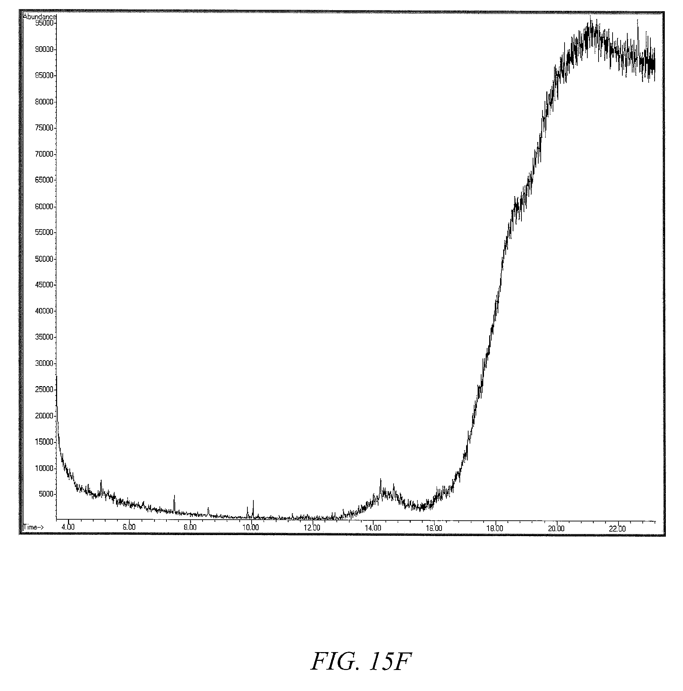

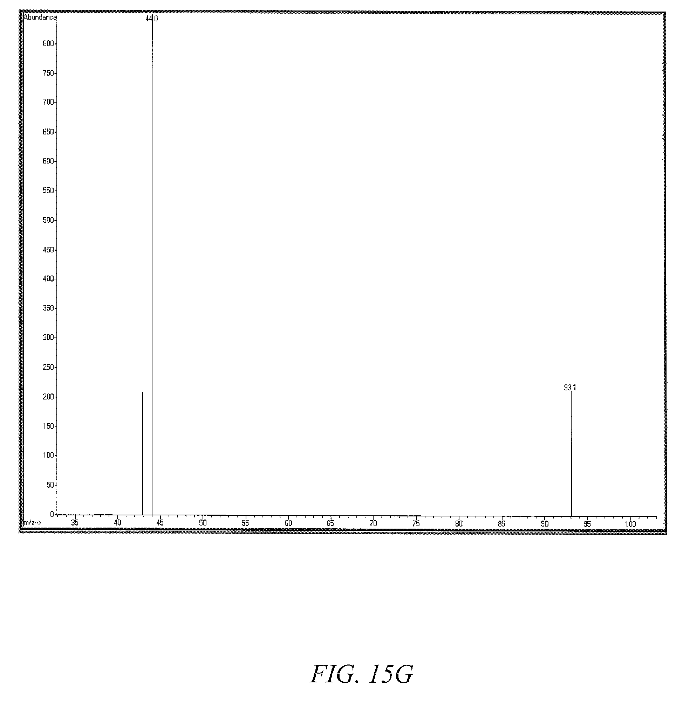

To determine if the FPP synthase produced by transformed algae cells was functional, FPP synthase activity was tested using an enzyme function assay. Algae cells were harvested from TAP media, resuspended in assay buffer (35mM HEPES, pH 7.4, 10mM MgCl₂, 5mM DTT) and lysed using zirconium beads in a bead beater. Crude lysate was clarified by centrifugation at 15,000 rpm for 20min. Isopentenyl diphosphate (IPP) and dimemthylallyl diphosphate (DMAPP) were added to clarified lysates and the reaction allowed to proceed at 30C overnight. Reactions were then CIP treated for 4-6hours @ 37C in glycine buffer, pH 10.6, 5mM ZnCl₂. The samples were then overlayed with heptane and analyzed via GC/MS **(****Figures 14A to G).** Additionally, IPP, DMAPP and *E. coli* purified amorpha-4, 11-diene were added to clarified lysates, the reactions allowed to proceed at 30 °C overnight, overlayed with heptane and analyzed via GC/MS **(****Figures 15A** to **G).** For both methods, the diagnostic ions at m/Z 204 and 189 were detected in the engineered *S. dimorphus,* but not in the wt samples.

These data demonstrate that the chloroplast of S. *dimorphus* can be transformed with foreign DNA containing an expression cassette with a selectable marker and a separate expression cassette with a gene encoding an FPP synthase, and the expressed proteins are functional. One of skill in the art will appreciate that many other methods known in the art may be substituted in lieu of the ones specifically described or referenced.

### Example 6: Production of Fusicoccadiene Synthase in S. dimorphus

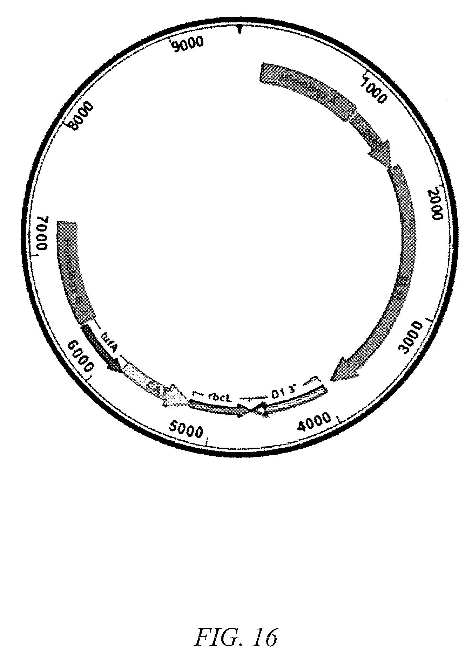

In this example a nucleic acid encoding fusicoccadiene synthase from *P. amygdali* was introduced into *S. dimorphus.* Transforming DNA is shown graphically in **Figure 16****.** In this instance the DNA segment labeled "Is88" is the fusicoccadiene synthase encoding gene (SEQ ID NO: 25, Is88), the segment labeled "psbD" is the promoter and 5' UTR for the *psbD* gene from *S. dimorphus,* the segment labeled "D1 3' " is the 3' UTR for the *psbA* gene from *S. dimorphus,* and the segment labeled "CAT" is the chloramphenicol acetyl transferase gene (CAT) from *E. coli,* which is regulated by the promoter and 5' UTR sequence for the *tufA* gene from *S. dimorphus* and the 3' UTR sequence for the *rbcL* gene from *S. dimorphus.* The transgene expression cassette and selection marker are targeted to the *S. dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus,* NC_008101) on the 5' and 3' sides, respectively. All segments were subcloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297,192-208, 1998.

Transforming DNA was introduced into *S. dimorphus* via particle bombardment according to the method described in **EXAMPLE 1** with DNA carried on 550 nm gold particles@ 500psi and a shooting distance of 4cm. Transformants were selected by growth on TAP-CAM agar medium under constant light 50-100uE @ RT for approximately 2 weeks. Transformants were streaked onto TAP-CAM agar medium to ensure single colony isolation and grown for 4 days under constant light.

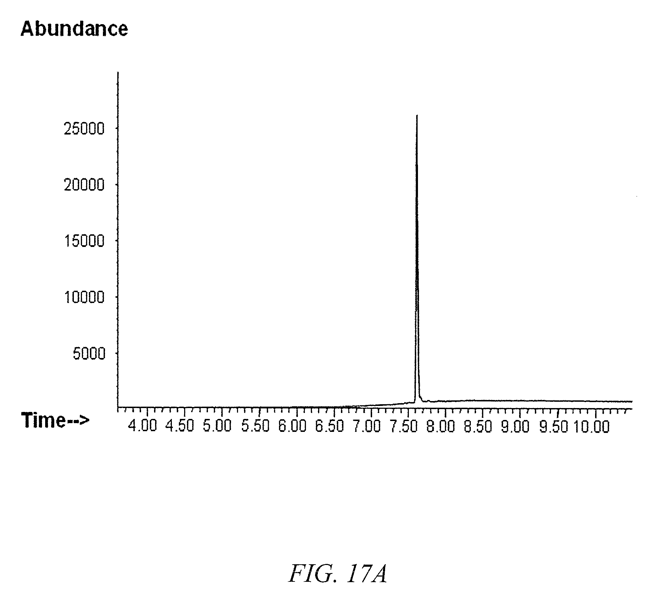

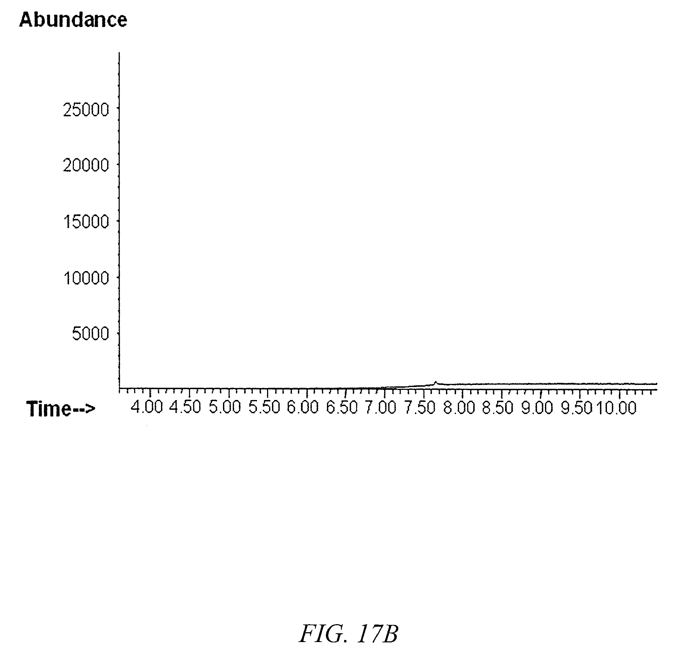

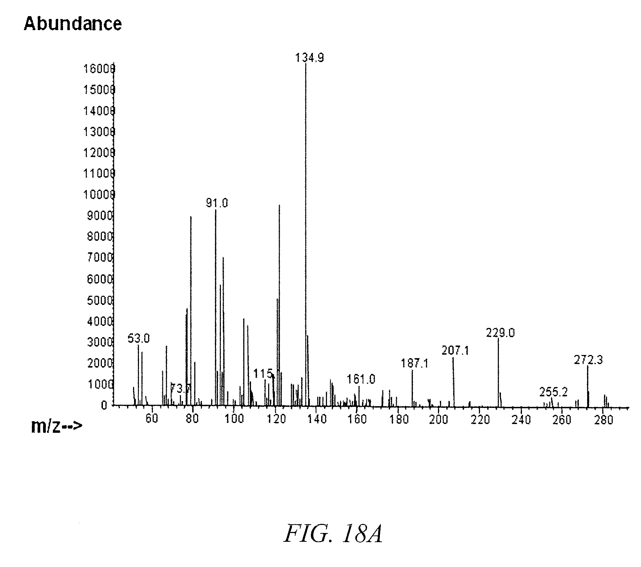

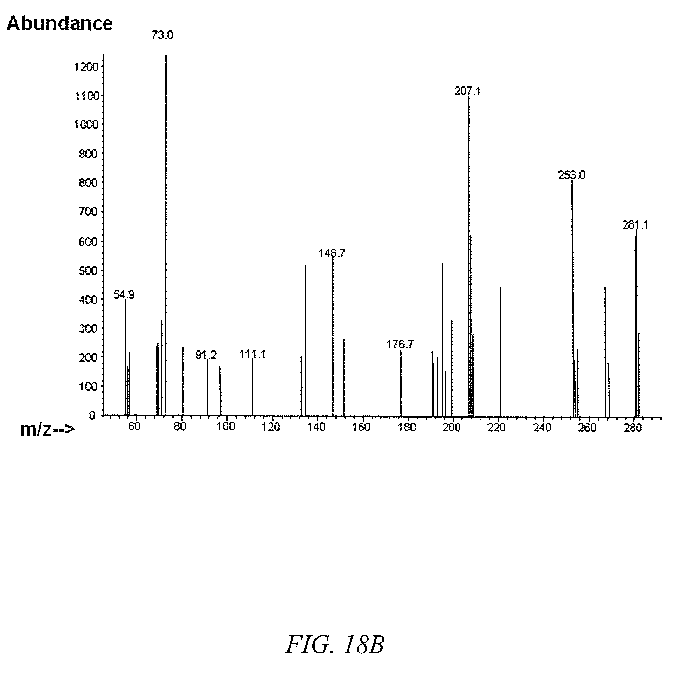

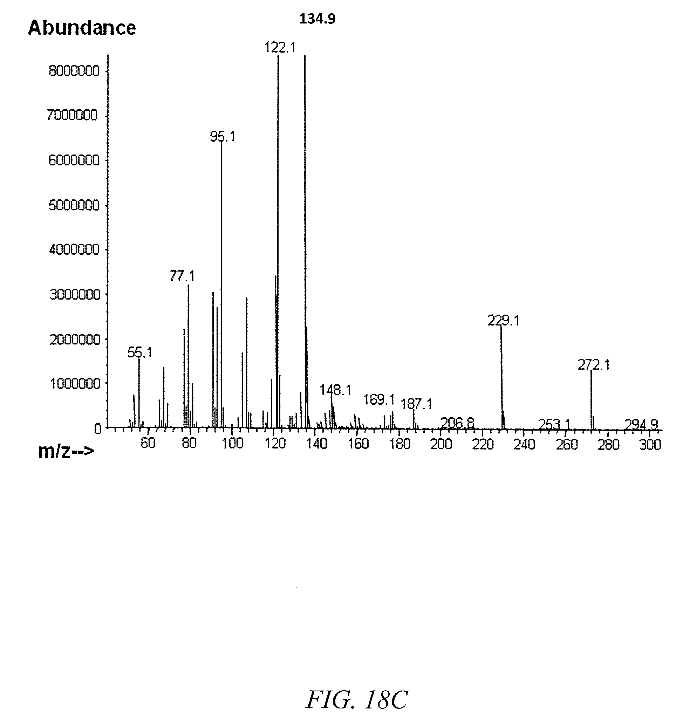

To determine if functional fusicoccadiene synthase is produced by transformed algal cells, cultures (2ml) of gene positive, homoplasmic algae were collected by centrifugation, resuspended in 250µl of methanol, and 500µl of saturated NaCl in water and 500µl of petroleum ether were added. The solution was vortexed for three minutes, then centrifuged at 14,000g for five minutes at room temperature to separate the organic and aqueous layers. The organic layer (100µl) was transferred to a vial insert in a standard 2ml sample vial and analyzed using GC/MS. The mass spectrum at 7.617 minutes for the sample from the engineered *S. dimorphus* is obtained. The diagnostic ions at m/Z=, 229, 135, and 122 are present in this spectrum, demonstrating the presence of fusicocca-2,10 (14)-diene and indole **(****Figure 17** and **Figure 18****).**

These data demonstrate that the chloroplast of *S. dimorphus* can be transformed with foreign DNA containing an expression cassette with a selectable marker and a separate expression cassette with a gene encoding a fusicoccadiene synthase that produces a novel hydrocarbon *in vivo.* One of skill in the art will appreciate that many other methods known in the art may be substituted in lieu of the ones specifically described or referenced.

### Example 7: Production of Phytase in S. dimorphus

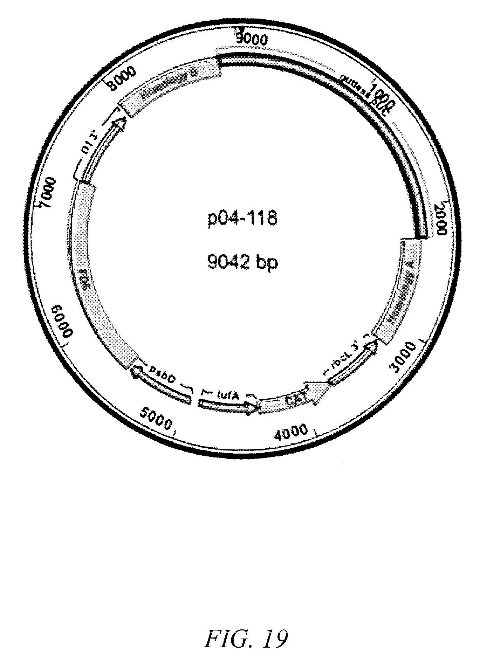

In this example a nucleic acid encoding phytase from *E. coli* was introduced into S. *dimorphus.* Transforming DNA is shown graphically in **Figure 19****.** In this instance the DNA segment labeled "FD6" is the phytase encoding gene (SEQ ID NO: 26, FD6), the segment labeled "psbD" is the promoter and 5' UTR for the *psbD* gene from *S. dimorphus,* the segment labeled "D1 3' " is the 3' UTR for the *psbA* gene from *S. dimorphus,* and the segment labeled "CAT" is the chloramphenicol acetyl transferase gene (CAT) from *E. coli,* which is regulated by the promoter and 5' UTR sequence for the *tufA* gene from *S. dimorphus* and the 3' UTR sequence for the *rbcL* gene from *S. dimorphus.* The transgene expression cassette and selection marker are targeted to the *S. dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All DNA segments were cloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP-CAM agar medium under constant light 50-100uE @ RT for approximately 2 weeks. Transformants were streaked onto TAP-CAM agar medium to ensure single colony isolation and grown for 4 days under constant light.

Transformants were analyzed by PCR screening (as described in **EXAMPLE 1)** and homoplasmic clones were identified and subcultured for further studies.

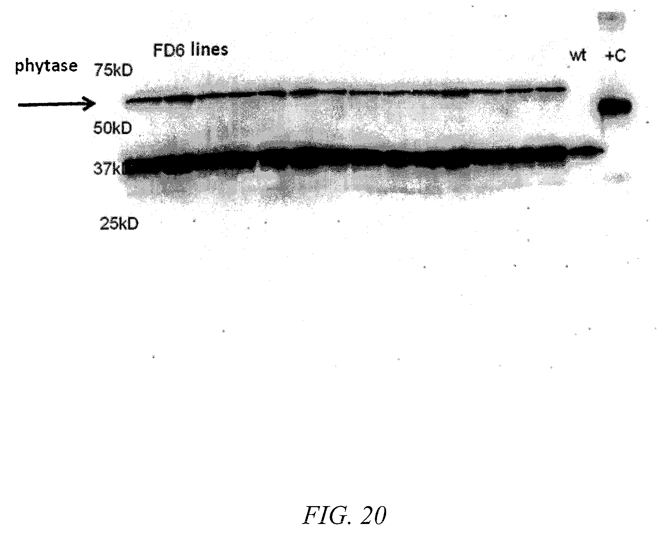

To ensure that the presence of the phytase-encoding gene led to expression of the phytase protein, a Western blot was performed (as described in **EXAMPLE 4).** Results from multiple clones **(****Figure 20****)** show that expression of the phytase gene in *S. dimorphus* cells resulted in production of the protein.

One of skill in the art will appreciate that many other methods known in the art may be substituted in lieu of the ones specifically described or referenced.

### Example 8: Use of Erythromycin Esterase as a Selection Marker in S. dimorphus and S. obliquus

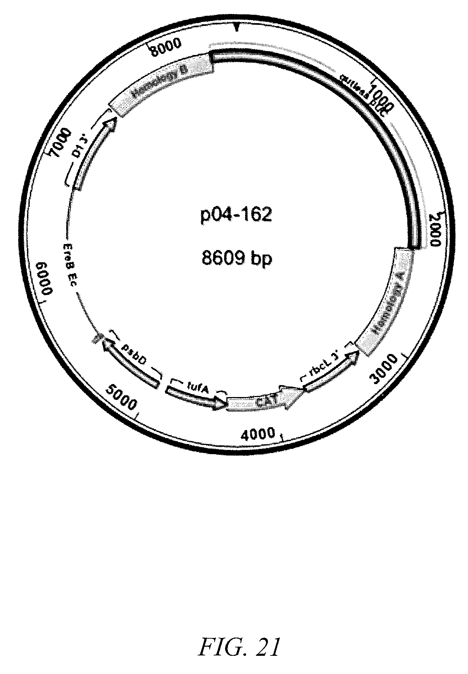

In this example, a nucleic acid encoding erythromycin esterase gene (EreB) (SEQ ID NO: 29) from *E*. *coli* was introduced into *S. dimorphus.* Transforming DNA is shown graphically in **Figure 21****.** In this instance the DNA segment labeled "EreB ec" is the erythromycin esterase gene (EreB) from *E. coli,* the segment labeled "psbD" is the promoter and 5' UTR sequence for the *psbD* gene from *S. dimorphus,* and the segment labeled " D1 3' " is the 3' UTR for the *psbA* gene from *S. dimorphus.* The selection marker cassette is targeted to the S. *dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All segments were cloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP agar medium + 50 µg/mL erythromycin (TAP-ERM50) under constant light 50-100uE @ RT for approximately 2 weeks. Transformants were streaked onto TAP-ERM50 agar medium to ensure single colony isolation and grown for 4 days under constant light.

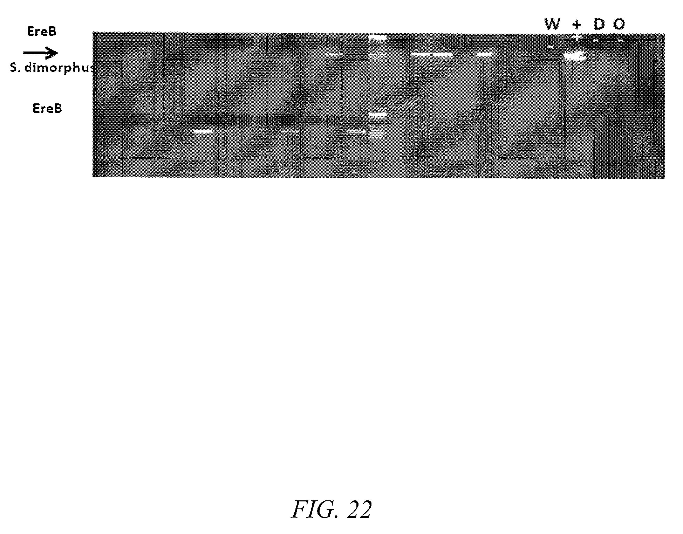

Transformants were analyzed by PCR screening (as described in **EXAMPLE 1).** The presence of the EreB selection marker was determined using primers that amplify a 555 bp region within the gene (SEQ ID NO: 7 and SEQ ID NO: 8). **Figure 22** shows that the EreB gene (SEQ ID NO: 29) was amplified from DNA from several transformants but not from wildtype DNA from *S. dimorphus.*

One of skill in the art will appreciate that many other methods known in the art may be substituted in lieu of the ones specifically described or referenced.

### Example 9: Use of codA as a Selection Marker in S. dimorphus

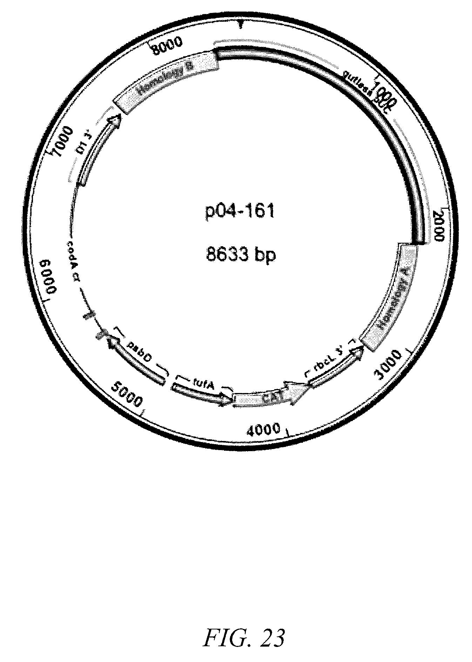

In this example, a nucleic acid encoding cytosine deaminase gene (codA) from *E. coli* was introduced into *S. dimorphus.* Transforming DNA is shown graphically in **Figure 23****.** In this instance the DNA segment labeled "codA cr" is the codA encoding gene (SEQ ID NO: 31, codA), the segment labeled "psbD" is the promoter and 5' UTR for the *psbD* gene from S. *dimorphus*, the segment labeled " D1 3' " is the 3' UTR for the psbA gene from *S*. *dimorphus,* and the segment labeled "CAT" is the chloramphenicol acetyl transferase gene (CAT) from *E*. *coli*, which is regulated by the promoter and 5' UTR sequence for the *tufA* gene from *S. dimorphus* and the 3' UTR sequence for the *rbcL* gene from *S. dimorphus.* The transgene expression cassette and selection marker are targeted to the *S. dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All DNA segments were cloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP-CAM agar medium under constant light 50-100uE @ RT for approximately 2 weeks. Transformants were streaked onto TAP-CAM agar medium to ensure single colony isolation and grown for 4 days under constant light.

Transformants were analyzed by PCR screening (as described in **EXAMPLE 1)** and homoplasmic clones were identified and subcultured for further studies.

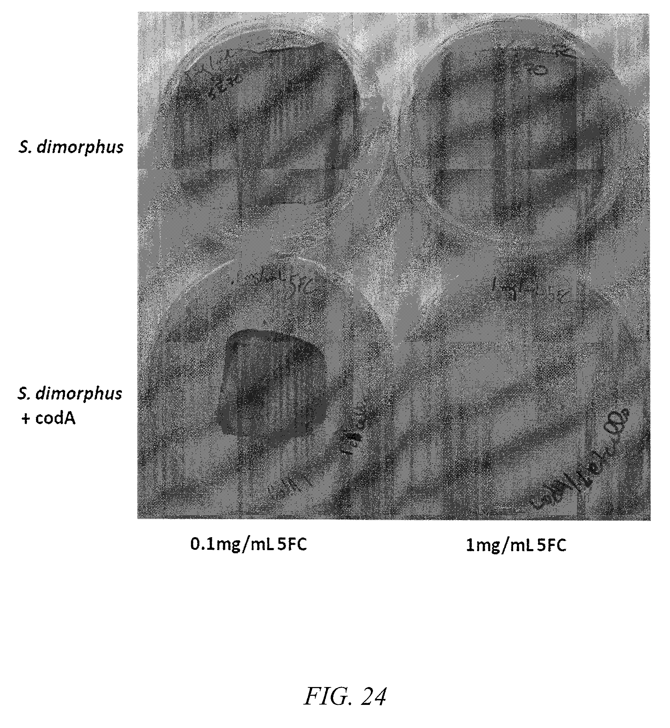

To determine if functional codA is produced by transformed algae cells, cells were grown in TAP media to log phase, pelleted and resuspended to 10⁸ cells/mL and plated onto TAP agar medium containing 1mg/mL 5-fluorocytosine (5FC). Wildtype *S. dimorphus,* survives on TAP agar containing 1mg/mL 5FC, while transformants containing the transgene do not **(****Figure 24****).** These data demonstrate that the chloroplast *of S. dimorphus* can be transformed with foreign DNA containing an expression cassette with a selectable marker and a separate expression cassette with a gene encoding a cytosine deaminase producing a cell with a negatively selectable phenotype.

This *S. dimorphus* homoplasmic codA line can now be transformed with either 1) a vector containing a gene of interest cassette without a selection marker in site 1 (the same site that the codA cassette is located within the genome) and after a recovery period on nonselective medium, selected for on medium containing 5FC, or 2) a vector containing a gene of interest cassette linked with an EreB cassette at site 1 and selected on medium containing erythromycin. In this instance, transformants can be streaked onto TAP medium + 50ug/mL erythromycin for single colony isolation and subclones can be patched onto TAP + 1mg/mL 5FC to select for clones homoplasmic for the EreB cassette.

One of skill in the art will appreciate that many other methods known in the art may be substituted in lieu of the ones specifically described or referenced.

### Example 10: Use of codA as a Selection Marker of S. obliquus

In this example, a nucleic acid encoding cytosine deaminase gene (codA) from *E. coli* was introduced into *S. obliquus*. Transforming DNA is shown graphically in **Figure 23****.** In this instance the DNA segment labeled "codA cr" is the codA encoding gene (SEQ ID NO: 27, codA), the segment labeled "psbD" is the promoter and 5' UTR for the *psbD* gene from S. *dimorphus,* the segment labeled "D1 3' " is the 3' UTR for the psbA gene from S. *dimorphus,* and the segment labeled "CAT" is the chloramphenicol acetyl transferase gene (CAT) from *E. coli,* which is regulated by the promoter and 5' UTR sequence for the *tufA* gene from *S. dimorphus* and the 3' UTR sequence for the *rbcL* gene from *S. dimorphus.* The transgene expression cassette and selection marker are targeted to the *S. obliquus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All DNA segments were cloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297,192-208, 1998.

Transforming DNA was introduced into *S. obliquus* via particle bombardment according to the method described in **EXAMPLE 1** with DNA carried on 550 nm gold particles @ 500psi and a shooting distance of 4cm. Transformants were selected by growth on TAP-CAM agar medium under constant light 50-100uE @ RT for approximately 2 weeks. Transformants were streaked onto TAP-CAM agar medium to ensure single colony isolation and grown for 4 days under constant light.

Transformants were analyzed by PCR screening (as described in **EXAMPLE 1)** and homoplasmic clones were identified and subcultured for further studies.

To determine if functional codA is produced by transformed algae cells, cells were plated onto TAP agar medium containing 1mg/mL 5-fluorocytosine (5FC). Wild type *S. dimorphus* survived on TAP agar containing 5FC, while transformants containing the transgene did not **(****Figure 24****).**

These data demonstrate that the chloroplast of S. *obliquus* can be transformed with foreign DNA containing an expression cassette with a selectable marker and a separate expression cassette with a gene encoding a cytosine deaminase producing a cell with a negatively selectable phenotype

This *S. obliquus* homoplasmic codA line can now be transformed with either 1) a vector containing a gene of interest cassette without a selection marker in site 1 (the same site that the codA cassette is located within the genome) and after a recovery period on nonselective medium, selected for on medium containing 5FC or 2) a vector containing a gene of interest cassette linked with an EreB cassette at site 1 and selected on medium containing erythromycin. In this instance, transformants can be streaked onto TAP medium + 50ug/mL erythromycin for single colony isolation and subclones can be patched onto TAP + 1mg/mL 5FC to select for clones homoplasmic for the EreB cassette.

One of skill in the art will appreciate that many other methods known in the art may be substituted in lieu of the ones specifically described or referenced.

### Example 11: Identification of Functional Promoters for Gene Expression in S. dimorplus

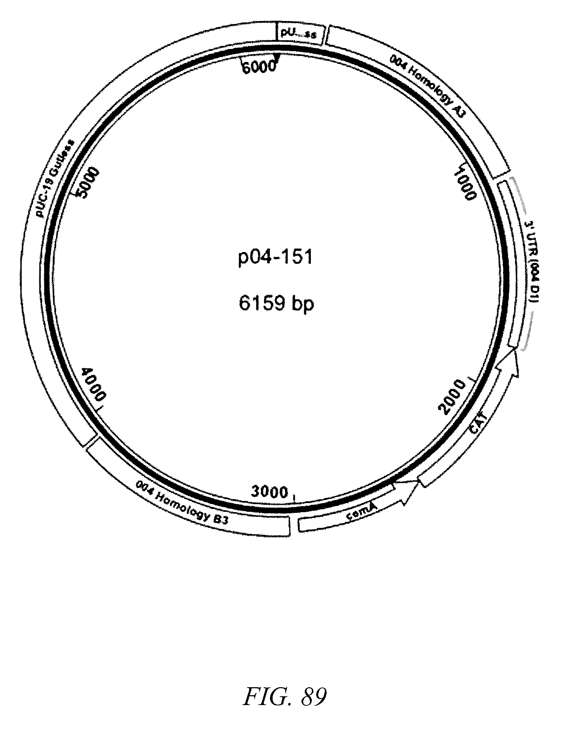

In this example, 8 promoters were amplified from *S. dimorphus* DNA and cloned upstream of the *E. coli* CAT gene. Transforming DNA (p04-151) is shown graphically in **Figure 89****.** In this instance the DNA segment labeled "CAT" is the chloramphenicol acetyl transferase gene (CAT) from *E. coli* (SEQ ID NO: 28, CAT), the segment labeled "tufA" is the promoter consisting of ∼500bp of the 5' UTR sequence for the *chlB* (SEQ ID NO: 51), *psbB* (SEQ ID NO: 39), *psbA* (SEQ ID NO: 37), *rpoA* (SEQ ID NO: 44), *rbcL* (SEQ ID NO: 49), *cemA* (SEQ ID NO: 45), *ftsH* (SEQ ID NO: 47), *petA* (SEQ ID NO: 53), *petB* (SEQ ID NO: 55) genes from *S*. dimorphus, and the segment labeled "D1 3 " is the 3' UTR for the *psbA* gene from *S. dimorphus.* The selection marker cassette is targeted to the S. *dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All DNA segments were subcloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP agar medium + 25 µg/mL chloramphenicol (TAP-CAM) under constant light 50-100uE @ RT for approximately 2 weeks. Each promoter *chlB* (SEQ ID NO: 51), *psbB* (SEQ ID NO: 39), *psbA* (SEQ ID NO: 37), *rpoA* (SEQ ID NO: 44), *rbcL* (SEQ ID NO: 49), *cemA* (SEQ ID NO: 45), *ftsH* (SEQ ID NO: 47), *petA* (SEQ ID NO: 53), *petB* (SEQ ID NO: 55) gave rise to chloramphenicol resistant transformants indicating that these promoter/ 5'UTR fragments were able to drive expression of the CAT gene.

### Example 12: Multiple Gene Expression in S. dimorphus

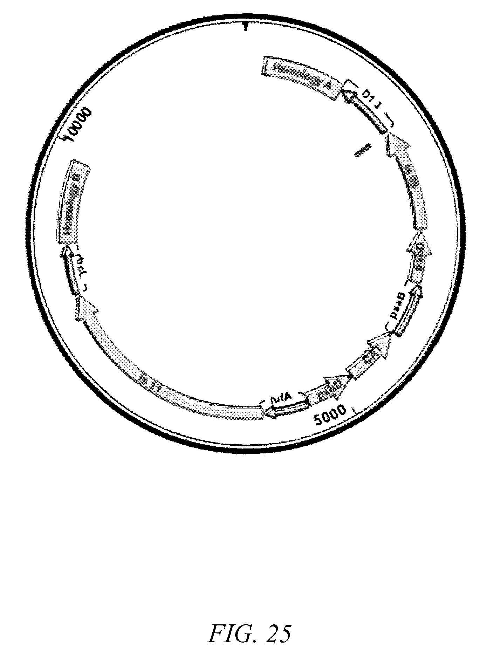

In this example a nucleic acid encoding FPP synthase from *G. gallus* and a nucleic acid encoding bisabolene synthase *from A. grandis* was introduced into *S. dimorphus.* Transforming DNA is shown graphically in **Figure 25****.** In this instance the DNA segment labeled "Is09" is the FPP synthase encoding gene (SEQ ID NO: 23, Is09), the segment labeled "psbD" is the promoter and 5' UTR for the *psbD* gene from *S. dimorphus,* the segment labeled "D1 3' " is the 3' UTR for the *psbA* gene from *S. dimorphus,* the segment labeled "Is11" is the bisabolene synthase encoding gene (SEQ ID NO: 35, Is011), the segment labeled "tufA" is the promoter and 5'UTR for the *tufA* gene from *S. dimorphus*, the segment labeled "rbcL" is the 3'UTR for the *rbcL* gene from *S. dimorphus*, and the segment labeled "CAT" is the chloramphenicol acetyl transferase gene (CAT) from *E. coli,* which is regulated by the promoter and 5' UTR sequence for the *psbD* gene from *S. dimorphus* and the 3' UTR sequence for *the psaB* gene (SEQ ID NO: 59)from *S. dimorphus.* The transgene expression cassette and selection marker are targeted to the *S. dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All DNA segments were subcloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP-CAM agar medium under constant light 50-100uE @ RT for approximately 2 weeks. Transformants were streaked onto TAP-CAM agar medium to ensure single colony isolation and grown for 4 days under constant light.

Transformants were analyzed by PCR screening (as described in **EXAMPLE 1)** and homoplasmic clones were identified and subcultured for further studies.

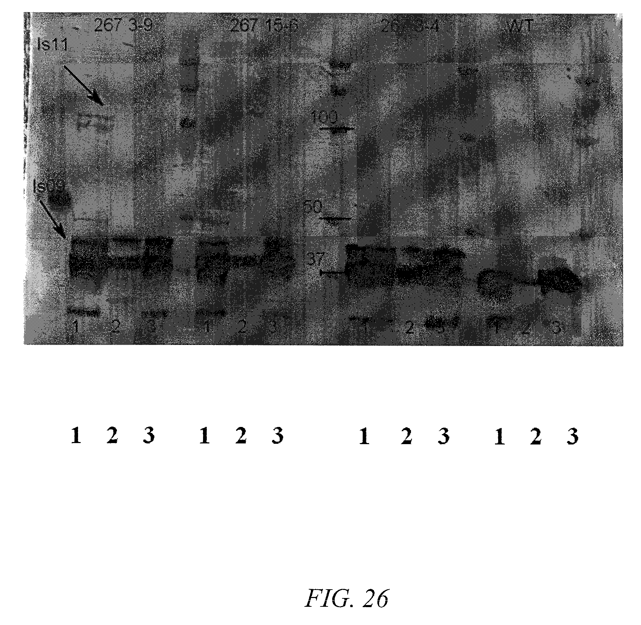

To ensure that the presence of the FPP synthase-encoding gene and the bisabolene-encoding gene led to expression of the FPP synthase and bisabolene synthase proteins, a Western blot was performed (as described in **EXAMPLE 4).** Proteins were visualized by a colormetric assay as per manufacturers instructions (1-step TMB blotting, Pierce). Results from multiple clones (267 3-9; 267 15-6; and 367 3-4) **(****Figure 26****)** show that expression of the FPP synthase gene (Is09) and bisabolene synthase (Is11) in *S. dimorphus* cells resulted in production of both proteins. WT is untransformed *S. dimorphus.* These data demonstrate that the chloroplast *of S. dimorphus* can be transformed with a vector of foreign DNA containing an expression cassette with a selectable marker and two separate expression cassette with a gene encoding an FPP synthase and an E-alpha-bisabolene synthase, and that both proteins are expressed.

### Example 13: Multiple Gene Expression in S. dimorphus

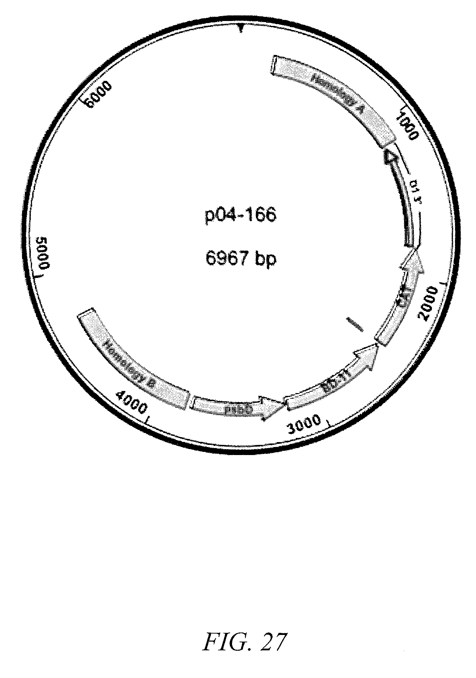

In this example, a nucleic acid encoding endoxylanase from *T. reesei* and chloramphenicol acetyl transferase gene (CAT) from *E. coli* linked together by a ribosome binding sequence from *E. coli* was introduced into *S. dimorphus.* Transforming DNA (BD11-RBS-CAT) is shown graphically in **Figure 27****.** In this instance the DNA segment labeled "BD11" is the endoxylanase encoding gene (SEQ ID NO: 21, BD11), the segment labeled "CAT" is the chloramphenicol acetyl transferase encoding gene (SEQ ID NO: 28, CAT), the segment labeled "RBS1" is the ribosome binding sequence (SEQ ID NO: 60, RBS1), the segment labeled "psbD" is the promoter and 5' UTR for the *psbD* gene from *S. dimorphus,* the segment labeled "psbA" is the 3' UTR for the *psbA* gene from *S. dimorphus,* and the segment labeled "CAT" is the chloramphenicol acetyl transferase gene (CAT) from *E. coli,* which is regulated by the promoter and 5' UTR sequence for the *tufA* gene from *S. dimorphus* and the 3' UTR sequence for the *rbcL* gene from S. *dimorphus.* The transgene expression cassette and selection marker are targeted to the *S. dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All DNA segments were subcloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP agar medium + 25 µg/mL chloramphenicol. Transformants were streaked onto TAP-CAM agar medium to ensure single colony isolation and grown for 4 days under constant light.

Transformants were analyzed by PCR screening (as described in **EXAMPLE 1)** and homoplasmic clones were identified and subcultured for further studies.

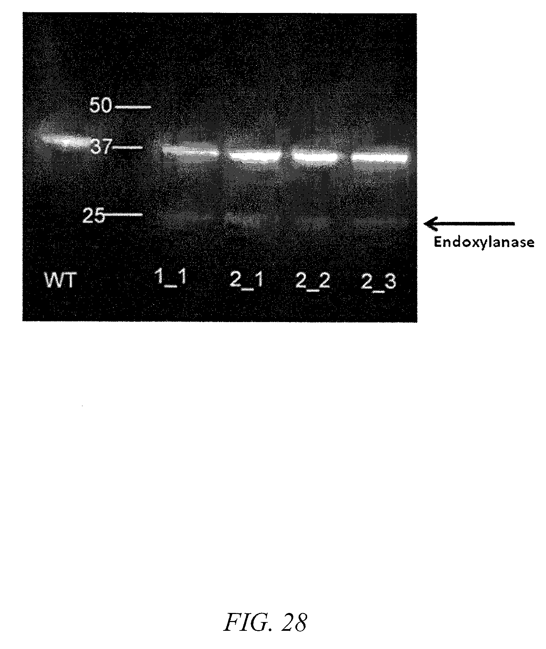

To ensure that the presence of the endoxylanase-encoding gene led to expression of the endoxylanase protein, a Western blot was performed (as described in **EXAMPLE 4).** Results from multiple clones **(****Figure 28****)** show that expression of the endoxylanase gene in *S. dimorphus* cells resulted in production of the protein of expected molecular weight and not of an endoxylanse-CAT fusion protein.

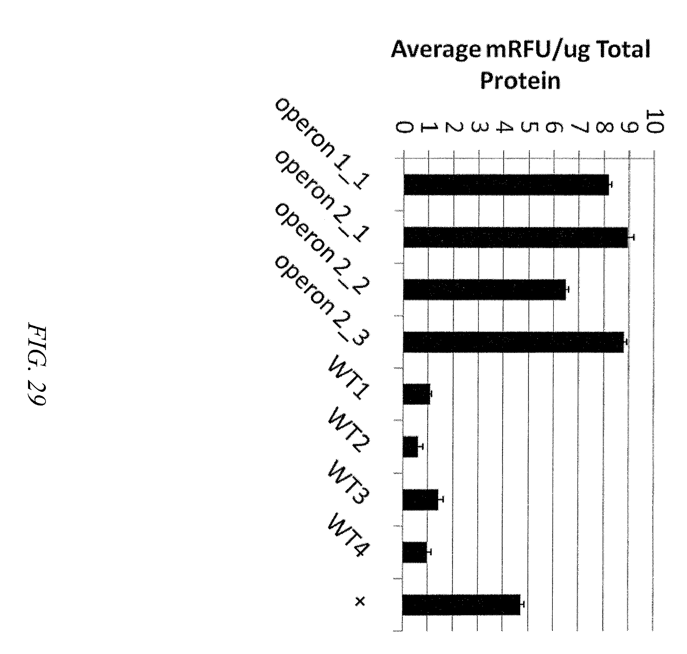

To determine if the endoxylanase produced by transformed algae cells was functional, endoxylanase activity was tested using an enzyme function assay (as described in **EXAMPLE 4).** **Figure 29** shows that endoxylanase activity is detected in clarified lysates *of S. dimorphus* engineered with the endoxylanase-RBS-CAT construct (operon 1_1, 2_1, 2_2, 2_3) and not in lysates of wt.

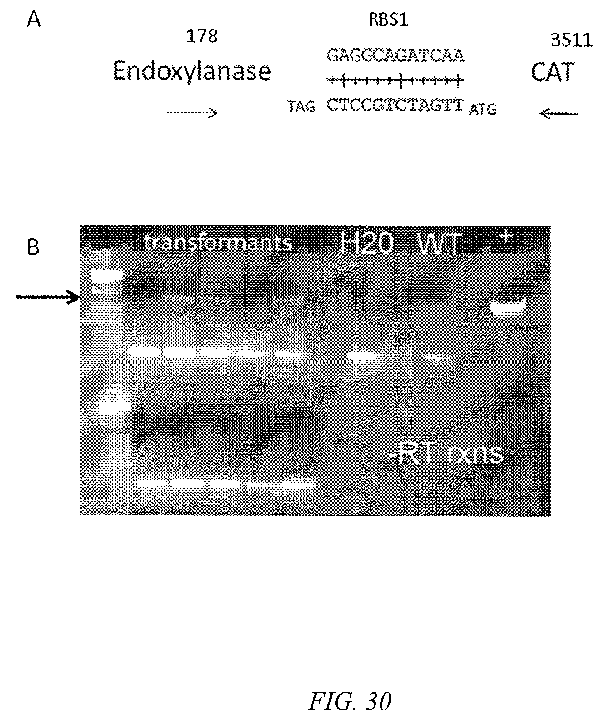

To determine whether both enzymes are produced from the same transcript, RNA was isolated from wildtype and engineered algae cells using the Concert Plant RNA Reagent kit (Invitrogen). RNA was DNase treated and cleaned using the RNeasy clean up kit (Qiagen). cDNA was synthesized from each of RNA using the iScrip kit (Biorad) and -reverse transcriptase (-RT) controls were included. cDNA (and -RT controls) was used as template in PCR with primers that hybridize to the endoxylanase gene and the CAT gene **(****Figure 30A****)** (SEQ ID NO: 11 and SEQ ID NO: 12, respectively) and amplify a product of 1.3kb. **Figure 30B** shows that a product of appropriate size was amplified from cDNA templates from 4 of the 5 transformants indicating that in these lines, the endoxylanase and the CAT gene are transcribed on a single transcript.

To further investigate variants of RBS1 (e.g., RBS2) and to understand the strength of these RBS sequences to recruit ribosomes, a nucleic acid encoding chloramphenicol acetyl transferase gene (CAT) from E. *coli* and endoxylanase from *T. reesei* linked together by two distinct ribosome binding sequences from *E. coli* were introduced into *S. dimorphus.* Transforming DNA (p04-231 or p04-232) is shown graphically in **Figure 31****.** In this instance the DNA segment labeled "CAT" is the chloramphenicol acetyl transferase encoding gene (SEQ ID NO: 28, CAT), the segment labeled "BD11" is the endoxylanase encoding gene (SEQ ID NO: 21, BD11), the segment labeled "RBS1" is the ribosome binding sequence (SEQ ID NO: 60, RBS1), the segment labeled "RBS2" is the ribosome binding sequence (SEQ ID NO: 61, RBS2) the segment labeled "psbD" is the promoter and 5' UTR for the *psbD* gene from *S. dimorphus,* the segment labeled "D1 3' " is the 3' UTR for the *psbA* gene from *S. dimorphus.* The transgene expression cassette and selection marker are targeted to the *S. dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for S. *obliquus*, NC_008101) on the 5' and 3' sides, respectively. All DNA segments were subcloned into pUC19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297,192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP agar medium + 25 µg/mL chloramphenicol. Transformants were streaked onto TAP-CAM agar medium to ensure single colony isolation and grown for 4 days under constant light.

Transformants were analyzed by PCR screening (as described in **EXAMPLE 1)** and homoplasmic clones were identified and subcultured for further studies.

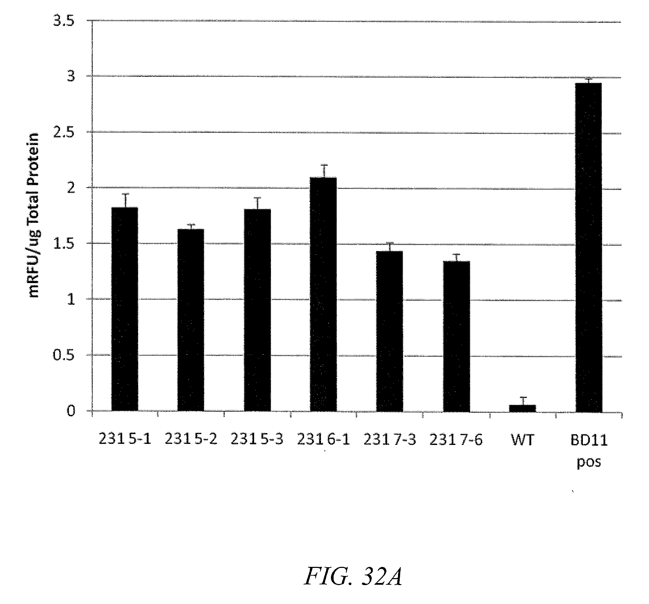

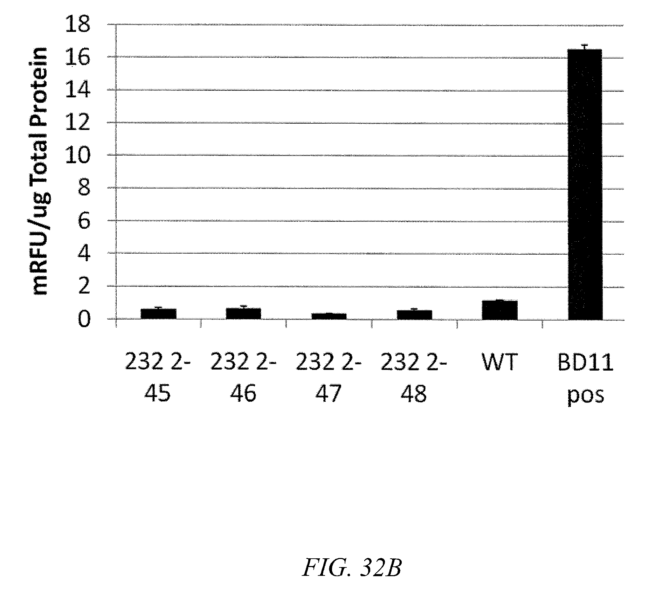

To determine if the endoxylanase produced by transformed algae cells was functional, endoxylanase activity was tested using an enzyme function assay (as described in **EXAMPLE 4).** **Figure 32A** shows that RBS1 between the two genes produces xylanase activity, however RBS2 does not produce active xylanase **(****Figure 32B****).**

These data demonstrate that the chloroplast of *S*. *dimorphus* can be transformed with a vector of foreign DNA containing an expression cassette that consists of a gene of interest linked to a selectable marker by a nucleotide sequence, allowing for the expression of multiple genes from one transcript, in this case a gene encoding an endoxylanase and a gene encoding chloramphenicol acetyl transferase. One of skill in the art will appreciate that many other methods known in the art may be substituted in lieu of the ones specifically described or referenced.

### Example 14: Use of Conserved Gene Cluster for an Integration Site in S. dimorphus

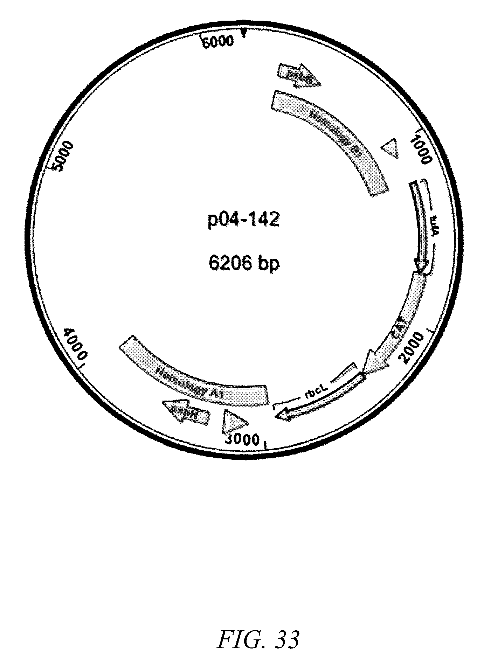

In this example, a nucleic acid encoding chloramphenicol acetyl transferase gene from *E. coli* was introduced into *S. dimorphus.* Transforming DNA is shown graphically in **Figure 33****.** In this instance the DNA segment labeled "CAT" is the chloramphenicol acetyl transferase gene from *E. coli,* the segment labeled "tufA" is the promoter and 5' UTR sequence for the *tufA* gene from *S. dimorphus,* and the segment labeled "rbcL" is the 3' UTR for the *rbcL* gene from *S. dimorphus.* The selection marker cassette is targeted to the *S. dimorphus* chloroplast genome via the segments labeled "Homology A1" and "Homology B1 which are approximately 1000 bp fragments homologous to sequences of DNA in the psbB-psbT-psbN-psbH cluster wherein the CAT cassette is inserted between psbT and psbN. All DNA segments were subcloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297, 192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP agar medium + 25 µg/mL chloramphenicol (TAP-CAM) under constant light 50-100uE @ RT for approximately 2 weeks. Transformants were streaked onto TAP-CAM agar medium to ensure single colony isolation and grown for 4 days under constant light.

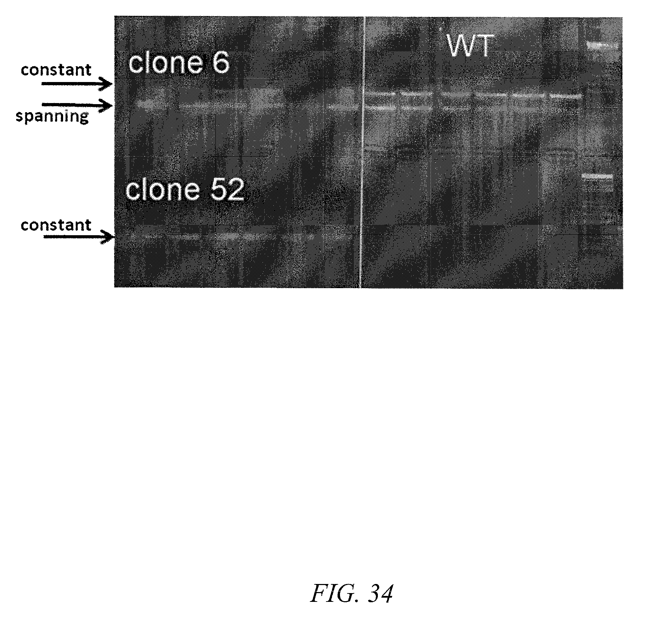

Cells from the transformants were analyzed by PCR screening (as described in **EXAMPLE 1).** The degree to which the transforming DNA was integrated into the chloroplast genome was determined using primers that amplify a 250 bp constant region (SEQ ID NO: 3 and SEQ ID NO: 4) and a 400 bp region spanning the integration site (SEQ ID NO: 15 and SEQ ID NO: 16). The homology regions target the integration site, the region of the chloroplast genome between psbT and psbN, approximately nucleotide 059,687 (nucleotide locations according to the sequence available from NCBI for *S. obliquus*, NC_008101). **Figure 34** shows that subclones from clone 52 are homoplasmic, i.e., only the constant region (250bp product) was amplified, while in the control reactions (WT) both the constant region and the region spanning the integration site (400bp) were amplified. Clone 6 is another parental clone, however subclones from clone 6 are not completely homoplasmic as the spanning region is still amplified. These data indicate that the psbB-psbH cluster can be utilized as an integration site in engineering *S. dimorphus.*

One of skill in the art will appreciate that many other methods known in the art may be substituted in lieu of the ones specifically described or referenced.

### Example 15: Strategy to Generate Markerless Transgenic S. dimorphus

In this example, the transgenic line generated in **EXAMPLE 12,** was used to inoculate nonselective media. A saturated culture was diluted 1:300 in nonselective media, allowed to grow to saturation and diluted 1:4 in nonselective media. Once saturated, the culture was plated onto nonselective TAP medium to ensure single colony formation. Single clones were then patched to 1) nonselective TAP medium and 2) TAP-CAM medium. Clones that failed to grow on TAP-CAM were further analyzed by PCR.

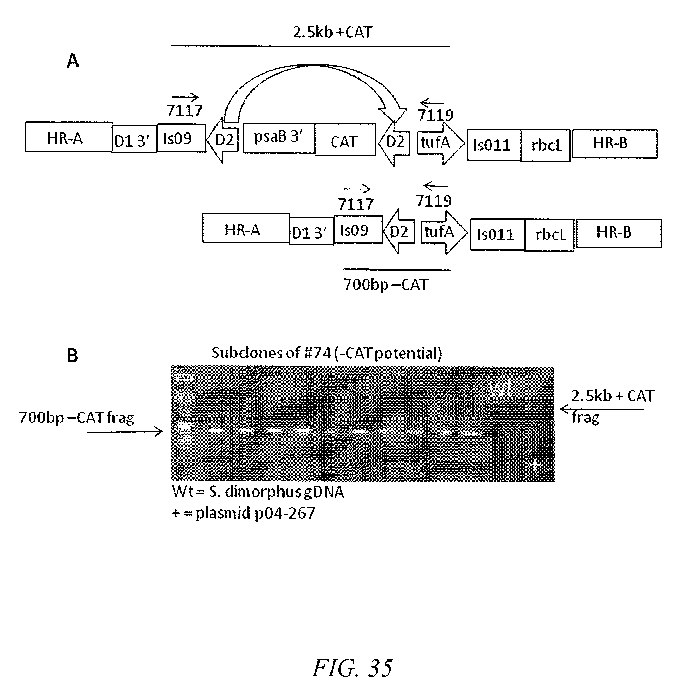

**Figure 35** **A** is a graphical representation of the transforming DNA (top) and loopout product (bottom) that results from recombination at the identical D2 (psbD) promoter segments. HR-A & HR-B represent the homology regions. D1 3', psaB 3' and rbcL represent the psbA 3'UTR, psaB 3'UTR, and rbcL 3'UTR, respectively. D2 and tufA is the psbD and tufA promoter, respectively. Is09 is FPP synthase and Is011 is bisabolene synthase.

To confirm the absence of the CAT gene, two methods were employed. First, PCR was performed using primers that amplify a 2.5kb +CAT fragment and/or a 700bp -CAT fragment (SEQ ID NO: 9 and SEQ ID NO: 10). **Figure 35** **B** is an agarose gel showing that in subclones of the #74 transformant only the 700bp -CAT product was amplified while in the plasmid DNA control, the 2.5kb +CAT fragment was amplified. The presence of the 700bp product in the plasmid DNA control is likely the result of recombination in the *E. coli* host as it is *RecB+.* Primers 7117 & 7119 (SEQ ID NO: 9 and SEQ ID NO: 10) were used to amplify the products. The "markerless" transgenic S. dimorphus shows amplification of 700bp -CAT loopout fragment and failure to amplify the 2.5kb +CAT fragment in subclones of clone #74.

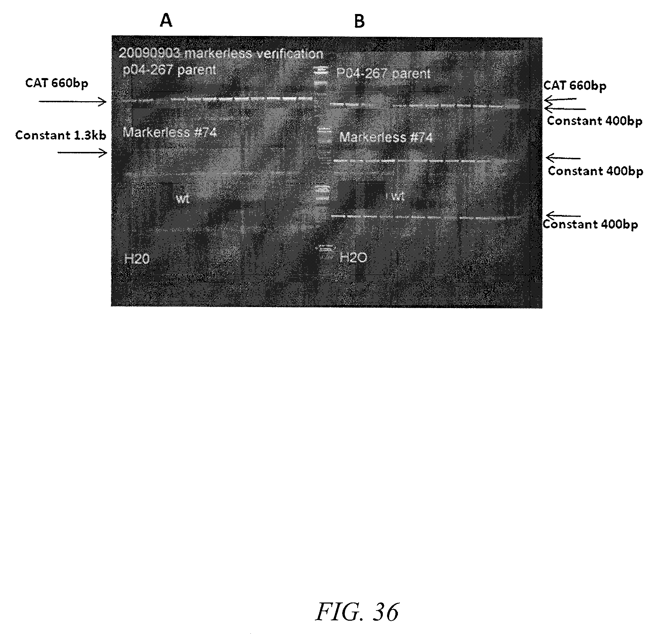

Second, PCR was performed using primers that amplify the 660bp CAT gene (SEQ ID NO: 18 and SEQ ID NO: 19), and either primers that amplify a 1.3kb constant region of the *psbA* gene (SEQ ID NO: 13 and SEQ ID NO: 14) or those that amplify a 400bp constant region of the *psbA* gene (SEQ ID NO: 1 and SEQ ID NO: 2). **Figure 36** shows that only the constant fragment was amplified in the #74 markerless line, while the CAT gene was amplified in the parental line that was always kept on CAT selection. **Panel A** shows multiplex PCR using primers that amplify a 660bp CAT fragment and primers that amplify a 1.3kb constant region of the endogenous psbA gene. Only the 1.3kb constant region is amplified in the #74 markerless potential. **Panel B** shows multiplex PCR using primers that amplify a 660bp CAT fragment and primers that amplify a 400bp constant region of the endogenous psbA gene. Only the 400bp constant region is amplified in the #74 markerless potential. The PCR reactions in both panel A and panel B had a 50-60 degree Celcius annealing gradient to rule out the possibility that the annealing of the primers was temperature sensitive.

These data demonstrate that *S. dimorphus* clones can be obtained consisting of a genetically engineered chloroplast and without an antibiotic resistance marker.

One of skill in the art will appreciate that many other methods known in the art may be substituted in lieu of the ones specifically described or referenced.

### Example 16: Use ofBetaine Aldehyde Dehydrogenase to Confer Salt Tolerance and/or as a Negative Selection Mechanism

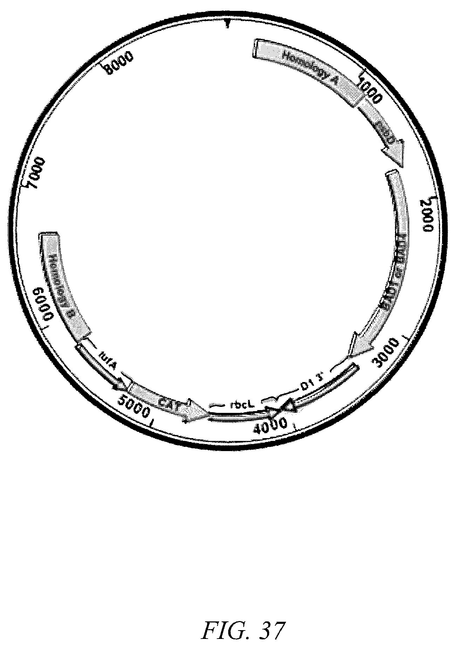

In this example, a nucleic acid sequence encoding betaine aldehyde dehydrogenase from spinach or sugar beet was engineered into *S. dimorphus* (as described in **EXAMPLE 4).** Transforming DNA is shown graphically in **Figure 37****.** In this instance the DNA segment labeled "BAD1 or BAD4" is the betaine aldehyde dehydrogenase encoding gene from spinach (BAD1) or sugar beet (BAD4) (SEQ ID NO: 32, BAD1 or SEQ ID NO: 34, BAD4), the segment labeled "psbD" is the promoter and 5' UTR for the *psbD* gene from *S. dimorphus,* the segment labeled "rbcL" is the 3' UTR for the *psbA* gene from *S. dimorphus,* and the segment labeled "CAT" is the chloramphenicol acetyl transferase gene from *E*. *coli,* which is regulated by the promoter and 5' UTR sequence for the *tufA* gene from *S. dimorphus* and the 3' UTR sequence for the *rbcL* gene from *S. dimorphus.* The transgene expression cassette and selection marker are targeted to the *S. dimorphus* chloroplast genome via the segments labeled "Homology A" and "Homology B" which are approximately 1000 bp fragments homologous to sequences of DNA adjacent to nucleotide 071,366 (Site 1; nucleotide locations according to the sequence available from NCBI for *S. obliquus*, NC_008101) on the 5' and 3' sides, respectively. All DNA segments are subcloned into pUC19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297,192-208, 1998.

Transforming DNA was introduced into *S. dimorphus* via particle bombardment according to the method described in **EXAMPLE 1** with DNA carried on 550 nm gold particles @ 500psi and a shooting distance of 4cm. Transformants were selected by growth on TAP-CAM agar medium under constant light 50-100uE @ RT for approximately 2 weeks. Transformants were streaked onto TAP-CAM agar medium to ensure single colony isolation and grown for 4 days under constant light.

Transformants were analyzed by PCR screening (as described in **EXAMPLE 1)** and homoplasmic clones were identified and subcultured for further studies.

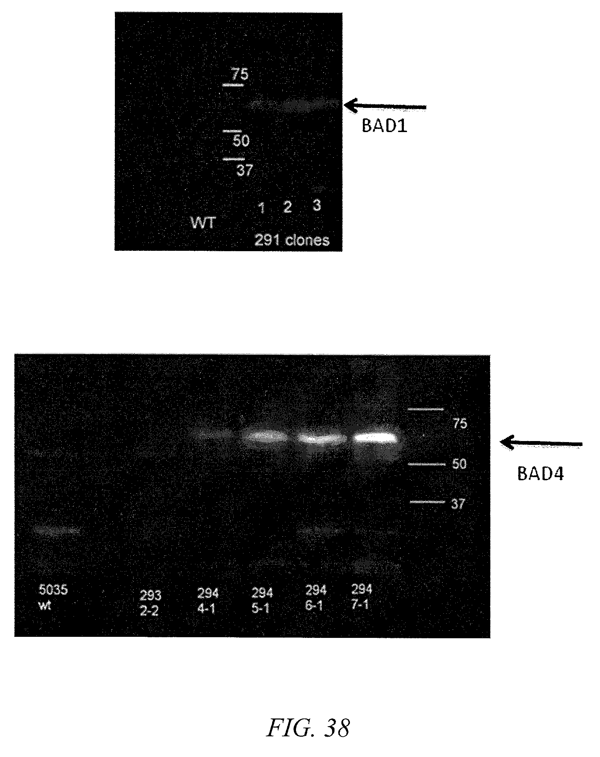

To ensure that the presence of the betaine aldehyde dehydrogenase encoding gene led to expression of the protein, a Western blot was performed (as described in **EXAMPLE 4).** In this instance, the BAD genes were tagged with an HA tag and the primary antibody was an anti-HA HRP conjugated antibody (clone 3F10, Roche) in which a 1:10,000 dilution of a 50U/mL stock was used as the antibody solution. Results from multiple clones **(****Figure 38****)** show that expression of the BAD gene from spinach and from sugar beet gene in *S. dimorphus* cells resulted in production of the protein.

To determine if this protein confers salt tolerance or causes the cells to become sensitive to betaine aldehyde (and therefore allows this strain to be used in negative selection experiments as proposed in examples 9 and 10), cells expressing the BAD genes can be grown side-by-side with wildtype cells and the media supplemented with increasing concentrations of salt and/or betaine aldehyde.

One of skill in the art will appreciate that many other methods known in the art may be substituted in lieu of the ones specifically described or referenced.

### Example 17: Development of a Transformation System for D. tertiolecta

In this example, a method for transformation of *D. tertiolecta* is described. Algae cells are grown to log phase (approximately 5.0 x 10⁶ cells/mL) in G32 medium (32g/L NaCl, 0.0476mM CaCl₂, 0.162mM H₃BO₃, 0.406mM Mg₂SO₄, 0.00021mM NaVO₃, 5g/L bicarbonate, 12.9mL/L each of F/2 A and B algae food (Aquatic Eco-systems, Inc.)) at 23°C under constant illumination of 50-100uE on a rotary shaker set at 100 rpm. Cells are harvested at 1000 x g for 5 min. The supernatant is decanted and cells are resuspended in G32 media at 10⁸ cells/mL. 5 x 10⁷ cells are spread on selective agar medium and transformed by particle bombardment with 550 nm diameter gold particles carrying the transforming DNA @ 300-400psi with the Helios Gene Gun (Bio-Rad) from a shot distance of 4cm. Desired algae clones are those that grow on selective media.

PCR is used to identify transformed algae strains. For PCR analysis, colony lysates are prepared by suspending algae cells (from agar plate or liquid culture) in lysis buffer (0.5% SDS, 100 mM NaCl, 10 mM EDTA, 75 mM Tris-HCl, pH 7.5) and heating to 98°C for 10 minutes, followed by cooling to near 23°C. Lysates are diluted 50-fold in 100mM Tris-HCl pH 7.5 and 2uL is used as template in a 25uL reaction. Alternatively, total genomic DNA preparations may be substituted for colony lysates. A PCR cocktail consisting of reaction buffer, dNTPs, PCR primer pair(s) (indicated in each example below), DNA polymerase, and water is prepared. Algae DNA is added to provide template for the reaction. Annealing temperature gradients are employed to determine optimal annealing temperature for specific primer pairs. In many cases, algae transformants are analyzed by PCR with primers that are specific for the transgene being introduced into the chloroplast genome. Desired algae transformants are those that give rise to PCR product(s) of expected size(s).

One of skill in the art will appreciate that many other transformation methods known in the art may be substituted in lieu of the ones specifically described or referenced herein.

### Example 18: Use of Conserved Gene Cluster for an Integration site in D. tertiolecta

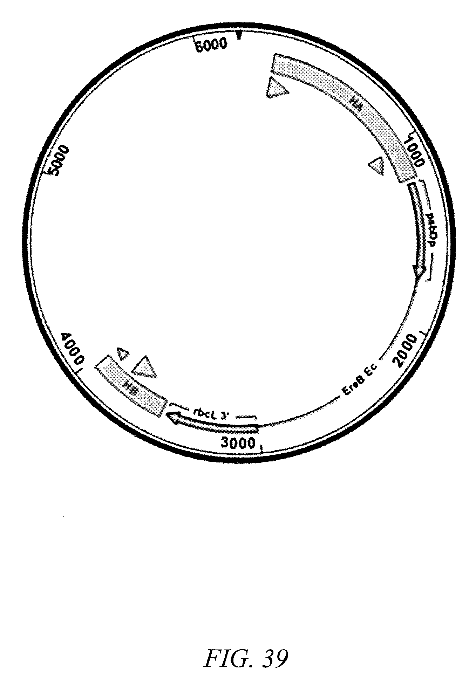

In this example, a nucleic acid encoding erythromycin esterase gene (EreB) (SEQ ID NO: 29) from *E. coli* was introduced into *D. tertiolecta.* Transforming DNA is shown graphically in **Figure 39****.** In this instance the DNA segment labeled "EreB ec" is the erythromycin esterase gene (EreB) from *E. coli,* the segment labeled "psbDp" is the promoter and 5' UTR sequence for the *psbD* or *tufA* gene from a D. *tertiolecta* (SEQ ID NO: 62, psbD2, SEQ ID NO: 63, tufA2), and the segment labeled "rbcL 3' " is the 3' UTR for the *rbcL* gene from *D. tertiolecta* (SEQ ID NO: 64, 2rbcL 3'). The selection marker cassette is targeted to the *D. tertiolecta* chloroplast genome via the segments labeled "HA" and "HB" which are approximately 1000 bp fragments homologous to sequences of DNA in the psbB-psbT-psbN-psbH cluster (SEQ ID NO: 133) wherein the EreB cassette is inserted between psbT and psbN at approximately nucleotide 2383 of SEQ ID NO: 133. All DNA segments were subcloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297,192-208, 1998.

Transforming DNA was introduced into *D. tertiolecta* via particle bombardment according to the method described in **EXAMPLE 17** with DNA carried on 550 nm gold particles @ 300psi and a shooting distance of 4cm. Transformants were selected by growth on G32 agar medium + 75 µg/mL erythromycin (G32-Erm) under constant light 50-100uE @ RT for approximately 4 weeks. Transformants were inoculated into nonselective G32 media and grown for ∼1week under constant light (50-100uE).

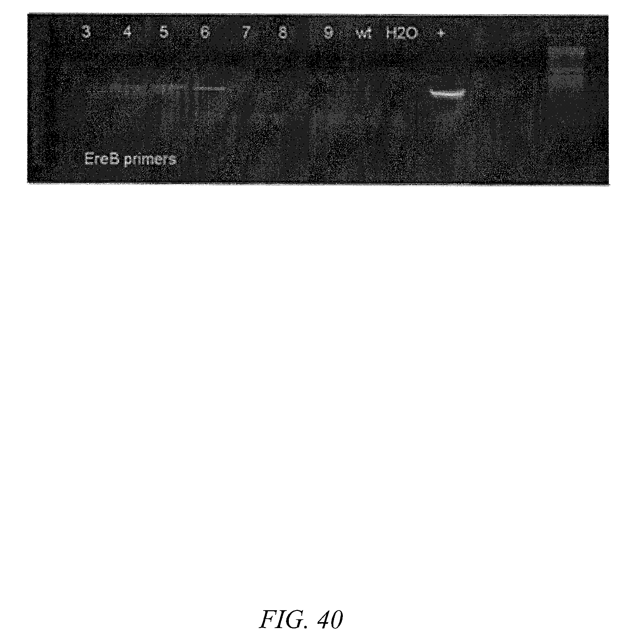

Cells from the transformants were analyzed by PCR screening (as described in **EXAMPLE 17).** The presence of the EreB selection marker was determined using primers that amplify a 555 bp region within the gene (SEQ ID NO: 7 and SEQ ID NO: 8). **Figure 40** shows that the EreB gene was amplified from DNA from transformants 4, 5, and 6 but not from wildtype DNA from *D. tertiolecta.*

These data demonstrate that the chloroplast *ofD. tertiolecta* can be transformed with foreign DNA containing an expression cassette with a selectable marker. One of skill in the art will appreciate that many other methods known in the art may be substituted in lieu of the ones specifically described or referenced.

### Example 19: Production of Endoxylanase in D. tertiolecta

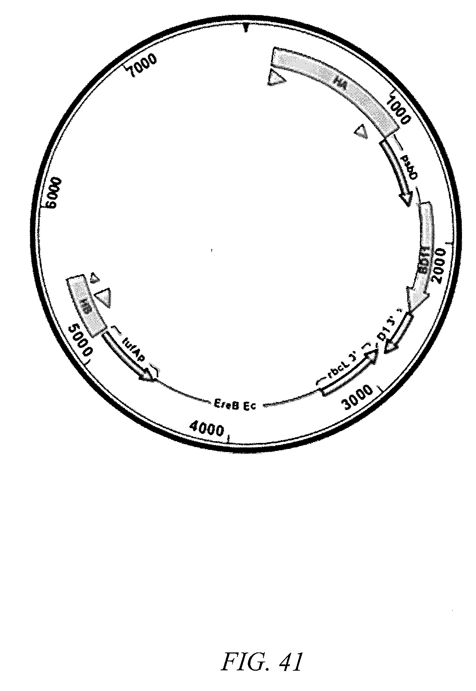

In this example a nucleic acid encoding endoxylanase from *T. reesei* was introduced into *D. tertiolecta.* Transforming DNA is shown graphically in **Figure 41****.** In this instance the DNA segment labeled "BD11" is the endoxylanase encoding gene (SEQ ID NO: 21, BD11), the segment labeled "psbD" is the promoter and 5' UTR for the *psbD* gene from *D. tertiolecta,* the segment labeled "D1 3"' is the 3' UTR for the *psbA* gene from *D. viridis* (SEQ ID NO: 65, 3 psbA 3'), and the segment labeled "EreB ec" is the erythromycin esterase gene from *E. coli,* which is regulated by the promoter and 5' UTR sequence for the *tufA* gene from *D. tertiolecta* and the 3' UTR sequence for the *rbcL* gene from *D. tertiolecta.* The transgene expression cassette and selection marker are targeted to the *D. tertiolecta* chloroplast genome via the segments labeled "HA" and "HB" which are approximately 1000 bp fragments homologous to sequences of DNA in the psbB-psbT-psbN-psbH cluster wherein the transgene cassette is inserted between psbT and psbN. All DNA segments were subcloned into pUC 19. All DNA manipulations carried out in the construction of this transforming DNA were essentially as described by Sambrook et al., Molecular Cloning: A Laboratory Manual (Cold Spring Harbor Laboratory Press 1989) and Cohen et al., Meth. Enzymol. 297,192-208, 1998.

Transforming DNA was introduced into *D. tertiolecta* via particle bombardment according to the method described in **EXAMPLE 17** with DNA carried on 550 nm gold particles @ 400 and a shooting distance of 4cm. Transformants were selected by growth on G32 agar medium + 75 µg/mL erythromycin (G32-Erm) under constant light 50-100uE @ RT for approximately 4 weeks. Transformants were inoculated into G32 media + 100ug/mL erythromycin and grown for ∼1week under constant light (50-100uE).

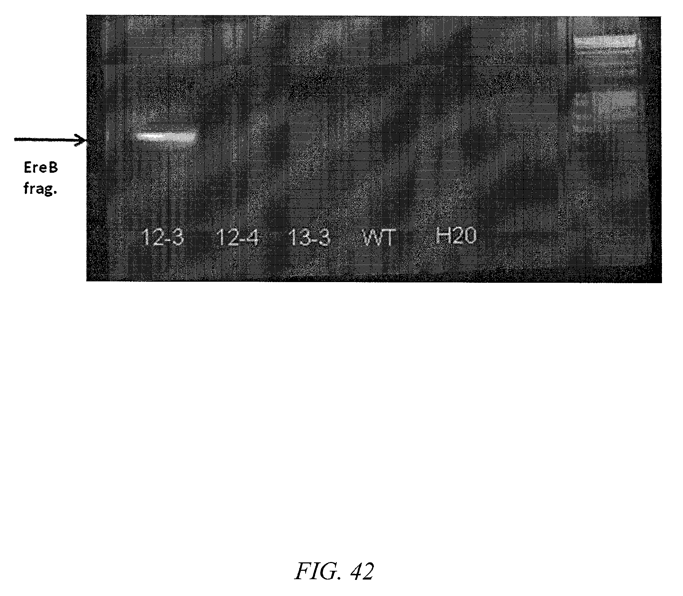

Cells from the transformants were analyzed by PCR screening (as described in **EXAMPLE 17).** The presence of the EreB selection marker was determined using primers that amplify a 555bp region within the gene (SEQ ID NO: 7and SEQ ID NO: 8). **Figure 42** shows that the EreB gene was amplified from DNA from transformant 12-3 but not from wildtype DNA from *D. tertiolecta.*

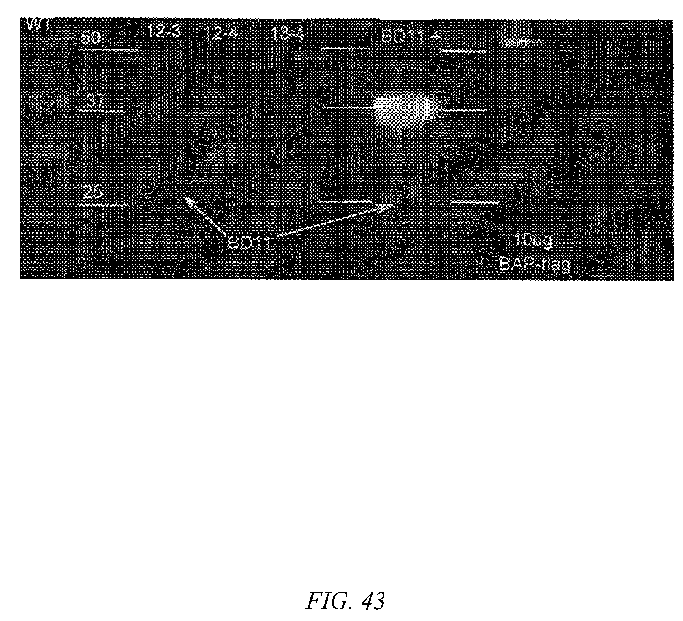

To ensure that the presence of the endoxylanase-encoding gene led to expression of the endoxylanase protein, a Western blot was performed (as described in **EXAMPLE 4).** Results from transformant 12-3 **(****Figure 43****)** show that expression of the endoxylanase gene in *D. tertiolecta* cells resulted in production of the protein.

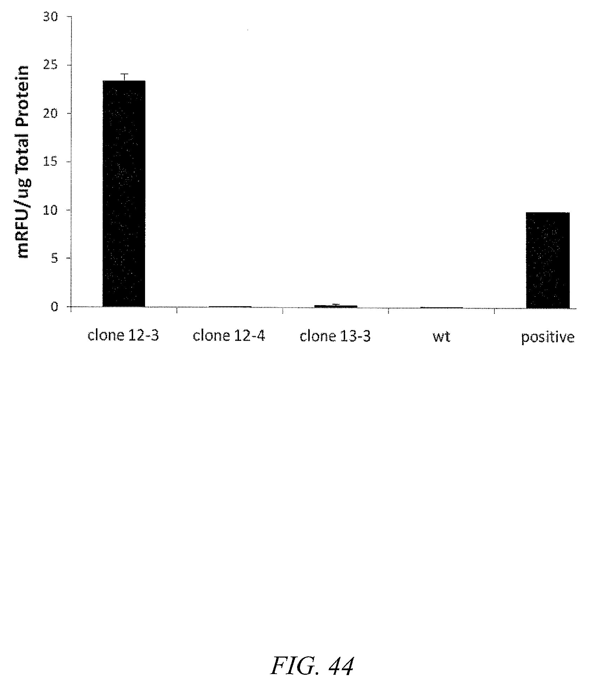

To determine if the endoxylanase produced by transformed algae cells was functional, endoxylanase activity was tested using an enzyme function assay (as described in **EXAMPLE 4).** **Figure 44** shows that endoxylanse activity is detected in the 12-3 transformant but not in wildtype cells.

These data demonstrate that the chloroplast ofD. *tertiolecta* can be transformed with foreign DNA containing an expression cassette with a selectable marker and a separate expression cassette with a gene encoding an endoxylanase, and that the proteins expressed were functional. One of skill in the art will appreciate that many other methods known in the art may be substituted in lieu of the ones specifically described or referenced.

### Example 20: Overview of Genetic Engineering

To engineer the chloroplast of an algae three things are required: a cassette expressing a selectable marker; a delivery method to deliver the plasmid DNA into the chloroplast; and a vector containing regions of DNA homologous to the chloroplast genome to be used in targeted homologous recombination (a homologous integration vector or homologous recombination vector).

In strains of algae that have little or no known chloroplast sequence information available, the identification of homologous regions of DNA and the construction of a vector containing those regions, are significant and time consuming tasks. Current methods for obtaining unknown sequence information, such as Inverse PCR (PCR Cloning Protocols Series: Methods in Molecular Biology, Volume: 192, Pub. Date: Apr-01-2002, Page Range: 301-307, DOI: 10.1385/1-59259-177-9:301) and Adaptor Ligated PCR (Nature Protocols 2, pp. 2910 - 2917 (2007) Published online: 8 November 2007) are time consuming in that they take multiple iterations in order to generate a DNA sequence that is long enough to be used in a homologous integration vector.

A method that allows for the quick identification of a large piece of chloroplast DNA sequence, sufficient in size to build a homologous integration vector, would be very useful in the engineering of algal genomes. The methods described herein, can be applied to all strains of an algae, for example, a green algae, for which there is little or no known DNA sequence information available. The methods described herein, can also be applied to an algae, for which there is incomplete sequence information available.

### Example 21: Use of a Conserved Gene Cluster to Generate Sequence Information

Across the chloroplast genomes sequenced to date, there are only a few clusters of genes that are consistently found adjacent to each other. Two examples of such gene clusters are ycf3-ycf4 and psbF-psbL. However, these two clusters are too small is size to yield enough DNA sequence information to be useful for homologous recombination.

Another gene cluster, psbB-psbT-psbN-psbH, is found together in the same orientation in most algae and plants. Knowledge of the presence of this gene cluster allows one to amplify a large region of chloroplast DNA that provides enough DNA sequence information to construct a vector for homologous recombination. This vector can then be used to modify the chloroplast genome of algal strains and plants that have not yet been genetically engineered.

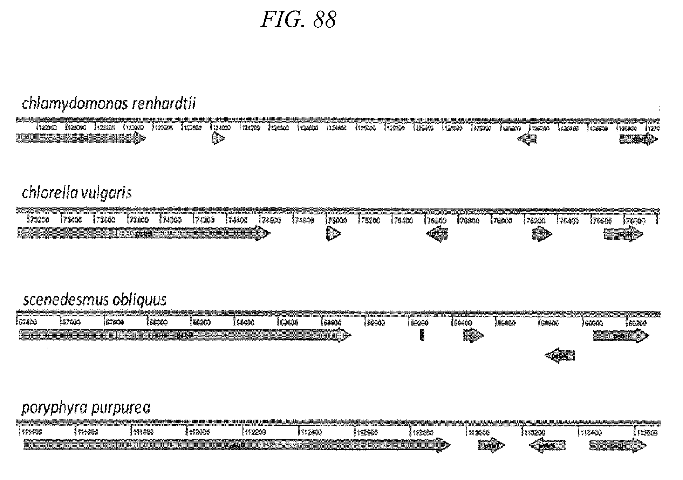

The gene cluster psbB-psbT-psbN-psbH is a region of chloroplast DNA that is highly conserved amongst algae and plants. However, this cluster may not be conserved at the nucleic acid level or in the spacing between the genes (the intergenic regions). In addition, the nucleic acid contents of the intergenic regions may vary. While at the nucleotide level there may be significant diversity, at the protein level this region is quite conserved. Figure 88 is an alignment of 4 algae that have had their chlorolast genomes sequenced: C. *reinhardtii* (NCBI NC_005353), *C. vulgaris* (NCBI NC_001865), *S. obliquus* (NCBI NC_008101), and *P. purpurea* NCBI NC_000925). This figure shows the high degree of conservation in terms of gene placement and orientation.

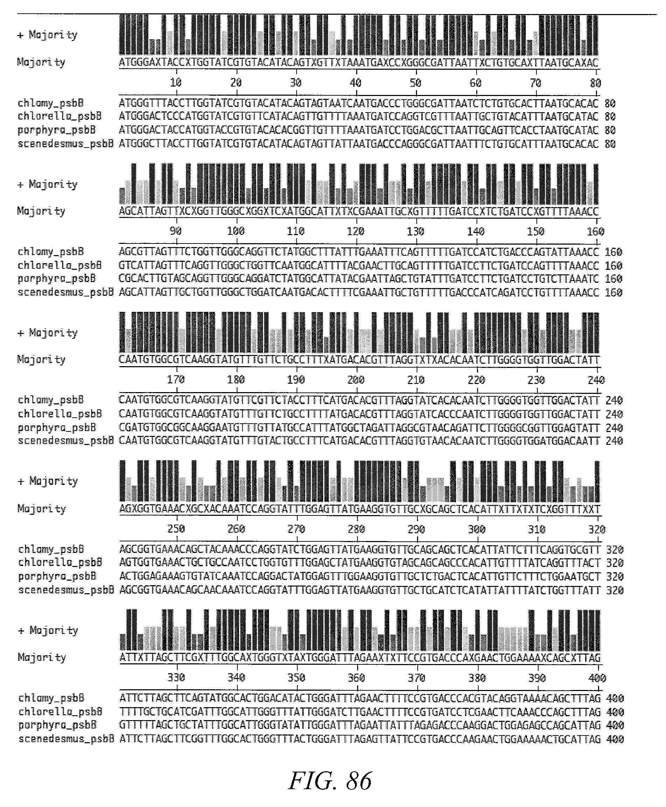

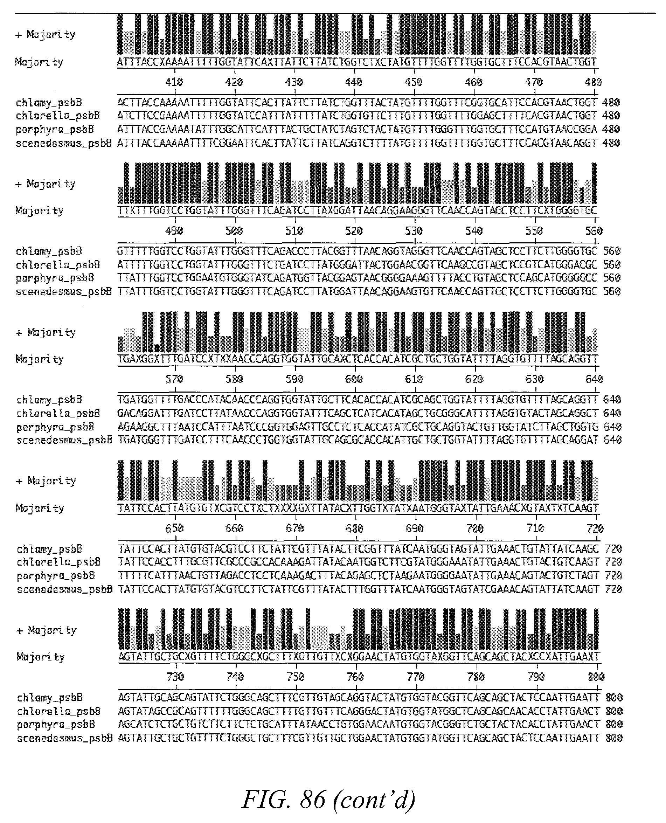

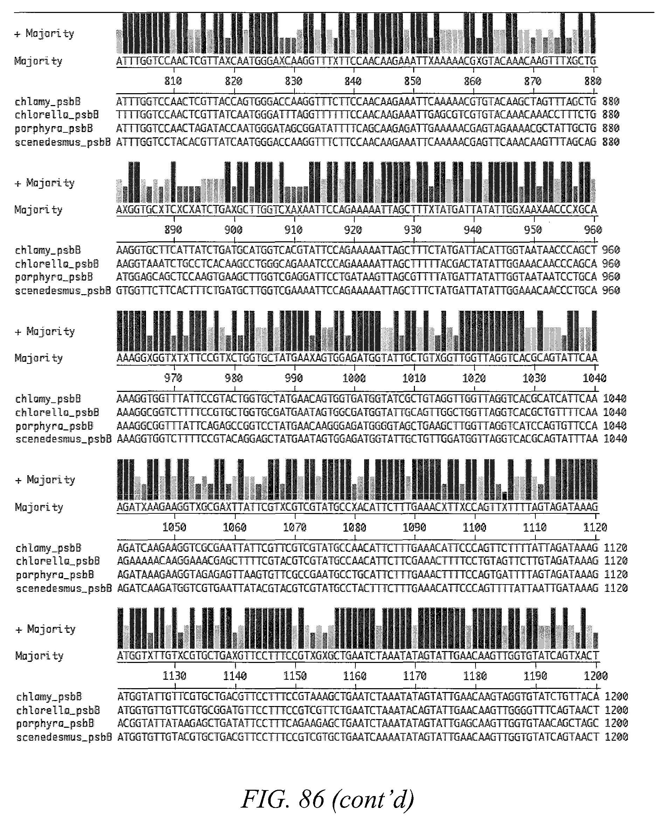

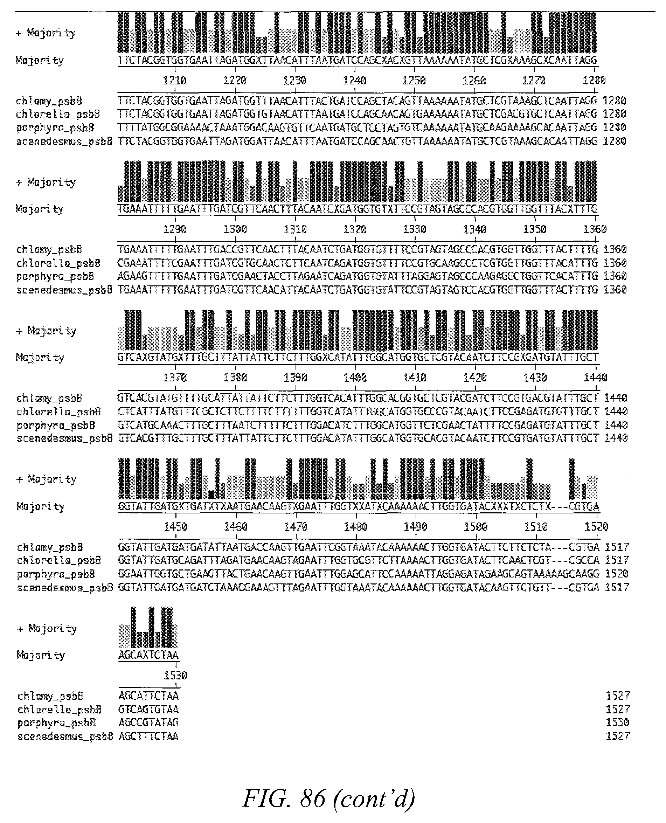

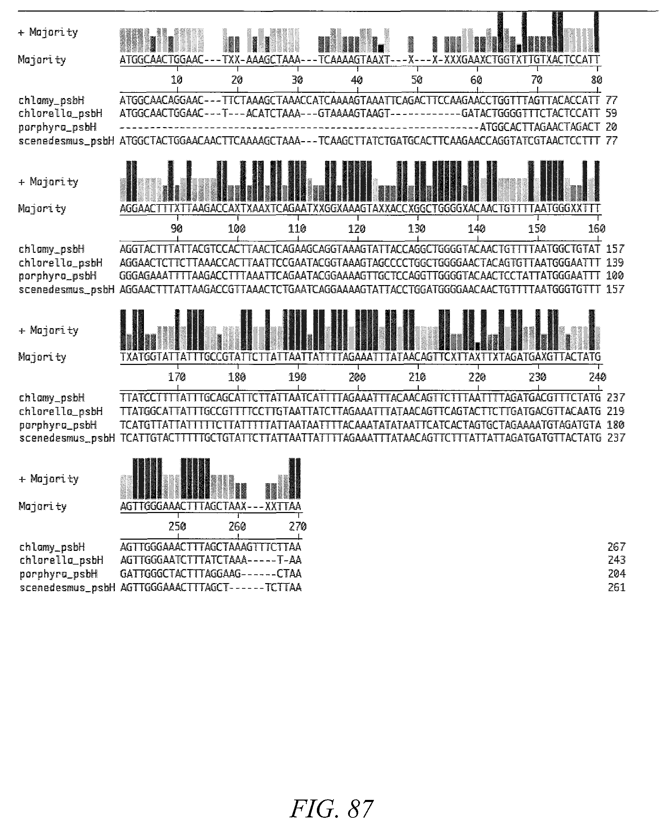

Although the gene cluster, psbB-psbT-psbN-psbH, may not be conserved at the nucleic acid level, the proteins on the terminal ends of this region, psbB and psbH, are highly conserved at the amino acid level and contain regions of high conservation at the nucleotide level. **Figure 86** is an alignment of the *psbB* gene from four algae that have had their chlorolast genomes sequenced: C. *reinhardtii, C. vulgaris*, *S. obliquus*, and *P. purpurea*, and **Figure 87** is an alignment of the *psbH* gene from the same algae. Both figures show regions of high nucleic acid homology. This allows for the design of degenerate primers that will anneal to regions within the nucleic acid sequences encoding for the proteins psbB and psbH, resulting in the amplification of the whole gene cluster in one step. This double stranded product can then be quickly sequenced directly from both ends, and enough sequence information can then be generated to construct a homologous recombination vector. The time it takes to generate the sequence data is much less than with other methods.

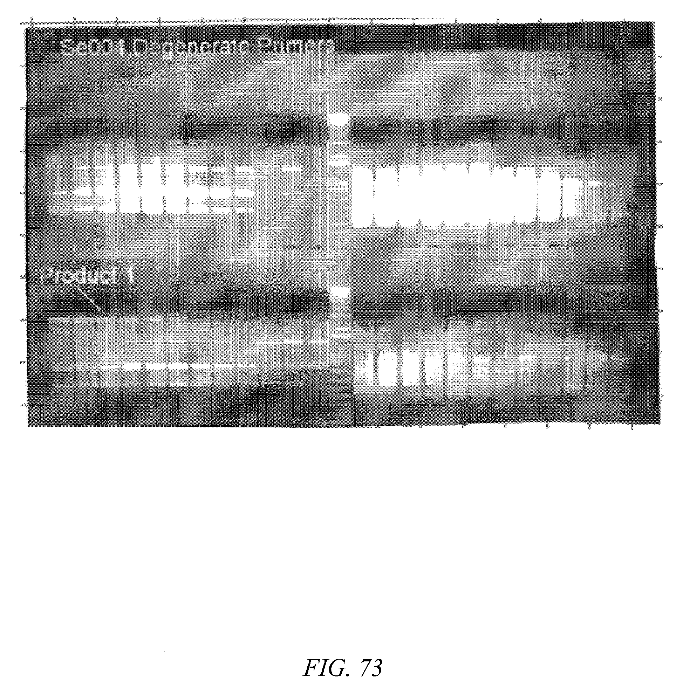

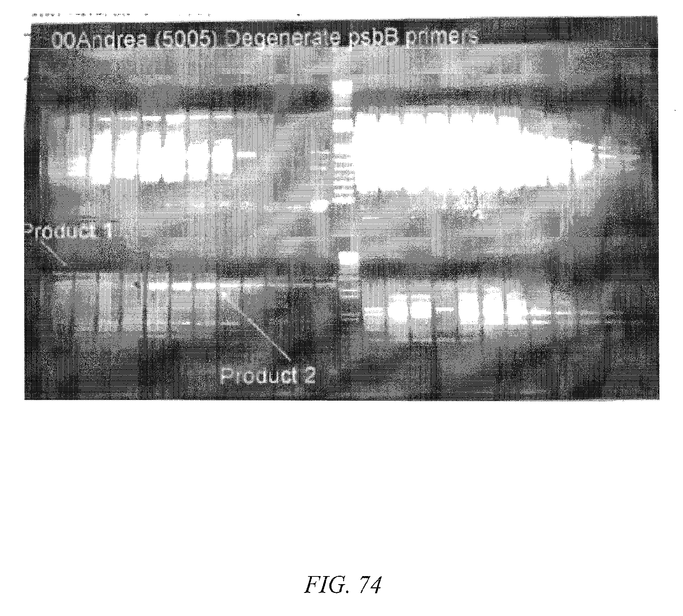

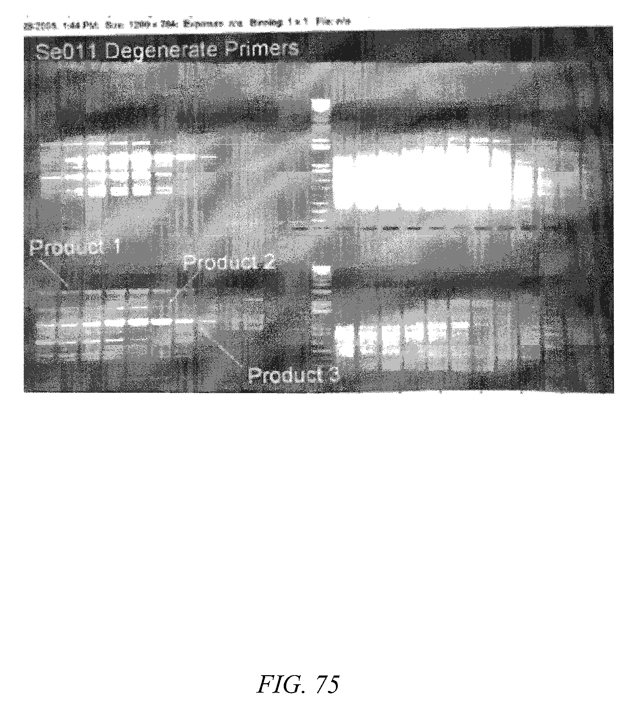

Two degenerate primers (forward primers 4099 and 4100) specific to the *psbB* gene (reverse primers SEQ ID NO: 129 and SEQ ID NO: 130) and 2 two degenerate primers (4101 and 4102) specific to the *psbH* gene (SEQ ID NO: 131 and SEQ ID NO: 132) were designed from the conserved nucleotide regions of the *psbB* and *psbH.* These primers have been used to generate the sequence of the psbB-psbT-psbN-psbH gene cluster from different species of algae that have little or no sequence information available in public databases including *D. tertiolecta* (SEQ ID NO: 133), an alga from the genus *Dunaliella* of unknown species (SEQ ID NO: 134), *N. abudans* (SEQ ID NO: 135) , an isolate of *C*. *vulgaris* differing from the published genome (SEQ ID NO: 136), and *T. suecica* (SEQ ID NO: 137). **Figures 74** **and** **75** show the degenerate primers amplifying a large fragment from the *Dunaliella* isolate and *N. abudans*, respectively. **Figure 73** shows the amplification from *S. dimorphus.* In each of these figures the center lane is occupied by a 1kb Plus ladder (Invitrogen). In each figure four different combinations of primers were used. The top left panel shows amplification with primers 4099 and 4101. The bottom left panel shows amplification with primers 4099 and 4102. The top right panel shows amplification with primers 4100 and 4101. The bottom left panel shows amplification with primers 4100 and 4102. After amplification the desired fragments are gel purified using the Qiaquick Gel Extraction Kit (Qiagen) and sequenced. In each of **Figures 73 to 75****,** Product 1 represents the full length psbB-pbsBH gene cluster.

An integration vector built from this region has been shown to transform *Dunaliella tertiolecta* (see **EXAMPLE 18).**

### Example 22: Additional Vectors constructed for Scenedesmus dimorphus

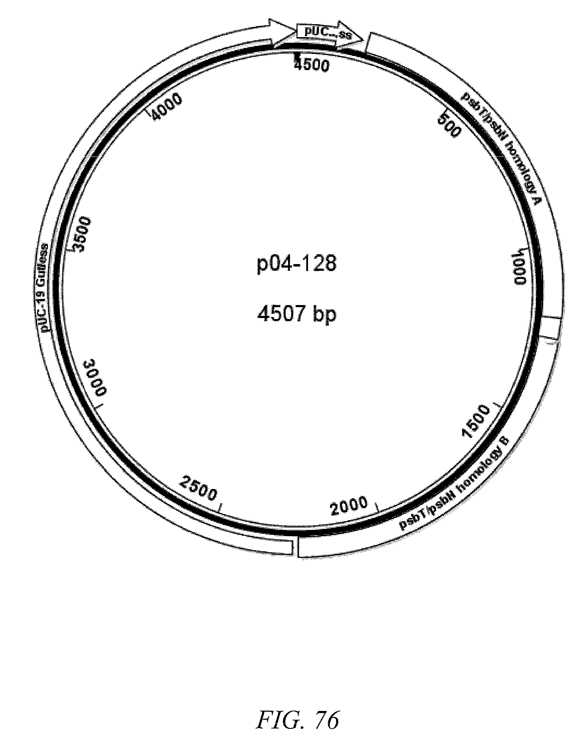

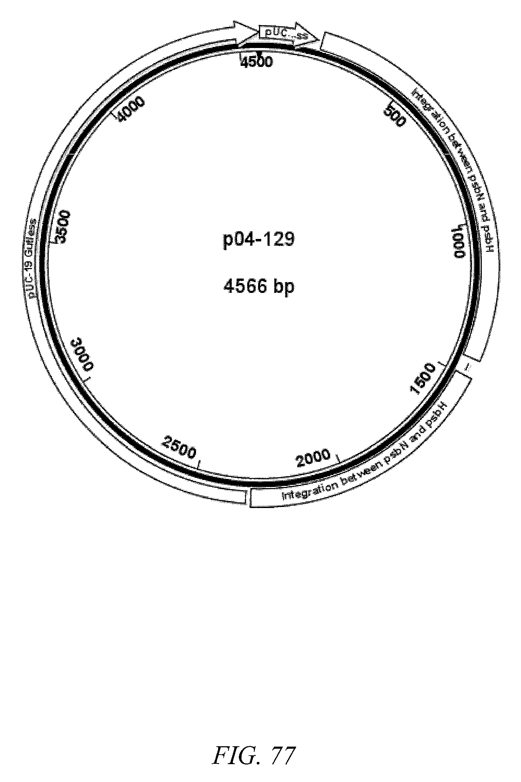

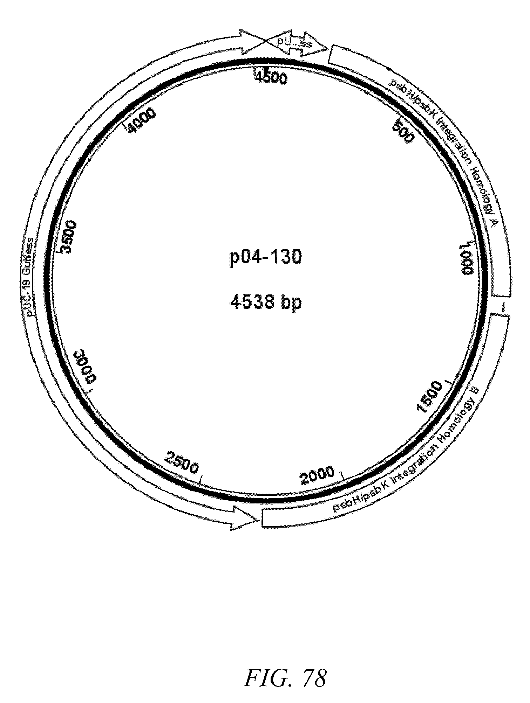

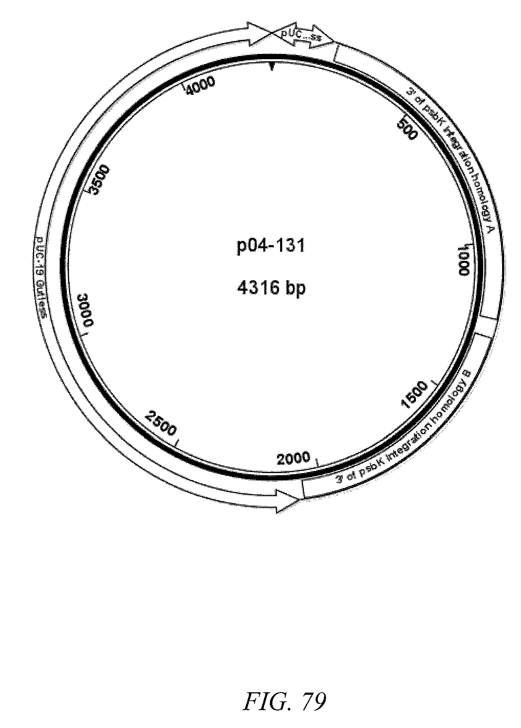

Additional vectors were constructed for *Scenedesmus dimorphus* since the sequence of a closely related species *Scenedesmus obliquus* is publicly available (NCBI for *S. obliquus*, NC_008101). These vectors were made to test integration sites and homoplasmicity along the entire region of psbB-psbT-psbN-psbH, as well as the next adjacent protein in *S. dimorphus,* psbK. This set of vectors targeted integration into the intergenic region between psbT and psbN, psbN and psbH, psbH and psbK, and the region 3' of psbK (p04-128, p04-129, p04-130, and p04-131 respectively) (**Figures 76** to **79** respectively). p04-128 targets integration at approximately nucleotide 059,587. p04-129 targets integration at approximately nucleotide 059,999. p04-130 targets integration at approximately nucleotide 060,429. p04-131 targets integration at approximately nucleotide 060,961 (nucleotide locations according to the sequence available from NCBI for *S*. *obliquus*, NC_008101).

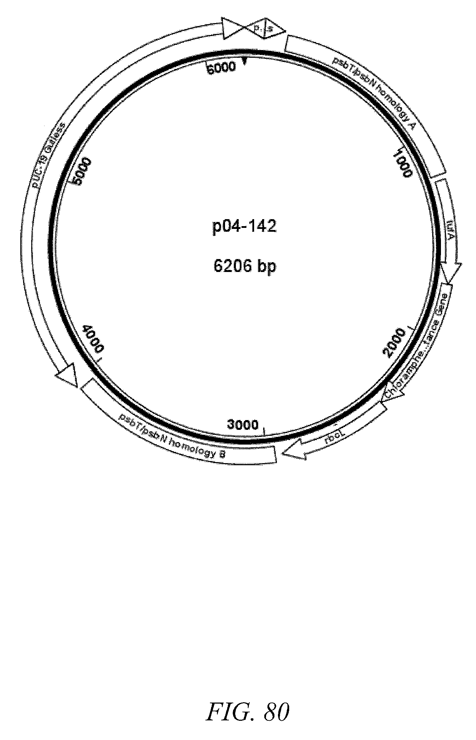

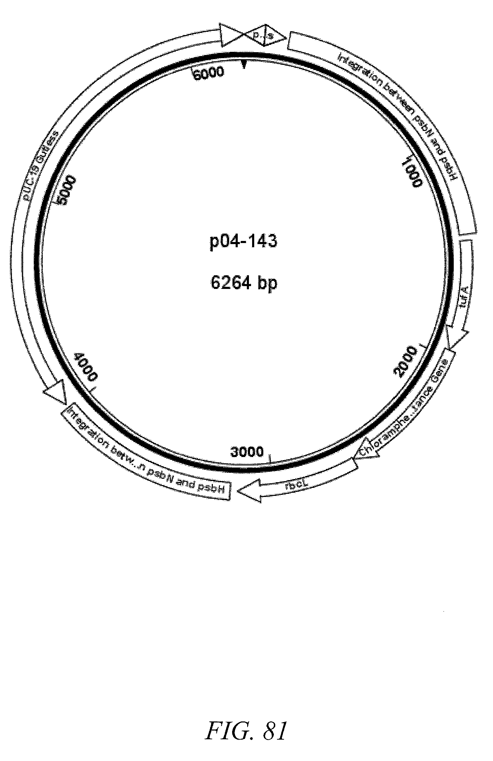

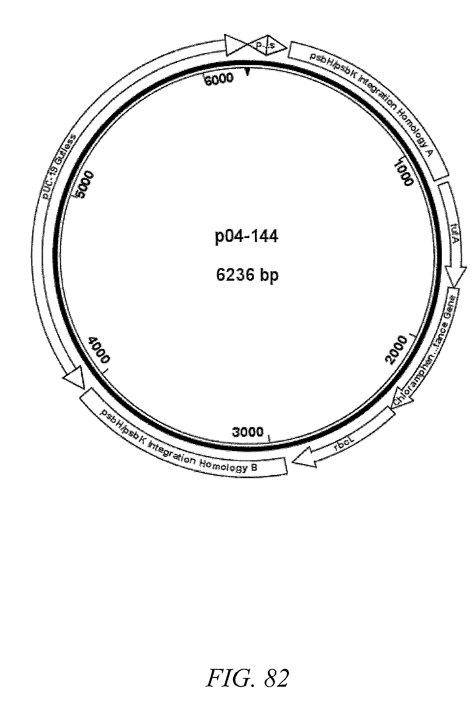

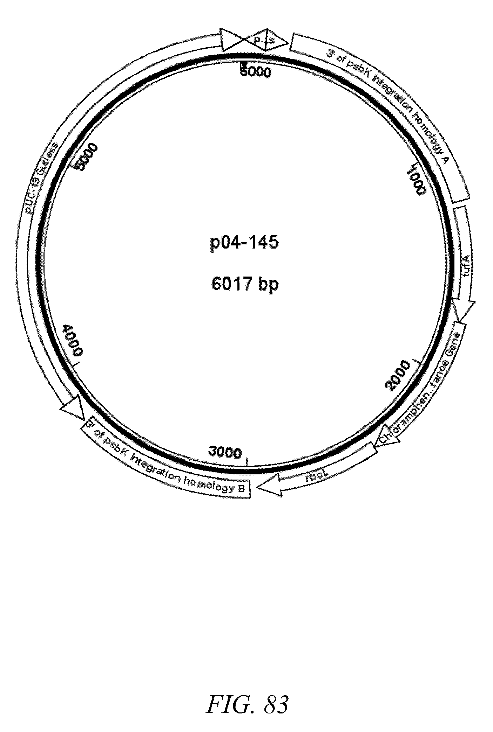

All vectors have an expression cassette consisting of a chloramphenicol (CAT) selectable marker, an endogenous promoter, and an endogenous terminator cloned between the Homology A and Homology B fragments. p04-128 had tufA-CAT-rbcL cloned between the Homology A and Homology B fragments (p04-142)(**Figure 80**). p04-129 had tufA-CAT-rbcL cloned between the Homology A and Homology B fragments (p04-143) (**Figure 81**). p04-130 had tufA-CAT-rbcL cloned between the Homology A and Homology B fragments (p04-144) (**Figure 82**). p04-131 had tufA-CAT-rbcL cloned between the Homology A and Homology B fragments (p04-145) (**Figure 83**). Vectors are shown graphically in their corresponding figures. In this instance the DNA segment labeled "CAT" is the chloramphenicol acetyl transferase gene from *E. coli,* the segment labeled "tufA" is the promoter and 5' UTR sequence for the *tufA* gene from *S. dimorphus,* and the segment labeled "rbcL" is the 3' UTR for the *rbcL* gene from *S. dimorphus.*

Transforming DNA was introduced into *S. dimorphus* via particle bombardment according to the method described in **EXAMPLE 1** with DNA carried on 550 nm gold particles@ 500psi and a shooting distance of 4cm. Transformants were selected by growth on TAP-CAM agar medium under constant light 50-100uE @ RT for approximately 2 weeks. Transformants were streaked onto TAP-CAM agar medium to ensure single colony isolation and grown for 4 days under constant light.

To test for integration of the CAT gene in between the *psbT* and *psbN* genes (p04-142), clones were screened for homoplasmicity using primers 3160 and 3162 (that amplify a 200bp constant band from the genome), and primers 4682 and 4982 (that amplify a 400 bp band that spanning the integration site).

To test for integration of the CAT gene in between the *psbN* and *psbH* genes (p04-143), clones were screened for homoplasmicity using primers 2922 and 2923 that amplify a 400bp constant band from the genome, and primers 4684 and 4685, that amplify a 200 bp band that spans the integration site.

To test for integration of the CAT gene in between the *psbH* and *psbK* genes (p04-144), clones were screened for homoplasmicity using primers 2922 and 2923 that amplify a 400bp constant band from the genome, and primers 4686 and 4687, that amplify a 300 bp band that spans the integration site.

To test for integration of the CAT gene 3' of the *psbK* gene (p04-145), clones were screened for homoplasmicity using primers 3160 and 3162 that amplify a 200bp constant band from the genome, and primers 4688 and 4689 amplify a 300 bp band that spans the integration site.

Primers used for each of the PCR screens are listed in **Table 5.**

**Table 5**

| | |
|---|---|
| **p04-142** | 3160 (SEQ ID NO: 3) GAACTACAACTAATTATTTTC |
| | 3162 (SEQ ID NO: 4) TGAAACCAGTCTTTGTAAAGCTCA |
| | 4682 (SEQ ID NO: 15) CCACCTCGTATGGTAAAATAATTG |
| | 4982 (SEQ ID NO: 16) GAAAGAATTATGGACAGTCCTGCT |
| **p04-143** | 2922 (SEQ ID NO: 1) AGAAGGAGCTTCTACAGATGC |
| | 2923 (SEQ ID NO: 2) TCATTAGTTACTTCATCTTTAATCCG |
| | 4684 (SEQ ID NO: 140) GAAGGAGGTCCAAAACTCACA |
| | 4685 (SEQ ID NO: 141) CCTGGTTCTTGAAGTGCATC |
| **p04-144** | 2922 (see above) |
| | 2923 (see above) |
| | 4686 (SEQ ID NO: 142) TGAGTTGGGAAACTTTAGCTTCTT |
| | 4787 (SEQ ID NO: 143) AAAAGATTGCCAAGACCAAA |
| **p04-145** | 3160 (see above) |
| | 3162 (see above) |
| | 4688 (SEQ ID NO: 144) AAAAAGAATGAAATTTTTATGTTCG |
| | 4689 (SEQ ID NO: 145) ATGGATGTCGTCCTCCAAAA |

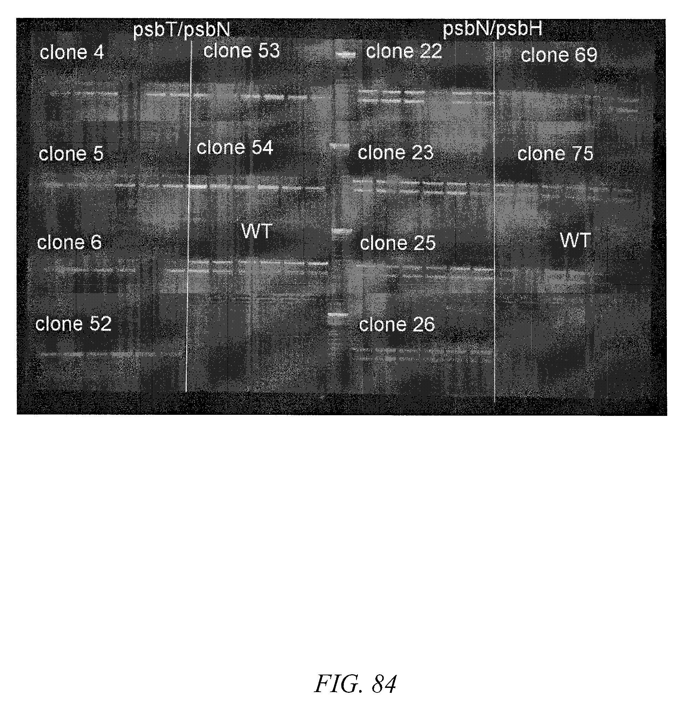

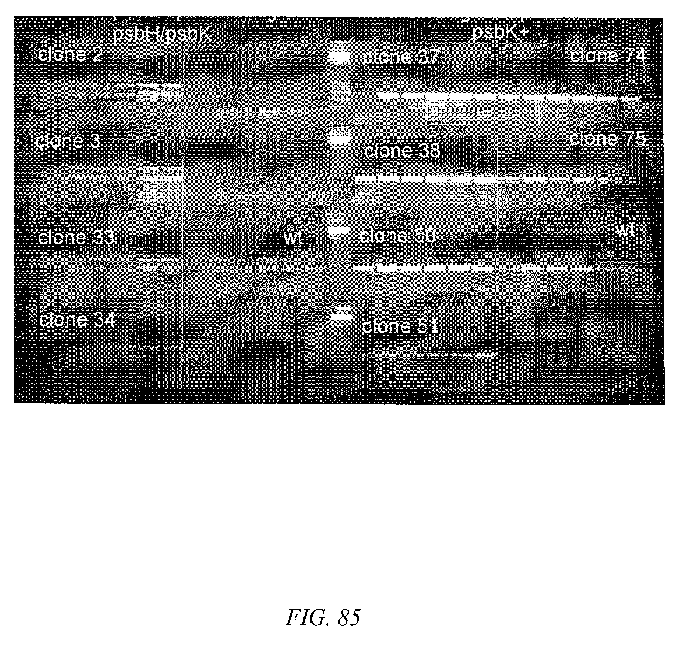

**Figure 84** and **Figure 85** shows that homoplasmic clones are recovered from integration between psbT and psbN (p04-142) and integration 3' ofpsbK (p04-145).

### Example 23: Creation of a Yeast-bacteria Shuttle Vector

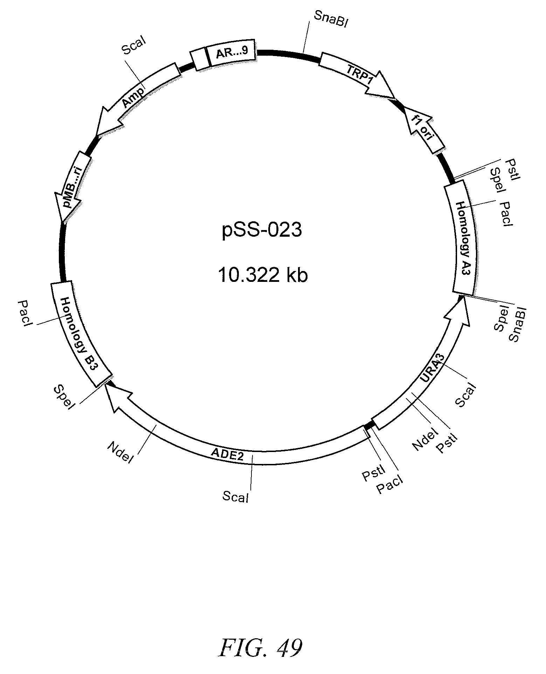

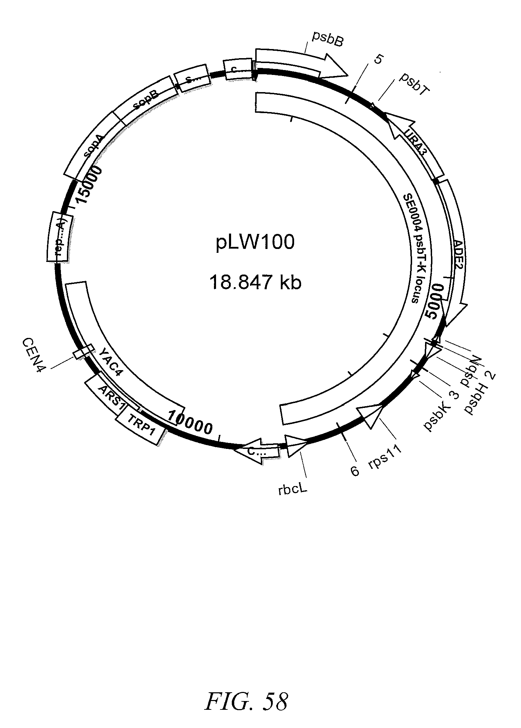

Heterologous (exogenous) gene introduction into the chloroplast by homologous recombination is efficient when a selectable marker and the gene of interest is flanked by 5' and 3' homology to a locus that can tolerate integration. To integrate more than one gene, one can target a separate locus and use a second selectable marker. Integration of two or more genes is problematic from a time and labor standpoint. In addition, availability of selectable markers becomes an issue. To contend with these issues, a yeast-based system was created wherein, in a single step, several exogenous genes can be assembled along with an algal selectable marker, and placed into a yeast-bacteria shuttle vector. Two versions of this vector were created. One version contains a 5.2 kb region from the *Scenedesmus obliquus* chloroplast (*Scenedesmus* chloroplast sequence NCBI reference sequence: NC_008101, 057,611-062850bp) (SEQ ID NO: 125). This 5.2 kb region is highly conserved (at the amino acid level) amongst algae species, and spans a region comprising psbB to rbcL genes. The second version of this vector contains two 1,000 bp "homology A3" (070,433-071,342bp) (SEQ ID NO: 126) and "homology B3" (071,379-072,254bp) (SEQ ID NO: 127) regions which target a locus immediately downstream of the psbA gene. The two shuttle vectors (Figures 49 and 58) comprise the above-mentioned sequences from the chloroplast genome of *Scenedesmus obliquus,* bacterial replication/selection elements, and yeast replication, segregration, and selection/counter-selection elements.

There are at least four advantages of the yeast-based system over the existing technology: 1) each of the 1, 2, 3, 4, or more gene expression cassettes can be amplified with primers containing 5' and 3' homology to adjacent cassettes, thereby alleviating the requirement to clone flanking homology into the gene cassette design; 2) several gene cassettes (for example, 2, 3, 4, 5, 6, or 20) can be assembled together as a contig in a single step and require a single selectable marker for chloroplast introduction; 3) this technology can be applied to other algal species due to the conserved nature of the psbB-rbcL locus across algae species; and 4) the 5.2 kb of homology contained within the shuttle vector (**Figure 58**) and the 2 kb of homology as shown in **Figure 49**, ensures that homologous recombination is accurate and efficient within the chloroplast.

It should be noted that, for example, more than 2, more than 5, more than 10, more than 15, more than 20, or more than 25 gene cassettes can be assembled in the shuttle vector.

### Example 24: Plasmid Construction

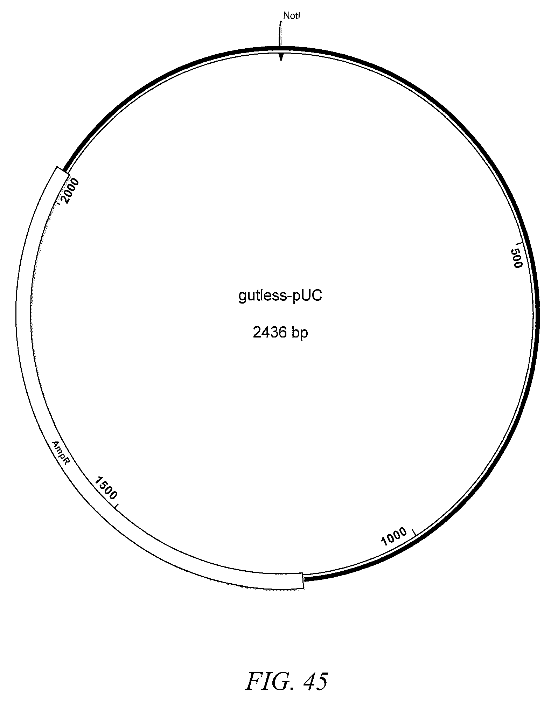

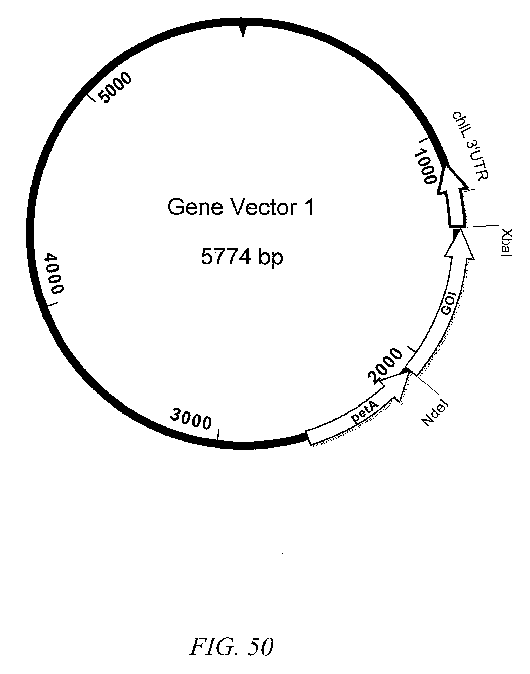

A derivative of plasmid vector pUC19 (New England Biolabs, U.S.A.; Yanisch-Perron, C., et al. (1985) Gene, 33, 103-119) lacking a multiple cloning site (herein referred to as gutless pUC) (**Figure 45**) was used to create the backbones for three gene expression cassettes. Three different gene expression cassettes comprising the promoter-terminator pairs: petA-chlL, D2-D1, and tufA-psaB, respectively were cloned into gutless pUC (**Figures 50**, **51** and **52**).

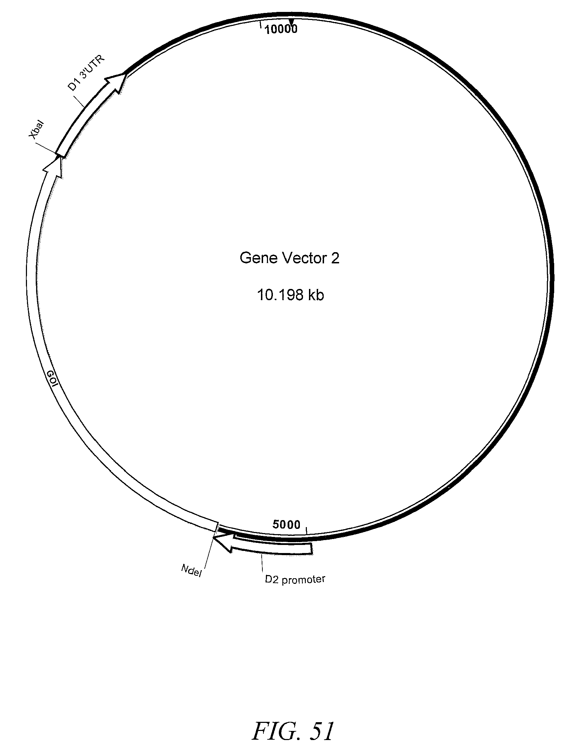

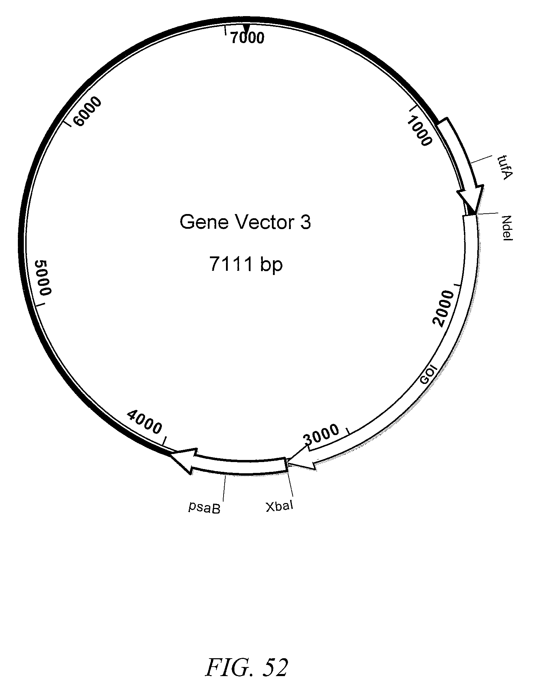

To insert the genes of interest ("GOI")(CC90, SEQ ID NO: 115; CC91, SEQ ID NO: 116; CC92, SEQ ID NO: 117; CC93, SEQ ID NO: 109; CC94, SEQ ID NO: 110; CC97, SEQ ID NO: 113; IS57, SEQ ID NO: 121; IS61, SEQ ID NO: 124; IS62, SEQ ID NO: 123; IS116, SEQ ID NO: 122; BD11, SEQ ID NO: 146; and IS99, SEQ ID NO: 147), each of the three vectors (Gene Vector 1 (**Figure 50**), Gene Vector 2 (**Figure 51**), and Gene Vector 3 (**Figure 52**)), along with the genes of interest, were double-digested with the restriction enzymes *NdeI* and *XbaI*, and ligated together resulting in 36 different vectors. Several of the 36 vectors served as PCR templates for the gene amplifications used in the 2-, 3-, or 4-gene contig assemblies described below.

The genes of interest are as follows:
CC90 glcD - glycolate oxidase subunit, FAD-linked NP_417453;
CC91 glcE - glycolate oxidase FAD binding subunit YP_026191;
CC92 glcF - glycolate oxidase iron-sulfur subunit YP_026190;
CC93 glyoxylate carboligase NP_415040;
CC94 tartronate semialdehyde reductase NP_417594; and
CC97 tartronate semialdehyde reductase - NADH dependent NP_415042.

These genes are described in Kebeish, R., et al., Nature Biotechnology (2007) 25(5) 593-599. All six genes are codon-optimized for the chloroplast genome of *Chlamydomonas reinhardtii.*

Additional genes of interest are as follows:
BD11 is an endoxylanase from T. reesei; and
IS99 is a mevalonate pyrophosphate decarboxylase from S. cerevisiae, codon optimized according to the tRNA usage of the C. *reinhardtii* chloroplast.

Other genes of interest are as follows:
IS57 is 1-Deoxy-D-xylulose 5-phosphate reductoisomerase (DXR);
IS-61 is Chlamydomonas chlorophyll synthase;
IS-62 is the same protein as IS-9, the chicken FPP synthase; the difference is that the C-terminal tag has been removed, and replaced with an N-terminal FLAG tag; and
IS-116 is 4-diphosphocytidyl-2-C- methylerythritol synthetase (CDP-ME synthase, it is the E. coli version of the gene).

These above four genes were all codon biased for expression in the Chlamydomonas chloroplast genome.

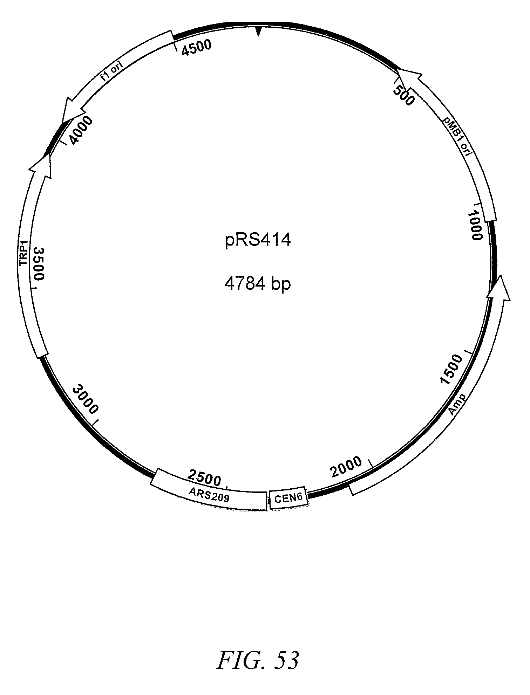

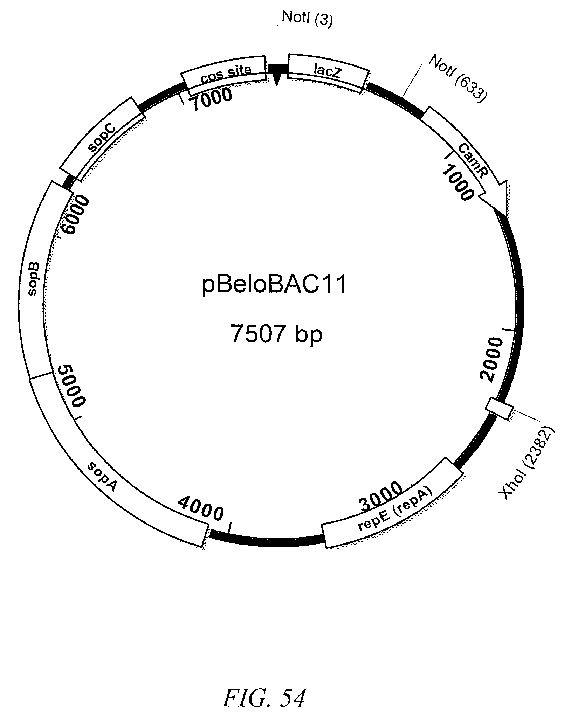

Plasmid vectors pRS414 (Sikorski and Hieter, Genetics. 1989 May;122(1): 19-27) (**Figure 53**) and pBeloBAC11 (NEB)(**Figure 54**) were used to construct transformation platform vectors. In all instances, pRS414, and Gene Vectors 1, 2, and 3 were selectively maintained in DH10B cells (Invitrogen, U.S.A.) by growth in Luria Bertani (LB) medium supplemented with 100µg/ml ampicillin. Similarly, the plasmid pBeloBAC11 was selectively maintained in its host bacterium, DH10B, by growth in LB medium supplemented with 12.5µg/ml chloramphenicol.

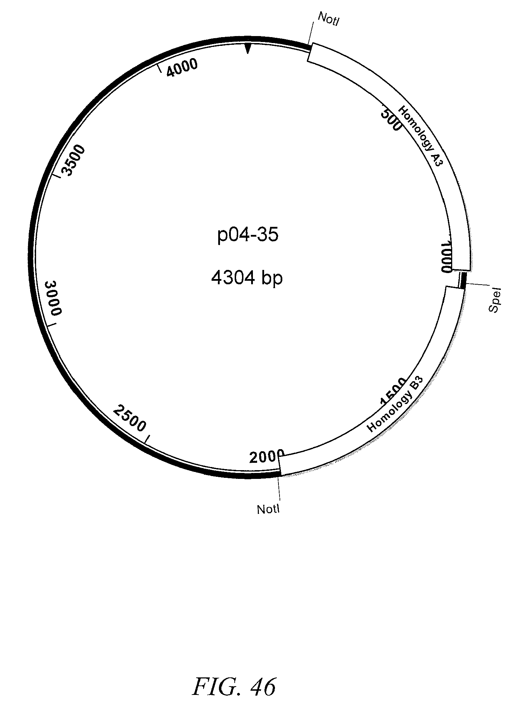

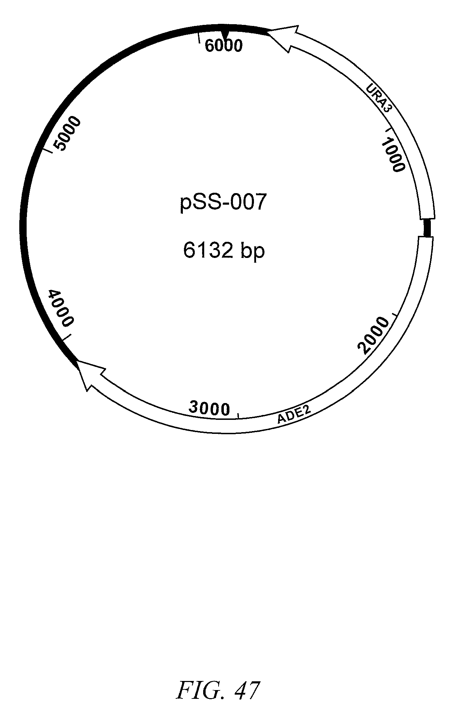

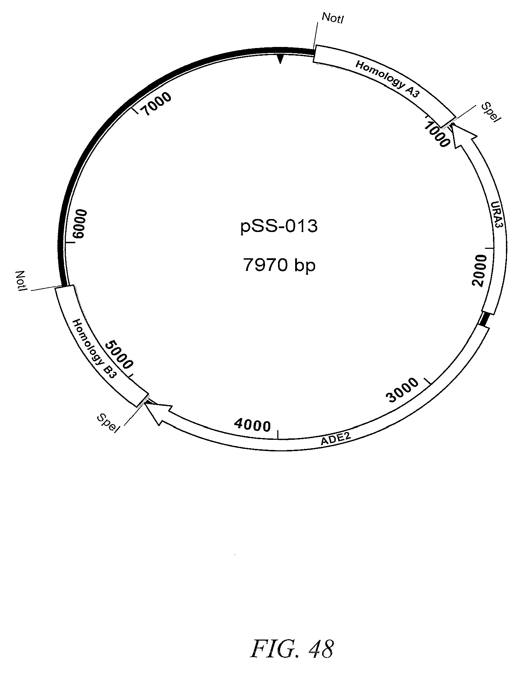

To construct the first of the two base platform vectors (**Figure 49**) that can be used for the introduction of two genes into the chloroplast of *Scenedesmus obliquus*, the homology region A3 (SEQ ID NO: 126) and the homology region B3 (SEQ ID NO: 127) were amplified from *Scenedesmus* chloroplast DNA using primers 34 (SEQ ID NO: 99) and 35(SEQ ID NO: 100), and 36 (SEQ ID NO: 101) and 37 (SEQ ID NO: 102), respectively, digested with *NotI* and *SpeI*, and ligated into *NotI* digested gutless pUC (**Figure 45**). Plasmid p04-35 (**Figure 46**) was then linearized with *SpeI* and ligated to a PCR product comprising the nucleotide sequence encoding the yeast genes URA3-ADE2 (SEQ ID NO: 105). The nucleotide sequence encoding the yeast genes URA3-ADE2 was obtained by PCR using as a DNA template, plasmid pSS-007 (**Figure 47**), and primers 30 (SEQ ID NO: 95) and primer 31 (SEQ ID NO: 96), which both contain *SpeI* restriction sites at their 5' termini. The resulting vector comprising the homology regions flanking the yeast genes (pSS-013) is shown in **Figure 48**.

The URA3-ADE2 cassette allows for positive selection in yeast that are deficient for *URA3* or *ADE2* gene function, respectively. Similarly, expression of the URA3 gene can be negatively selected against in the presence of 5-floroorotic acid (5-FOA) as URA3 converts 5-FOA to 5-fluorouracil, which is toxic to the cell. In addition, the presence or absence of a functional *ADE2* gene results in white or red yeast colonies, respectively - thereby allowing for another level of selection when picking colonies.

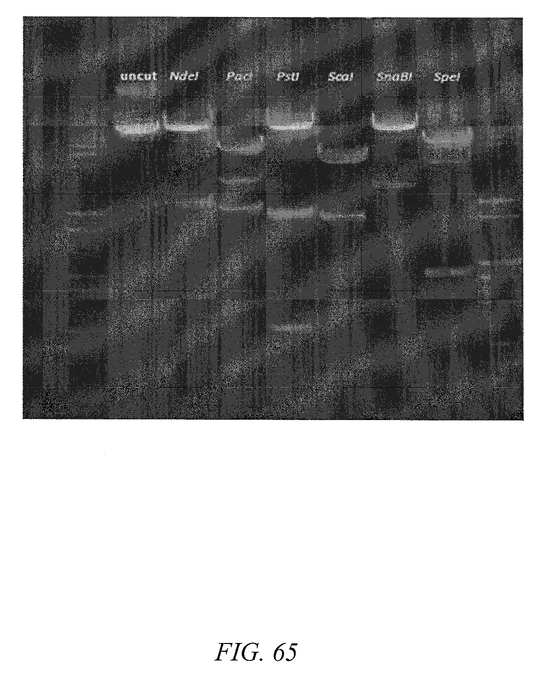

To create the yeast-bacterial shuttle vector for two-gene contig assembly, which targets the A3-B3 region, pSS-013 (**Figure 48**) was digested with *NotI*, liberating the fragment containing A3-URA3-ADE2-B3, which was then ligated into *NotI* digested pRS414 (**Figure 53**), resulting in the vector pSS-023 (**Figure 49**). pSS-023 was confirmed by sequencing and restriction digest mapping with *NdeI*, *PacI*, *PstI*, *ScaI, SnaBI*, and *SpeI* (**Figure 65**). Order of lanes from left to right: 1kb DNA plus ladder (Invitrogen), uncut pSS-023, *NdeI, PacI, PstI, ScaI, SnaBI, SpeI,* 1kb DNA plus ladder (Invitrogen, U.S.A.). Expected bands are as follows: NdeI, 2187 bp and 8135bp; PacI, 2051 bp, 2981 bp, and 5290bp; PstI, 493 bp, 1872 bp, and 7957 bp; ScaI, 1761 bp, 4050 bp, and 4511 bp; SnaBI, 2587 bp and 7735 bp; and SpeI, 950 bp, 3694 bp, and 5678 bp. pSS-023 was used in all two-gene contig assemblies that target homology A3 and homology B3 regions.

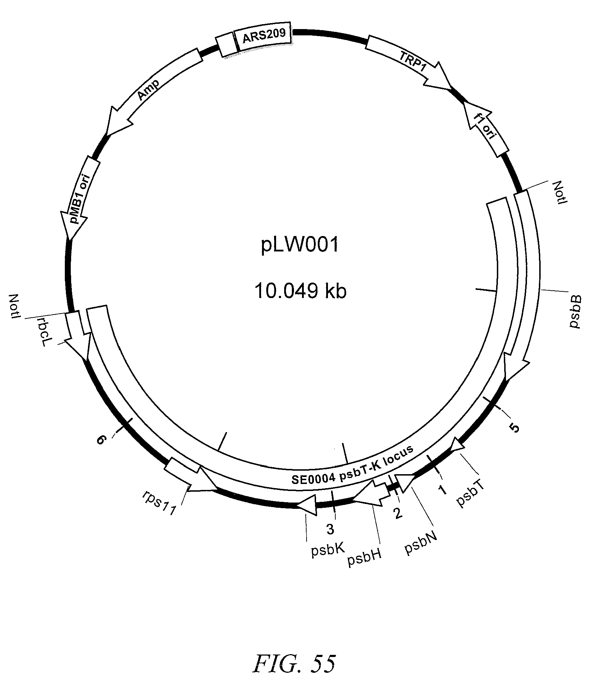

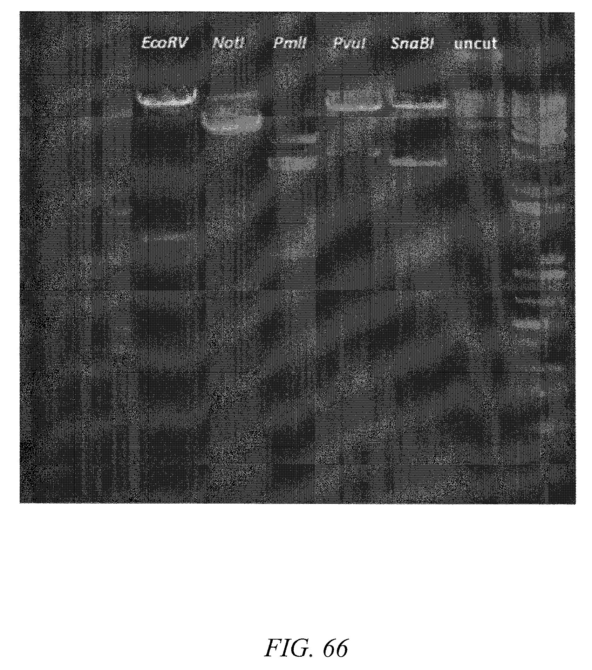

To construct the base platform vector used for the three-gene, four-gene, and the second two-gene contig assembly (which all target the psbB-rbcL locus in *Scenedesmus)*, primer 1 (SEQ ID NO: 66) and primer 2 (SEQ ID NO: 67), both of which contain *NotI* restriction sites at their 5' termini, were used to amplify the 5.2 kb sequence (SEQ ID NO: 125) spanning from the psbB gene to the rbcL gene. The resultant 5.2 kb PCR product and plasmid vector pRS414 (Figure 53) were both digested with *NotI* and ligated together, resulting in pLW001 (Figure 55). pLW001 was confirmed by sequencing and restriction digest mapping with *EcoRV, NotI, PmlI, PvuI*, and *SnaBI* (Figure 66). Order of lanes from left to right: 1kb DNA plus ladder (Invitrogen, U.S.A.), *EcoRV, NotI, PmlI, PvuI, SnaBI,* uncut, and 1kb DNA plus ladder (Invitrogen, U.S.A.). Expected bands are as follows: *EcoRV,* 1182 bp and 8867 bp; *NotI,* 4784 bp and 5265 bp; *PmlI,* 995 bp, 2644 bp, and 2695 bp; *PvuI*, 2868 bp and 7181 bp; and *SnaBI,* 2526 bp and 7523bp.

To assemble contigs of two, three, and four genes in pLW001, using negative selection, a PCR product containing the *Saccharomyces cerevisiae* genes URA3-ADE2 (SEQ ID NO: 105) was amplified with primer 27 (SEQ ID NO: 92) and primer 28 (SEQ ID NO: 93), which contain 5' tails homologous to the locus in the chloroplast sequence between psbT and psbN. This PCR product, along with pLW001 (**Figure 55**), were simultaneously transformed into *S. cerevisiae.* Transformants were selected for on complete synthetic media (CSM) lacking tryptophan, uracil, and adenine (CSM - TRP - URA - ADE) using a standard lithium acetate transformation protocol (for example, as described in Gietz, R.D. and Woods, R.A., Methods Enzymol. (2002) 350:87-96).

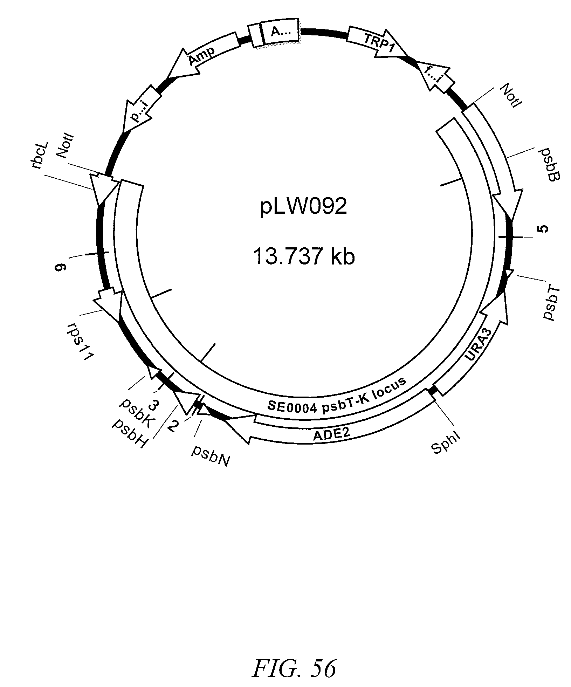

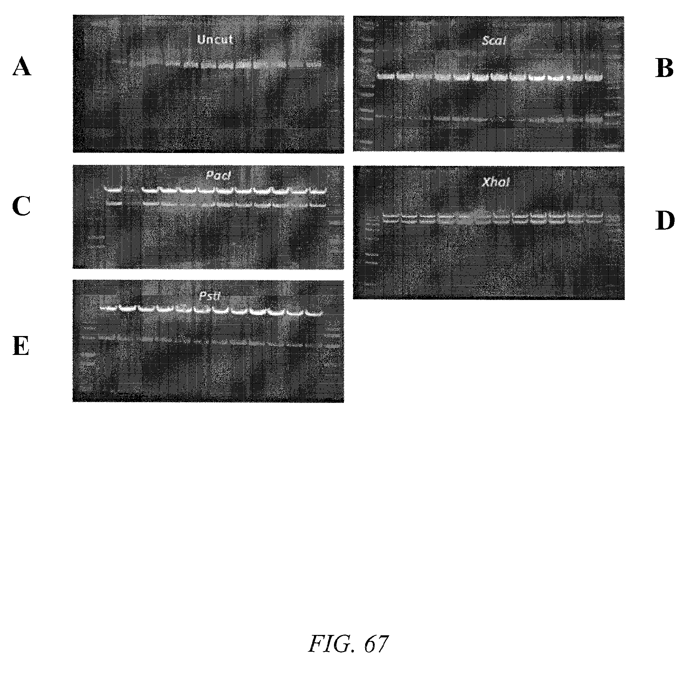

Resultant yeast colonies were patched to CSM - TRP - URA- ADE and PCR screened for the correct homologous insertion of the URA3-ADE2 construct. Plasmid DNA was then harvested from PCR positive yeast clones and electroporated into *E. coli* DH10B cells (Invitrogen). Bacterial colonies were PCR screened. PCR positive clones were then harvested for plasmid DNA (Qiagen miniprep protocol). Twelve independent plasmid isolates from the above-mentioned yeast colonies were sequence confirmed and restriction enzyme mapped with *PacI, PstI, ScaI,* and *XhoI* (**Figures 67A-E**). **Figure 67A** is uncut plasmid DNA. **Figure 67B** is the plasmid DNA digested with *ScaI,* the expected fragments are 1761 bp, 5646 bp, and 6330 bp. **Figure 67C** is the plasmid DNA digested with *PacI;* the expected fragments are 4847 bp and 8890 bp. **Figure 67D** is the plasmid DNA digested with *XhoI*; expected fragments are 5830 bp and 7907 bp. **Figure 67E** is the plasmid DNA digested with *PstI*; the resulting fragments are 493 bp, 3011 bp, and 10233 bp. The resulting platform construct was designated as pLW092 (**Figure 56**).

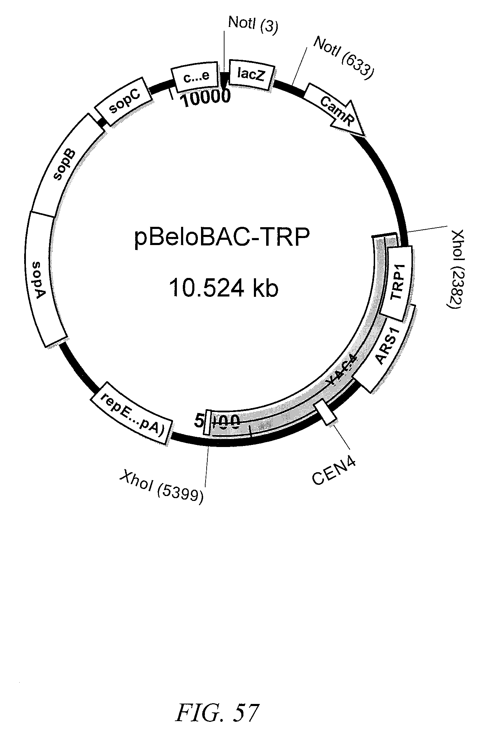

The size of the contig becomes an issue in assembling contigs of three or more genes as the colE1 origin present in the pLW092 backbone (**Figure 56**) is unable to support faithful duplication of plasmids greater than 20kb. To contend with this issue, a platform vector was created that is capable of larger assemblies based on the BAC cloning vector, pBeloBAC11 (**Figure 54**), which contains the OriS origin capable of maintaining very large DNA fragments, for example, upwards of 300kb. Briefly, pBeloBAC11 was linearized using the restriction enzyme *XhoI*. The *TRP1*-*ARS1*-*CEN4* gene sequences (SEQ ID NO: 107) was PCR-amplified from pYAC4 (ATCC; GenBank number U01086; Burke, D.T., et al., Science (1987) 236: 806-812) with primer 3 (SEQ ID NO: 68) and primer 4 (SEQ ID NO: 69), which both contain *XhoI* ends. The *XhoI-*digested BeloBAC11 and pYAC4 sequences were ligated together. Resultant bacterial colonies were PCR screened for the correct ligation event, restriction enzyme mapped, and sequence confirmed. The resultant plasmid was designated pBeloBAC-TRP (**Figure 57**).

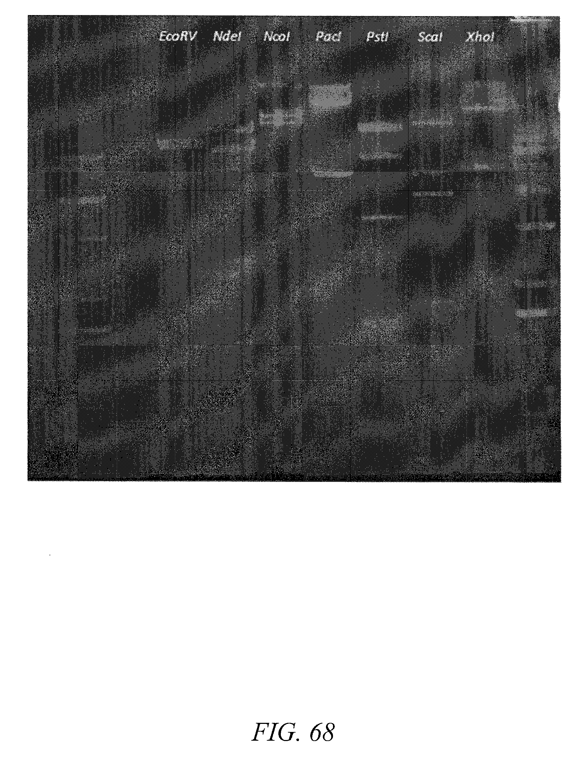

pBeloBAC-TRP was further modified to incorporate the *Scenedesmus* psbB-rbcL locus (containing URA3-ADE2 between psbT and psbN, from pLW092). Briefly, the *Scenedesmus* psbB-rbcL locus was digested away from pLW092 (**Figure 56**) using *NotI* and ligated into pBeloBAC 11-TRP (**Figure 57**) (also digested with *NotI*)*.* Resultant bacterial clones were sequence confirmed and restriction enzyme mapped with *EcoRV*, *NdeI*, *NotI*, *PacI*, *PstI*, *ScaI* and *XhoI* (**Figure 68**). Order of lanes from left to right: 1kb DNA plus ladder (Invitrogen, U.S.A.), empty, *EcoRV, NdeI*, *NcoI*, *PacI*, *PstI*, *ScaI*, *XhoI,* and 1kb DNA plus ladder (Invitrogen, U.S.A.). Expected bands are as follows: *EcoRV,* 229 bp, 1290 bp, 1461 bp, 2261 bp, 6558 bp, and 7048 bp; *NdeI*, 2187 bp, 2470 bp, 6183bp, and 8007 bp; *NotI*, 8953 bp and 9894 bp; *PacI*, 4847 bp and 14000 bp; *PstI*, 493 bp, 1541 bp, 3179 bp, 5559bp, and 8075 bp; *ScaI*, 1761 bp, 3835 bp, 4704 bp, and 8547bp; and *XhoI*, 3017 bp, 4942 bp, and10888 bp. The resultant platform construct was designated as pLW100 (**Figure 58**) and is used in all of the 3- and 4-gene contig assemblies.

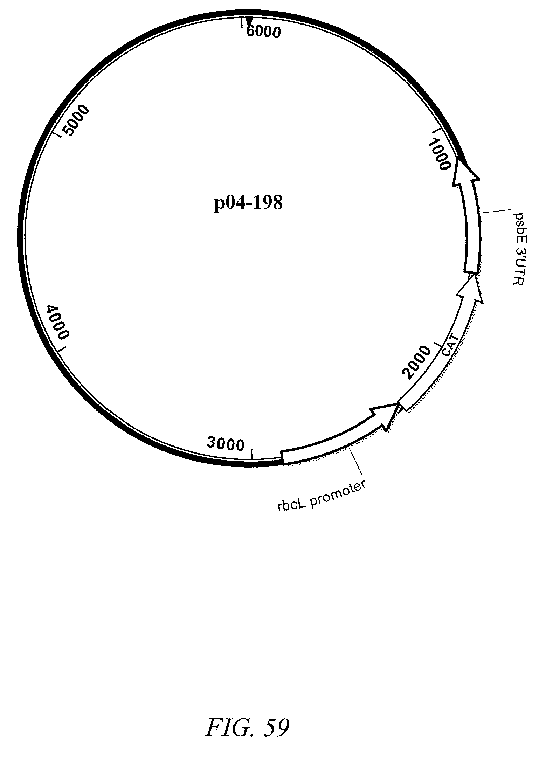

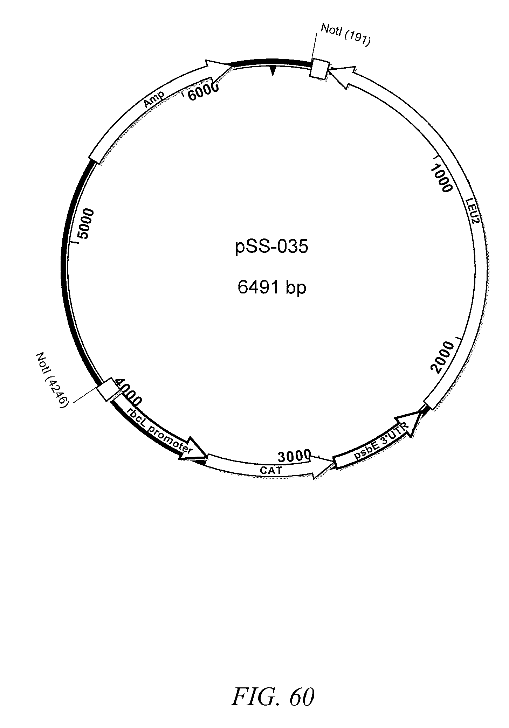

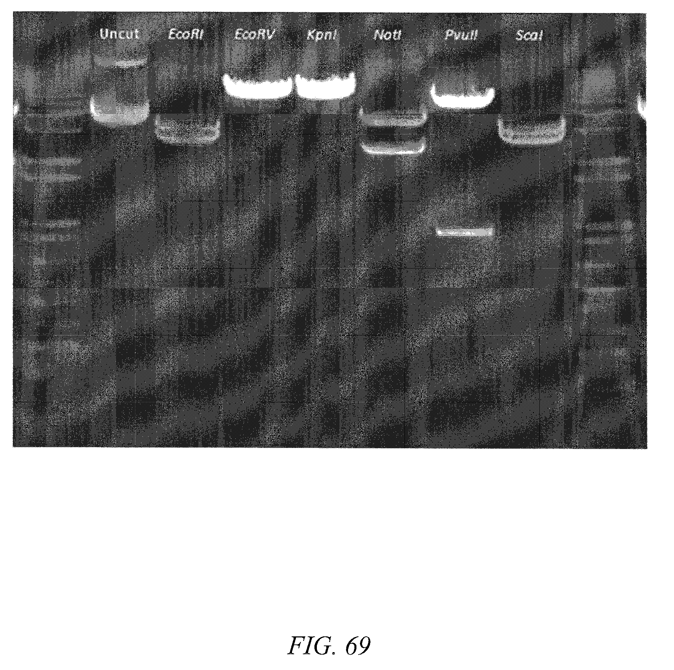

In addition to the genes of interest assembled into the 2- 3- and 4- gene contigs, a yeast positive selection marker and a *Scenedesmus* positive selection marker were also included. The yeast auxotrophic marker, LEU2 (SEQ ID NO: 108), along with the chlorampenicol acetyltransferase (CAT) gene (SEQ ID NO: 148) driven by the rbcL promoter (which confers resistance to chloramphenicol in *Scenedesmus*) (**Figure 59**) were ligated into gutless-pUC. Homology regions flanking these two genes were also cloned, which correspond to the adjacent genes of interest in contig assembly. Briefly, the *Saccharomyces cerevisiae* gene LEU2 (SEQ ID NO: 108), was amplified from total genomic DNA with primer 5 (SEQ ID NO: 70), which contains a *PstI* restriction site, and primer 6 (SEQ ID NO: 71), which contains a *NotI* restriction site (at the 5' terminus) and 80bp of DNA, which are homologous to adjacent genes in 2-, 3-, and 4-gene contig assembly. In addition, the rbcL-CAT-psbE gene (SEQ ID NO: 128) was amplified from vector p04-198 (**Figure 59**) using primer 7 (SEQ ID NO: 72), which contains a *NotI* restriction site (at the 5' terminus) and 80bp of DNA which are homologous to adjacent genes in 2-, 3-, and 4-gene contig and primer 8 (SEQ ID NO: 73), which contains a *PstI* restriction site. The LEU2 and rbcL-CAT-psbE fragments were digested with *PstI and NotI* and ligated to *NotI* digested gutless-pUC. Resultant bacterial clones were sequence confirmed and restriction enzyme mapped with *EcoRI*, *EcoRV, KpnI*, *NotI, PvuII,* and *ScaI* (**Figure 69**). The order of lanes is as follows: 1kb DNA plus ladder (Invitrogen, U.S.A.), uncut DNA, *EcoRI*, *EcoRV, KpnI, NotI, PvuII, ScaI,* and 1kb DNA plus ladder (Invitrogen, U.S.A.). Expected bands are as follows: *EcoRI,* 3033 bp and 3458 bp; *EcoRV,* 6491 bp; *KpnI,* 6491 bp; *NotI,* 2436 bp and 4055 bp; *PvuII,* 958 bp and 5533 bp; *ScaI* 3023 bp and 3468 bp. This construct was designated as pSS-035 (**Figure 60**) and is used in all of the gene contigs to promote proper assembly and also to provide for a positive selection element during *Scenedesmus* transformation.

### Example 25:Contig Assemblies

The *Saccharomyces cerevisiae* strain, YPH858 (*MATa*, *ura3-52*, *lys2*-*801, ade2-101*, *trp1Δ63*, *his3Δ200*, *leu2Δ1*, *cyh2R),* was used in all contig assembly reactions.

For two-gene contig assemblies targeting the A3-B3 region, the following were combined:
1) 1µg of pSS-023 (**Figure 49**) linearized between URA3 and ADE2 with *SphI*;
2) 500 ng of a gel purified fragment, obtained by digesting pSS-035 (**Figure 60**) with NotI, and comprising the rbcL-CAT-psbE/LEU2 construct;
3) 500ng of PCR amplified petA-CC94-chlL (gene vector 1) (**Figure 50**), amplified with a forward primer, primer 9 (SEQ ID NO: 74), which is comprised of 60bp of homology to the *NotI* digestion product from pSS-035, and a reverse primer, primer 32 (SEQ ID NO: 97), which is comprised of 60bp of homology to pSS-023 just downstream of the nucleotide sequence encoding for ADE2; and 4) 500ng of PCR amplified tufA-CC93-psaB (gene vector 3) (**Figure 52**), amplified with a forward primer, primer 33 (SEQ ID NO: 98), which comprises 60bp of homology to pSS-023 just upstream of the nucleotide sequence encoding for URA3, and a reverse primer, primer 12 (SEQ ID NO: 77), which comprises 60bp of homology to the *NotI* digestion product described in step 2 above.

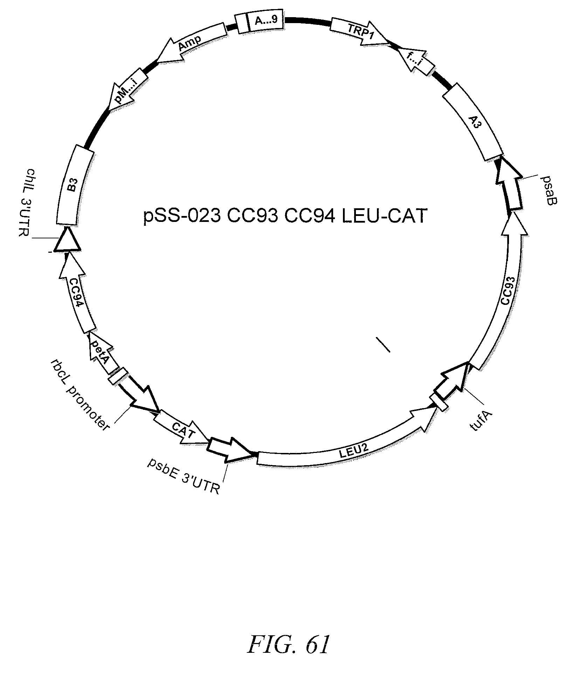

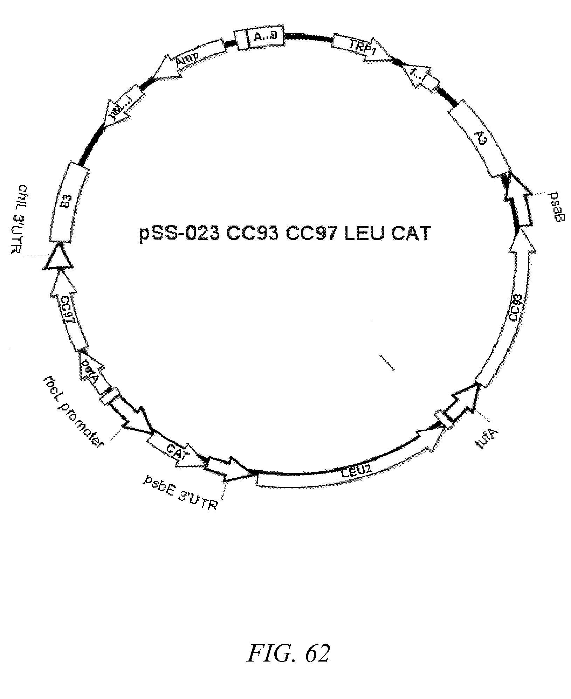

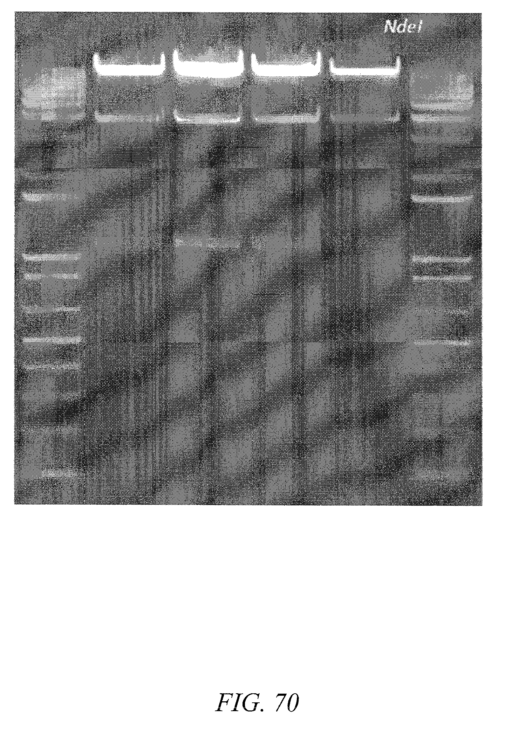

Cells were transformed with the mixture of DNA described above, using a standard lithium acetate transformation protocol. Transformants were selected for on CSM -TRP-LEU +5-FOA plates. After two days at 30 °C, yeast colonies were picked and patched to a CSM-TRP-LEU plate. The next day, yeast patches were PCR screened for the correct gene assembly. Plasmid DNA was then harvested from PCR positive yeast clones and electroporated into *E. coli* DH10B cells (Invitrogen). Bacterial colonies were also PCR screened. Four PCR positive clones were then harvested for the preparation of plasmid DNA (Qiagen miniprep protocol), which were subsequently restriction enzyme mapped with *NdeI* (**Figure 70**; expected band sizes, 1097 bp, 3703 bp, and 10283 bp; and 1kb DNA plus ladder (Invitrogen, U.S.A.). One of the four clones was picked and the sequence of that clone was confirmed. The resulting two-gene contig assembly is shown in **Figure 61****.** Another example of this assembly is shown in **Figure 62****.**

For two-gene contig assemblies targeting the 5.2kb psbB-rbcL region, the following were combined:
1) 1µg of pLW092 (**Figure 56**) linearized between URA3 and ADE2 with *SphI*;
2) 500 ng of a gel purified fragment, obtained by digesting pSS-035 (**Figure 60**) with NotI, and comprising the rbcL-CAT-psbE/LEU2 construct;
3) 500ng of PCR amplified petA-BD11-chL (gene vector 1) (**Figure 50**), amplified with a reverse primer, primer 1001 (SEQ ID NO: 150), which is comprised of 60bp of homo logy to the *NotI* digestion product from pSS-035, and a forward primer, primer 1000 (SEQ ID NO: 149), which is comprised of 60bp of homology to pLW092 just upstream of the nucleotide sequence encoding for URA3; and
4) 500ng of PCR amplified tufA-IS99-psaB (gene vector 3) (**Figure 52**), amplified with a reverse primer, primer 1002 (SEQ ID NO: 151), which comprises 60bp of homology to pLW092 just downstream of the nucleotide sequence encoding for ADE2, and a forward primer, primer 1003 (SEQ ID NO: 152), which comprises 60bp of homology to the *NotI* digestion product described in step 2 above.

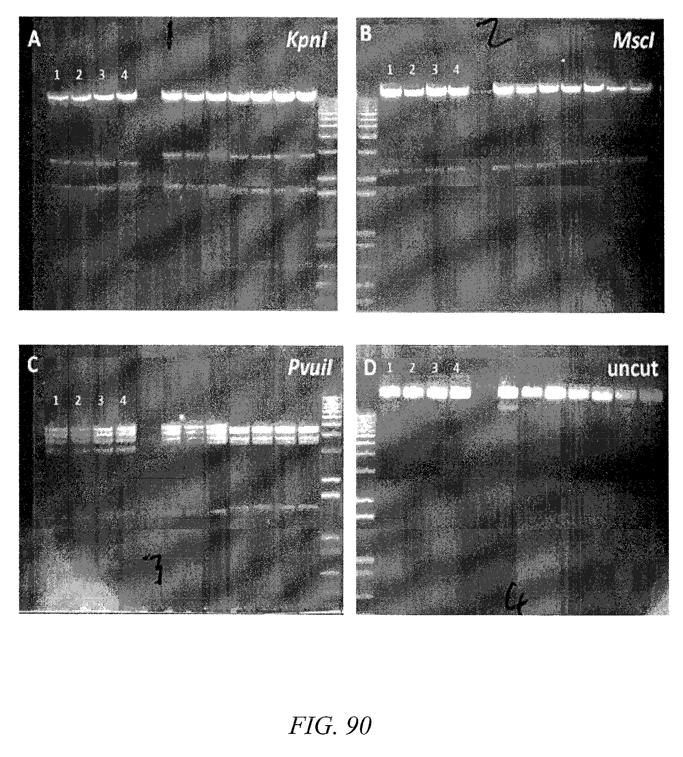

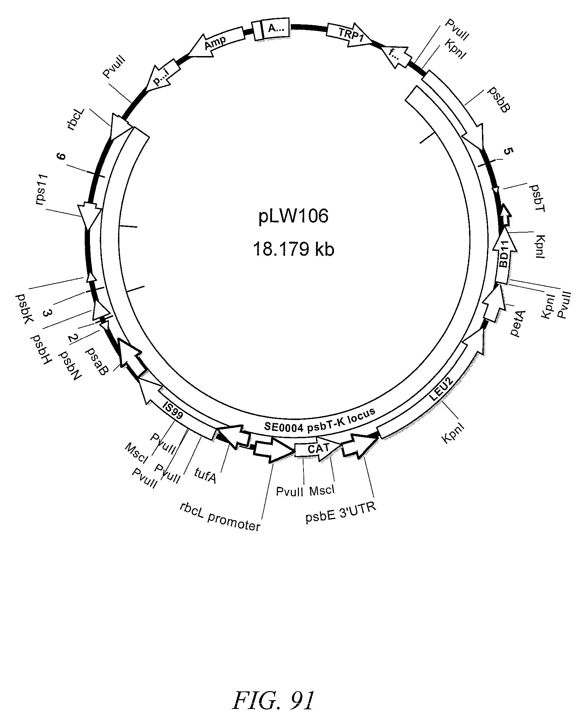

Cells were transformed with the mixture of DNA described above, using a standard lithium acetate transformation protocol. Transformants were selected for on CSM -TRP -LEU +5-FOA plates. After two days at 30°C, yeast colonies were picked and patched to a CSM-TRP-LEU plate. The next day, yeast patches were PCR screened for the correct gene assembly. Plasmid DNA was then harvested from PCR positive yeast clones and electroporated into *E. coli* DH10B cells (Invitrogen). Bacterial colonies were also PCR screened. Four PCR positive clones were then harvested for the preparation of plasmid DNA (Qiagen miniprep protocol), which were subsequently restriction enzyme mapped. **Figure 90A-D** depicts mapping of the two gene contig assembly with the restriction enzymes: *KpnI* (A), *MscI* (B), *PvuII* (C), and also uncut DNA (D). Expected band sizes are as follows: *KpnI:* 670 bp, 1791 bp, 2555bp, and 13163 bp; *MscI:* 2206bp and 15973bp; and *PvuII:* 21bp, 195bp, 1421bp, 3289bp, 3908bp, 4336bp, and 5009bp (note: the 21bp and 195bp bands have run off the gel in **Figure 90C**). One of the four clones was picked and the sequence of that clone was confirmed. The resulting two-gene contig assembly targeting the psbB-rbcL locus is shown in **Figure 91****.**

For a three-gene contig assembly, the following were combined:
1) 1µg of pLW100 (**Figure 58**) linearized between URA3 and ADE2 with *SphI;*
2) 500 ng of a gel purified fragment, obtained by digesting pSS-035 (**Figure 60**) with NotI, and comprising the rbcL-CAT-psbE/LEU2 construct;
3) 500ng of PCR amplified petA-CC90-chlL (gene vector 1) (**Figure 50**), amplified with a forward primer, primer 13 (SEQ ID NO: 78), which comprises 60bp of homology to the NotI digestion product from pSS-035, and a reverse primer, primer 14 (SEQ ID NO: 79), which comprises 60bp of homology to pLW100 just upstream of the nucleotide sequence encoding for URA3;
4) 500ng of PCR amplified tufA-CC91-psaB (gene vector 3) (**Figure 52**), amplified with a forward primer, primer 15 (SEQ ID NO: 80), which comprises 60bp of homology to the NotI digestion product from step 2, and a reverse primer, primer 16 (SEQ ID NO: 81), which comprises 60bp of homology to the PCR amplified gene vector 2 (**Figure 51**); and
5) 500ng of PCR amplified D2-CC92-D1 (gene vector 2) (**Figure 51**), amplified with a forward primer, primer 29 (SEQ ID NO: 94), which comprises 60bp of homology to PCR amplified gene vector 2, and a reverse primer, primer 17 (SEQ ID NO: 82), which comprises 60bp of homology to pLW100, just downstream of the nucleotide sequence encoding for ADE2.

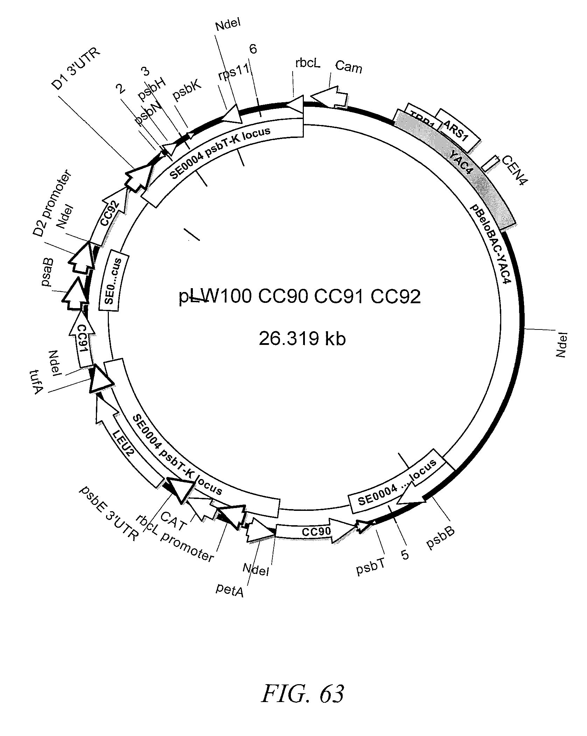

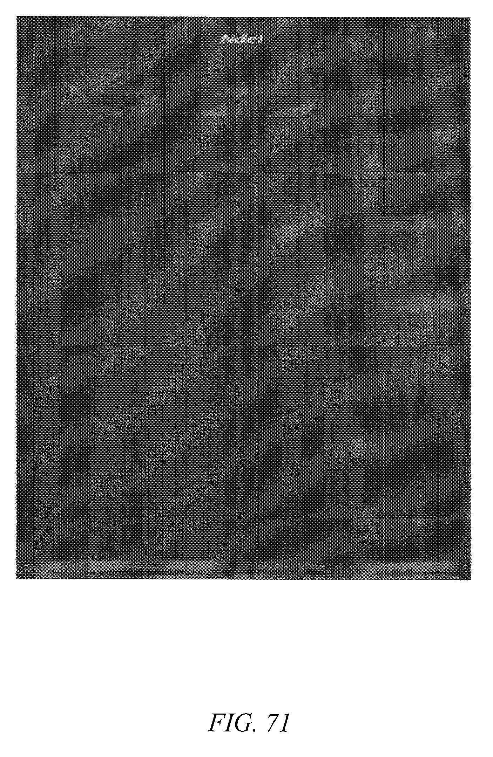

Cells were transformed with the mixture of DNA described above, using a standard lithium acetate transformation protocol. Transformants were selected for on CSM -TRP -LEU +5-FOA plates. After two days at 30°C, yeast colonies were picked and patched to a CSM -TRP -LEU plate. The next day, yeast patches were PCR screened for the correct gene assembly. Plasmid DNA was then harvested from PCR positive yeast clones and electroporated into E. *coli* DH10B cells (Invitrogen). Bacterial colonies were also PCR screened. Two PCR positive clones were then harvested for plasmid DNA (Qiagen maxiprep protocol), which were subsequently restriction enzyme mapped with *NdeI* (**Figure 71**; expected bands, 2396 bp, 3873 bp, 5114 bp, 6929 bp, and 8007 bp; and 1kb DNA plus ladder (Invitrogen, U.S.A.)). One of the two clones was picked and the sequence of that clone was confirmed. The resulting three-gene contig assembly is shown in **Figure 63****.**

To facilitate proper assembly of the 4-gene contig assembly, two positive selection yeast auxotrophic markers, HIS3 (SEQ ID NO: 118) and LYS2 (SEQ ID NO: 119), were added to the contig assembly.

For four-gene contig assemblies, the following were combined:
1) 1µg of pLW100 (**Figure 58**) linearized between URA3 and ADE2 with *SphI*;
2) 500 ng of a gel purified fragment, obtained by digesting pSS-035 (**Figure 60**) with NotI, and comprising the rbcL-CAT-psbE/LEU2 construct;
3) 500ng of PCR amplified tufA-IS57-psaB (gene vector 3) (**Figure 52**), amplified with a forward primer, primer 19 (SEQ ID NO: 84, which contains 60bp of homology to PCR amplified HIS3, and a reverse primer, primer 20 (SEQ ID NO: 85), which contains 60bp of homology to pLW100 just upstream of the nucleotide sequence encoding for URA3;
4) 500ng of PCR amplified HIS3, amplified with a forward primer, primer 21 (SEQ ID NO: 86), which contains 60bp of homology to PCR amplified gene vector 3 and a reverse primer, primer 22 (SEQ ID NO: 87), which contains 60bp of homology to PCR amplified gene vector 1 (**Figure 50**);
5) 500ng of PCR amplified petA-IS116-chlL (gene vector 1), amplified with a forward primer, primer 13 (SEQ ID NO: 78), which contains 60bp of homology to the *NotI* digestion product from step 2, and a reverse primer, primer 23 (SEQ ID NO: 88), which contains 60bp of homology to PCR amplified HIS3;
6) 500ng of PCR amplified tufA-IS62-psaB (gene vector 3), amplified with a forward primer, primer 16 (SEQ ID NO: 81), which contains 60bp of homology to the *NotI* digestion product from step 2 and a reverse primer, primer 15 (SEQ ID NO: 80), which contains 60bp of homology to PCR amplified LYS2;
7) 500ng of PCR amplified LYS2, amplified with a forward primer, primer 24 (SEQ ID NO: 89), which contains 60bp of homology to PCR amplified gene vector 3 and a reverse primer, primer 25 (SEQ ID NO: 90), which contains 60bp of homology to PCR amplified gene vector 2 (**Figure 51**); and
8) 500ng of PCR amplified D2-IS61-D1 (gene vector 2), amplified with a forward primer, primer 26 (SEQ ID NO: 91), which contains 60bp of homology to PCR amplified LYS2 and a reverse primer, primer 18 (SEQ ID NO: 83), which contains 60bp of homology to pLW100 just downstream of ADE2.

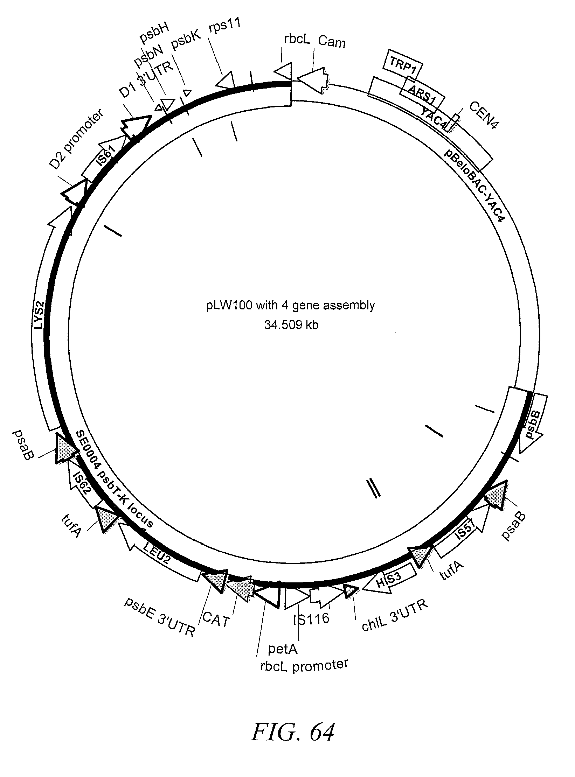

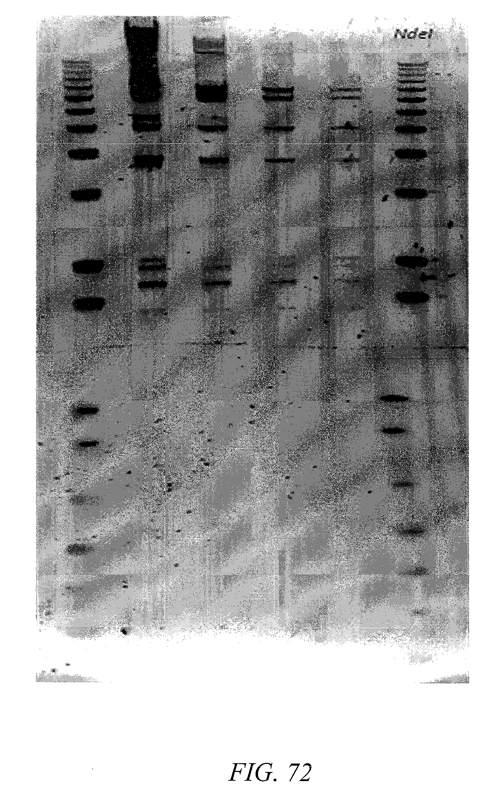

Cells were transformed with this mixture of DNA using a standard lithium acetate transformation protocol. Transformants were selected for on CSM-TRP-LEU-HIS-LYS +5-FOA plates. After two days at 30°C, yeast colonies were picked and patched to a CSM-TRP -LEU-HIS-LYS plate. The next day, yeast patches were PCR screened for the correct gene assembly. Plasmid DNA was then harvested from PCR positive yeast clones and electroporated into *E. coli* DH10B cells (Invitrogen). Bacterial colonies were also PCR screened. Four PCR positive clones were then harvested for plasmid DNA (Qiagen maxiprep protocol), which were subsequently restriction enzyme mapped with *NdeI* (**Figure 72**; expected bands, 553 bp, 564 bp, 1570 bp, 1791 bp, 1824 bp, 1969, 2040 bp, 3858 bp, 5114 bp, 7219 bp, and 8007 bp; and 1kb DNA plus ladder (Invitrogen, U.S.A.)). One of the four clones was picked and the sequence of that clone was confirmed. The resulting four-gene contig assembly is shown in **Figure 64****.**

### Example 26: Scenedesmus chloroplast transformation

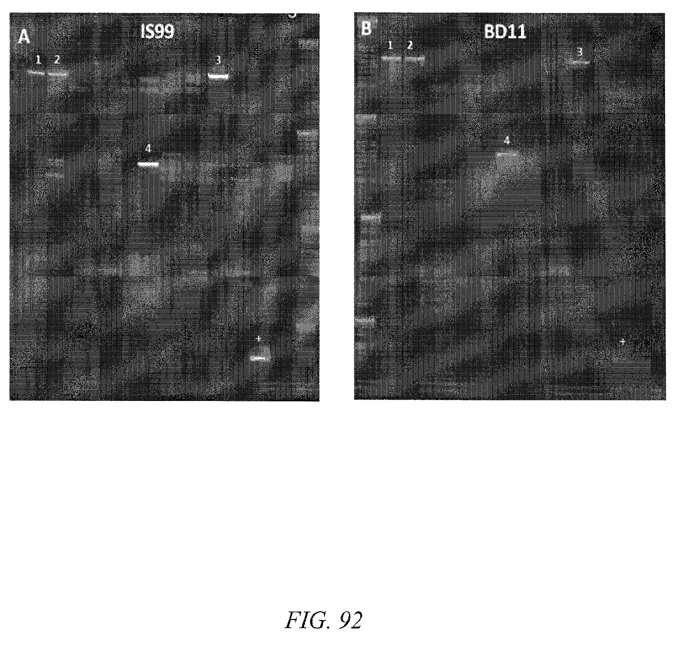

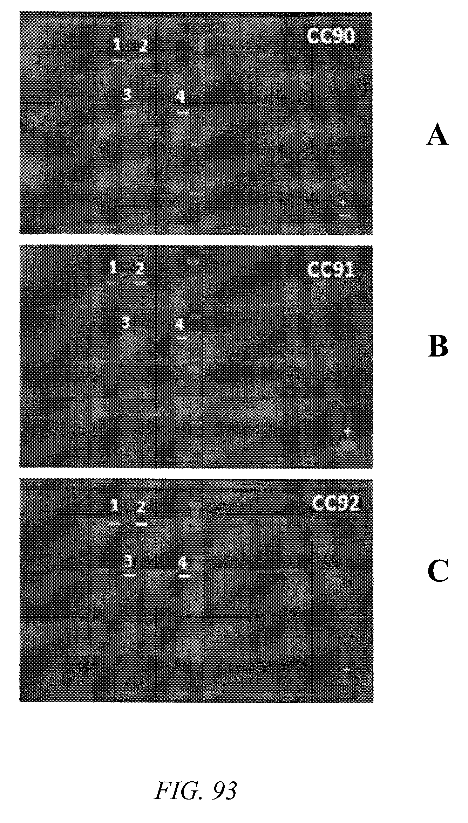

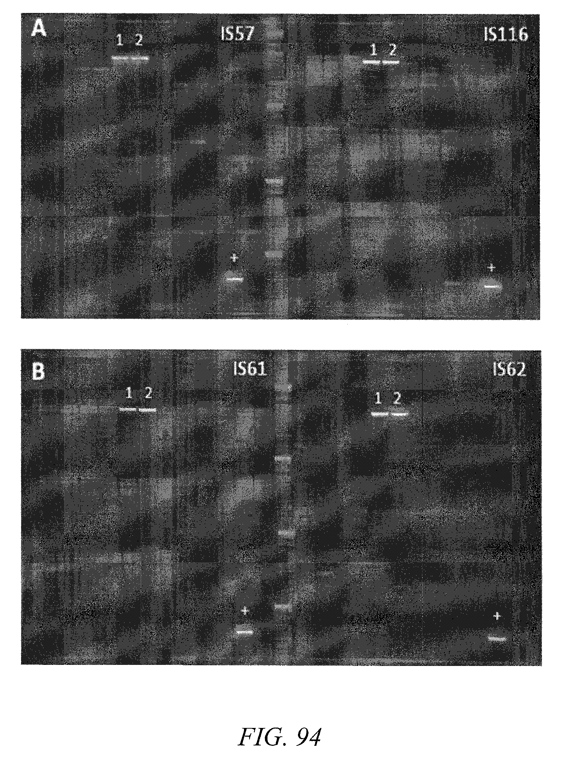

Once construct integrity was confirmed for each of the gene assemblies (2-, 3-, and 4-gene contigs), each of the gene assemblies were individually transformed into *Scenedesmus obliquus.* Briefly, cells were grown to mid-log phase and harvested. Approximately 5x10⁷ cells were spread onto TAP plates containing 25µg/ml chloramphenicol and allowed to dry in a sterile culture hood. While plates were drying, 10µg of plasmid DNA (from each of the contig assemblies) was bound to gold beads and transformation was conducted using a biolistic gene gun (Bio-rad) at 500psi. 2µg of DNA was loaded into each shot and each plate was shot five times. Plates were placed under constant light for about 10 days. After which, chloramphenicol resistant colonies were picked and patched to a TAP plate containing 25µg/ml chloramphenicol. Three to four days later, algae patches were picked into 10mM EDTA, boiled for 10 minutes and then used in a standard PCR reaction to screen for the introduction of the genes into the chloroplast. Chloramphenicol resistant transformants potentially containing the 2-gene contig, targeting the psbB-rbcL locus, were screened for the presence of BD11 and IS99. Primers 1004 (SEQ ID NO: 153) and 1005 (SEQ ID NO: 154) screen for the presence of BD11, while primers 1006 (SEQ ID NO: 155) and 1007 (SEQ ID NO: 156) screen for the presence of IS99. **Figure 92A and 92B** depict 4 clones that screen PCR positive for both IS99 and BD11, respectively. Chloramphenicol resistant transformants potentially containing the 3-gene contig, targeting the psbB-rbcL locus, were screened for the presence of CC90, CC91, and CC92. Primers 1008 (SEQ ID NO: 157) and 1009 (SEQ ID NO: 158) screen for the presence of CC90, primers 1010 (SEQ ID NO: 159) and 1011 (SEQ ID NO: 160) screen for the presence of CC91, and primers 1012 (SEQ ID NO: 161) and 1013 (SEQ ID NO: 162) screen for the presence of CC92. **Figures 93A-C** depict 4 clones that screen PCR positive for CC90, CC91, and CC92. Chloramphenicol resistant transformants potentially containing the 4-gene contig, targeting the psbB-rbcL locus, were screened for the presence of IS61, IS62, IS57, and IS116. Primers 1014 (SEQ ID NO: 163) and 1015 (SEQ ID NO: 164) screen for the presence of IS61, primers 1016 (SEQ ID NO: 165) and 1017 (SEQ ID NO: 166) screen for the presence of IS62, primers 1018 (SEQ ID NO: 167) and 1019 (SEQ ID NO: 168) screen for the presence of IS57, and primers 1020 (SEQ ID NO: 169) and 1021 (SEQ ID NO: 170) screen for the presence of IS 116. **Figure 94A and 94B** depict 2 clones that screen PCR positive for IS57, IS116 (A), and IS61, IS62 (B). Taken together these data demonstrate that one skilled in the art can integrate multiple gene contigs of varying sizes (2 gene: 8.1kb, 3 gene: 11.2kb, and 4 gene: 19.4kb) into the chloroplast genome of *Scenedesmus* in a single step.

One of skill in the art will appreciate that many other methods known in the art may be substituted in lieu of the ones specifically described or referenced.

While certain examples have been shown and described herein, it will be obvious to those skilled in the art that such examples are provided byway of example only. Numerous variations, changes, and substitutions will now occur to those skilled in the art without departing from the disclosure. It should be understood that various alternatives to the examples of the disclosure described herein may be employed in practicing the disclosure. It is intended that the following claims define the scope of the disclosure and that methods and structures within the scope of these claims and their equivalents be covered thereby.

## Claims

1. A method of transforming a chloroplast genome of a *Scenedesmus sp.* with at least one exogenous nucleotide sequence, comprising:
a) obtaining an exogenous nucleotide sequence flanked by two sequences that are homologous to the chloroplast genome, wherein the exogenous nucleotide sequence comprises a nucleic acid sequence encoding a protein and a further nucleic acid sequence encoding a selectable marker;
b) binding the exogenous nucleotide sequence onto a particle; and
c) shooting the exogenous nucleotide sequence into the *Scenedesmus sp.* by particle bombardment carried out by a biolistic device with a helium pressure of at least 300psi, wherein the chloroplast genome is transformed with the exogenous nucleotide sequence.

2. The method of claim 1, wherein:
i) in the method, transformation of the chloroplast comprises introducing regions of chloroplast DNA flanking the exogenous nucleotide sequence, such that there is homologous recombination of exogenous DNA into the target chloroplast genome; or
ii) one or more exogenous nucleotide sequences are encoded in an expression vector, or a linearized portion thereof, and the exogenous sequence is flanked by two sequences homologous to a nucleotide sequence found naturally in the host cell, wherein the first and second homologous sequences enable recombination of the exogenous sequence into the genome of the *Scenedesmus sp.* host.

3. The method of claim 2, comprising step ii), and wherein, in step ii), the exogenous sequence is flanked by two homologous sequences having at least 50%, at least 60%, at least 70%, at least 80%, at least 90%, at least 95%, at least 98%, or at least 99% sequence identity to a nucleotide sequence found naturally in the host cell.

4. The method of claim 3, wherein, in step ii), the first and second homologous sequences are at least 100, at least 200, at least 300, at least 400, at least 500, or at least 1000 nucleotides in length.

5. The method of claim 1, which is a method of transforming a chloroplast genome of a *Scenedesmus sp.* with a vector, wherein the vector comprises:
i) a first nucleotide sequence of a *Scenedesmus sp.* chloroplast genome;
ii) a second nucleotide sequence of a *Scenedesmus sp.* chloroplast genome;
iii) a third nucleotide sequence comprising the exogenous nucleotide sequence, wherein the exogenous nucleotide sequence comprises a nucleic acid encoding the protein which is a protein of interest, wherein the third nucleotide sequence is located between the first and second nucleotide sequences, and wherein the vector is used to transform the chloroplast genome of the *Scenedesmus sp*.; and
iv) a promoter configured for expression of the protein of interest;
wherein the first nucleotide sequence is at least 500 bp, at least 1000 bp, or at least 1,500 bp in length, and the first nucleotide sequence is homologous to a first portion of the chloroplast genome of the *Scenedesmus sp*.;
and wherein the second nucleotide sequence is at least 500 bp, at least 1000 bp, or at least 1,500 bp in length, and the second nucleotide sequence is homologous to a second portion of the chloroplast genome of the *Scenedesmus sp.*

6. The method of claim 5, wherein the exogenous sequence is flanked by the two homologous sequences which have at least 50%, at least 60%, at least 70%, at least 80%, at least 90%, at least 95%, at least 98%, or at least 99% sequence identity to a nucleotide sequence found naturally in the host cell.

7. The method of claim 1, 2, 3 or 4, wherein said particle bombardment is carried out by a biolistic device with a helium pressure of 300 psi to 500 psi, or at least 350 psi, or at least 400 psi, or at least 425 psi, or at least 450 psi.

8. The method of claim 1, 2, 3 or 4, wherein the exogenous nucleotide sequence bound to the particle is shot at a distance of 2 to 4 cm from the *Scenedesmus sp.*

9. The method of claim 1, 2, 3 or 4, wherein the *Scenedesmus sp.* is *S. dimorphus* or *S. obliquus.*

10. The method of claim 1, 2, 3 or 4, wherein the particle is a gold particle or a tungsten particle.

11. The method of claim 1, 2, 3 or 4,
wherein the selectable marker is a chloramphenicol acetyltransferase (CAT), an erythromycin esterase, or a cytosine deaminase; or wherein the selectable marker results in resistance to DCMU, which is 3-(3,4-dichlorophenyl)-1,1-dimethylurea;
or wherein the exogenous nucleotide sequence comprises a nucleic acid sequence which encodes for a betaine aldehyde dehydrogenase.

12. An isolated *Scenedesmus sp.* comprising a chloroplast genome that has been transformed with an exogenous nucleotide sequence, wherein the exogenous nucleotide sequence comprises a nucleic acid sequence encoding at least one protein and a further nucleic acid sequence encoding a selectable marker,
and wherein the isolated *Scenedesmus sp.* is obtainable by a method as defined in any one of claims 1 to 11.

13. The isolated *Scenedesmus sp.* of claim 12,
wherein the selectable marker is a chloramphenicol acetyltransferase (CAT), an erythromycin esterase, or a cytosine deaminase; or wherein the selectable marker results in resistance to DCMU, which is 3-(3,4-dichlorophenyl)-1,1-dimethylurea;
or wherein the exogenous nucleotide sequence comprises a nucleic acid sequence which encodes for a betaine aldehyde dehydrogenase;
and wherein the meaning of a selectable marker being a chloramphenicol acetyltransferase (CAT) encompasses a modified chloramphenicol acetyltransferase gene according to SEQ ID NO: 28;
and wherein the meaning of a selectable marker being an erythromycin esterase encompasses a modified erythromycin esterase gene according to SEQ ID NO: 29.

14. The isolated *Scenedesmus sp.* of claim 12 or 13,
wherein the nucleic acid sequence is codon optimized for expression in the chloroplast genome of the *Scenedesmus sp*.; or
wherein one or more codons of an encoding polynucleotide is or are optimized to reflect chloroplast codon usage in *Chlamydomonas reinhardtii.*

15. The isolated *Scenedesmus sp.* of claim 12, wherein the nucleic acid sequence or the further nucleic acid sequence is a nucleotide sequence of SEQ ID NO: 28, SEQ ID NO: 29, SEQ ID NO: 30, SEQ ID NO: 31, SEQ ID NO: 32, SEQ ID NO: 34, or SEQ ID NO: 148.

16. The isolated *Scenedesmus sp.* of claim 12, wherein the *Scenedesmus sp.* is *S. dimorphus* or *S. obliquus.*

17. The isolated *Scenedesmus sp.* of claim 12, wherein the exogenous nucleotide sequence comprises a nucleic acid sequence which encodes for a biomass-degrading enzyme.

## Patentansprüche

1. Verfahren zum Transformieren eines Chloroplastgenoms einer *Scenedesmus sp.* mit wenigstens einer exogenen Nukleotidsequenz, Folgendes umfassend:
a) Erhalten einer exogenen Nukleotidsequenz, die von zwei Sequenzen flankiert wird, welche zu dem Chloroplastgenom homolog sind, wobei die exogene Nukleotidsequenz eine Nukleinsäuresequenz, die für ein Protein kodiert, und eine weitere Nukleinsäuresequenz, die für einen auswählbaren Marker kodiert, umfasst;
b) Binden der exogenen Nukleotidsequenz auf ein Partikel; und
c) Schießen der exogenen Nukleotidsequenz in die *Scenedesmus sp.* durch Partikelbeschuss, durchgeführt mit einer biolistischen Vorrichtung mit einem Heliumdruck von wenigstens 300 psi, wobei das Chloroplastgenom mit der exogenen Nukleotidsequenz transformiert wird.

2. Verfahren nach Anspruch 1, wobei:
i) in dem Verfahren die Transformation des Chloroplasten das Einfügen von Regionen von Chloroplast-DNA umfasst, welche die exogene Nukleotidsequenz flankieren, sodass eine homologe Rekombination von exogener DNA in das Zielchloroplastgenom stattfindet; oder
ii) eine oder mehrere exogene Nukleotidsequenzen in einem Expressionsvektor oder in einem linearisierten Abschnitt davon kodiert sind und die exogene Sequenz durch zwei Sequenzen flankiert wird, die homolog zu einer Nukleotidsequenz sind, die in der Natur in der Wirtszelle vorkommt, wobei die erste und die zweite homologe Sequenz eine Rekombination der exogenen Sequenz in das Genom der Wirts-*Scenedesmus sp.* ermöglichen.

3. Verfahren nach Anspruch 2, Schritt ii) umfassend, und wobei in Schritt ii) die exogene Sequenz von zwei homologen Sequenzen flankiert wird, die wenigstens 50 %, wenigstens 60 %, wenigstens 70 %, wenigstens 80 %, wenigstens 90 %, wenigstens 95 %, wenigstens 98 % oder wenigstens 99 % Sequenzidentität zu einer Nukleotidsequenz aufweisen, die in der Natur in der Wirtszelle vorkommt.

4. Verfahren nach Anspruch 3, wobei in Schritt ii) die erste und die zweite homologe Sequenz wenigstens 100, wenigstens 200, wenigstens 300, wenigstens 400, wenigstens 500 oder wenigstens 1000 Nukleotide lang sind.

5. Verfahren nach Anspruch 1, wobei es sich um ein Verfahren zum Transformieren eines Chloroplastgenoms einer *Scenedesmus sp.* mit einem Vektor handelt, wobei der Vektor Folgendes umfasst:
i) eine erste Nukleotidsequenz eines *Scenedesmus sp*.-Chloroplastgenoms;
ii) eine zweite Nukleotidsequenz eines *Scenedesmus sp*.-Chloroplastgenoms;
iii) eine dritte Nukleotidsequenz, die die exogene Nukleotidsequenz umfasst, wobei die exogene Nukleotidsequenz eine Nukleinsäure umfasst, die für das Protein kodiert, welches ein Protein von Interesse ist, wobei die dritte Nukleotidsequenz zwischen der ersten und der zweiten Nukleotidsequenz angeordnet ist und wobei der Vektor eingesetzt wird, um das Chloroplastgenom der *Scenedesmus sp.* zu transformieren; und
iv) einen Promoter, der für die Expression des Proteins von Interesse konfiguriert ist; wobei die erste Nukleotidsequenz wenigstens 500 bp, wenigstens 1000 bp oder wenigstens 1500 bp lang ist und die erste Nukleotidsequenz homolog zu einem ersten Abschnitt des Chloroplastgenoms der *Scenedesmus sp.* ist;
und wobei die zweite Nukleotidsequenz wenigstens 500 bp, wenigstens 1000 bp oder wenigstens 1500 bp lang ist und die zweite Nukleotidsequenz homolog zu einem zweiten Abschnitt des Chloroplastgenoms der *Scenedesmus sp.* ist.

6. Verfahren nach Anspruch 5, wobei die exogene Sequenz von zwei homologen Sequenzen flankiert wird, die wenigstens 50 %, wenigstens 60 %, wenigstens 70 %, wenigstens 80 %, wenigstens 90 %, wenigstens 95 %, wenigstens 98 % oder wenigstens 99 % Sequenzidentität zu einer Nukleotidsequenz aufweisen, die in der Natur in der Wirtszelle vorkommt.

7. Verfahren nach Anspruch 1,2,3 oder 4, wobei der Partikelbeschuss mit einer biolistischen Vorrichtung mit einem Heliumdruck von 300 psi bis 500 psi oder wenigstens 350 psi oder wenigstens 400 psi oder wenigstens 425 psi oder wenigstens 450 psi durchgeführt wird.

8. Verfahren nach Anspruch 1, 2, 3 oder 4, wobei die an das Partikel gebundene exogene Nukleotidsequenz aus einer Entfernung von 2 bis 4 cm von der *Scenedesmus sp.* abgeschossen wird.

9. Verfahren nach Anspruch 1, 2, 3 oder 4, wobei die *Scenedesmus sp. S. dimorphus* oder S. *obliquus* ist.

10. Verfahren nach Anspruch 1, 2, 3 oder 4, wobei das Partikel ein Goldpartikel oder ein Wolframpartikel ist.

11. Verfahren nach Anspruch 1, 2, 3 oder 4,
wobei der auswählbare Marker eine Chloramphenicol-Acetyltransferase (CAT), eine Erythromycinesterase oder eine Cytosindeaminase ist; oder wobei der auswählbare Marker zu einer Resistenz gegen DCMU führt, wobei es sich um 3-(3,4-Dichlorphenyl)-1,1-dimethylharnstoff handelt;
oder wobei die exogene Nukleotidsequenz eine Nukleinsäuresequenz umfasst, die für eine Betainaldehyddehydrogenase kodiert.

12. Isolierte *Scenedesmus sp.,* umfassend ein Chloroplastgenom, das mit einer exogenen Nukleotidsequenz transformiert worden ist, wobei die exogene Nukleotidsequenz eine Nukleinsäuresequenz, die für wenigstens ein Protein kodiert, und eine weitere Nukleinsäuresequenz, die für einen auswählbaren Marker kodiert, umfasst,
und wobei die isolierte *Scenedesmus sp.* mit einem Verfahren nach einem der Ansprüche 1 bis 11 erhältlich ist.

13. Isolierte *Scenedesmus sp.* nach Anspruch 12,
wobei der auswählbare Marker eine Chloramphenicol-Acetyltransferase (CAT), eine Erythromycinesterase oder eine Cytosindeaminase ist; oder wobei der auswählbare Marker zu einer Resistenz gegen DCMU führt, wobei es sich um 3-(3,4-Dichlorphenyl)-1,1-dimethylharnstoff handelt;
oder wobei die exogene Nukleotidsequenz eine Nukleinsäuresequenz umfasst, die für eine Betainaldehyddehydrogenase kodiert;
und wobei, wenn ein auswählbarer Marker eine Chloramphenicol-Acetyltransferase (CAT) ist, dies für ein modifiziertes Chloramphenicol-Acetyltransferasegen nach SEQ ID NO: 28 steht;
und wobei, wenn ein auswählbarer Marker eine Erythromycinesterase ist, dies für ein modifiziertes Erythromycinesterasegen nach SEQ ID NO: 29 steht.

14. Isolierte *Scenedesmus sp.* nach Anspruch 12 oder 13,
wobei die Nukleinsäuresequenz für Expression in dem Chloroplastgenom der *Scenedesmus sp.* codonoptimiert ist; oder
wobei ein oder mehrere Codons eines kodierenden Polynukleotids optimiert sind, Chloroplastcodonnutzung in *Chlamydomonas reinhardtii* widerzuspiegeln.

15. Isolierte *Scenedesmus sp.* nach Anspruch 12, wobei die Nukleinsäuresequenz oder die weitere Nukleinsäuresequenz eine Nukleotidsequenz aus SEQ ID NO: 28, SEQ ID NO: 29, SEQ ID NO: 30, SEQ ID NO: 31, SEQ ID NO: 32, SEQ ID NO: 34 oder SEQ ID NO: 148 ist.

16. Isolierte *Scenedesmus sp.* nach Anspruch 12, wobei die *Scenedesmus sp. S. dimorphus* oder S. *obliquus* ist.

17. Isolierte *Scenedesmus sp.* nach Anspruch 12, wobei die exogene Nukleotidsequenz eine Nukleinsäuresequenz umfasst, die für ein Biomasse zersetzendes Enzym kodiert.

## Revendications

1. Procédé de transformation d'un génome de chloroplaste d'une *Scenedesmus sp*. avec au moins une séquence nucléotidique exogène, comprenant :
a) l'obtention d'une séquence nucléotidique exogène flanquée par deux séquences qui sont homologues au génome de chloroplaste, la séquence nucléotidique exogène comprenant une séquence d'acide nucléique codant pour une protéine et une autre séquence d'acide nucléique codant pour un marqueur sélectionnable ;
b) la liaison de la séquence nucléotidique exogène sur une particule ; et
c) le tir de la séquence nucléotidique exogène dans *Scenedesmus sp.* par bombardement de particules réalisé par un dispositif biolistique avec une pression d'hélium d'au moins 300 psi, le génome de chloroplaste étant transformé avec la séquence nucléotidique exogène.

2. Procédé selon la revendication 1, dans lequel :
i) dans le procédé, la transformation du chloroplaste comprend l'introduction de régions d'ADN de chloroplaste flanquant la séquence nucléotidique exogène, de telle sorte qu'il y a une recombinaison homologue de l'ADN exogène dans le génome de chloroplaste cible ;
ou
ii) une ou plusieurs séquences nucléotidiques exogènes sont codées dans un vecteur d'expression, ou une partie linéarisée de celui-ci, et la séquence exogène est flanquée par deux séquences homologues à une séquence nucléotidique présente naturellement dans la cellule hôte, les première et deuxième séquences homologues permettant la recombinaison de la séquence exogène dans le génome de l'hôte *Scenedesmus sp.*

3. Procédé selon la revendication 2, comprenant l'étape ii), et dans lequel, à l'étape ii), la séquence exogène est flanquée par deux séquences homologues ayant au moins 50 %, au moins 60 %, au moins 70 %, au moins 80 %, au moins 90 %, au moins 95 %, au moins 98 % ou au moins 99 % d'identité de séquences avec une séquence nucléotidique présente naturellement dans la cellule hôte.

4. Procédé selon la revendication 3, dans lequel, à l'étape ii), les première et deuxième séquences homologues ont au moins 100, au moins 200, au moins 300, au moins 400, au moins 500 ou au moins 1 000 nucléotides en longueur.

5. Procédé selon la revendication 1, qui est un procédé de transformation d'un génome de chloroplaste de *Scenedesmus sp.* avec un vecteur, dans lequel le vecteur comprend :
i) une première séquence nucléotidique d'un génome de chloroplaste de *Scenedesmus sp. ;*
ii) une deuxième séquence nucléotidique d'un génome de chloroplaste de *Scenedesmus sp. ;*
iii) une troisième séquence nucléotidique comprenant la séquence nucléotidique exogène, la séquence nucléotidique exogène comprenant un acide nucléique codant pour la protéine qui est une protéine d'intérêt, la troisième séquence nucléotidique étant située entre les première et deuxième séquences nucléotidiques, et le vecteur étant utilisé pour transformer le génome de chloroplaste de *Scenedesmus sp. ;* et
iv) un promoteur configuré pour l'expression de la protéine d'intérét ;
dans lequel la première séquence nucléotidique a au moins 500 pb, au moins 1 000 pb ou au moins 1 500 pb en longueur, et la première séquence nucléotidique est homologue à une première partie du génome de chloroplaste de *Scenedesmus sp. ;*
et dans lequel la deuxième séquence nucléotidique a au moins 500 pb, au moins 1 000 pb ou au moins 1 500 pb en longueur, et la deuxième séquence nucléotidique est homologue à une deuxième partie du génome de chloroplaste de Scenedesmus sp.

6. Procédé selon la revendication 5, dans lequel la séquence exogène est flanquée par les deux séquences homologues qui ont au moins 50 %, au moins 60 %, au moins 70 %, au moins 80 %, au moins 90 %, au moins 95 %, au moins 98 % ou au moins 99 % d'identité de séquences avec une séquence nucléotidique présente naturellement dans la cellule hôte.

7. Procédé selon la revendication 1, 2, 3 ou 4, dans lequel ledit bombardement de particules est réalisé par un dispositif biolistique avec une pression d'hélium de 300 psi à 500 psi, ou d'au moins 350 psi, ou d'au moins 400 psi, ou d'au moins 425 psi ou d'au moins 450 psi.

8. Procédé selon la revendication 1, 2, 3 ou 4, dans lequel la séquence nucléotidique exogène liée à la particule est tirée à une distance de 2 à 4 cm de *Scenedesmus sp.*

9. Procédé selon la revendication 1, 2, 3 ou 4, dans lequel *Scenedesmus sp.* est S. *dimorphus* ou S. *obliquus.*

10. Procédé selon la revendication 1, 2, 3 ou 4, dans lequel la particule est une particule d'or ou une particule de tungstène.

11. Procédé selon la revendication 1, 2, 3 ou 4,
dans lequel le marqueur sélectionnable est une chloramphénicol acétyltransférase (CAT), une érythromycine estérase ou une cytosine désaminase ; ou dans lequel le marqueur sélectionnable aboutit à la résistance à la DCMU, qui est la 3-(3,4-dichlorophényl)-1,1-diméthylurée ;
ou dans lequel la séquence nucléotidique exogène comprend une séquence d'acide nucléique qui code pour une bétaïne aldéhyde déshydrogénase.

12. *Scenedesmus sp.* isolée comprenant un génome de chloroplaste qui a été transformé avec une séquence nucléotidique exogène, dans laquelle la séquence nucléotidique exogène comprend une séquence d'acide nucléique codant pour au moins une protéine et une autre séquence d'acide nucléique codant pour un marqueur sélectionnable,
et dans laquelle la *Scenedesmus sp.* isolée peut être obtenue par un procédé tel que défini dans l'une quelconque des revendications 1 à 11.

13. *Scenedesmus sp.* isolée selon la revendication 12,
dans laquelle le marqueur sélectionnable est une chloramphénicol acétyltransférase (CAT), une érythromycine estérase ou une cytosine désaminase ; ou dans laquelle le marqueur sélectionnable aboutit à la résistance à la DCMU, qui est la 3-(3,4-dichlorophényl)-1,1-diméthylurée ;
ou dans laquelle la séquence nucléotidique exogène comprend une séquence d'acide nucléique qui code pour une bétaïne aldéhyde déshydrogénase ;
et dans laquelle la signification d'un marqueur sélectionnable qui est une chloramphénicol acétyltransférase (CAT) inclut un gène de chloramphénicol acétyltransférase modifiée selon la SEQ ID N° : 28 ;
et dans laquelle la signification d'un marqueur sélectionnable qui est une érythromycine estérase inclut un gène d'érythromycine estérase modifié selon la SEQ ID N° :29.

14. *Scenedesmus sp.* isolée selon la revendication 12 ou 13,
dans laquelle la séquence d'acide nucléique est un codon optimisé pour l'expression dans le génome de chloroplaste de *Scenedesmus sp. ;* ou
dans laquelle un ou plusieurs codons d'un polynucléotide codeur est ou sont optimisés pour refléter l'utilisation du codon de chloroplaste dans *Chlamydomonas rheinardtii.*

15. *Scenedesmus sp.* isolée selon la revendication 12, dans laquelle la séquence d'acide nucléique ou l'autre séquence d'acide nucléique est une séquence nucléotidique de SEQ ID N° : 28, SEQ ID N° : 29, SEQ ID N° : 30, SEQ ID N° : 31, SEQ ID N° : 32, SEQ ID N° : 34 ou SEQ ID N° : 148.

16. *Scenedesmus sp.* isolée selon la revendication 12, dans laquelle *Scenedesmus sp*. est *S*. *dimorphus* ou S. *obliquus.*

17. *Scenedesmus sp.* isolée selon la revendication 12, dans laquelle la séquence nucléotidique exogène comprend une séquence d'acide nucléique qui code pour une enzyme dégradant la biomasse.
